# EUROPEAN PATENT SPECIFICATION

(11) **EP 0 772 600 B1**
(45) Date of publication and mention of the grant of the patent: **18.09.2002**
(21) Application number: 95928164.3
(22) Date of filing: 27.07.1995
(51) Int. Cl.: C07D 233/54, A61K 31/415, C07D 233/32, C07D 233/90, C07D 401/04, C07D 403/04, C07D 405/04, C07D 409/04, C07D 417/04

(54) **1,2-SUBSTITUTED IMIDAZOLYL COMPOUNDS FOR THE TREATMENT OF INFLAMMATION**
1,2-SUBSTITUIERTE IMIDAZOLYLVERBINDUNGEN ZUR BEHANDLUNG VON ENTZÜNDUNGEN
COMPOSES D'IMIDAZOLYLE SUBSTITUES EN POSITIONS 1 ET 2, CONVENANT AU TRAITEMENT DE L'INFLAMMATION

(30) Priority: 28.07.1994 US 282395; 05.06.1995 US 464154
(43) Date of publication of application: 14.05.1997
(73) Proprietor: G.D. SEARLE & CO., Chicago, IL 60680-5110 (US)
(72) Inventor: KHANNA, Ish, K., Vernon Hills, IL 60061 (US); WEIER, Richard, M., Lake Bluff, IL 60044 (US); COLLINS, Paul, W., Deerfield, IL 60015 (US); YU, Yi, Skokie, IL 60077 (US); XU, Xiangdong, Evanston, IL 60202 (US); HUFF, Renee, M., Park Ridge, IL 60068 (US); PARTIS, Richard, A., Evanston, IL 60201 (US); KOSZYK, Francis, J., Prospect Heights, IL 60070 (US)
(74) Representative: Beil, Hans Christoph, Dr.
(86) International application number: PCT/US95/09506
(87) International publication number: WO 96/003388

(56) References cited:
- EP-A- 0 257 897
- EP-A- 0 554 829

## Description

### FIELD OF THE INVENTION

This invention is in the field of inflammatory pharmaceutical agents and specifically relates to compounds, compositions and the use thereof for preparing a medicament for treating inflammation and inflammation-associated disorders, such as arthritis.

### BACKGROUND OF THE INVENTION

Prostaglandins play a major role in the inflammation process and the inhibition of prostaglandin production, especially production of PGG₂, PGH₂ and PGE_{2,} has been a common target of antiinflammatory drug discovery. However, common non-steroidal antiinflammatory drugs (NSAIDs) that are active in reducing the prostaglandin-induced pain and swelling associated with the inflammation process are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous NSAIDs have been found to prevent the production of prostaglandins by inhibiting enzymes in the human arachidonic acid/prostaglandin pathway, including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

The references below that disclose antiinflammatory activity, show continuing efforts to find a safe and effective antiinflammatory agent. The novel imidazoles disclosed herein are such safe and also effective antiinflammatory agents furthering such efforts. The invention compounds are found to show usefulness *in vivo* as antiinflammatory agents with minimal side effects. The substituted imidazoles disclosed herein preferably selectively inhibit cyclooxygenase-2 over cyclooxygenase-1.

Diaryl oxazoles have been described in WO-A-9427980 as having antiinflammatory activity. Substituted 4,5-diarylimidazoles have been described in WO-A-9500501

2-Alkylimidazoles have been described as having angiotensin II activity. For example, see US-A-5,185,351 and WO-A-9100277.

US-A-5,207,820 to Wriede et al. describes 1-arylimidazole carboxylic esters as herbicide safeners. Specifically, ethyl [1-[2,6-dinitro-4-(methylsulfonyl)phenyl]-2-methyl-1H-imidazol-3-yl]carboxylate is described.

WO-A-9314082, published July 22, 1993, describes 1-pyridyl-2-phenyl-imidazole derivatives for the treatment of interleukin-1 mediated diseases. 1-(4-Pyridyl)-2-(4-fluorophenyl)-4-methylimidazole is described. WO-A-9502591, published January 26, 1995, describe trisubstituted imidazoles for the treatment of cytokine mediated diseases.

US-A-3,487,087, to Sarett et al., describes a method of nitration of imidazoles and specifically 1-methyl-2-[4-(methylsulfonyl)phenyl]-5-nitroimidazole.

US-A-5,112,532, to Ninomiya et al., describes imidazoles as an organic non-linear optical material. Specifically, 4-(4-hydroxyphenyl)-2-[2-formyl-4-(methylsulfonyl)phenyl]imidazole is described.

US-A-3,682,949 and US-A-3,719,759, to Sarett et al., describe 2-aryl-nitroimidazoles as agents for the treatment of parasites and bacteria. Specifically, 1-(2-hydroxyethyl)-2-(4-sulfonamidophenyl)-5-nitroimidazole is described.

US-A-4,822,805, corresponding to EP-A-0 257 897, to Takasugi et al., describes pyridyl-imidazoles as antiinflammatory agents. Specifically, 2-[2-methoxy-4-(mehtylsulfonyl)phenyl]-4-methyl-5-(3-pyridyl)imidazole is described.

EP-A-0 554 829 discloses 1-aryl-5-aryl-substituted pyrazole derivatives useful as antiinflammatory, analgesic and antithrombotic agents.

The invention's imidazolyl compounds are found to show usefulness in vivo as antiinflammatory agents with minimal side effects.

### DESCRIPTION OF THE INVENTION

A class of substituted imidazolyl compounds useful in treating inflammation-related disorders is defined by Formula I: 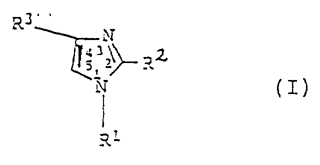 wherein R³ is a radical selected from C₁-C₆-alkyl, C₁-C₆-haloalkyl, aryl-C₁-C₆-alkyl, formyl, C₂-C₆-alkanoyl, cyano, C₁-C₆-alkylthio-C₁-C₆-alkyl,
C₃-C₁₀-cycloalkylthio-C₁-C₆-alkyl, C₁-C₆-hydroxyalkyl, C₁-C₆-alkoxy-C₁-C₆-alkyl,
arylcarbonyl, aryl-C₁-C₆-alkylcarbonyl, C₁-C₆-cyanoalkyl, C₁-C₆-carboxyalkyl, C₁-C₆-alkoxycarbonyl-C₁-C₆-alkyl, C₁-C₆-alkoxycarbonyl, C₁-C₆-haloalkylcarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxy-C₁-C₆-alkyl and aryl;
wherein R¹ and R² are independently selected from aryl and heteroaryl, wherein R¹ and R² are optionally substituted at a substitutable position with one or more radicals independently selected from C₁-C₆-alkylsulfonyl, aminosulfonyl, halo, C₁-C₆-alkylthio, C₁-C₆-alkyl, cyano, carboxyl, C₁-C₅-alkoxycarbonyl, C₁-C₆-haloalkyl, hydroxyl, C₁-C₆-alkoxy, C₁-C₆-hydroxyalkyl, C₁-C₆-alkoxy-C₁-C₆-alkyl, C₁-C₆-haloalkoxy, amino, C₁-C₆-alkylamino, arylamino and nitro; provided at least one or R¹ and R² is 4-methylsulfonylphenyl or 4-aminosulfonylphenyl; wherein aryl whenever occurring means a phenyl or naphthyl ring; wherein heteroaryl whenever occuring means an unsaturated heterocyclic radical selected from pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g. 4H-1,2,4-triazolyl, 1H-1,2,3-triazolyl, 2H-1,2,3-triazolyl] tetrazolyl [e.g. 1H-tetrazolyl, 2H-tetrazolyl], indolyl, isoindolyl, indolizinyl, benzimida zolyl, quinolyl, isoquinolyl, indazolyl, benzotriazolyl, tetrazolopyridazinyl [e.g. tetrazolo[1,5-b]pyridazinyl], pyranyl, 2-furyl, 3-furyl, 2-thienyl, 3-thienyl, oxazolyl, isoxazolyl, oxadiazolyl [e.g. 1,2,4-oxadiazolyl, 1,3,4-oxadiazolyl, 1,2,5-oxadiazolyl], benzoxazolyl, benzoxadiazolyl, thiazolyl, thiadiazolyl [e.g. 1,2,4-thiadiazolyl, 1,3,4-thiadiazolyl, 1,2,5-thiadiazolyl], benzothiazolyl, benzothiadiazolyl, benzofuran, benzothiophene, wherein said heterocyclic radical may be substituted at a substitutable position with one or more substituents selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and halo-C₁-C₆-alkoxy;
or a pharmaceutically-acceptable salt thereof.

Compounds of Formula I would be useful for, but not limited to, the treatment of inflammation in a subject, and for treatment of other inflammation-associated disorders, such as, as an analgesic in the treatment of pain and headaches, or as an antipyretic for the treatment of fever. For example, compounds of the invention would be useful to treat arthritis, including but not limited to rheumatoid arthritis, spondyloarthopathies, gouty arthritis, osteoarthritis, systemic lupus erythematosus and juvenile arthritis. Such compounds of the invention would be useful in the treatment of asthma, bronchitis, menstrual cramps, tendinitis, bursitis, and skin related conditions such as psoriasis, eczema, burns and dermatitis. Compounds of the invention also would be useful to treat gastrointestinal conditions such as inflammatory bowel disease, Crohn's disease, gastritis, irritable bowel syndrome and ulcerative colitis and for the prevention of colorectal cancer. Compounds of the invention would be useful in treating inflammation in such diseases as vascular diseases, migraine headaches, periarteritis nodosa, thyroiditis, aplastic anemia, Hodgkin's disease, sclerodoma, rheumatic fever, type I diabetes, myasthenia gravis, multiple sclerosis, sarcoidosis, nephrotic syndrome, Behcet's syndrome, polymyositis, gingivitis, hypersensitivity, conjunctivitis, swelling occurring after injury, myocardial ischemia, and the like. The compounds were also be useful in the treatment of ophthalmic diseases such as retinitis, retinopathies, uveitis, and of acute injury to the eye tissue. The compounds would also be useful for the treatment of certain central nervous system disorders such as alzheimers disease and dementia. The compounds of the invention are useful as anti-inflammatory agents, such as for the treatment of arthritis, with the additional benefit of having significantly less harmful side effects. These compounds would also be useful in the treatment of allergic rhinitis, respiratory distress syndrome, endotoxin shock syndrome, atherosclerosis and central nervous system damage resulting from stroke, ischemia and trauma.

Besides being useful for human treatment, these compounds are also useful in medicaments for treatment of mammals, including horses, dogs, cats, rats, mice, sheep, pigs, etc.

The present compounds may also be used in co-therapies, partially or completely, in place of other conventional antiinflammatories, such as together with steroids, NSAIDs, 5-lipoxygenase inhibitors, LTB₄ antagonists and LTA₄ hydrolase inhibitors.

Suitable LTB₄ inhibitors include, among others, ebselen, Bayer Bay-x-1005, Ciba Geigy compound CGS-25019C, Leo Denmark compound ETH-615, Lilly compound LY-293111, Ono compound ONO-4057, Terumo compound TMK-688, Lilly compounds LY-213024, 264086 and 292728, ONO compound ONO-LB457, Searle compound SC-53228, calcitrol, Lilly compounds LY-210073, LY223982, LY233469, and LY255283, ONO compound ONO-LB-448, Searle compounds SC-41930, SC-50605 and SC-51146, and SK&F compound SKF-104493. Preferably, the LTB₄ inhibitors are selected from ebselen, Bayer Bay-x-1005, Ciba Geigy compound CGS-25019C, Leo Denmark compound ETH-615, Lilly compound LY-293111, Ono compound ONO-4057, and Terumo compound TMK-688.

Suitable 5-LO inhibitors include, among others, masoprocol, tenidap, zileuton, pranlukast, tepoxalin, rilopirox, flezelastine hydrochloride, enazadrem phosphate, and bunaprolast.

The present invention preferably includes compounds which selectively inhibit cyclooxygenase-2 over cyclooxygenase-1. Preferably, the compounds have a cyclooxygenase-2 IC₅₀ equal to or less than about 0.2 µM, and also have a selectivity ratio of cyclooxygenase-2 inhibition over cyclooxygenase-1 inhibition of at least 50, and more preferably of at least 100. Even more preferably, the compounds have a cyclooxygenase-1 IC₅₀ of greater than about 1.0 µM, and more preferably of greater than 10 µM. Such preferred selectivity may indicate an ability to reduce the incidence of common NSAID-induced side effects.

A preferred class of compounds consists of those compounds of Formula I wherein R³ is a radical selected from methyl, ethyl, isopropyl, *tert*-butyl, isobutyl, pentyl, hexyl, fluoromethyl, difluoromethyl, trifluoromethyl, chloromethyl, dichloromethyl, trichloromechyl, pentafluoroethyl, heptafluoropropyl, difluorochloromethyl, dichlorofluoromethyl, difluoroethyl, difluoropropyl, dichloroethyl, dichloropropyl, benzyl, phenylethyl, phenylpropyl, formyl, cyano, hydroxymethyl, hydroxyethyl, methoxymethyl, ethoxymethyl, methylthiomethyl, isopropylthiomethyl, cyclohexylthiomethyl, methylcarbonyl, ethylcarbonyl, phenylcarbonyl, trifluoromechylcarbonyl, difluoromethylcarbonyl, fluoromethylcarbonyl, benzylcarbonyl, cyanomethyl, cyanobutyl, methoxycarbonylmethyl, ethoxycarbonylethyl, carboxymethyl, carboxypropyl, phenylthiomethyl, 2-chlorophenylthiomethyl, 2,6-dichlorophenylthiomechyl, 4-methylphenylthiomethyl, 2-isopropylphenylthiomethyl, 2-methylphenylthiomethyl, phenyloxymethyl, 4-chlorophenyloxymethyl, 4-methylphenyloxymethyl, benzyloxymethyl, 4-methoxybenzyloxymethyl, naphthyl and phenyl, wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trifluoromethoxy;
wherein R¹ and R² is selected from phenyl, imidazolyl, thienyl, thiazolyl, pyrrolyl, oxazolyl, isoxazolyl, triazolyl, pyrazinyl, pyrimidinyl, quiolinyl, indolyl, benzimidazolyl, pyrazolyl and pyridyl, wherein R¹ and R² are optionally substituted at a substitutable position with one or more radicals independently selected from methylsulfonyl, aminosulfonyl, fluoromethylsulfonyl, difluoromethylsulfonyl, fluoro, chloro, bromo, methylthio, methylsulfinyl, methyl, ethyl, isopropyl, *tert*-butyl, isobutyl, pentyl, hexyl, cyano, carboxyl, methoxycarbonyl, ethoxycarbonyl, isopropoxycarbonyl *tert*-butoxycarbonyl, propoxycarbonyl, butoxycarbonyl, isobutoxycarbonyl, pentoxycarbonyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl, methoxymethyl, ethoxymethyl, trifluoromethoxy, amino, methylamino, N,N-diethylamino, phenylamino and nitro; provided that at least one of R¹ and R² is 4-methyl-sulfonylphenyl or 4-aminosulfonylphenyl or a pharmaceutically-acceptable salt thereof.

A class of compounds of particular interest consists of those compounds of Formula I, wherein R³ is a radical selected from methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl;
wherein R¹ is phenyl substituted with 4-methylsulfonyl or 4-aminosulfonyl; and wherein R² is selected from imidazolyl, thienyl, thiazolyl, pyrrolyl, oxazolyl, isoxazolyl, triazolyl, pyrimidinyl, quiolinyl, indolyl, benzimidazolyl, pyrazolyl and pyridyl, wherein R² is optionally substituted at a substitutable position with one or more radicals independently selected from methylthio, methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, methylenedioxy, ethoxy, propoxy, n-butoxy, hydroxymethyl, hydroxyethyl, methoxymethyl, ethoxymethyl, and trifluoromethoxy; or a pharmaceutically-acceptable salt thereof.

Especially preferred compounds are th ose of the following group:
1-methyl-3-[1-[4-(methylsulfonyl)phenyl]-4-trifluoromethyl-1H-imidazol-2-yl]-1H-indole;
2-[1-[4-(methylsulfonyl)phenyl]-4-(trifluoromethyl)-1H-imidazol-2-yl]thiophene;
3-[1-[4-(methylsulfonyl)phenyl]-4-(trifluoromethyl)-1H-imidazol-2-yl]thiophene;
2-methyl-3-[1-[4-(methylsulfonyl)phenyl]-4-(trifluoromethyl)-1H-imidazol-2-yl]pyridine;
3-fluoro-5-[1-[4-(methylsulfonyl)phenyl]-4-(trifluoromethyl)-1H-imidazol-2-yl]pyridine,
3-chloro-5-[1-[4-(methylsulfonyl)phenyl]-4-(trifluoromethyl)-1H-imidazol-2-yl]pyridine;
5-methyl-2-[1-[4-(methylsulfonyl)phenyl]-4-(trifluoromethyl)-1H-imidazol-2-yl]pyridine;
4-methyl-2-[1-[4-(methylsulfonyl)phenyl]-4-(trifluoromethyl)-1H-imidazol-2-yl]pyridine;
2-methoxy-6-[1-[4-(methylsulfonyl)phenyl]-4-(trifluoromethyl)-1H-imidazol-2-yl]pyridine;
5-methoxy-2-[1-[4-(mezhylsulfonyl)phenyl]-4-(trifluoromethyl)-1H-imidazo-1-2-yl]pyridine;
4-methoxy-2-[1-(4-(methylsulfonyl)phenyl]-4-(trifluoromethyl)-1H-imidazol-2-yl]pyridine;
2-chloro-6-[1-[4-(methylsulfonyl)phenyl]-4-(trifluoromethyl)-1H-imidazol-2-yl]pyridine;
5-chloro-2-[1-[4-(methylsulfonyl)phenyl]-4-(trifluoromethyl)-1H-imidazol-2-yl]pyridine;
4-chloro-2-[1-[4-(methylsulfonyl)phenyl]-4-(trifluoromethyl)-1H-imidazol-2-yl]pyridine;
2-fluoro-6-[1-[4-(methylsulfonyl)phenyl]-4-(trifluoromethyl)-1H-imidazol-2-yl]pyridine;
4-fluoro-2-[1-[4-(methylsulfonyl)phenyl]-4-(trifluoromethyl)-1H-imidazol-2-yl]pyridine;
4-fluoro-2-[1-[4-(methylsulfonyl)phenyl]-4-(trifluoromethyl)-1H-imidazol-2-yl]pyridine;
3-methoxy-5-[1-[4-(methylsulfonyl)phenyl]-4-(trifluoromethyl)-1H-imidazol-2-yl]pyridine;
3-[4-methyl-1-[4-(mehtylsulfonyl)phenyl]-4-(trifluoromethyl)-1H-imidazol-2-yl]pyridine;
3-[4-[4-(fluorophenyl)-1-[4-(methylsulfonyl)phenyl]-1H-imidazol-2-yl]pyridine;
5-[1-[4-(methylsulfonyl)phenyl]-4-(trifluoromethyl)-1H-imidazol-2-yl]-2-(methylthio)pyridine;
3-[4-(difluoromethyl)-1-[4-(methylsulfonyl)phenyl]-1H-imidazol-2-yl]pyridine;
3-[1-[4-(methylsulfonyl)phenyl]-4-(trifluoromethyl)-1H-imidazol-2-yl]pyridine;
2-[1-[4-(methylsulfonyl)phenyl-4-(trifluoromethyl)-1H-imidazol-2-yl]pyridine;
2-methyl-4-[1-[4-(methylsulfonyl)phenyl-4-(trifluoromethyl)-1H-imidazol-2-yl]pyridine;
4-[1-[4-(methylsulfonyl)phenyl-4-(trifluoromethyl)-1H-imidazol-2 -yl]pyridine;
2-methyl-6-[1-[4-(methylsulfonyl)phenyl-4-(trifluoromethyl)-1H-imidazol-2-yl]pyridine;
3-methyl-5-[1-[4-(methylsulfonyl)phenyl]-4-(trifluoromethyl)-1H-imidazol-2-yl]pyridine;
2-methoxy-5-[1-[4-(methylsulfonyl)phenyl]-4-(trifluoromethyl)-1H-imidazol-2-yl]pyridine; and
2-[4-(4-fluorophenyl-1-[4-(methylsulfonyl)phenyl]-4-(trifluoromethyl)-1H-imidazol-2-yl]pyridine.

Also preferred are compounds of the group
4-[2-(4-methylpyridin-2-yl)-4-(trifluoromethyl)-1H-imidazol-1-yl]benzenesulfonamide;
4-[2-(5-methylpyridin-3-yl)-4-(trifluoromethyl)-1H-imidazol-1-yl]benzenesulfonamide;
4-[2-(2-methylpyridin-3-yl)-4-(trifluoromethyl)-1H-imidazol-1-yl]benzenesulfonamide;
4-[2-(5-fluoropyridin-3-yl)-4-(trifluoromethyl)-1H-imidazol-1-yl]benzenesulfonamide;
4-[2-(5-chloropyridin-3-yl)-4-(trifluoromethyl)-1H-imidazol-1-yl]benzenesulfonamide;
4-[2-(5-methylpyridin-2-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(6-methoxypyridin-2-yl)-4-(trifluoromethyl)-1H-imidazol-1-yl]benzenesulfonamide;
4-[2-(5-methoxypyridin-2-yl)-4-(trifluoromethyl)-1H-imidazol-1-yl]benzenesulfonamide;
4-[2-(4-methoxypyridin-2-yl)-4-(trifluoromethyl)-1H-imidazol-1-yl]benzenesulfonamide;
4-[2-(6-chloropyridin-2-yl)-4-(trifluoromethyl)-1H-imidazol-1-yl]benzenesulfonamide;
4-[2-(5-chloropyridin-2-yl)-4-(trifluoromethyl)-1H-imidazol-1-yl]benzenesulfonamide;
4-[2-(4-chloropyridin-2-yl)-4-(trifluoromethyl)-1H-imidazol-1-yl]benzenesulfonamide;
4-[2-(6-fluoropyridin-2-yl)-4-(trifluoromethyl)-1H-imidazol-1-yl]benzenesulfonamide;
4-[2-(5-fluoropyridin-2-yl)-4-(trifluoromethyl)-1H-imidazol-1-yl]benzenesulfonamide;
4-[2-(4-fluoropyridin-2-yl)-4-(trifluoromethyl)-1H-imidazol-1-yl]benzenesulfonamide;
4-[2-(5-methoxypyridin-3-yl)4-(trifluoromethyl)-1H-imidazol-1-yl]benzenesulfonamide;
4-[4-methyl-2-(3-pyridinyl)-1H-imidazol-1-yl]benzenesulfonamide;
4-[2-(pyridin-3-yl)-4-(trifluoromethyl)-1H-imidazol-1-yl]benzenesulfonamide;
4-[4-(4-fluorophenyl)-2-(3-pyridinyl)-1H-imidazol-1-yl]benzenesulfonamide;
4-[2-[6-(methylthio)pyridin-3-yl]-4-trifluoromethyl)-1H-imidazol-1-yl]benzenesulfonamide;
4-[4-(difluoromethyl)*-*2-(3-pyridinyl)-1H-imidazol-1-yl]benzenesulfonamide; and
4-[2-(6-methylpyridin-3-yl)-4-(trifluoromethyl)-1H-imidazol-1-yl]benzenesulfonamide.

Within Formula I there is a subclass of compounds of high interest represented by Formula II: 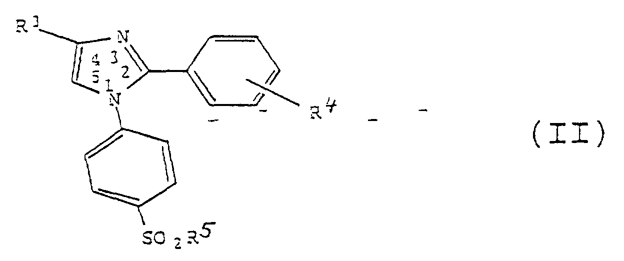 wherein R³ is a radical selected from C₁-C₆-alkyl, C₁-C₆-haloalkyl, formyl, C₁-C₆-alkanoyl, C₆-aroyl, cyano, C₁-C₆-alkylthio-C₁-C₆-alkyl, C₃-C₁₀-cycloalkylthio-C₁-C₆-alkyl, C₁-C₆-hydroxyalkyl, C₁-C₆-alkoxy-C₁-C₆-alkyl, C₁-C₆-alkylcarbonyl, C₁-C₆-cyanoalkyl, C₁-C₆-carboxyalkyl, C₁-C₆-alkoxycarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxy-C₁-C₆-alkyl and aryl; wherein R⁵ is a radical selected from methyl and amino; and wherein R⁴ is one or more radicals selected from hydrido, halo, C₁-C₆-alkyl, C₁-C₆-haloalkyl, C₁-C₆-alkoxy, amino, C₁-C₆-haloalkoxy, cyano, carboxyl, hydroxyl, C₁-C₆-hydroxyalkyl, C₁-C₆-alkoxy-C₁-C₆-alkyl, C₁-C₆-alkylamino, nitro and C₁-C₆-alkylthio; or a pharmaceutically-acceptable salt thereof.

A class of compounds of particular interest consists of those compounds of Formula II wherein R³ is selected from methyl, ethyl, isopropyl, *tert*-butyl, isobutyl, pentyl, hexyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, formyl, cyano, hydroxymethyl, hydroxyethyl, methoxymethyl, ethoxymethyl, methylthiomethyl, isopropylthiomethyl, cyclohexylthiomethyl, methylcarbonyl, cyanomethyl, cyanobutyl, methoxycarbonyl, ethoxycarbonyl, isopropoxycarbonyl, *tert*-butoxycarbonyl, propoxycarbonyl, carboxymethyl, carboxypropyl, phenylthiomethyl, 2-chlorophenylthiomethyl, 2, 6-dichlorophenylthiomethyl, 4-methylphenylthiomethyl, 2-isopropylphenylthiomethyl, 2-methylphenylthiomethyl, phenyloxymethyl, 4-chlorophenyloxymethyl, 4-methylphenyloxymethyl, benzyloxymethyl, 4-methoxybenzyloxymethyl, naphthyl and phenyl, wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difiuorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trilfluoromethoxy; wherein R⁵ is methyl or amino; and wherein R⁴ is a radical selected from hydrido, methylsulfonyl, fluoro, chloro, bromo, methylthio, methyl, ethyl, isopropyl, *tert*-butyl, isobutyl, pentyl, hexyl, cyano, carboxyl, methoxycarbonyl, ethoxycarbonyl, isopropoxycarbonyl, *tert*-butoxycarbonyl, propoxycarbonyl, butoxycarbonyl, isobutoxycarbonyl, pentoxycarbonyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl, methoxymethyl, ethoxymethyl, trifluoromethoxy, amino, methylamino, N,N-dimethylamino, phenylamino and nitro; or a pharmaceutically-acceptable salt thereof.

Preferred compounds of Formula II are those of the group consisting of
2-(4-fluorophenyl)-1-[4-(methylsulfonyl)phenyl]-4-(trifluoromethyl)-1H-imidazole;
2-(3,4-difluorophenyl)-1-[4-(methylsulfonyl)phenyl]-4-(trifluoromethyl)-1H-imidazole;
2-(2-fluorophenyl)-1-[4-(methylsulfonyl)phenyl]-4-(trifluoromethyl)-1H-imidazole;
2-(3-fluorophenyl)-1-[4-(methylsulfonyl)phenyl]-4-(trifluoromethyl)-1H-imidazole;
2-(4-fluoro-3-methylphenyl)-1-[4-(methylsulfonyl)phenyl]-4-(trifluoromethyl)-1H-imidazole;
2-(3-chloro-4-(N,N-dimethylamino)phenyl)-1-[4-(methylsulfonyl)phenyl]-4-(trifluoromethyl)-1H-imidazole;
2-(3-fluoro-4-(N,N-dimethylamino)phenyl)-1-[4-(methylsulfonyl)phenyl]-4-(trifluoromethyl)-1H-imidazole;
2-(3-bromophenyl)-1-[4-(methylsulfonyl)phenyl]-4-(trifluoromethyl)-1H-imidazole;
2-(3-nitrophenyl)-1-[4-(methylsulfonyl)phenyl]-4-(trifluoromethyl)-1H-imidazole;
2-(4-chlorophenyl)-1-[4-(methylsulfonyl)phenyl]-4-(trifluoromethyl)-1H-imidazole;
2-(4-chlorophenyl)-1-[4-(methylsulfonyl)phenyl]-4-methyl-1H-imidazole;
2-(4-chlorophenyl)-1-[4-(methylsulfonyl)phenyl]-4-phenyl-1H-imidazole;
2-(4-chlorophenyl)-4-(4-fluorophenyl)-1-[4-(methylsulfonyl)phenyl]-1H-imidazole;
2-(3-fluoro-4-methoxyphenyl)-1-[4-(methylsulfonyl)phenyl-4-(trifluoromethyl)-1H-imidazole;
1-[4-(methylsulfonyl)phenyl]-2-phenyl-4-trifluoromethyl-1H-imidazole;
2-(4-methylphenyl)-1-[4-(methylsulfonyl)phenyl]-4-trifluoromethyl-1H-imidazole;
2-(3-fluoro-5-methylphenyl)-1-[4-(methylsulfonyl)phenyl]-4-(trifluoromethyl)-1H-imidazole;
2-(3-methylphenyl)-1-[4-(methylsulfonyl)phenyl]-4-trifluoromethyl-1H-imidazole;
2-(3,5-difluoro-4-methoxyphenyl)-1-[4-(methylsulfonyl)phenyl]-4-trifluoromethyl-1H-imidazole;
1-[4-(methylsulfonyl)phenyl]-2-(3-chlorophenyl)-4-trifluoromethyl-1H-imidazole;
- 1-[4-(methylsulfonyl)phenyl]-2-(4-methoxy-3-chlorophenyl)-4-trifluoromethyl-1H-imidazole; and
2-(2-methylphenyl)-1-[4-(methylsulfonyl)phenyl]-4-trifluoromethyl-1H-imidazole;
and those of the group consising of
4-[2-(4-fluorophenyl)-4-(trifluoromethyl)-1H-imidazol-1-yl]benzenesulfonamide;
4-[2-(4-methylphenyl)-4-(trifluoromethyl)-1H-imidazol-1-yl]benzenesulfonamide;
4-[2-(3,4-difluorophenyl)-4-(trifluoromethyl)-1H-imidazol-1-yl]benzenesulfonamide;
4-[2-(2-fluorophenyl)-4-(trifluoromethyl)-1H-imidazol-1-yl]benzenesulfonamide;
4-[2-(3-fluorophenyl)-4-(trifluoromethyl)-1H-imidazol-1-yl]benzenesulfonamide;
4-[2-(4-chloro-3-methoxyphenyl)-4-(trifluoromethyl)-1H-imidazol-1-yl]benzenesulfonamide;
4-[2-(3,5-difluoro-4-methoxyphenyl)-4-(trifluoromethyl)-1H-imidazol-1-yl]benzenesulfonamide;
4-[2-(4-chlorophenyl)-4-(trifluoromethyl)-1H-imidazol-1-yl]benzenesulfonamide;
4-[2-(3-chloro-4-methylphenyl)-4-(trifluoromethyl)-1H-imidazol-1-yl]benzenesulfonamide;
4-[2-(3-fluoro-4-methoxyphenyl)-4-(trifluoromethyl)-1H-imidazol-1-yl]benzenesulfonamide;
4-[2-(3-fluoro-5-methylphenyl-4-(trifluoromethyl)-1H-imidazol-1-yl]benzenesulfonamide;
4-[2-(3-methylphenyl)-4-trifluoromethyl-1H-imidazol-1-yl]benzenesulfonamide;
4-[2-(3-chlorophenyl)-4-trifluoromethyl-1H-imidazol-1-yl]benzenesulfonamide;
4-[2-phenyl-4-trifluoromethyl-1H-imidazol-1-yl]benzenesulfonamide; and
4-[2-(4-methoxy-3-chlorophenyl)-4-trifluoromethyl-1H-imidazol-1-yl ]benzenesulfonamide.

Another group of preferred compounds are those of formula (III) 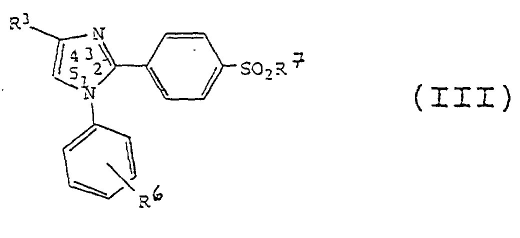 wherein R³ is a radical selected from C₁-C₆-alkyl, C₁-C₆-haloalkyl, formyl, C₁-C₆-alkanoyl, C₆-aroyl, cyano, C₁-C₆-alkylthio-C₁-C₆₋alkyl, C₃-C₁₀-cycloalkylthio-C₁-C₆-alkyl, C₁-C₆-hydroxyalkyl, C₁-C₆-alkoxyalkyl, C₁-C₆-alkylcarbonyl, C₁-C₆-cyanoalkyl, C₁-C₆-azidoalkyl, C₁-C₆-carboxyalkyl, C₁-C₆-alkoxycarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxyalkyl and aryl; wherein the aryl radicals are optionally substituted an a substitutable position with one or more radicals selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and C₁-C₆-haloalkoxy; wherein R⁶ is one or more radicals selected from hydrido, halo, C₁-C₆-alkyl, C₁-C₆-haloalkyl, C₁-C₆-alkoxy, amino, C₁-C₆-haloalkoxy, cyano, carboxyl, hydroxyl, C₁-C₆-hydroxyalkyl, C₁-C₆-alkoxy-C₁-C₆-alkyl,C₁-C₆-alkylamino, nitro and C₁-C₆-alkylthio; and wherein R⁷ is a radical selected-from methyl and amino; or a pharmaceutically-acceptable salt thereof.

Preferred compounds of formula III are those from methyl, ethyl, isopropyl, *tert*-butyl, isobutyl, pentyl, hexyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, formyl, cyano, hydroxymethyl, hydroxyethyl, methoxymethyl, ethoxymethyl, methylthiomethyl, methylcarbonyl, cyanomethyl, cyanobutyl, azidomethyl, methoxycarbonyl, ethoxycarbonyl, isopropoxycarbonyl, *tert*-butoxycarbonyl, propoxycarbonyl, phenylthiomethyl, phenyloxymethyl, 4-chlorophenyloxymethyl, 4-methylphenyloxymethyl, benzyloxymethyl, 4-methoxybenzyloxymethyl, naphthyl and phenyl, wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trifluoromethoxy;
wherein R⁶ is a radical selected from hydrido, fluoro, chloro, bromo, iodo, methyl, ethyl, isopropyl, *tert*-butyl, isobutyl, fluoromethyl, difluoromethyl, trifluoromethyl, chloromethyl, dichloromethyl, trichloromethyl pentafluoroethyl, heptafluoropropyl, difluorochloromethyl, dichlorofluoromethyl, difluoroethyl, difluoropropyl, dichloroethyl, dichloropropy-1, methoxy, ethoxy, isopropoxy, *tert*-butoxy, propoxy, butoxy, isobutoxy, pentoxy, methylenedioxy, amino, trifluoromethoxy, cyano, carboxyl, hydroxyl, nitro, hydroxymethyl, methoxymethyl, ethoxymethyl, methylamino, mechylthio, ethylthio, propylthio and butylthio; and wherein R⁷ is methyl, fluoromethyl or amino, or a pharmaceutically-acceptable salt thereof.

A family of other specific compounds of particular interest within Formula II consists of compounds and pharmaceutically-acceptable salts thereof as follows:
2-(3,4-difluorophenyl)-1-[4-(methylsulfonyl)phenyl]-4-(trifluoromethyl)-1H-imidazole;
2-(4-chlorophenyl)-4-[(4-methylphenoxy)methyl]-1-[4-(methylsulfonyl)phenyl]-1H-imidazole;
2-(4-chlorophenyl)-1-[4-(methylsulfonyl)phenyl]-4-[(methylthio)methyl]-1H-imidazole;
1,2-bis[4-(methylsulfonyl)phenyl]-4-(trifluoromethyl)-1H-imidazole;
4-[2-(3,4-difluorophenyl)-4-(trifluoromethyl)-1H-imidazol-l-yl]benzenesulfonamide;
4-[2-(3-bromo-4-methoxyphenyl)-4-(trifluoromethyl)-1H-imidazol-1-yl]benzenesulfonamide;
2-(4-chlorophenyl)-1-[4-(methylsulfonyl)phenyl]-1H-imidazole-4-carboxaldehyde;
2-(4-chlorophenyl)-4-[[(4-methylphenyl)thiomethyl]-1-[4-(methylsulfonyl)phenyl]-1H-imidazole;
2-(3-methoxyphenyl)-1-[4-(methylsulfonyl)phenyl]-4-(trifluoromethyl)-1H-imidazole;
2-(4-chlorophenyl)-4-fluoromethyl)-1-[4-(methylsulfonyl)phenyl]-1H-imidazole;
2-(2-fluorophenyl)-1-[4-(methylsulfonyl)phenyl]-4-(trifluoromethyl)-1H-imidazole;
2-(4-chlorophnyl)-1-[4-(methylsulfonyl)phenyl]-4-(phenylmethoxymethyl)-1H-imidazole;
4-[2-(2-fluorophenyl)-4-(trifluoromethyl)-1H-imidazol-1-yl]benzenesulfonamide;
N,N-dimethyl-4-[1-[4-(methylsulfonyl)phenyl]-4-(trifluoromethyl)-1H-imidazol-2-yl]benzenamine;
2-(4-chlorophenyl)-4-[[(1-methylethyl)thio]methyl]-1-[4-(methylsulfonyl)phenyl]-1H-imidazole;
2-(4-chlorophenyl)-4-[[(cyclohexyl)thio]methyl]-1-[4-(methysulfonyl)phenyl-1H-imidazole;
1-[4-(methylsulfonyl)phenyl]-2-[3-(trifluoromethyl)phenyl]-4-(trifluoromethyl)-1H-imidazole;
2-[3-(methoxymethyl)phenyl]-1-[4-(methylsulfonyl)phenyl]-4-(trifluoromethyl)-1H-imidazole;
2-fluoro-N,N-dimethyl-4-[1-[4-(methylsulfonyl)phenyl]-4-(trifluoromethyl)-1H-imidazol-2-yl]benzeneamine;
2-(3-bromophenyl)-1-[4-(methylsulfonyl)phenyl]-4-(trifluoromethyl)-1H-imidazole;
4-[4-(trifluoromethyl)-2-[3-(trifluoromethyl)phenyl]-1H-imidazol-l-yl]benzenesulfonamide;
2-(4-chlorophenyl)-4-[[(2-chlorophenyl)thio]methyl]-1-[4-(methylsulfonyl)phenyl]-1H-imidazole;
1-[4-(methylsulfonyl)phenyl]-2-(3-nitrophenyl)-4-(trifluoromethyl)-1H-imidazole;
2-(4-chlorophenyl)-4-[[(2-methylphenyl)thio]methyl]-1-[4-(methylsulfonyl)phenyl-1H-imidazole;
N-methyl-4-[1-[4-(methylsulfonyl)phenyl]-4-(trifluoromethyl)-1H-imidazol-2-yl]benzenamine;
3-[1-[4-(methylsulfonyl)phenyl]-4-(trifluoromethyl)-1H-imidazol-2-yl] benzeneamine;
N,N-dimethyl-3-[1-[4-(methylsulfonyl)phenyl]-4-(trifluoromethyl)-1H-imidazol-2-yl]benzenamine;
N-methyl-3-[1-[4-(methylsulfonyl)phenyl]-4-(trifluoromethyl)-1H-imidazol-2-yl]benzenamine;
2-fluoro-N-methyl-4-[1-[4-(methylsulfonyl)phenyl]-4-(trifluoromethyl)-1H-imidazol-2-yl]benzeneamine;
2-(4-chlorophenyl)-4-[[(2,6-dichlorophenyl)thio]methyl]-1-[4-(methylsulfonyl)phenyl]-1H-imidazole;
2-(4-chlorophenyl)-4-[[(2-(1-methylethyl)phenyl]thio]methyl]-1-[4-(methylsulfonyl)phenyl]-1H-imidazole;
1-[2-(4-chlorophenyl)-1-[4-(methylsulfonyl)phenyl]-1H-imidazol-4-yl]ethanone;
1-[4-(methylsulfonyl)phenyl]-2-[3-(methylthio)phenyl]-4-(trifluoromethyl)-1H-imidazole;
4-[2-(3-bromophenyl)-4-(trifluoromethyl)-1H-imidazol-1-yl]benzenesulfonamide;
2-(3-chloro-5-methylphenyl)-1-[4-(methylsulfonyl)phenyl]-4-(trifluoromethyl)-1H-imidazole;
4-[2-(3-chloro-5-methylphenyl)-4-(trifluoromethyl)-1H-imidazol-1-yl]benzenesulfonamide;
2-(4-chlorophenyl)-1-[4-(methylsulfonyl)phenyl]-1H-imidazole-4-acetonitrile;
2-(3-fluoro-5-methylphenyl)-1-[4-(methylsulfonyl)phenyl]-4-(trifluoromethyl)-1H-imidazole;
2-(3-fluoro-5-methoxyphenyl)-1-[4-(methylsulfonyl)phenyl]-4-(trifluoromethyl)-1H-imidazole;
4-[2-(3-fluoro-5-methylphenyl)-4-(trifluoromethyl)-1H-imidazol-1-yl]benzenesulfonamide;
2-(4-chlorophenyl)-1-[4-(methylsulfonyl)phenyl]-1H-imidazole-4-acetic acid;
4-[2-(3-fluoro-5-methoxyphenyl)-4-(trifluoromethyl)-1H-imidazol-1-yl]benzenesulfonamide;
2-(4-chlorophenyl)-4-trifluoromethyl-1-[4-(methylsulfonyl)phenyl]-1H-imidazole;
2-(4-chlorophenyl)-4-difluoromethyl-1-[4-(methylsulfonyl)phenyl]-1H-imidazole;
2-(4-chlorophenyl)-4-methyl-1-[4-(methylsulfonyl)phenyl]-1H-imidazole;
2-(4-chlorophenyl)-4-ethyl-1-[4-(methylsulfonyl)phenyl]-1H-imidazole;
2-(4-chlorophenyl)-4-phenyl-1-[4-(methylsulfonyl)phenyl]-1H-imidazole;
2-(4-chlorophenyl)-4-(4-fluorophenyl)-1-[4-(methylsulfonyl)phenyl]-1H-imidazole;
2-(4-chlorophenyl)-4-(4-bromophenyl)-1-[4-(methylsulfonyl)phenyl]-1H-imidazole;
2-(4-chlorophenyl)-4-(4-chlorophenyl)-1-[4-(methylsulfonyl)phenyl]-1H-imidazole;
2-(4-chlorophenyl)-4-(2-naphthyl)-1-[4-(methylsulfonyl)phenyl]-1H-imidazole;
2-(4-chlorophenyl)-4-[4-(trifluoromethoxy)phenyl]-1-[4-(methylsulfonyl)phenyl]-1H-imidazole;
2-(4-chlorophenyl)-4-(3-chlorophenyl)-1-[4-(methylsulfonyl]phenyl]-1H-imidazole;
2-(4-chlorophenyl)-4-(3-fluorophenyl)-1-[4-(methylsulfonyl)phenyl]-1H-imidazole;
2-(4-chlorophenyl)-4-(4-methoxyphenyl)-1-[4-(methylsulfonyl)phenyl]-1H-imidazole;
2-(4-chlorophenyl)-4-phenoxymethyl-1-[4-(methylsulfonyl)phenyl]-1H-imidazole;
2-(4-chlorophenyl)-4-(4-chlorophenoxy)methyl-1-[4-(methylsulfonyl)phenyl]-1H-imidazole;
2-(4-chlorophenyl)-4-(4-fluorophenoxy)methyl-1-[4-(methylsulfonyl)phenyl]-1H-imidazole;
2-(4-chlorophenyl)-4-phenylthiomethyl-1-[4-(methylsulfonyl)phenyl]-1H-imidazole;
2-(4-chlorophenyl)-4-(N-phenyl-N-methylamino)methyl-1-[4-(methylsulfonyl)phenyl]-1H-imidazole;
2-(4-chlorophenyl)-4-(2-quinolyl)methoxymethyl-1-[4-(methylsulfonyl)phenyl]-1H-imidazole;
2-(4-chlorophenyl)-4-methoxymethyl-1-[4-(methylsulfonyl)phenyl]-1H-imidazole;
2-(4-chlorophenyl)-4-(4-methoxybenzyloxy)methyl-1-[4-(methylsulfonyl)phenyl]-1H-imidazole;
2-(4-chlorophenyl)-1-(4-(methylsulfonyl)phenyl]-1H-imidazole-4-methanol;
2-(4-chlorophenyl)-4-formyl-1-[4-(methylsulfonyl)phenyl]-1H-imidazole;
2-(4-chlorophenyl)-1-[4-(methylsulfonyl)phenyl]-1H-imidazole-4-carbonitrile;
2-(4-chlorophenyl)-4-benzyl-1-[4-(methylsulfonyl)phenyl]-1H-imidazole;
2-(4-chlorophenyl)-4-phenylethyl-1-[4-(methylsulfonyl)phenyl]-1H-imidazole;
2-(4-chlorophenyl)-4-hexyl-1-[4-(methylsulfonyl)phenyl]-1H-imidazole;
2-(4-chlorophenyl)-4-hexylcarbonyl-1-[4-(methylsulfonyl)phenyl]-1H-imidazole;
2-(4-chlorophenyl)-4-phenylcarbonyl-1-[4-(methylsulfonyl)phenyl]-1H-imidazole;
2-(4-chlorophenyl)-4-benzylcarbonyl-1-[4-(methylsulfonyl)phenyl]-1H-imidazole;
2-(4-chlorophenyl)-4-(1-hydroxy-1-phenyl-methyl)-1-[4-(methylsulfonyl)phenyl]-1H-imidazole;
2-(4-chlorophenyl)-4-(1-hexanol)-1-[4-(methylsulfonyl)phenyl]-1H-imidazole;
2-(4-chlorophenyl)-1-((methylsulfonyl)phenyl]-1H-imidazole;
2-(4-chlorophenyl)-4-octyl-1-[4-(methylsulfonyl)phenyl]-1H-imidazole;
2-(4-chlorophenyl)-4-methoxy-1-[4-(methylsulfonyl)phenyl]-1H-imidazole;
2-(4-chlorophenyl)-4-butoxy-1-[4-(methylsulfonyl)phenyl]-1H-imidazole;
2-(4-chlorophenyl)-4-methylthio-1-[4-(methylsulfonyl)phenyl]-1H-imidazole;
2-(4-chlorophenyl)-1-[4-(methylsulfonyl)phenyl]-4-trifluoromethylsulfonyl-1H-imidazole;
2-(4-chlorophenyl)-1-[4-(methylsulfonyl)phenyl]-4-trifluoromethylcarbonyl-1H-imidazole;
2-(4-chlorophenyl)-4-(2-thienyl)-1-[4-(methylsulfonyl)phenyl]-1H-imidazole;
2-(4-chlorophenyl)-4-(3-furyl)-1-[4-(methylsulfonyl)phenyl]-1H-imidazole;
2-(4-chlorophenyl)-4-(4-pyridyl)-1-[4-(methylsulfonyl)phenyl]-1H-imidazole;
2-(4-chlorophenyl)-4-chloro-1-[4-(methylsulfonyl)phenyl]-1H-imidazole;
2-(4-chlorophenyl)-4-fluoro-1-[4-(methylsulfonyl)phenyl]-1H-imidazole;
methyl [2-(4-chlorophenyl)-1-[4-(methylsulfonyl)phenyl]-1H-imidazol-4-yl]carboxylate;
[2-(4-chlorophenyl)-1-[4-(methylsulfonyl)phenyl]-1H-imidazol-4-yl] carboxamide;
methyl [2-(4-chlorophenyl)-1-[4-(methylsulfonyl)phenyl]-1H-imidazol-4-yl]carboxamide;
4-[2-(4-chlorophenyl)-4-trifluoromethyl-1H-imidazol-1-yl]benzenesulfonamide;
4-[2-(4-chlorophenyl)-4-difluoromethyl-1H-imidazol-1-yl]benzenesulfonamide;
4-[2-(4-chlorophenyl)-4-methyl-1H-imidazol-1-yl]benzenesulfonamide;
4-[2-(4-chlorophenyl)-4-ethyl-1H-imidazol-1-yl]benzenesulfonamide;
4-[2-(4-chlorophenyl)-4-phenyl-1H-imidazol-1-yl]benzenesulfonamide;
4-[2-(4-chlorophenyl)-4-(4-fluorophenyl)-1H-imidazol-1-yl]benzenesulfonamide;
4-[2-(4-chlorophenyl)-4-(4-bromophenyl)-1H-imidazol-1-yl]benzenesulfonamide;
4-[2-(4-chlorophenyl)-4-(4-chlorophenyl)-1H-imidazol-1-yl]benzenesulfonamide;
4-[2-(4-chlorophenyl)-4-(2-naphthyl)-1H-imidazol-1-yl]benzenesulfonamide;
4-[2-(4-chlorophenyl)-4-[4-(trifluoromethoxy)phenyl]-lH-imidazol-1-yl]benzenesulfonamide;
4-[2-(4-chlorophenyl)-4-(3-chlorophenyl)-1H-imidazol-1-yl]benzenesulfonamide;
4-[2-(4-chlorophenyl)-4-(3-fluorophenyl)-1H-imidazol-1-yl]benzenesulfonamide;
4-[2-(4-chlorophenyl)-4-(4-methoxyphenyl)-1H-imidazol-1-yl]benzenesulfonamide;
4-[2-(4-chlorophenyl)-4-phenoxymethyl-1H-imidazol-1-yl]benzenesulfonamide;
4-[2-(4-chlorophenyl)-4-(4-chlorophenoxy)methyl-1H-imidazol-1-yl]benzenesulfonamide;
4-[2-(4-chlorophenyl)-4-(4-fluorophenoxy)methyl-1H-imidazol-1-yl]benzenesulfonamide;
4-[2-(4-chlorophenyl)-4-phenylthiomethyl-1H-imidazol-1-yl]benzenesulfonamide;
4-[2-(4-chlorophenyl)-4-(N-phenyl-N-methylamino)methyl-1H-imidazol-1-yl]benzenesulfonamide;
4-[2-(4-chlorophenyl)-4-(2-quinolyl)methoxymethyl-1H-imidazol-1-yl]benzenesulfonamide;
4-[2-(4-chlorophenyl)-4-methoxymethyl-1H-imidazol-1-yl]benzenesulfonamide;
4-[2-(4-chlorophenyl)-4-(4-methoxybenzyloxy)methyl-1H-imidazol-1-yl]benzenesulfonamide;
4-[2-(4-chlorophenyl)-4-hydroxymethyl-1H-imidazol-1-yl]benzenesulfonamide;
4-[2-(4-chlorophenyl)-4-formyl-1H-imidazol-l-yl]benzenesulfonamide;
4-[2-(4-chlorophenyl)-4-cyano-1H-imidazol-1-yl]benzenesulfonamide;
4-[2-(4-chlorophenyl)-4-benzyl-1H-imidazol-1-yl]benzenesulfonamide;
4-[2-(4-chlorophenyl)-4-phenylethyl-1H-imidazol-1-yl]benzenesulfonamide;
4-[2-(4-chlorophenyl)-4-hexyl-1H-imidazol-1-yl]benzenesulfonamide;
4-[2-(4-chlorophenyl)-4-hexylcarbonyl-1H-imidazol-1-yl]benzenesulfonamide;
4-[2-(4-chlorophenyl)-4-phenylcarbonyl-1H-imidazol-1-yl]benzenesulfonamide;
4-[2-(4-chlorophenyl)-4-benzylcarbonyl-1H-imidazol-1-yl]benzenesulfonamide;
4-[2-(4-chlorophenyl)-4-(1-hydroxy-1-phenyl-methyl)-1H-imidazol-1-yl]benzenesulfonamide;
4-[2-(4-chlorophenyl)-4-(1-hexanol)-1H-imidazol-1-yl]benzenesulfonamide;
4-[2-(4-chlorophenyl)-1H-imidazol-1-yl]benzenesulfonamide;
4-[2-(4-chlorophenyl)-4-octyl-1H-imidazol-1-yl]benzenesulfonamide;
4-[2-(4-chlorophenyl)-4-methoxy-1H-imidazol-1-yl]benzenesulfonamide;
4-[2-(4-chlorophenyl)-4-butoxy-1H-imidazol-1-yl]benzenesulfonamide;
4-[2-(4-chlorophenyl)-4-methylthio-1H-imidazol-1-yl]benzenesulfonamide;
4-[2-(4-chlorophenyl)-4-(2-thienyl)-1H-imidazol-1-yl]benzenesulfonamide;
4-[2-(4-chlorophenyl)-4-(3-furyl)-1H-imidazol-1-yl]benzenesulfonamide;
4-[2-(4-chlorophenyl)-4-(4-pyridyl)-1H-imidazol-1-yl]benzenesulfonamide;
4-[2-(4-chlorophenyl)-4-chloro-1H-imidazol-1-yl]benzenesulfonamide;
4-[2-(4-chlorophenyl)-4-fluoro-1H-imidazol-1-yl]benzenesulfonamide;
[2-(4-chlorophenyl)-1-[4-(aminosulfonyl)phenyl)-1H-imidazol-4-yl]carboxylic acid;
methyl[2-(4-chlorophenyl)-1-[4-(aminosulfonyl)phenyl)-1H-imidazol-4-yl]carboxylate;
[2-(4-chlorophenyl)-1-[4-(aminosulfonyl)phenyl)-1H-imidazol-4-yl]carboxamide;
methyl[2-(4-chlorophenyl)-1-[4-(aminosulfonyl)phenyl)-1H-imidazol-4-yl]carboxamide;
2-(3-methylphenyl)-1-[4-(methylsulfonyl)phenyl]-4-trifluoromethyl-1H-imidazole;
2-(2-methylphenyl)-1-[4-(methylsulfonyl)phenyl]-4-trifluoromethyl-1H-imidazole;
2-(4-fluoro-3-methylphenyl)-1-[4-(methylsulfonyl)phenyl]-4-trifluoromethyl-1H-imidazole;
2-(4-chloro-3-methylphenyl)-1-[4-(methylsulfonyl)phenyl]-4-trifluoromethyl-1H-imidazole;
2-(4-fluoro-3-methoxyphenyl)-1-[4-(methylsulfonyl)phenyl]-4-trifluoromethyl-lH-imidazole;
2-(4-chloro-3-methoxyphenyl)-1-[4-(methylsulfonyl)phenyl]-4-trifluoromethyl-1H-imidazole;
2-(3-fluoro-4-methylphenyl)-1-[4-(methylsulfonyl)phenyl]-4-trifluoromethyl-lH-imidazole;
2-(3-chloro-4-methylphenyl) -1-[4-(methylsulfonyl)phenyl]-4-trifluoromethyl-lH-imidazole;
2-(3-fluoro-4-methylthiophenyl)-1-[4-(methylsulfonyl)phenyl]-4-trifluoromethyl-1H-imidazole;
2-(3-chloro-4-methylthiophenyl)-1-[4-(methylsulfonyl)phenyl]-4-trifluoromethyl-1H-imidazole;
2-(4-fluoro-3-methylthiophenyl)-1-[4-(methylsulfonyl)phenyl]-4-trifluoromethyl-lH-imidazole;
2-(4-chloro-3-methylthiophenyl)-1-[4-(methylsulfonyl)phenyl] -4-trifluoromethyl-lH-imidazole;
2-(3,5-dimethyl-4-methoxyphenyl)-1-[4-(methylsulfonyl]phenyl]-4-trifluoromethyl-1H-imidazole;
2-(3,5-dichloro-4-methoxyphenyl)-1-[4-(methylsulfonyl)phenyl]-4-trifluoromethyl-1H-imidazole;
2-(3,5-difluoro-4-methoxyphenyl)-1-[4-(methylsulfonyl)phenyl]-4-trifluoromethyl-1H-imidazole;
2-(3,4-dimethylphenyl)-1-[4-(methylsulfonyl)phenyl]-4-trifluoromethyl-1H-imidazole;
2-(3,5-dichlorophenyl)-1-[4-(methylsulfonyl)phenyl]-4-trifluoromethyl-1H-imidazole;
4-[2-(3-methylphenyl)-4-trifluoromethyl-1H-imidazol-1-yl]benzenesulfonamide;
4-[2-(2-methylphenyl)-4-trifluoromethyl-1H-imidazol-1-yl]benzenesulfonamide;
4-[2-(4-fluoro-3-methylphenyl)-4-trifluoromethyl-1H-imidazol-1-yl]benzenesulfonamide ;
4-[2-(4-chloro-3-methylphenyl)-4-trifluoromethyl-lH-imidazol-1-yl]benzenesulfonamide;
4-[2-(4-fluoro-3-methoxyphenyl)-4-trifluoromethyl-1H-imidazol-1-yl]benzenesulfonamide;
4-[2-(4-chloro-3-methoxyphenyl)-4-trifluoromethyl-1H-imidazol-1-yl]benzenesulfonamide;
4-[2-(3-fluoro-4-methylphenyl)-4-trifluoromethyl-lH-imidazol-1-yl]benzenesulfonamide;
4-[2-(3-chloro-4-methylthiophenyl)-4-trifluoromethyl-1H-imidazol-1-yl]benzenesulfonamide;
4-[2-(3-fluoro-4-methylthiophenyl)-4-trifluoromethyl-1H-imidazol-1-yl]benzenesulfonamide;
4-[2-(4-fluoro-3-methylthiophenyl)-4-trifluoromethyl-1H-imidazol-1-yl]benzenesulfonamide;
4-[2-(4-chloro-4-methylthiophenyl)-4-trifluoromethyl-1H-imidazol-1-yl]benzenesulfonamide;
4-[2-(3,5-dimethyl-4-methoxyphenyl)-4-trifluoromethyl-1H-imidazol-1-yl]benzenesulfonamide;
4-[2-(3,5-dichloro-4-methoxyphenyl)-4-trifluoromethyl-1H-imidazol-1-yl]benzenesulfonamide;
4-[2-(3,5-difluoro-4-methoxyphenyl)-4-trifluoromethyl-1H-imidazol-1-yl]benzenesulfonamide;
4-[2-(3,4-dimethylphenyl)-4-trifluoromethyl-1H-imidazol-1-yl]benzenesulfonamide;
4-[2-(3,5-dichlorophenyl)-4-trifluoromethyl-1H-imidazol-l-yl]benzenesulfonamide;
ethyl [2-(4-chlorophenyl)-1-[4-(methylsulfonyl)phenyl]-1H-imidazol-4-yl]carboxylate;
ethyl [1-[4-(aminosulfonyl)phenyl]-2-(4-chlorophenyl)-1H-imidazol-4-yl]carboxylate;
1-[4-(methylsulfonyl)phenyl]-2-(4-methylphenyl)-4-trifluoromethyl-1H-imidazole;
1-[4-(methylsulfonyl)phenyl]-2-(3-chlorophenyl)-4-trifluoromethyl-1H-imidazole;
1-[4-(methylsulfonyl)phenyl]-2-(4-methyl-3-chlorophenyl)-4-trifluoromethyl-1H-imidazole;
5-[1-[4-(methylsulfonyl)phenyl]-4-trifluoromethyl-1H-imidazol-2-yl]-1,3-benzodioxole;
1-[4-(methylsulfonyl)phenyl]-2-(4-methoxyphenyl)-4-trifluoromethyl-1H-imidazole;
1-[4-(methylsulfonyl)phenyl]-2-(3-fluorophenyl)-4-trifluoromethyl-1H-imidazole;
1-[4-(methylsulfonyl)phenyl]-2-(4-fluorophenyl)-4-trifluoromethyl-1H-imidazole;
1-[4-(methylsulfonyl)phenyl]-2-(4-methoxy-3-fluorophenyl)-4-trifluoromethyl-1H-imidazole;
1-[4-(methylsulfonyl)phenyl]-2-phenyl-4-trifluoromethyl-1H-imidazole;
1-[4-(methylsulfonyl)phenyl]-2-(4-methoxy-3-chlorophenyl)-4-trifluoromethyl-1H-imidazole;
1-[4-(methylsulfonyl)phenyl]-2-[4-(trifluoromethyl)phenyl]-4-trifluoromethyl-lH-imidazole;
1-[4-(methylsulfonyl)phenyl]-2-(4-bromophenyl)-4-trifluoromethyl-1H-imidazole;
1-[4-(methylsulfonyl)phenyl]-2-(2-chlorophenyl) -4-trifluoromethyl-1H-imidazole;
1-[4-(methylsulfonyl)phenyl]-2-(4-ethylphenyl)-4-trifluoromethyl-1H-imidazole;
1-[4-(methylsulfonyl)phenyl]-2-(4-butylphenyl)-4-trifluoromethyl-1H-imidazole;
1-[4-(methylsulfonyl)phenyl]-2-[4-(difluoromethyl)phenyl]-4-trifluoromethyl-lH-imidazole;
1-[4-(methylsulfonyl)phenyl]-2-(4-butoxyphenyl)-4-trifluoromethyl-1H-imidazole;
1-[4-(methylsulfonyl)phenyl]-2-(4-(methylthio)phenyl]-4-trifluoromethyl-1H-imidazole;
1-[4-(methylsulfonyl)phenyl]-2-(4-methoxy-3,5-dichloro-phenyl)-4-trifluoromethyl-1H-imidazole;
1-[4-(methylsulfonyl)phenyl]-2-(4-methyl-3,5-difluorophenyl)-4-trifluoromethyl-1H-imidazole;
1-[4-(methylsulfonyl)phenyl]-2-(2,4-dichlorophenyl)-4-trifluoromethyl-1H-imidazole;
1-[4-(methylsulfonyl)phenyl]-2-(3,4-dichlorophenyl)-4-trifluoromethyl-1H-imidazole;
1-[4-(methylsulfonyl)phenyl]-2-[4-(trifluoromethyl)phenyl]-4-trifluoromethoxy-1H-imidazole;
1-[4-(methylsulfonyl)phenyl]-2-(3,5-dimethyl-4-methoxy-phenyl)-4-trifluoromethyl-1H-imidazole;
1-[4-(methylsulfonyl)phenyl]-2-(3,4-dimethylphenyl)-4-trifluoromethyl-1H-imidazole;
1-[4-(methylsulfonyl)phenyl]-2-(4-aminophenyl)-4-trifluoromethyl-1H-imidazole;
4-[2-(4-methylphenyl)-4-trifluoromethyl-1H-imidazol-1-yl]benzenesulfonamide;
4-[2-(3-chlorophenyl)-4-trifluoromethyl-1H-imidazol-1-yl]benzenesulfonamide;
4-[2-(4-methyl-3-chlorophenyl)-4-trifluoromethyl-lH-imidazol-1-yl]benzenesulfonamide;
5-[1-[4-(aminosulfonyl)phenyl]-4-trifluoromethyl-lH-imidazol-2-yl]-1,3-benzodioxole;
4-[2-(4-methoxyphenyl)-4-trifluoromethyl-1H-imidazol-1-yl]benzenesulfonamide;
4-[2-(3-fluorophenyl)-4-trifluoromethyl-1H-imidazol-1-yl]benzenesulfonamide;
4-[2-(4-fluorophenyl)-4-trifluoromethyl-1H-imidazol-1-yl]benzenesulfonamide;
4-[2-(4-methoxy-3-fluorophenyl)-4-trifluoromethyl-lH-imidazol-1-yl]benzenesulfonamide;
4-[2-phenyl-4-trifluoromethyl-1H-imidazol-1-yl]benzenesulfonamide;
4-[2-(4-methoxy-3-chlorophenyl)-4-trifluoromethyl-lH-imidazol-1-yl]benzenesulfonamide;
4-[2-[4-(trifluoromethyl)phenyl]-4-trifluoromethyl-lH-imidazol-1-yl]benzenesulfonamide;
4-[2-(4-bromophenyl)-4-trifluoromethyl-1H-imidazol-1-yl]benzenesulfonamide;
4-[2-(2-chlorophenyl)-4-trifluoromethyl-1H-imidazol-1-yl]benzenesulfonamide;
4-[2-(4-ethylphenyl)-4-trifluoromethyl-1H-imidazol-1-yl]benzenesulfonamide;
4-[2-(4-butylphenyl)-4-trifluoromethyl-1H-imidazol-1-yl]benzenesulfonamide;
4-[2-[4-(difluoromethyl)phenyl]-4-trifluoromethyl-1H-imidazol-1-yl]benzenesulfonamide;
4-[2-(4-butoxyphenyl) -4-trifluoromethyl-1H-imidazol-1-yl] benzenesulfonamide;
4-[2-[4-(methylthio)phenyl] -4-trifluoromethyl-1H-imidazol-1-yl]benzenesulfonamide;
4-[2-(4-methoxy-3,5-dichloro-phenyl)-4-trifluoromethyl-1H-imidazol-1-yl]benzenesulfonamide;
4-[2-(4-methyl-3,5-difluorophenyl)-4-trifluoromethyl-1H-imidazol-1-yl]benzenesulfonamide;
4-[2-(2,4-dichlorophenyl)-4-trifluoromethyl-1H-imidazol-1-yl]benzenesulfonamide;
4-[2- (3,4-dichlorophenyl) -4-trifluoromethyl-1H-imidazol-1-yl]benzenesulfonamide;
4-[2-[4-(trifluoromethoxy)phenyl]-4-trifluoromethyl-1H-imidazol-1-yl]benzenesulfonamide;
4-[2-(3,5-dimethyl-4-methoxy-phenyl)-4-trifluoromethyl-1H-imidazol-1-yl]benzenesulfonamide;
4-[2-(3,4-dimethylphenyl)-4-trifluoromethyl-1H-imidazol-1-yl]benzenesulfonamide; and
4-(2-(4-aminophenyl)-4-trifluoromethyl-1H-imidazol-1-yl]benzenesulfonamide.

A family of specific compounds of particular interest within Formula III consists of compounds and pharmaceutically-acceptable salts thereof as follows:
1-(4-chlorophenyl)-4-trifluoromethyl-2-[4-(methylsulfonyl)phenyl]-1H-imidazole;
1-(4-chlorophenyl)-4-difluoromethyl-2-[4-(methylsulfonyl)phenyl]-1H-imidazole;
1-(4-chlorophenyl)-4-methyl-2-[4-(methylsulfonyl)phenyl]-1H-imidazole;
1-(4-chlorophenyl)-4-ethyl-2-[4-(methylsulfonyl)phenyl]-1H-imidazole;
1-[4-chlorophenyl)-4-phenyl-2-[4-(methylsulfonyl)phenyl]-1H-imidazole;
1-(4-chlorophenyl)-4-(4-fluorophenyl)-2-[4-(methylsulfonyl)phenyl]-1H-imidazole;
1-(4-chlorophenyl)-4-(4-bromophenyl)-2-[4-(methylsulfonyl)phenyl]-1H-imidazole;
1-(4-chlorophenyl)-4-(4-chlorophenyl)-2-[4-(methylsulfonyl)phenyl]-1H-imidazole;
1-(4-chlorophenyl)-4-(2-naphthyl)-2-[4-(methylsulfonyl)phenyl]-1H-imidazole;
1-(4-chlorophenyl)-4-[4-(trifluoromethoxy)phenyl]-2-[4-(methylsulfonyl)phenyl]-1H-imidazole;
1-(4-chlorophenyl)-4-(3-chlorophenyl)-2-[4-(methylsulfonyl)phenyl]-1H-imidazole;
1-(4-chlorophenyl)-4-(3-fluorophenyl)-2-[4-(methylsulfonyl)phenyl]-1H-imidazole;
1-(4-chlorophenyl)-4-(4-methoxyphenyl)-2-[4-(methylsulfonyl)phenyl]-1H-imidazole;
1-(4-chlorophenyl)-4-phenoxymethyl-2-[4-(methylsulfonyl)phenyl]-1H-imidazole;
1-(4-chlorophenyl)-4-(4-chlorophenoxy)methyl-2-[4-(methylsulfonyl)phenyl]-1H-imidazole;
1-(4-chlorophenyl)-4-(4-fluorophenoxy)methyl-2-[4-(methylsulfonyl)phenyl]-1H-imidazole;
1-(4-chlorophenyl)-4-phenylthiomethyl-2- [4-(methylsulfonyl)phenyl]-1H-imidazole;
1-(4-chlorophenyl)-4-(N-phenyl-N-methylamino)methyl-2-[4-(methylsulfonyl)phenyl]-1H-imidazole;
1-(4-chlorophenyl)-4-(2-quinolyl)methoxymethyl-2-[4-(methylsulfonyl)phenyl]-1H-imidazole;
1-(4-chlorophenyl)-4-methoxymethyl-2-[4-(methylsulfonyl)phenyl]-1H-imidazole;
1-(4-chlorophenyl)-4-(4-methoxybenzyloxy)methyl-2-[4-(methylsulfonyl)phenyl]-1H-imidazole;
1-(4-chlorophenyl)-4-hydroxymethyl-2-[4-(methylsulfonyl)phenyl]-1H-imidazole;
1-(4-chlorophenyl)-4-formyl-2-[4-(methylsulfonyl)phenyl]-1H-imidazole;
1-(4-chlorophenyl)-4-cyano-2-[4-(methylsulfonyl)phenyl]-1H-imidazole;
1-(4-chlorophenyl)-4-benzyl-2-[4-(methylsulfonyl)phenyl]-1H-imidazole;
1-(4-chlorophenyl)-4-phenylethyl-2-[4-(methylsulfonyl)phenyl]-1H-imidazole;
1-(4-chlorophenyl)-4-hexyl-2-[4-(methylsulfonyl)phenyl]-1H-imidazole;
1-(4-chlorophenyl)-4-hexylcarbonyl-2-[4-(methylsulfonyl)phenyl]-1H-imidazole;
1-(4-chlorophenyl)-4-phenylcarbonyl-2-[4-(methylsulfonyl)phenyl]-1H-imidazole;
1-(4-chlorophenyl)-4-benzylcarbonyl-2-[4-(methylsulfonyl)phenyl]-1H-imidazole;
1-(4-chlorophenyl)-4-(1-hydroxy-2-phenyl-methyl)-2-[4-(methylsulfonyl)phenyl]-1H-imidazole;
1-(4-chlorophenyl)-4-(1-hexanol)-2-[4-(methylsulfonyl)phenyl]-1H-imidazole;
1-(4-chlorophenyl)-2-[(methylsulfonyl)phenyl]-1H-imidazole;
1-(4-chlorophenyl)-4-octyl-2-[4-(methylsulfonyl)phenyl]-1H-imidazole;
1-(4-chlorophenyl)-4-methoxy-2-[4-(methylsulfonyl)phenyl]-1H-imidazole;
1-(4-chlorophenyl)-4-butoxy-2-[4-(methylsulfonyl)phenyl]-1H-imidazole;
1-(4-chlorophenyl)-4-methylthio-2-[4-(methylsulfonyl)phenyl]-1H-imidazole;
1-(4-chlorophenyl)-4-(2-thienyl)-2-[4-(methylsulfonyl)phenyl]-1H-imidazole;
1-(4-chlorophenyl)-4-(3-furyl)-2-[4-(methylsulfonyl)phenyl]-1H-imidazole;
1-(4-chlorophenyl)-4-(4-pyridyl)-2-[4-(methylsulfonyl)phenyl]-1H-imidazole;
1-(4-chlorophenyl)-4-chloro-2-[4-(methylsulfonyl)phenyl]-1H-imidazole;
1-(4-chlorophenyl) -4-fluoro-2-[4-(methylsulfonyl)phenyl]-1H-imidazole;
[1-(4-chlorophenyl)-2-[4-(methylsulfonyl)phenyl)-1H-imidazol-4-yl]carboxylic acid;
methyl[1-(4-chlorophenyl)-2-[4-(methylsulfonyl)phenyl)-1H-imidazol-4-yl]carboxylate;
[1-(4-chlorophenyl)-2-[4-(methylsulfonyl)phenyl)-1H-imidazol-4-yl]carboxamide;
methyl[1-(4-chlorophenyl)-2-[4-(methylsulfonyl)phenyl)-1H-imidazol-4-yl]carboxamide;
4-[1-(4-chlorophenyl)-4-trifluoromethyl-1H-imidazol-2-yl]benzenesulfonamide;
4-[1-(4-chlorophenyl)-4-difluoromethyl-1H-imidazol-2-yl]benzenesulfonamide;
4-[1-(4-chlorophenyl)-4-methyl-1H-imidazol-2-yl]benzenesulfonamide;
4-[1-(4-chlorophenyl)-4-ethyl-1H-imidazol-2-yl]benzenesulfonamide;
4-[1-(4-chlorophenyl)-4-phenyl-1H-imidazol-2-yl]benzenesulfonamide;
4-[1-(4-chlorophenyl)-4-(4-fluorophenyl)-1H-imidazol-2-yl]benzenesulfonamide;
4-[1-(4-chlorophenyl)-4-(4-bromophenyl)-1H-imidazol-2-yl]benzenesulfonamide;
4-[1-(4-chlorophenyl)-4-(4-chlorophenyl)-1H-imidazol-2-yl]benzenesulfonamide;
4-[1-(4-chlorophenyl)-4-(2-naphthyl)-1H-imidazol-2-yl]benzenesulfonamide;
4-[1-(4-chlorophenyl)-4-[4-(trifluoromethoxy)phenyl]-1H-imidazol-2-yl]benzenesulfonamide;
4-[1-(4-chlorophenyl)-4-(3-chlorophenyl)-1H-imidazol-2-yl]benzenesulfonamide;
4-[1-(4-chlorophenyl)-4-(3-fluorophenyl)-1H-imidazol-2-yl]benzenesulfonamide;
4-[1-(4-chlorophenyl)-4-(4-methoxyphehyl)-1H-imidazol-2-yl]benzenesulfonamide;
4-[1-(4-chlorophenyl)-4-phenoxymethyl-1H-imidazol-2-yl]benzenesulfonamide;
4-[1-(4-chlorophenyl)-4-(4-chlorophenoxy)methyl-1H-imidazol-2-yl]benzenesulfonamide;
4-[1-(4-chlorophenyl)-4-(4-fluorophenoxy)methyl-1H-imidazol-2-yl]benzenesulfonamide;
4-[1-(4-chlorophenyl)-4-phenylthiomethyl-1H-imidazol-2-yl] benzenesulfonamide;
4-[1-(4-chlorophenyl)-4-(N-phenyl-N-methylamino)methyl-1H-imidazol-2-yl]benzenesulfonamide;
4-[1-(4-chlorophenyl)-4-(2-quinolyl)methoxymethyl-1H-imidazol-2-yl]benzenesulfonamide;
4-[1-(4-chlorophenyl)-4-methoxymethyl-1H-imidazol-2-yl]benzenesulfonamide;
4-[1-(4-chlorophenyl)-4-(4-methoxybenzyloxy)methyl-1H-imidazol-2-yl]benzenesulfonamide;
4-[1-(4-chlorophenyl)-4-hydroxymethyl-1H-imidazol-2-yl]benzenesulfonamide;
4-[1-(4-chlorophenyl)-4-formyl-1H-imidazol-2-yl]benzenesulfonamide;
4-[1-(4-chlorophenyl)-4-cyano-1H-imidazol-2-yl]benzenesulfonamide;
4-[1-(4-chlorophenyl)-4-benzyl-1H-imidazol-2-yl]benzenesulfonamide;
4-[1-(4-chlorophenyl)-4-phenylethyl-1H-imidazol-2-yl]benzenesulfonamide;
4-[1-(4-chlorophenyl)-4-hexyl-1H-imidazol-2-yl]benzenesulfonamide;
4-[1-(4-chlorophenyl)-4-hexylcarbonyl-1H-imidazol-2-yl]benzenesulfonamide;
4-[1-(4-chlorophenyl)-4-phenylcarbonyl-1H-imidazol-2-yl]benzenesulfonamide;
4-[1-(4-chlorophenyl)-4-benzylcarbonyl-1H-imidazol-2-yl]benzenesulfonamide;
4-[1-(4-chlorophenyl)-4-(1-hydroxy-2-phenyl-methyl)-1H-imidazol-2-yl]benzenesulfonamide;
4-[1-(4-chlorophenyl)-4-(1-hexanol)-1H-imidazol-2-yl]benzenesulfonamide;
4-[1-(4-chlorophenyl)-1H-imidazol-2-yl]benzenesulfonamide;
4-[1-(4-chlorophenyl)-4-octyl-1H-imidazol-2-yl]benzenesulfonamide;
4-[1-(4-chlorophenyl) -4-methoxy-1H-imidazol-2-yl]benzenesulfonamide;
4-[1-(4-chlorophenyl)-4-butoxy-1H-imidazol-2-yl] benzenesulfonamide;
4-[1-(4-chlorophenyl)-4-methylthio-1H-imidazol-2-yl]benzenesulfonamide;
4-[1-(4-chlorophenyl)-4-(3-thienyl)-1H-imidazol-2-yl]benzenesulfonamide;
4-[1-(4-chlorophenyl)-4-(2-furyl)-1H-imidazol-2-yl]benzenesulfonamide;
4-[1-(4-chlorophenyl)-4-(3-pyridyl)-1H-imidazol-2-yl]benzenesulfonamide;
4-[1-(4-chlorophenyl)-4-chloro-1H-imidazol-2-yl]benzenesulfonamide;
4-[1-(4-chlorophenyl)-4-fluoro-1H-imidazol-2-yl]benzenesulfonamide;
[1-(4-chlorophenyl)-2-[4-(aminosulfonyl)phenyl)-1H-imidazol-4-yl]carboxylic acid;
methyl [1-(4-chlorophenyl)-2-[4-(aminosulfonyl)phenyl)-1H-imidazol-4-yl]carboxylate;
[1-(4-chlorophenyl)-2-[4-(aminosulfonyl)phenyl)-1H-imidazol-4-yl]carboxamide;
methyl[1-(4-chlorophenyl)-2-[4-(aminosulfonyl)phenyl)-1H-imidazol-4-yl]carboxamide;
2-[4-(methylsulfonyl)phenyl]-1-(4-methylphenyl)-4-trifluoromethyl-1H-imidazole;
2- [4-(methylsulfonyl)phenyl]-1-(3-chlorophenyl)-4-trifluoromethyl-1H-imidazole;
2-[4-(methylsulfonyl)phenyl]-2-(4-methyl-3-chlorophenyl)-4-trifluoromethyl-1H-imidazole;
5-[2-[4-(methylsulfonyl)phenyl]-4-trifluoromethyl-1H-imidazol-1-yl]-1,3-benzodioxole;
2-[4-(methylsulfonyl)phenyl]-2-(4-methoxyphenyl)-4-trifluoromethyl-1H-imidazole;
2-[4-(methylsulfonyl)phenyl]-1-(3-fluorophenyl)-4-trifluoromethyl-1H-imidazole;
2-[4-(methylsulfonyl)phenyl]-1-(4-fluorophenyl)-4-trifluoromethyl-1H-imidazole;
2-[4-(methylsulfonyl)phenyl]-1-(4-methoxy-3-fluorophenyl)-4-trifluoromethyl-1H-imidazole;
2-[4-(methylsulfonyl)phenyl]-1-phenyl-4-trifluoromethyl-1H-imidazole;
2-[4-(methylsulfonyl)phenyl]-1-(4-methoxy-3-chlorophenyl)-4-trifluoromethyl-1H-imidazole;
2-[4-(methylsulfonyl)phenyl]-1-[4-(trifluoromethyl)phenyl]-4-trifluoromethyl-lH-imidazole;
2-[4-(methylsulfonyl)phenyl]-1-(4-bromophenyl)-4-trifluoromethyl-1H-imidazole;
2-[4-(methylsulfonyl)phenyl]-1-(2-chlorophenyl)-4-trifluoromethyl-1H-imidazole;
2-[4-(methylsulfonyl)phenyl]-1-(4-ethylphenyl)-4-trifluoromethyl-1H-imidazole;
2-[4-(methylsulfonyl)phenyl]-1-(4-butylphenyl)-4-trifluoromethyl-1H-imidazole;
2-[4-(methylsulfonyl)phenyl]-1-[4-(difluoromethyl)phenyl]-4-trifluoromethyl-1H-imidazole;
2-[4-(methylsulfonyl)phenyl]-1-(4-butoxyphenyl)-4-trifluoromethyl-1H-imidazole;
2-[4-(methylsulfonyl)phenyl]-1-[4-(methylthio)phenyl]-4-trifluoromethyl-1H-imidazole;
2-[4-(methylsulfonyl)phenyl]-1-(4-methoxy-3,5-dichloro-phenyl)-4-trifluoromethyl-1H-imidazole;
2-[4-(methylsulfonyl)phenyl]-1-(4-methyl-3,5-difluorophenyl)-4-trifluoromethyl-1H-imidazole;
2-[4-(methylsulfonyl)phenyl]-1-(2,4-dichlorophenyl)-4-trifluoromethyl-1H-imidazole;
2-[4-(methylsulfonyl)phenyl]-1-(3,4-dichlorophenyl)-4-trifluoromethyl-1H-imidazole;
2-[4-(methylsulfonyl)phenyl] -1-[4-(trifluoromethyl)phenyl]-4-trifluoromethoxy-1H-imidazole;
2-[4-(methylsulfonyl)phenyl]-1-(3,5-dimethyl-4-methoxy-phenyl)-4-trifluoromethyl-1H-imidazole;
2-[4-(methylsulfonyl)phenyl]-1-(3,4-dimethylphenyl)-4-trifluoromethyl-1H-imidazole;
2-[4-(methylsulfonyl)phenyl]-1-(4-aminophenyl)-4-trifluoromethyl-1H-imidazole;
4-[1-(4-methylphenyl)-4-trifluoromethyl-1H-imidazol-2-yl]benzenesulfonamide;
4-[1-(3-chlorophenyl)-4-trifluoromethyl-1H-imidazol-2-yl]benzenesulfonamide;
4-[1-(4-methyl-3-chlorophenyl)-4-trifluoromethyl-lH-imidazol-2-yl]benzenesulfonamide;
5-[2-[4-(aminosulfonyl)phenyl]-4-trifluoromethyl-1H-imidazol-1-yl]-1,3-benzodioxole;
4-[1-(3-fluorophenyl)-4-trifluoromethyl-1H-imidazol-2-yl]benzenesulfonamide;
4-[1-(4-fluorophenyl)-4-trifluoromethyl-1H-imidazol-2-yl]benzenesulfonamide;
4-[1-(4-methoxy-3-fluorophenyl)-4-trifluoromethyl-lH-imidazol-2-yl]benzenesulfonamide;
4-[1-phenyl-4-trifluoromethyl-1H-imidazol-2-yl]benzenesulfonamide;
4-[1-(4-methoxy-3-chlorophenyl)-4-trifluoromethyl-1H-imidazol-2-yl]benzenesulfonamide;
4-[1-[4-(trifluoromethyl)phenyl]-4-trifluoromethyl-lH-imidazol-2-yl]benzenesulfonamide;
4-[1-(4-bromophenyl)-4-trifluoromethyl-1H-imidazol-2-yl]benzenesulfonamide;
4-[1-(2-chlorophenyl)-4-trifluoromethyl-1H-imidazol-2-yl]benzenesulfonamide;
4-[1-(4-ethylphenyl)-4-trifluoromethyl-1H-imidazol-2-yl]benzenesulfonamide;
4-[1-(4-butylphenyl)-4-trifluoromethyl-1H-imidazol-2-yl]benzenesulfonamide;
4-[1-[4-(difluoromethyl)phenyl]-4-trifluoromethyl-1H-imidazol-2-yl]benzenesulfonamide;
4-[1-(4-butoxyphenyl)-4-trifluoromethyl-1H-imidazol-2-yl]benzenesulfonamide;
4-[1-[4-(methylthio)phenyl]-4-trifluoromethyl-lH-imidazol-2-yl]benzenesulfonamide;
4-[1-(4-methoxy-3,5-dichloro-phenyl)-4-trifluoromethyl-1H-imidazol-2-yl]benzenesulfonamide;
4-[1-(4-methyl-3,5-difluorophenyl)-4-trifluoromethyl-1H-imidazol-2-yl]benzenesulfonamide;
4-[1-(2,4-dichlorophenyl)-4-trifluoromethyl-1H-imidazol-2-yl]benzenesulfonamide;
4-[1-(3,4-dichlorophenyl)-4-trifluoromethyl-1H-imidazol-2-yl]benzenesulfonamide;
4-[1-(4-(trifluoromethoxy)phenyl]-4-trifluoromethyl-lH-imidazol-2-yl]benzenesulfonamide;
4-[1-(3,5-dimethyl-4-methoxy-phenyl)-4-trifluoromethyl-1H-imidazol-2-yl]benzenesulfonamide;
4-[1-(3,4-dimethylphenyl)-4-trifluoromethyl-1H-imidazol-2-yl]benzenesulfonamide; and
4-[1-(4-aminophenyl)-4-trifluoromethyl-1H-imidazol-2-yl]benzenesulfonamide.

Within Formula I there is a third subclass of compounds of high interest represented by Formula IV: 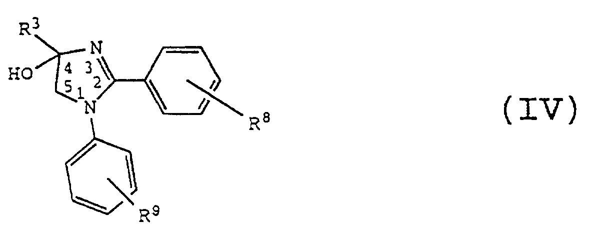 wherein R³ is selected from C₁-C₆-haloalkyl;
wherein R⁸ and R⁹ are independently selected from hydrido, halo, C₁-C₆-alkyl, C₁-C₆-haloalkyl, C₁-C₆-alkoxy, C₁-C₆-alkylsulfonyl, C₁-C₆-haloalkylsulfonyl and sulfamyl; or a pharmaceutically-acceptable salt thereof.

A preferred class of compounds consists of those compounds of Formula IV wherein R³ is selected from from fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl; and wherein R⁸ and R⁹ are independently selected from hydrido, fluoro, chloro, bromo, iodo, methyl, ethyl, isopropyl, *tert*-butyl, isobutyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, ethoxy, isopropoxy, *tert*-butoxy, propoxy, butoxy, isobutoxy, pentoxy, methylenedioxy, methylsulfonyl, fluoromethylsulfonyl and sulfamyl; or a pharmaceutically-acceptable salt thereof.

A family of specific compounds of particular interest within Formula IV consists of compounds and pharmaceutically-acceptable salts thereof as follows:
2-(4-chlorophenyl)-4-trifluoromethyl-1-[4-(methylsulfonyl)phenyl]-4-hydroxy-4,5-dihydro-1H-imidazole;
2-(4-chlorophenyl)-4-difluoromethyl-1-[4-(methylsulfonyl)phenyl]-4-hydroxy-4,5-dihydro-1H-imidazole;
4-[2-(4-chlorophenyl)-4-difluoromethyl-4-hydroxy-4,5-dihydro-1H-imidazol-1-yl]benzenesulfonamide;
2-[4-(methylsulfonyl)phenyl]-1-(4-methylphenyl)-4-trifluoromethyl-4-hydroxy-4,5-dihydro-1H-imidazole;
2-[4-(methylsulfonyl)phenyl]-1-(3-chlorophenyl)-4-trifluoromethyl-4-hydroxy-4,5-dihydro-1H-imidazole;
1-[4-(methylsulfonyl)phenyl]-2-(4-methyl-3-chlorophenyl)-4-trifluoromethyl-4-hydroxy-4,5-dihydro-1H-imidazole;
5-[1-[4-(methylsulfonyl)phenyl]-4-hydroxy-4-trifluoromethyl-4,5-dihydro-1H-imidazol-2-yl]-1,3-benzodioxole;
1-[4-(methylsulfonyl)phenyl]-2-(4-methoxyphenyl)-4-trifluoromethyl-4-hydroxy-4,5-dihydro-1H-imidazole;
1-[4-(methylsulfonyl)phenyl]-2-(3-fluorophenyl)-4-trifluoromethyl-4-hydroxy-4,5-dihydro-1H-imidazole;
1-[4-(methylsulfonyl)phenyl]-2-(4-fluorophenyl)-4-trifluoromethyl-4-hydroxy-4,5-dihydro-1H-imidazole;
1-[4-(methylsulfonyl)phenyl]-2-(4-methoxy-3-fluorophenyl)-4-trifluoromethyl-4-hydroxy-4,5-dihydro-1H-imidazole;
1-[4-(methylsulfonyl)phenyl]-2-phenyl-4-trifluoromethyl-4-hydroxy-4,5-dihydro-1H-imidazole;
1-[4-(methylsulfonyl)phenyl]-2-(4-methoxy-3-chlorophenyl)-4-trifluoromethyl-4-hydroxy-4,5-dihydro-1H-imidazole;
1-[4-(methylsulfonyl)phenyl]-2-[4-(trifluoromethyl)phenyl]-4-trifluoromethyl-4-hydroxy-4,5-dihydro-1H-imidazole;
1-[4-(methylsulfonyl)phenyl]-2-(4-bromophenyl)-4-trifluoromethyl-4-hydroxy-4,5-dihydro-1H-imidazole;
1-[4-(methylsulfonyl)phenyl]-2-(4-ethylphenyl)-4-trifluoromethyl-4-hydroxy-4,5-dihydro-1H-imidazole;
1-[4-(methylsulfonyl)phenyl]-2-(4-butylphenyl)-4-trifluoromethyl-4-hydroxy-4,5-dihydro-1H-imidazole;
1-[4-(methylsulfonyl)phenyl]-2-[4-(difluoromethyl)phenyl]-4-trifluoromethyl-4-hydroxy-4,5-dihydro-1H-imidazole;
1-[4-(methylsulfonyl)phenyl]-2-(4-butoxyphenyl)-4-trifluoromethyl-4-hydroxy-4,5-dihydro-1H-imidazole;
1-[4-(methylsulfonyl)phenyl]-2-[4-(methylthio)phenyl]-4-trifluoromethyl-4-hydroxy-4,5-dihydro-1H-imidazole;
1-[4-(methylsulfonyl)phenyl]-2-(4-methoxy-3,5-dichloro-phenyl)-4-trifluoromethyl-4-hydroxy-4,5-dihydro-1H-imidazole;
1-[4-(methylsulfonyl)phenyl]-2-(4-methyl-3,5-difluorophenyl)-4-trifluoromethyl-4-hydroxy-4,5-dihydro-1H-imidazole;
1-[4-(methylsulfonyl)phenyl]-2-(2,4-dichlorophenyl)-4-trifluoromethyl-4-hydroxy-4, 5-dihydro-1H-imidazole;
1-[4-(methylsulfonyl)phenyl]-2-(3,4-dichlorophenyl)-4-trifluoromethyl-4-hydroxy-4,5-dihydro-1H-imidazole;
1-[4-(methylsulfonyl)phenyl]-2-[4-(trifluoromethyl)phenyl]-4-trifluoromethoxy-4-hydroxy-4,5-dihydro-1H-imidazole;
2-(3,5-dimethyl-4-methoxy-phenyl)-1-[4-(methylsulfonyl)phenyl]-4-trifluoromethyl-4-hydroxy-4,5-dihydro-1H-imidazole;
2-(3,4-dimethylphenyl)-1-[4-(methylsulfonyl)phenyl]-4-trifluoromethyl-4-hydroxy-4,5-dihydro-1H-imidazole;
2-(4-aminophenyl)-1-[4-(methylsulfonyl)phenyl]-4-trifluoromethyl-4-hydroxy-4,5-dihydro-1H-imidazole;
4- [2-(4-methylphenyl)-4-trifluoromethyl-4-hydroxy-4,5-dihydro-1H-imidazol-1-yl]benzenesulfonamide;
4-[2-(3-chlorophenyl)-4-trifluoromethyl-4-hydroxy-4,5-dihydro-1H-imidazol-1-yl]benzenesulfonamide;
4-[2-(4-methyl-3-chlorophenyl)-4-trifluoromethyl-4-hydroxy-4,5-dihydro-1H-imidazol-1-yl]benzenesulfonamide;
5-[1-[4-(aminosulfonyl)phenyl]-4-hydroxy-4-trifluoromethyl-4,5-dihydro-1H-imidazol-2-yl]-1,3-benzodioxole;
4-[2-(4-methoxyphenyl)-4-trifluoromethyl-4-hydroxy-4,5-dihydro-1H-imidazol-1-yl]benzenesulfonamide;
4-[2-(3-fluorophenyl)-4-trifluoromethyl-4-hydroxy-4,5-dihydro-1H-imidazol-1-yl]benzenesulfonamide;
4-[2-(4-fluorophenyl)-4-trifluoromethyl-4-hydroxy-4,5-dihydro-1H-imidazol-1-yl]benzenesulfonamide;
4-[2-(4-methoxy-3-fluorophenyl)-4-trifluoromethyl-4-hydroxy-4,5-dihydro-1H-imidazol-1-yl]benzenesulfonamide;
4-[2-phenyl-4-trifluoromethyl-4-hydroxy-4,5-dihydro-1H-imidazol-1-yl]benzenesulfonamide;
4-[2-(4-methoxy-3-chlorophenyl) -4-trifluoromethyl-4-hydroxy-4,5-dihydro-1H-imidazol-1-yl]benzenesulfonamide;
4-[2-[4-(trifluoromethyl)phenyl]-4-trifluoromethyl-4-hydroxy-4,5-dihydro-1H-imidazol-1-yl]benzenesulfonamide;
4-[2-(4-bromophenyl)-4-trifluoromethyl-4-hydroxy-4,5-dihydro-1H-imidazol-1-yl]benzenesulfonamide;
4-[2-(2-chlorophenyl)-4-trifluoromethyl-4-hydroxy-4,5-dihydro-1H-imidazol-1-yl]benzenesulfonamide;
4-[2-(4-ethylphenyl)-4-trifluoromethyl-4-hydroxy-4,5-dihydro-1H-imidazol-1-yl]benzenesulfonamide;
4-[2-(4-butylphenyl)-4-trifluoromethyl-4-hydroxy-4,5-dihydro-1H-imidazol-1-yl]benzenesulfonamide;
4-[2-[4-(difluoromethyl)phenyl]-4-trifluoromethyl-4-hydroxy-4,5-dihydro-1H-imidazol-1-yl]benzenesulfonamide;
4-[2-(4-butoxyphenyl)-4-trifluoromethyl-4-hydroxy-4,5-dihydro-1H-imidazol-1-yl]benzenesulfonamide;
4-[2-[4-(methylthio)phenyl]-4-trifluoromethyl-4-hydroxy-4,5-dihydro-1H-imidazol-1-yl]benzenesulfonamide;
4-[2-(4-methoxy-3,5-dichloro-phenyl)-4-trifluoromethyl-4-hydroxy-4,5-dihydro-1H-imidazol-1-yl]benzenesulfonamide;
4-[2-(4-methyl-3,5-difluorophenyl)-4-trifluoromethyl-4-hydroxy-4,5-dihydro-1H-imidazol-1-yl]benzenesulfonamide;
4-[2-(2,4-dichlorophenyl)-4-trifluoromethyl-4-hydroxy-4,5-dihydro-1H-imidazol-1-yl]benzenesulfonamide;
4-[2-(3,4-dichlorophenyl)-4-trifluoromethyl-4-hydroxy-4,5-dihydro-1H-imidazol-1-yl]benzenesulfonamide;
4-[2-[4-(trifluoromethoxy)phenyl]-4-trifluoromethyl-4-hydroxy-4,5-dihydro-1H-imidazol-1-yl]benzenesulfonamide;
4-[2-(3,5-dimethyl-4-methoxy-phenyl)-4-trifluoromethyl-4-hydroxy-4,5-dihydro-1H-imidazol-1-yl]benzenesulfonamide;
4-[2-(3,4-dimethylphenyl)-4-trifluoromethyl-4-hydroxy-4,5-dihydro-1H-imidazol-1-yl]benzenesulfonamide;
4-[2-(4-aminophenyl)-4-trifluoromethyl-4-hydroxy-4,5-dihydro-1H-imidazol-1-yl]benzenesulfonamide;
1-(4-chlorophenyl)-4-trifluoromethyl-2-[4-(methylsulfonyl)phenyl]-4-hydroxy-4,5-dihydro-1H-imidazole;
1-(4-chlorophenyl)-4-difluoromethyl-2-[4-(methylsulfonyl)phenyl]-4-hydroxy-4,5-dihydro-lH-imidazole;
4-[1-(4-chlorophenyl)-4-trifluoromethyl-4-hydroxy-4,5-dihydro-1H-imidazol-2-yl]benzenesulfonamide;
4-[1-(4-chlorophenyl)-4-difluoromethyl-4-hydroxy-4,5-dihydro-1H-imidazol-2-yl]benzenesulfonamide;
2-[4-(methylsulfonyl)phenyl]-1-(3-chlorophenyl)-4-trifluoromethyl-4-hydroxy-4,5-dihydro-1H-imidazole;
2-[4-(methylsulfonyl)phenyl]-1-(4-methyl-3-chlorophenyl)-4-trifluoromethyl-4-hydroxy-4,5-dihydro-1H-imidazole;
5-[2- [4-(methylsulfonyl)phenyl]-4-hydroxy-4-trifluoromethyl-4,5-dihydro-1H-imidazol-1-yl]-1,3-benzodioxole;
2-[4-(methylsulfonyl)phenyl]-1-(4-methoxyphenyl)-4-trifluoromethyl-4-hydroxy-4, 5-dihydro-1H-imidazole;
2-[4-(methylsulfonyl)phenyl]-1-(3-fluorophenyl)-4-trifluoromethyl-4-hydroxy-4,5-dihydro-1H-imidazole;
2- [4- (methylsulfonyl)phenyl]-1- (4-fluorophenyl) -4-trifluoromethyl-4-hydroxy-4,5-dihydro-1H-imidazole;
2-[4-(methylsulfonyl)phenyl]-1-(4-methoxy-3-fluorophenyl)-4-trifluoromethyl-4-hydroxy-4,5-dihydro-1H-imidazole;
2-[4-(methylsulfonyl)phenyl]-1-phenyl-4-trifluoromethyl-4-hydroxy-4,5-dihydro-1H-imidazole;
2-[4-(methylsulfonyl)phenyl]-1-(4-methoxy-3-chlorophenyl) -4-trifluoromethyl-4-hydroxy-4,5-dihydro-1H-imidazole;
2-[4-[methylsulfonyl)phenyl]-1-[4-(trifluoromethyl)phenyl]-4-trifluoromethyl-4-hydroxy-4,5-dihydro-1H-imidazole;
2-[4-(methylsulfonyl]phenyl]-1-(4-bromophenyl)-4-trifluoromethyl-4-hydroxy-4,5-dihydro-1H-imidazole;
2-[4-(methylsulfonyl)phenyl]-1-(1-chlorophenyl)-4-trifluoromethyl-4-hydroxy-4,5-dihydro-1H-imidazole;
2-[4-(methylsulfonyl)phenyl]-1- (4-ethylphenyl)-4-trifluoromethyl-4-hydroxy-4,5-dihydro-1H-imidazole;
2-[4-(methylsulfonyl)phenyl]-1-(4-butylphenyl)-4-trifluoromethyl-4-hydroxy-4,5-dihydro-1H-imidazole;
2-[4-(methylsulfonyl)phenyl]-1-[4-(difluoromethyl)phenyl]-4-trifluoromethyl-4-hydroxy-4,5-dihydro-1H-imidazole;
2-[4-(methylsulfonyl)phenyl]-1-(4-butoxyphenyl)-4-trifluoromethyl-4-hydroxy-4,5-dihydro-1H-imidazole;
2-[4-(methylsulfonyl)phenyl]-1-[4-(methylthio)phenyl]-4-trifluoromethyl-4-hydroxy-4,5-dihydro-1H-imidazole;
2-[4-(methylsulfonyl)phenyl]-1-(4-methoxy-3,5-dichloro-phenyl)-4-trifluoromethyl-4-hydroxy-4,5-dihydro-1H-imidazole;
2-[4-(methylsulfonyl)phenyl]-1-(4-methyl-3,5-difluorophenyl)-4-trifluoromethyl-4-hydroxy-4,5-dihydro-1H-imidazole;
2-[4-(methylsulfonyl)phenyl]-1-(2,4-dichlorophenyl)-4-trifluoromethyl-4-hydroxy-4,5-dihydro-1H-imidazole;
2-[4-(methylsulfonyl)phenyl]-1-(3,4-dichlorophenyl)-4-trifluoromethyl-4-hydroxy-4,5-dihydro-1H-imidazole;
2-[4-(methylsulfonyl)phenyl]-1-[4-(trifluoromethyl)phenyl]-4-trifluoromethoxy-4-hydroxy-4,5-dihydro-1H-imidazole;
2-[4-(methylsulfonyl)phenyl]-1-(3,5-dimethyl-4-methoxy-phenyl)-4-trifluoromethyl-4-hydroxy-4,5-dihydro-1H-imidazole;
2-[4-(methylsulfonyl)phenyl]-1-(3,4-dimethylphenyl) -4-trifluoromethyl-4-hydroxy-4,5-dihydro-1H-imidazole;
2-[4-(methylsulfonyl)phenyl]-1-(4-aminophenyl)-4-trifluoromethyl-4-hydroxy-4,5-dihydro-1H-imidazole;
4-[1-(4-methylphenyl) -4-trifluoromethyl-4-hydroxy-4,5-dihydro-1H-imidazol-2-yl]benzenesulfonamide;
4-[1-(3-chlorophenyl)-4-trifluoromethyl-4-hydroxy-4,5-dihydro-1H-imidazol-2-yl]benzenesulfonamide;
4-[1-(4-methyl-3-chlorophenyl) -4-trifluoromethyl-4-hydroxy-4,5-dihydro-1H-imidazol-2-yl]benzenesulfonamide;
5-[2-[4-(aminosulfonyl)phenyl]-4-hydroxy-4,5-dihydro-4-trifluoromethyl-1H-imidazol-1-yl]-1,3-benzodioxole;
4- [1(3-fluorophenyl)-4-trifluoromethyl-4-hydroxy-4,5-dihydro-1H-imidazol-2-yl]benzenesulfonamide;
4-[1-(4-fluorophenyl)-4-trifluoromethyl-4-hydroxy-4,5-dihydro-1H-imidazol-2-yl]benzenesulfonamide;
4-[1-(4-methoxy-3-fluorophenyl)-4-trifluoromethyl-4-hydroxy-4,5-dihydro-1H-imidazol-2-yl]benzenesulfonamide;
4-[1-phenyl-4-trifluoromethyl-4-hydroxy-4,5-dihydro-lH-imidazol-2-yl]benzenesulfonamide;
4-[1-(4-methoxy-3-chlorophenyl)-4-trifluoromethyl-4-hydroxy-4,5-dihydro-1H-imidazol-2-yl]benzenesulfonamide;
4-[1-[4-(trifluoromethyl)phenyl]-4-trifluoromethyl-4-hydroxy-4,5-dihydro-1H-imidazol-2-yl]benzenesulfonamide;
4-[1-(4-bromophenyl)-4-trifluoromethyl-4-hydroxy-4,5-dihydro-1H-imidazol-2-yl]benzenesulfonamide;
4-[1-(1-chlorophenyl)-4-trifluoromethyl-4-hydroxy-4,5-dihydro-1H-imidazol-2-yl]benzenesulfonamide;
4-[1-(4-ethylphenyl)-4-trifluoromethyl-4-hydroxy-4,5-dihydro-1H-imidazol-2-yl]benzenesulfonamide;

The claimed invention also comprises a process of making a sulphonamide of Formula (I) 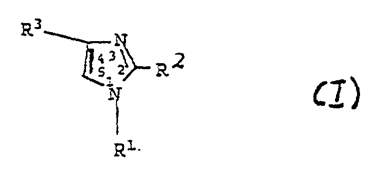 or a pharmaceutically acceptable salt thereof,
wherein R³ is a radical selected from C₁-C₆-alkyl, C₁-C₆-haloalkyl, aryl-C₁-C₆-alkyl, formyl, C₂₋₆ alkanoyl, cyano, C₁-C₆-alkylthio C₁-C₆ alkyl, C₃-C₁₀-cycloalkylthio-C₁-C₆-alkyl, C₁-C₆-hydroxyalkyl, C₁-C₆-alkoxy C₁-C₆ alkyl, arylcarbonyl, aryl-C₁-C₆-alkylcarbonyl, C₁-C₆-cyanoalkyl, C₁-C₆-carboxyalkyl, C₁-C₆-alkoxycarbonyl C₁-C₆ alkyl, C₁-C₆-alkoxycarbonyl, C₁-C₆-haloalkylcarbonyl, arylthio-C₁-C₆-alkyl, aryloxy-C₁-C₆-alkyl, aryl-C₁-C₆-alkylthio C₁-C₆ alkyl, aryl-C₁-C₆-alkoxyalkyl aryl;
wherein R¹ and R² are independently selected from aryl and heteroaryl, wherein R¹ and R² are optionally substituted at a substitutable position with one or more radicals independently selected from C₁-C₆-alkylsulfonyl, aminosulfonyl, halo, C₁-C₆-alkylthio, C₁-C₆-alkyl, cyano, carboxyl, C₁-C₆-alkoxycarbonyl, C₁-C₆-haloalkyl, hydroxyl, C₁-C₆-alkoxy, C₁-C₆-hydroxyalkyl, C₁-C₆-alkoxy C₁-C₆ alkyl, C₁-C₆-haloalkoxy, amino, C₁-C₆-alkylamino, arylamino and nitro; provided one of R¹ and R² is 4-aminosulfonylphenyl; wherein aryl whenever occurring means a phenyl or naphthyl ring;
wherein heteroaryl whenever occurring means an a heterocyclic radical selected from the possibilities as indicated in claim 1
said process comprising the steps of
treating a compound of Formula I wherein one of R¹ and R² is 4-methylsulfonylphenyl with a base and an substituted trialkylsilane in an appropriate solvent to form a silylalkylsulfone,
treating said silylalkylsulfone with an alkylammonium halide to form a sulfinic acid salt, and
forming said sulfonamide by treating the sulfinic acid salt with an aminating agent.

A family of specific compounds of particular interest within Formula I consists of compounds and pharmaceutically-acceptable salts thereof as follows:
1-methyl-3-[1-[4-(methylsulfonyl)phenyl]-4-trifluoromethyl-1H-imidazol-2-yl]-1H-indole;
4- [2-(1-methylindol-3-yl)-4-(trifluoromethyl)-1H-imidazol-1-yl]benzenesulfonamide;
4-[2-(isoquinol-2-yl)-4-(trifluoromethyl)-1H-imidazol-1-yl]benzenesulfonamide;
4-[2-(2-methyloxazol-4-yl)-4-(trifluoromethyl)-1H-imidazol-1-yl]benzenesulfonamide;
4-[2-(2-methylthiazol-4-yl)-4-(trifluoromethyl)-1H-imidazol-1-yl]benzenesulfonamide;
4-[2- (oxazol-4-yl)-4-(trifluoromethyl)-1H-imidazol-1-yl]benzenesulfonamide;
4-[2-(1-methylpyrazol-3-yl)-4-(trifluoromethyl)-1H-imidazol-1-yl]benzenesulfonamide;
4-[2-(5-methylisoxazol-3-yl)-4- (trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
2-[1-[4-(methylsulfonyl)phenyl]-4-(trifluoromethyl)-1H-imidazol-2-yl]thiophene;
3-[1-[4-(methylsulfonyl)phenyl]-4-(trifluoromethyl)-1H-imidazol-2-yl]thiophene;
4-[2-(5-methylpyridin-3-yl)-4-(trifluoromethyl)-1H-imidazol-1-yl]benzenesulfonamide;
2-methyl-3-[1-[4-(methylsulfonyl)phenyl]-4-(trifluoromethyl)-1H-imidazol-2-yl]pyridine;
4-[2-(2-methylpyridin-3-yl)-4-(trifluoromethyl)-1H-imidazol-1-yl]benzenesulfonamide;
4-[2-(pyridin-3-yl)-4-(trifluoromethyl)-1H-imidazol-1-yl]benzenesulfonamide;
3-fluoro-5-[1-[4-(methylsulfonyl)phenyl]-4-(trifluoromethyl)-1H-imidazol-2-yl]pyridine;
3-chloro-5-[1-[4-(methylsulfonyl)phenyl]-4-(trifluoromethyl)-1H-imidazol-2-yl]pyridine;
4-[2-(5-fluoropyridin-3-yl)-4-(trifluoromethyl)-1H-imidazol-1-yl]benzenesulfonamide;
4-[2-(5-chloropyridin-3-yl)-4-(trifluoromethyl)-1H-imidazol-1-yl]benzenesulfonamide;
5-methyl-2-[1-[4-(methylsulfonyl)phenyl]-4-(trifluoromethyl)-1H-imidazol-2-yl]pyridine;
4-methyl-2-[1-[4-(methylsulfonyl)phenyl]-4-(trifluoromethyl)-1H-imidazol-2-yl]pyridine;
2-methoxy-6-[1-[4-(methylsulfonyl)phenyl]-4-(trifluoromethyl)-1H-imidazol-2-yl]pyridine;
5-methoxy-2-[1-[4-(methylsulfonyl)phenyl] -4-(trifluoromethyl)-1H-imidazol-2-yl]pyridine;
4-methoxy-2-[1-[4-(methylsulfonyl)phenyl]-4-(trifluoromethyl)-1H-imidazol-2-yl]pyridine;
2-chloro-6-[1-[4-(methylsulfonyl)phenyl]-4-(trifluoromethyl)-1H-imidazol-2-yl]pyridine;
5-chloro-2-[1-[4-(methylsulfonyl)phenyl]-4-(trifluoromethyl)-1H-imidazol-2-yl]pyridine;
4-chloro-2-[1-[4- (methylsulfonyl)phenyl]-4-(trifluoromethyl)-1H-imidazol-2-yl]pyridine;
2-fluoro-6-[1-[4-(methylsulfonyl)phenyl] -4-(trifluoromethyl)-1H-imidazol-2-yl]pyridine ;
4-fluoro-2-[1-[4-(methylsulfonyl)phenyl]-4-(trifluoromethyl)-1H-imidazol-2-yl]pyridine;
4-fluoro-2-[1-[4-(methylsulfonyl)phenyl]-4-(trifluoromethyl)-1H-imidazol-2-yl]pyridine;
4-[2- (5-methylpyridin-2-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(6-methoxypyridin-2-yl)-4-(trifluoromethyl)-1H-imidazol-1-yl]benzenesulfonamide;
4-[2-(5-methoxypyridin-2-yl)-4-(trifluoromethyl)-1H-imidazol-1-yl]benzenesulfonamide;
4-[2-(4-methoxypyridin-2-yl)-4-(trifluoromethyl)-1H-imidazol-1-yl]benzenesulfonamide;
4-[2-(6-chloropyridin-2-yl)-4-(trifluoromethyl)-1H-imidazol-1-yl]benzenesulfonamide;
4-[2-(5-chloropyridin-2-yl)-4-(trifluoromethyl)-1H-imidazol-1-yl]benzenesulfonamide;
4-[2-(4-chloropyridin-2-yl)-4-(trifluoromethyl)-1H-imidazol-1-yl]benzenesulfonamide;
4-[2-(6-fluoropyridin-2-yl)-4-(trifluoromethyl)-1H-imidazol-1-yl]benzenesulfonamide;
4-[2-(5-fluoropyridin-2-yl)-4-(trifluoromethyl)-1H-imidazol-1-yl]benzenesulfonamide;
4-[2-(4-fluoropyridin-2-yl)-4-(trifluoromethyl)-1H-imidazol-1-yl]benzenesulfonamide;
3-methoxy-5-[1-[4-(methylsulfonyl)phenyl]-4-(trifluoromethyl)-1H-imidazol-2-yl]pyridine;
3-[4-methyl-1-[4-(methylsulfonyl)phenyl]-4-(trifluoromethyl)-1H-imidazol-2-yl]pyridine;
3-[1-[4-(methylsulfonyl)phenyl]-4-(trifluoromethyl)-1H-imidazol-2-yl]pyridine 1-oxide;
3-[4-[4-(fluorophenyl)-1-(4-(methylsulfonyl)phenyl]-1H-imidazol-2-yl]pyridine;
5-[1-[4-(methylsulfonyl)phenyl]-4-(trifluoromethyl)-1H-imidazol-2-yl]-2-(methylthio)pyridine;
3-[4-(difluoromethyl)-1-[4-(methylsulfonyl)phenyl]-1H-imidazol-2-yl]pyridine;
4-[2-(5-methoxypyridin-3-yl)-4-(trifluoromethyl)-1H-imidazol-1-yl]benzenesulfonamide;
4-[4-methyl-2-(3-pyridinyl)-1H-imidazol-1-yl]benzenesulfonamide;
4-[2-(3-pyridinyl)-4-(trifluoromethyl)-1H-imidazol-1-yl]benzenesulfonamide 1-oxide;
4-[4-(4-fluorophenyl)-2-(3-pyridinyl)-1H-imidazol-1-yl]benzenesulfonamide;
4-[2-(3-pyridinyl)-4-(trifluoromethyl)-1H-imidazol-1-yl]benzenesulfonamide 1-oxide;
4-[4-(4-fluorophenyl)-2-(3-pyridinyl)-1H-imidazol-1-yl]benzenesulfonamide;
4-[2-[6-(methylthio)pyridin-3-yl]-4-trifluoromethyl)-1H-imidazol-1-yl]benzenesulfonamide;
4-[4-(difluoromethyl)-2-(3-pyridinyl)-1H-imidazol-1-yl]benzenesulfonamide;
3-[1-[4-(methylsulfonyl)phenyl]-4-(trifluoromethyl)-lH-imidazol-2-yl]pyridine;
2-[1-[4-(methylsulfonyl)phenyl-4-(trifluoromethyl)-1H-imidazol-2-yl]pyridine;
2-methyl-4-[1-[4-(methylsulfonyl)phenyl-4-(trifluoromethyl)-1H-imidazol-2-yl]pyridine;
4-[1-[4-(methylsulfonyl)phenyl-4-(trifluoromethyl)-1H-imidazol-2-yl]pyridine;
2-methyl-6-[1-[4-(methylsulfonyl)phenyl-4-(trifluoromethyl)-1H-imidazol-2-yl]pyridine;
4-[2-(6-methylpyridin-3-yl)-4-(trifluoromethyl)-1H-imidazol-1-yl]benzenesulfonamide;
1-methyl-3-[1-[4-(methylsulfonyl)phenyl]-4-(trifluoromethyl)-1H-imidazol-2-yl]pyridinium iodide;
2-methyl-5-[1-[4-(methylsulfonyl)phenyl]-4-(trifluoromethyl)-1H-imidazol-2-yl]pyridine 1-oxide;
3-methyl-5-{1-[4-(methylsulfonyl)phenyl]-4-(trifluoromethyl)-1H-imidazol-2-yl]pyridine;
2-methoxy-5-[1-[4-(methylsulfonyl)phenyl]-4-(trifluoromethyl)-1H-imidazol-2-yl]pyridine;
2-[4-(4-fluorophenyl-1-[4-(methylsulfonyl)phenyl]-4-(trifluoromethyl)-1H-imidazol-2-yl]pyridine;
3-methyl-5-[1-[4-(methylsulfonyl)phenyl]-4-(trifluoromethyl)-1H-imidazol-2-yl]pyridine 1-oxide;
3-methoxy-5-[1-[4-(methylsulfonyl)phenyl]-4-(trifluoromethyl)-1H-imidazol-2-yl]pyridine;
4-[2-(3-methoxypyridin-5-yl)-4-(trifluoromethyl)-1H-imidazol-1-yl]benzenesulfonamide;
2-[1-[4-(methylsulfonyl)phenyl]-4-(trifluoromethyl)-1H-imidazol-2-yl]quinoline;
2-[1-[4-(methylsulfonyl)phenyl]-4-(trifluoromethyl)-1H-imidazol-2-yl]pyrazine;
2-methyl-4-[1-[4-(methylsulfonyl)phenyl]-4-(trifluoromethyl)-1H-imidazol-2-yl]thiazole; and
4-[2-(5-methylpyridin-2-yl)-4-(trifluoromethyl)-1H-imidazol-1-yl]benzenesulfonamide.

Compounds of Formula I, especially where R² is pyridyl, may form N-oxides, which may be active forms or prodrugs which would be converted to compounds of Formula V *in vivo*.

Compounds of Formula I would also be capable of inhibiting cytokines, such as TNF, IL-1, IL-6, and IL-8. As such, the compounds can be used in the manufacture of a medicament or in a method for the treatment for the prophylactic or therapeutic treatment of diseases mediated by cytokines, such as TNF, IL-1, IL-6, and IL-8.

The term "hydrido" denotes a single hydrogen atom (H). This hydrido radical may be attached, for example, to an oxygen atom to form a hydroxyl radical or two hydrido radicals may be attached to a carbon atom to form a methylene (-CH2-) radical. Where used, either alone or within other terms such as "haloalkyl", "alkylsulfonyl", "alkoxyalkyl" and "hydroxyalkyl", the term "alkyl" embraces linear or branched radicals having one to six carbon atoms. Examples of such radicals include methyl, ethyl, n-propyl, isopropyl, n-butyl, isobutyl, sec-butyl, *tert*-butyl, pentyl, isoamyl, hexyl. The term "halo" means halogens such as fluorine, chlorine, bromine or iodine. The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either an iodo, bromo, chloro or fluoro atom within the radical. Dihalo and polyhaloalkyl radicals may have two or more of the same halo atoms or a combination of different halo radicals. Examples of haloalkyl radicals include fluoromethyl, difluoromethyl, trifluoromethyl, chloromethyl, dichloromethyl, trichloromethyl, trichloromethyl, pentafluoroethyl, heptafluoropropyl, difluorochloromethyl, dichlorofluoromethyl, difluoroethyl, difluoropropyl, dichloroethyl and dichloropropyl. The term "hydroxyalkyl" embraces linear or branched alkyl radicals having one to six carbon atoms and one or more hydroxyl radicals. Examples of such radicals include hydroxymethyl, hydroxyethyl, hydroxypropyl, hydroxybutyl and hydroxyhexyl. The terms "alkoxy" and "alkoxyalkyl" embrace linear or branched oxy-containing radicals each having alkyl portions of one to six carbon atoms. Examples of such radicals include methoxy, ethoxy, propoxy, butoxy and tert-butoxy. The term "alkoxyalkyl" also embraces alkyl radicals having two or more alkoxy radicals attached to the alkyl radical, that is, to form monoalkoxyalkyl and dialkoxyalkyl radicals radicals having one to six carbon atoms and one or two alkoxy radicals. Examples of such radicals include methoxymethyl, methoxyethyl, ethoxyethyl, methoxybutyl and methoxypropyl. Haloalkoxy radicals have one to six carbon atoms and one or more halo radicals. Examples of such radicals include fluoromethoxy, chloromethoxy, trifluoromethoxy, trifluoroethoxy, fluoroethoxy and fluoropropoxy. The term "cyanoalkyl" having a cyano or nitrile (-CN) radical attached to an alkyl radical as having one to six carbon atoms. Examples of such cyanoalkyl radicals include cyanomethyl, cyanopropyl, cyanoethyl and cyanobutyl. The term "aryl", alone or in combination, means a phenyl or naphthyl, Such aryl radicals may be substituted at a substitutable position with one or more substituents selected from halo, C₁₋₆ alkylthio, C₁₋₆ alkyl, cyano, C₁₋₆ haloalkyl, hydroxyl, C₁₋₆ alkoxy, C₁₋₆ hydroxyalkyl and halo C₁₋₆ alkoxy. The term "heteroaryl" means unsaturated heterocyclic radicals related from pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl] tetrazolyl [e.g. 1H-tetrazolyl, 2H-tetrazolyl], indolyl, isoindolyl, indolizinyl, benzimidazolyl, quinolyl, isoquinolyl, indazolyl, benzotriazolyl, tetrazolopyridazinyl [e.g., tetrazolo [1,5-b]pyridazinyl], pyranyl, 2-furyl, 3-furyl, 2-thienyl, 3-thienyl, oxazolyl, isoxazolyl, oxadiazolyl [e.g., 1,2,4-oxadiazolyl, 1,3,4-oxadiazolyl, 1,2,5-oxadiazolyl] benzoxazolyl, benzoxadiazolyl, thiazolyl, thiadiazolyl [e.g., 1,2,4- thiadiazolyl, 1,3,4-thiadiazolyl, 1,2,5-thiadiazolyl], benzothiazolyl, benzothiadiazolyl, benzofuran, benzothiophene. Said "heterocyclic radical" may be substituted at a substitutable position with one or more substituents selected from halo, C₁₋₆ alkylthio, alkylsulfinyl, C₁₋₆ alkyl, cyano, C₁₋₆ haloalkyl, hydroxyl, C₁₋₆ alkoxy, C₁₋₆ hydroxyalkyl and C₁₋₆ haloalkoxy. More preferred heteroaryl radicals include five to six membered heteroaryl radicals. The term "alkylthio" embraces radicals containing a linear or branched alkyl radical, of one to six carbon atoms. Examples of such alkylthio radicals are methylthio, ethylthio, propylthio, butylthio and hexylthio. The term "alkylthioalkyl" embraces alkylthio radicals attached to an alkyl radical having alkyl radicals of one. to six carbon atoms and an alkylthio radical as described above. Examples of such radicals include methylthiomethyl. The term "arylthio" embraces radicals containing an aryl radical, attached to a divalent sulfur atom, such as a phenylthio radical. The term "arylthioalkyl" embraces arylthio radicals attached to an alkyl radical having alkyl radicals of one to six carbon atoms and an arylthio radical as described above. Examples of such radicals include phenylthiomethyl. The term "sulfonyl", whether used alone or linked to other terms such as alkylsulfonyl, denotes respectively divalent radicals -SO₂-. "Alkylsulfonyl" embraces alkyl radicals attached to a sulfonyl radical, where alkyl has one to six carbon atoms. Examples of such alkylsulfonyl radicals include methylsulfonyl, ethylsulfonyl and propylsulfonyl. The terms "sulfamyl", "aminosulfonyl" and "sulfonamidyl" denotes NH₂O₂S-. The terms "carboxy" or "carboxyl", whether used alone or with other terms, such as "carboxyalkyl", denotes -CO₂H. The term "carbonyl", whether used alone or with other terms, such as "alkoxycarbonyl", denotes -(C=O)-. The term "alkoxycarbonyl" means a radical containing an alkoxy radical, of one to six carbon atoms as defined above, attached via an oxygen atom to a carbonyl radical. Examples of such "alkoxycarbonyl" ester radicals include methoxycarbonyl, ethoxycarbonyl, propoxycarbonyl, butoxycarbonyl and hexyloxycarbonyl. The term "aralkyl" embraces aryl-substituted alkyl radicals having aryl radicals attached to alkyl radicals having one to six carbon atoms. Examples of such radicals include benzyl, diphenylmethyl, triphenylmethyl, phenylethyl and diphenylethyl. The aryl in said aralkyl may be substituted as indicated above. The terms benzyl and phenylmethyl are interchangeable. The terms "alkylcarbonyl", "arylcarbonyl" and "aralkylcarbonyl" include radicals having alkyl, aryl and aralkyl radicals, respectively, as defined above, attached via an oxygen atom to a carbonyl radical. The alkylcarbonyl radicals have one to six carbon atoms. Examples of such radicals include methylcarbonyl and ethylcarbonyl. The aralkylcarbonyl radicals are radicals having aryl radicals attached to alkyl radicals having one to six carbon atoms. Examples of such aralkylcarbonyl radicals include benzylcarbonyl. An example of an arylcarbonyl radical is phenylcarbonyl. The term "alkoxycarbonylalkyl" embraces radicals having "alkoxycarbonyl", as defined above substituted to an alkyl radical having one to six carbon atoms. Examples of such lower alkoxycarbonylalkyl radicals include methoxycarbonylmethyl. The term "haloalkylcarbonyl" embraces radicals having a C₁₋₆ haloalkyl radical attached to a carbonyl radical. The term "carboxyalkyl" embraces radicals having a carboxy radical as defined above, attached to an alkyl radical. The alkanoyl radicals include formyl, acetyl, propanoyl, butanoyl, isobutanoyl, valeryl, isovaleryl, pivaloyl, hexanoyl. The term "aryloxy" embraces aryl radicals, as defined above, attached to an oxygen atom. Examples of such radicals include phenoxy. The term "aralkoxy" embraces oxy-containing aralkyl radicals attached through an oxygen atom to other radicals. The term "ar C₁₋₆ alkoxy C₁₋₆ alkyl" embraces alkyl radicals having an aralkoxy radical attached to the alkyl radical, E.g. benzyloxymethyl. The term "cycloalkylthio" embraces radicals containing a cycloalkyl radical, of three to ten carbon atoms attached to a divalent sulfur atom. More preferred cycloalkylthio radicals are radicals having cycloalkyl radicals of four to six carbon atoms. Examples are cyclobutylthio, cyclopentylthio and cyclohexylthio. The term "cycloalkylthio C₁₋₆ alkyl" embraces radicals containing a cycloalkylthio radical, as described above, attached to C₁₋₆ alkyl radical. The term "mono or di C₁₋₆ "alkylamino" denotes amino groups which have been substituted with one C₁₋₆ alkyl radical. E.g. as N-methylamino, N-ethylamino, N,N-dimethylamino, N,N-diethylamino. The term "arylamino" denotes amino groups which have been substituted with an aryl radical, such as N-phenylamino. The "arylamino" radicals may be further substituted on the aryl ring portion of the radical.

The present invention comprises a pharmaceutical composition comprising a therapeutically-effective amount of a compound of Formula I in association with at least one pharmaceutically-acceptable carrier, adjuvant or diluent.

The present invention also comprises a method of treating inflammation or inflammation-associated disorders in a subject, the method comprising administering to the subject having or susceptible to such inflammation or disorder a therapeutically-effective amount of a compound of Formula I.

Also included in the family of compounds of Formula I are the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, *p*-hydroxybenzoic, phenylacetic, mandelic, embonic (pamoic), methanesulfonic, ethylsulfonic, benzenesulfonic, pantothenic, toluenesulfonic, 2-hydroxyethanesulfonic, sulfanilic, stearic, cyclohexylaminosulfonic, algenic, β-hydroxybutyric, salicylic, galactaric and galacturonic acid. Suitable pharmaceutically-acceptable base addition salts of compounds of Formula I include metallic salts made from aluminum, calcium, lithium, magnesium, potassium, sodium and zinc or organic salts made from N,N'-dibenzylethylenediamine, choline, chloroprocaine, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

### GENERAL SYNTHETIC PROCEDURES

The compounds of the invention can be synthesized according to the following procedures of Schemes I-XIV, wherein the R¹-R⁵ substituents are as defined for Formula I-IV, above, except where further noted.

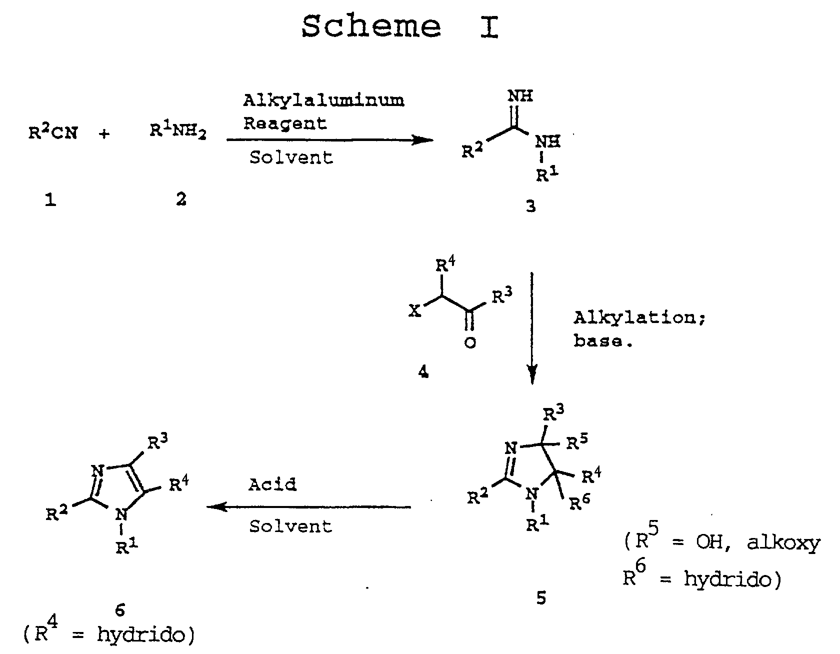

Scheme I shows the three step preparation of the 4,5-dihydro imidazoles **5** and substituted imidazoles **6** of the present invention. In step 1, the reaction of substituted nitriles (R²CN) **1** with primary amines (R¹NH₂) **2** in the presence of alkylaluminum reagents such as trimethylaluminum, triethylaluminum, dimethylaluminum chloride, diethylaluminum chloride in the presence of inert solvents such as toluene, benzene, and xylene, gives amidines **3**. In step 2, the reaction of amidine 3 with 2-halo-ketones **4** (where X is Br or Cl) in the presence of bases, such as sodium bicarbonate, potassium carbonate, sodium carbonate, potassium bicarbonate or hindered tertiary amines such as *N,N'*-diisopropylethylamine, gives the 4,5-dihydroimidazoles **5** (where R⁵ is hydroxyl and R⁶ is hydrido). Some of the suitable solvents for this reaction are isopropanol, acetone and dimethylformamide. The reaction may be carried out at temperatures of 20°C to 90°C. In step 3, the 4,5-dihydroimidazoles **5** may be dehydrated in the presence of an acid catalyst such as 4-toluenesulfonic acid or mineral acids to form the 1,2-disubstituted imidazoles **6** of the invention. Suitable solvents for this dehydration step are e.g., toluene, xylene and benzene. Trifluoroacetic acid can be used as solvent and catalyst for this dehydration step.

In some cases (e.g., where R³ = methyl or phenyl) the intermediate **5** may not be readily isolable. The reaction, under the conditions described above, proceeds to give the targeted imidazoles directly.

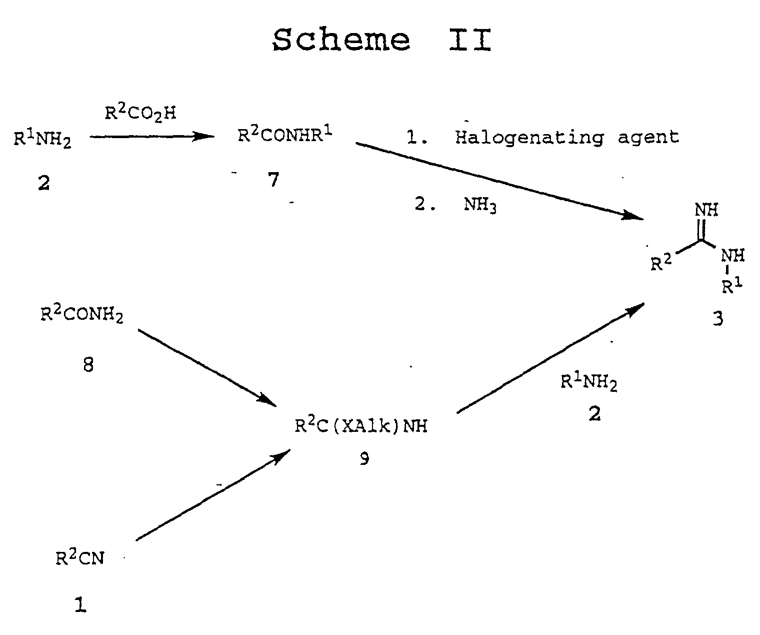

Scheme II shows alternative methods of forming amidines **3**. Amidines **3** are also available by the two step conversion of amide **7** (R²CONHR¹ formed by the conversion of primary amine **2**). In Step 1; the amide **7** is converted to the corresponding imidoyl chloride by treatment with a halogenating agent such as phosphorus oxychloride. In step two, treatment of the imidoyl chloride with ammonia forms the desired amidine **3**. In addition, amidines **3** may also be obtained by conversion of primary amides **8** (e. g., R²CONH₂) or nitriles **1** (R²CN) to their corresponding iminothioethers or iminoethers, (**9** where X is sulfur and oxygen, respectively) followed by reaction with amine **2** R¹NH₂.

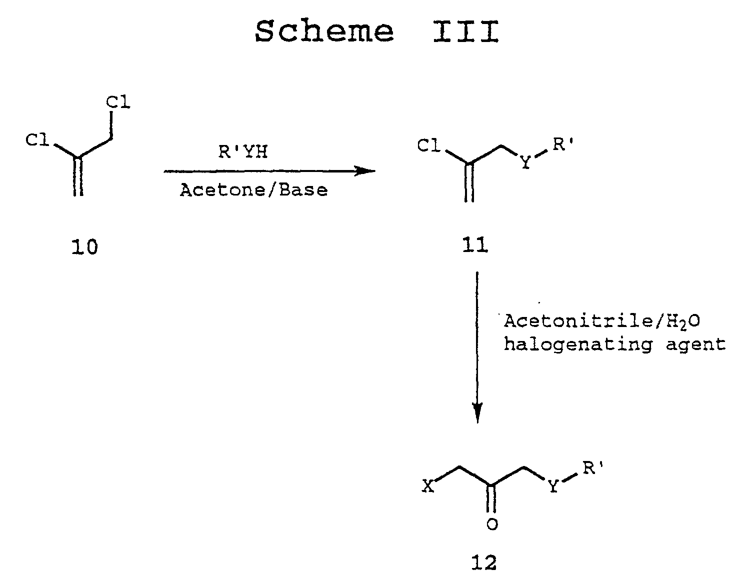

Scheme III shows the two step method of preparing certain 2-halo-ketones **12** (compound **4** from Scheme I where X is bromo or chloro, R³ is -CH₂YR' [Y is oxygen, sulfur or -NH] and R⁴ is hydrido) which are not commercially available, from 1,2-dihalo-propenes **10**. In step 1, 2,3-dichloro-1-propene **10** is added to a mixture of alcohol, amine or mercaptan (R'YH) and base, such as potassium carbonate in acetone, to form the 2-chloropropene **11**, where R' is an alkyl or aryl group and Y is an oxygen, nitrogen or sulfur atom. In step 2, the 2-chloropropene **11** is converted to 2-haloketones **12** via a method as described by H. E. Morton and M. R.Leanna (*Tet. Letters*, **34,** 4481 (1993)).

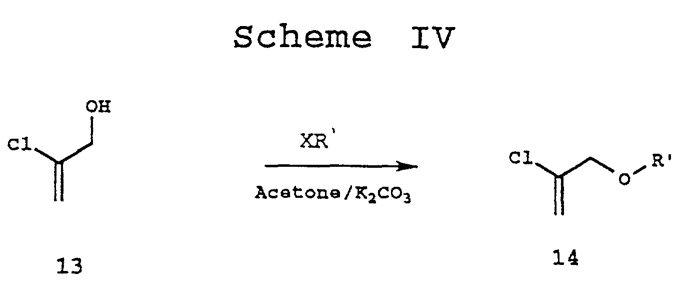

Scheme IV shows a method of forming 2-chloropropenes **14** (compound **11** in Scheme III where Y is oxygen). The 2-chloro-2-propen-1-ol **13** is added to a mixture of an alkyl, aralkyl or heteroaralkyl halide (XR') and base, such as potassium carbonate in acetone, to form the 2-chloropropene **14**.

Alternatively, 2-chloropropenes **14** can be formed from the corresponding 2,3-dichloro-1-propenes **10** (Scheme III) by reaction with a metal alkoxide in an appropriate solvent. Sodium methoxide in methanol is an example of one such alkoxide and solvent.

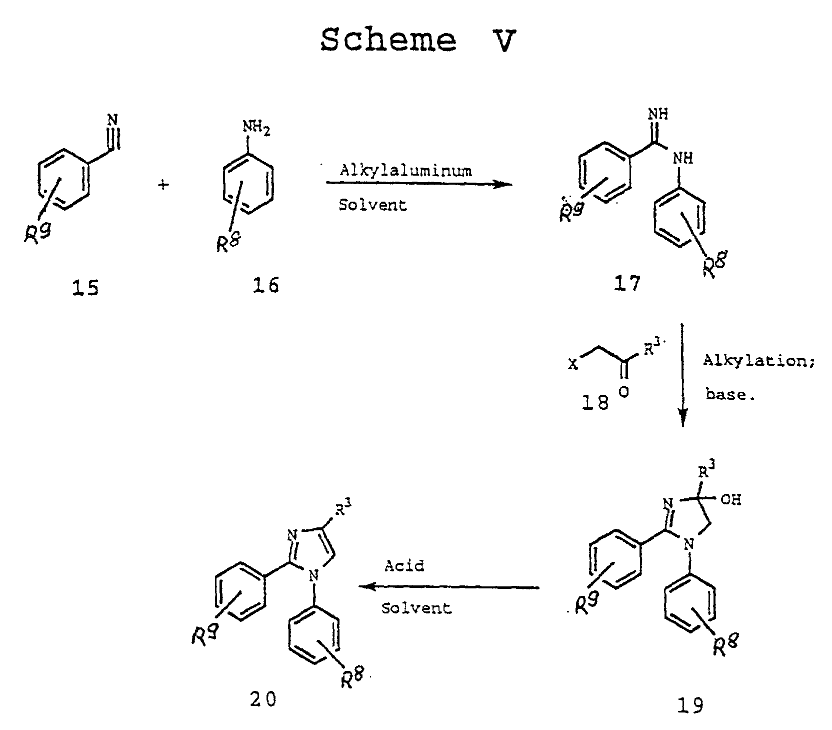

Scheme V shows the three step preparation of 1,2-diarylimidazoles **20** of the present invention. In step 1, the reaction of substituted benzonitriles **15** with substituted anilines **16** in the presence of alkylaluminum reagents such as trimethylaluminum, triethylaluminum, dimethylaluminum chloride, diethylaluminum chloride gives amidines **17**. In step 2, the reaction of amidines **17** with haloketones **18** (compound **4** in Scheme I where X is Br or Cl and R⁴ is hydrido) in the presence of bases, such as sodium bicarbonate, potassium carbonate, sodium carbonate, potassium bicarbonate or hindered tertiary amines such as *N,N*'-diisopropylethylamine, gives the 1,2-diaryl-4,5-dihydro-imidazoles **19**. Some of the suitable solvents for this reaction are isopropanol, acetone and dimethylformamide. The reaction may be carried out at a temperature between 20°C to 90°C. In step 3, the 1,2-diaryl-4,5-dihydro-imidazoles **19** may be dehydrated in the presence of an acid catalyst such as 4-toluenesulfonic acid to form the 1,2-diarylimidazoles **20** of the present invention. Suitable solvents for this dehydration step are, for example, toluene, xylene and benzene. Trifluoroacetic acid can be used as solvent and catalyst for this dehydration step.

In some cases (e.g., where R³ is methyl or phenyl), the intermediate **19** may not be readily isolable. The reaction, under the conditions described above, proceeds to give the targeted imidazoles **20** directly.

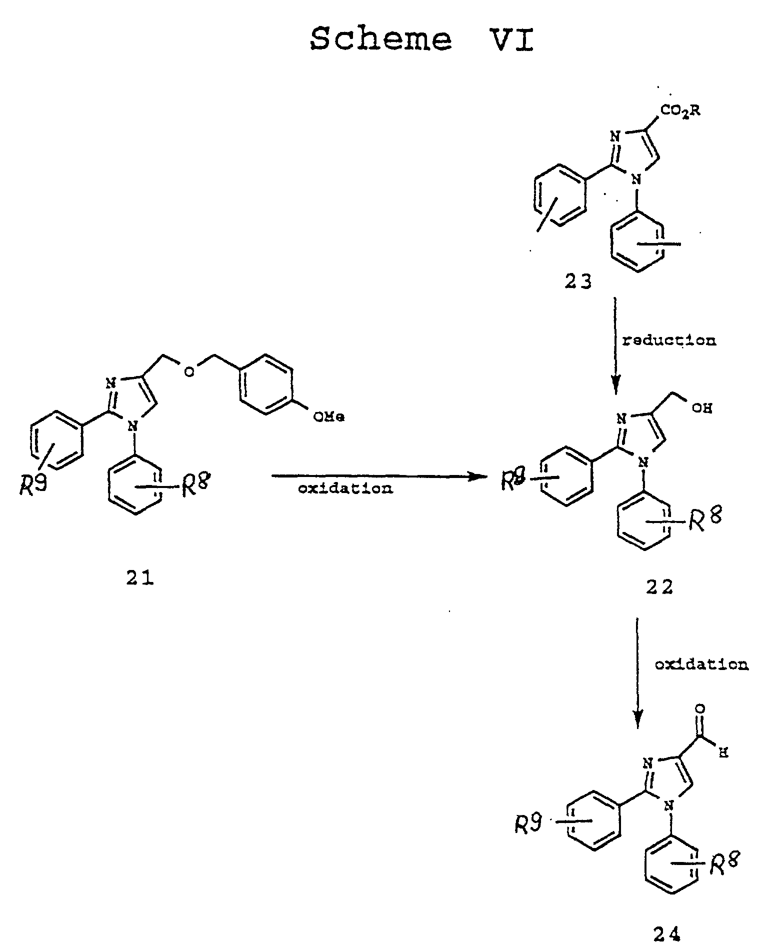

Scheme VI shows the formation of 4-hydroxymethyl imidazoles **22** and 4-formyl-imidazoles **23** from benzyloxy-protected imidazoles **21** and from 4-carboalkoxy imidazoles **23**. In step 1, the oxidative deprotection of 4-methoxybenzyl group in **21**, such as with ceric ammonium nitrate, gives the hydroxymethyl imidazoles **22**. Alternatively, the alkoxycarbonyl group of **23** may be reduced to the hydroxymethyl group. Suitable reducing agents include lithium borohydride. In step 2, the hydroxymethyl imidazoles **22** are oxidized, for example, with pyridinium chlorochromate, to give the 4-formyl-imidazoles **24**.

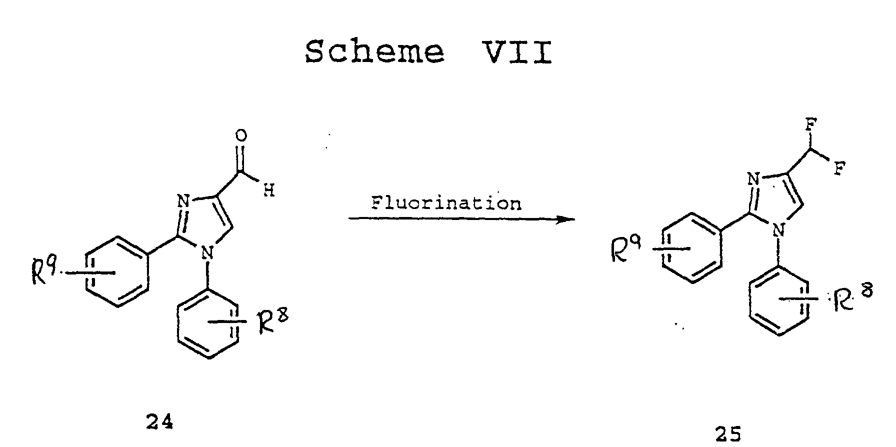

Scheme VII shows the formation of 4-difluoromethyl-imidazoles **25** from 4-formyl-imidazoles **24**. The 4-formyl-imidazoles **24** are converted to desired 4-difluoromethyl-imidazoles **25** by direct fluorination using the known reagents such as SF4 or diethylaminosulfur trifluoride (DAST). For discussion of the reaction and the representative procedures, see e.g., *Organic Reactions,* **34,** 319 (1987), *Organic Reactions,* **35,** 513 (1988), *Organic Reactions,* **21,** 319 (1974) and *Chem. Soc. Reviews,* **16**, 381 (1987), Alternatively, the imidazoles **25** can be synthesized by reaction of hydrazones of **24** with N--bromosuccinimide/pyridinium poly(hydrogen fluoride). This transformation has been developed by Olah and coworkers (see, *Synlett*, 594 (1990).

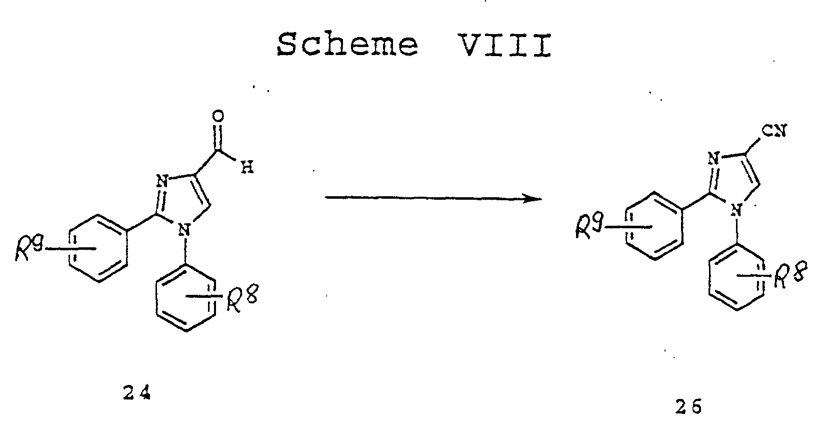

Scheme VIII shows the conversion of the 4-formyl-imidazoles **24** to 4-cyanoimidazoles **26.** The 4-formyl-imidazoles **24** are converted to the target nitrile derivatives **26** by following the literature procedures [see, e.g., Chem. Letters, 773 (1984), *Synthesis*, 510 (1984), *Tetrahedron Lett.*, 1781 (1976), *Synthesis*, 739 (1981), *Synth. Communications*, **18**, 2179 (1988), *Bull. Chem. Soc. Japan*, **54,** 1579 (1981), *Synthesis*, 201 (1985), *Synthesis,* 190 (1982), *Synthesis*, 56 (1979), and the references cited therein].

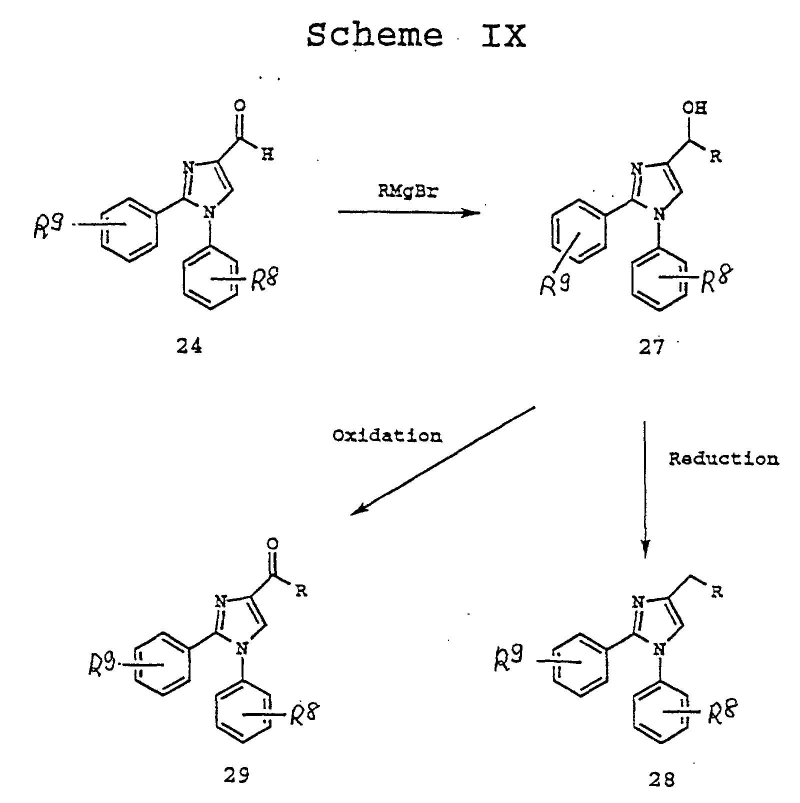

Scheme IX shows other 1,2-diarylimidazoles that can be synthesized from the 4-formyl-imidazoles **24** in two steps. In step 1, the 4-formyl-imidazoles **24** are converted to carbinol derivatives (where R is aralkyl or alkyl) by addition of Grignard reagents (RMgBr). In step 2, the hydroxy derivatives **27** are reduced by catalytic hydrogenation (using e.g., Pd/C or Pt/C), preferably in the presence of a small amount of acid (e.g., acetic acid or aqueous HCl) to form the alkyl or aralkyl derivatives **28**. Alternatively, the ketones **29** are synthesized by oxidation (e.g., using pyridinium chlorochromate) of the hydroxy derivatives **27**.

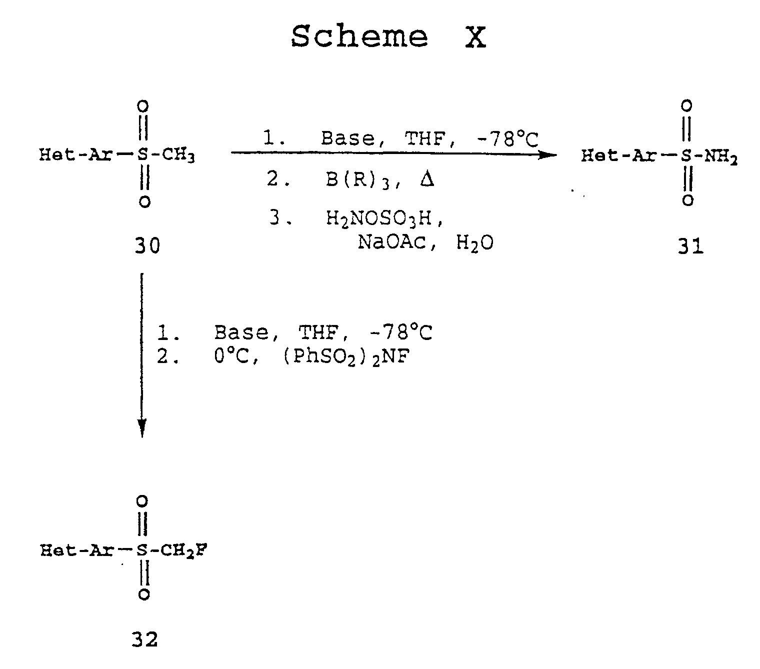

Synthetic Scheme X shows the three step procedure used to prepare sulfonamide antiinflammatory agents **31** and the two step procedure used to prepare fluoromethyl sulfone antiinflammatory agents **32** from their corresponding methyl sulfones **30**. In step one, THF solutions of the methyl sulfones **30** at -78°C are treated with a base such as alkyllithium reagents, lithioamides and Grignard reagents. Examples of such bases include n-butyllithium, methyllithium, lithium diisopropylamide (LDA), butylmagnesium chloride, phenylmagnesium bromide and methylmagnesium chloride. In step two, the anions generated in step one are treated with an organoborane, e.g., triethylborane, tributylborane, etc., at -78°C then warmed to ambient temperature prior to stirring at reflux. An alternative to the boron chemistry involves room temperature alkylation, such as with haloalkyltrialkylsilanes, followed by treatment with silylalkyl-elimination agents. Examples of such haloalkyltrialkylsilanes include trimethylsilylmethylhalides such as (iodomethyl)trimethylsilane and (chloromethyl)trimethylsilane. Suitable silylalkyl-elimination agents include compounds which produce a fluoride ion. Examples of such compounds include alkylammonium fluorides and cesium fluoride. tetrabutylammonium fluoride (1M in THF) is preferred. The deprotonation of sulfone is conveniently carried out in the temperature range of about -70°C to about 25°C, preferably at about 0°C. The formation of silylalkylsulfone is conveniently carried out in the temperature range of about 0°C to about 35°C, preferably at about 20°C. In step three, an aqueous solution of sodium acetate and hydroxylamine-O-sulfonic acid is added to provide the corresponding sulfonamide antiinflammatory agents **31** of this invention. Alternatively, the anion solutions generated in step one may be warmed to 0°C and treated with N-fluorodibenzenesulfonamide-to provide the corresponding fluoromethyl sulfone antiinflammatory agents **32** of this invention.

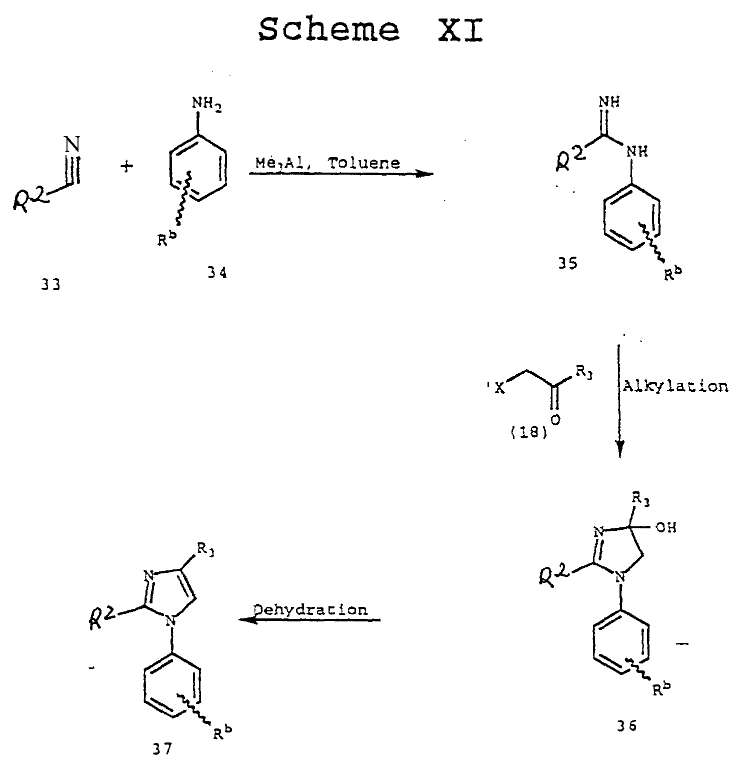

1-Phenyl-2-heterocycloimidazoles of the current invention **37** are synthesized by following the generic synthesis shown in Scheme XI. The reaction of a substituted heterocyclonitrile **33** with substituted anilines **34** (where Rb is as defined above for aryl and heteroaryl radicals) in the presence of alkylaluminum reagents such as trimethylaluminum, triethylaluminum, dimethylaluminum chloride, diethylaluminum chloride gives the amidine **35**. The reaction of amidine **35** with a 2-halo-ketone derivative **18** (X' = Br or Cl) in the presence of bases such as sodium bicarbonate, potassium carbonate, sodium carbonate, potassium bicarbonate or *N,N'*-diisopropylethylamine gives the alkylated product **36**. Some of the suitable solvents for this reaction are i-propanol, acetone and dimethylformamide. The reaction may be carried out at 20 to 90°C. The intermediate **36** may be dehydrated in the presence of an acid catalyst such as 4-toluenesulfonic acid to give the targeted 1,2-diarylimidazoles **37.** Suitable solvents for this dehydration step are e.g., toluene, xylene and benzene. Alternatively, trifluoroacetic acid may be used both as solvent and catalyst in this dehydartion step.

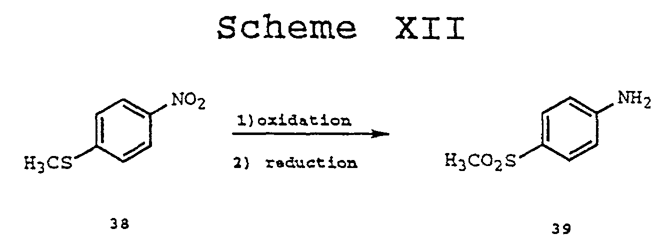

Scheme XII shows a two step method of forming sulfonyl anilines **39** from nitro compounds **38**. In step one, the 4-methylthio-nitrobenzene **38** is oxidized to the sulfone with an oxidizing reagent such as hydrogen peroxide, Oxone® or MCPBA. In step 2, the 4-methylsulfonyl-nitrobenzene is reduced to the corresponding aniline **39**.

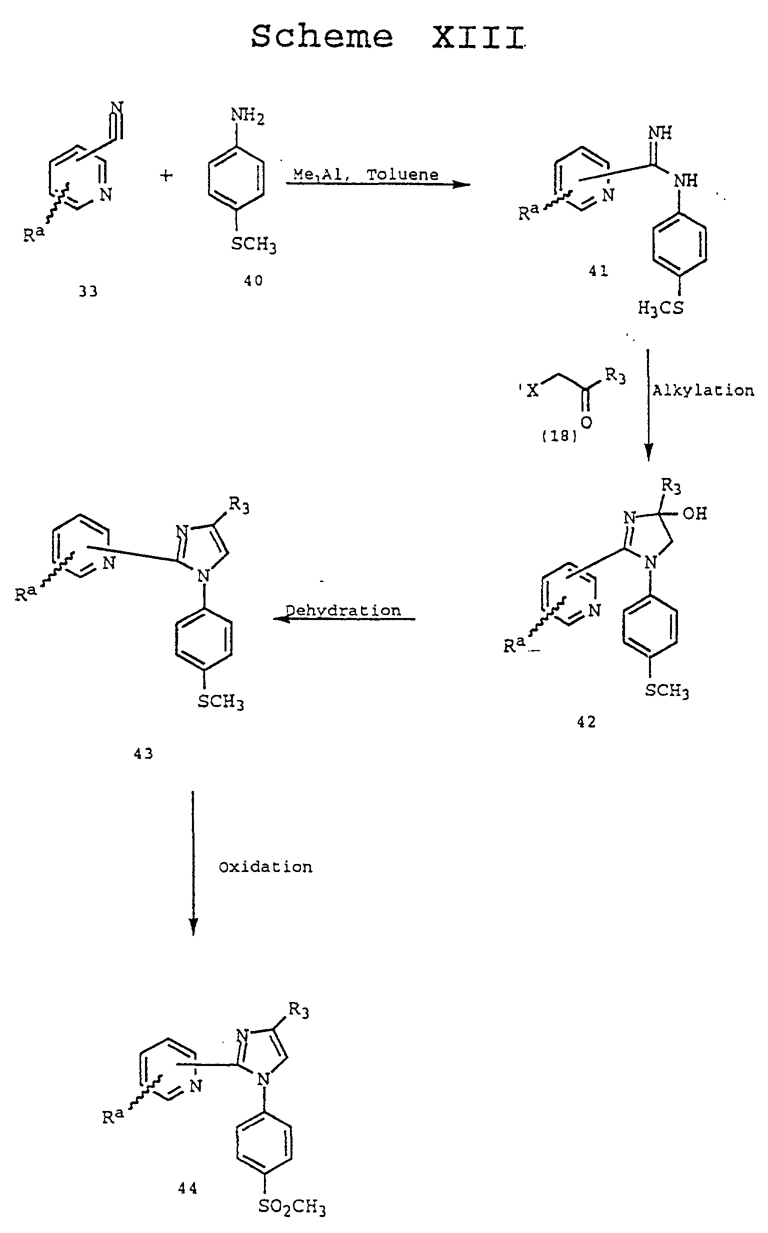

Synthetic Scheme XIII describes an alternative method of forming 1-aryl-2-pyridyl-imidazoles **44** from 4-alkylthioanilines **40**. The reaction of a substituted cyanopyridine **33** (where Rₐ is as defined above for aryl and heteroaryl radicals) with substituted anilines **40** in the presence of alkylaluminum reagents such as trimethylaluminum, triethylaluminum, dimethylaluminum chloride, diethylaluminum chloride gives the amidine **41**. Alternatively, amidine **41** may be synthesized by reaction of aniline **40** first with a suitable base, and then with nitrile **33**. Examples of suitable bases include sodium hydride, sodium methoxide, n-butyllithium and lithium diisopropylamide. These reactions may be run in solvents such as dimethyl sulfoxide, tetrahydrofuran, dimethoxyethane and methanol or the like. The reaction of amidine **41** with a 2-halo-ketone derivative **18** (X' = Br or Cl) in the presence of bases such as sodium bicarbonate, potassium carbonate, sodium carbonate, potassium bicarbonate or *N,N*'-diisopropylethylamine gives the alkylated product **42**. Some of the suitable solvents for this reaction are *i*-propanol, acetone and dimethylformamide. The reaction may be carried out at 20 to 90°C. The intermediate **42** is dehydrated in the presence of an acid catalyst such as 4-toluenesulfonic acid to give the 1-(4-alkylthio)aryl-2-pyridylimidazoles **43**. Suitable solvents for this dehydration step are e.g., toluene, xylene and benzene. Oxidation of the alkylthio **43**, with an oxidizing reagent such as hydrogen peroxide, Oxone® or MCPBA, yields the sulfones **44**.

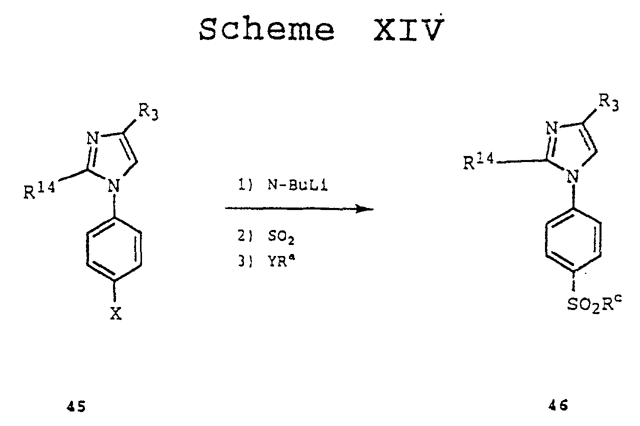

Scheme XIV shows a method of forming sulfones and sulfonamides **46** from the corresponding 1-phenylimidazoles **45**, where X is a leaving group such as halo. Treatment of **45** with base, such as butyl lithium, followed by addition of sulfur dioxide and a substituted alkyl or amine yields the corresponding sulfone or sulfonamide **46** (where R^{a} is alkyl or amino).

The following examples contain detailed descriptions of the methods of preparation of compounds of Formula I-V. All parts are by weight and temperatures are in Degrees centigrade unless otherwise indicated. All compounds showed NMR spectra consistant with their assigned structures. In some cases, the assigned structures were confirmed by nuclear Overhauser effect (NOE) experiments.

### Example 1

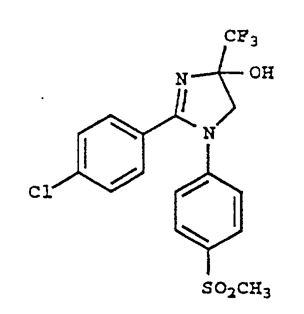

### 2-(4-Chlorophenyl)-4-hydroxy-1-[4-(methylsulfonyl)phenyl]-4-(trifluoromethyl)-4,5-dihydro-1H-imidazole

### Step 1: Preparation of 4-chloro-N-[4-(methylsulfonyl)phenyl]benzenecarboximidamide

To a suspension of 4-(methylsulfonyl)aniline (7 g, 41 mmol) in toluene (400 mL), trimethylaluminum (2M solution in toluene, 30.5 mL, 61 mmol) was added over 15 minutes. The reaction mixture was warmed to room temperature and stirred for 2.5 hours. A solution of 4-chlorobenzonitrile (11.3 g, 82 mmol) in toluene (200 mL) was added over 10 minutes and the reaction mixture was heated to 80-85°C. After 16 hours, the reaction mixture was cooled to room temperature and poured over a slurry of silica gel in chloroform. After filtration, the residue was washed with a mixture of methylene chloride/mechanol. The combined filtrates were concentrated *in vacuo* and the resulting yellowish solid was stirred with a mixture of hexane/ether (2/1, 1000 mL). The intermediate was filtered and washed with more of hexane/ether (2/1). The pale yellow solid 4-chloro-N-[4-(methylsulfonyl)phenyl] benzenecarboximidamide (10.93 g, 86%) was used in the next reaction without further purification: mp (DSC) 191°C. Anal. Calc'd. for C₁₄H₁₃N₂SO₂Cl: C, 54.46, H, 4.24, N, 9.07. Found: C, 54.42, H, 4.30, N, 9.07.

### Step 2 : Preparation of 2-(4-chlorophenyl)-4-hydroxy-1-[4-(methylsulfonyl)phenyl]-4-(trifluoromethyl)-4,5-dihydro-1H-imidazole

To a mixture of 4-chloro-N-[4-(methylsulfonyl)phenyl]benzenecarboximidamide from Step 1 (8 g, 26 mmol) and sodium bicarbonate (4.36 g, 52 mmol) in isopropanol (240 mL), 3-bromo-1,1,1-trifluoroacetone (5.4 mL, 52 mmol) was added. After heating the reaction mixture at 75-80°C for 24 hours, the solvent was removed. The residue was redissolved in methylene chloride and washed with water. The organic fractions were combined, dried over sodium sulfate, filtered and concentrated *in vacuo*. The crude product (16.2 g) was purified by chromatography (silica gel, hexane/ethyl acetate, 55/45) to give pure 2-(4-chlorophenyl)-4-hydroxy-1-[4-(methylsulfonyl)phenyl]-4-(trifluoromethyl)-4,5-dihydro-1H-imidazole (6.7 g, 62%) as a white solid: Anal. Calc'd. for C₁₇H₁₄N₂SO₃ClF₃: C, 48.75, H, 3.37, N, 6.69. Found: C, 48.56, H, 3.22, N, 6.51.

### Example 2

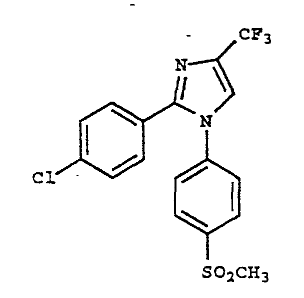

### 2-(4-Chlorophenyl)-1-[4-(methylsulfonyl)phenyl]-4-(trifluoromethyl)-1H-imidazole

A mixture of 2-(4-chlorophenyl)-4-hydroxy-1-[4-(methylsulfonyl)phenyl]-4-(trifluoromethyl)-4,5-dihydro-1H-imidazole from Example 1 (6.2 g, 15.4 mmol) and *p*-toluenesulfonic acid monohydrate (0.9 g, 4.7 mmol) in toluene (300 mL) was heated to reflux for 84 hours. The reaction mixture was cooled and the solvent removed under reduced pressure. The crude residue was redissolved in methylene chloride and washed with water, aqueous sodium bicarbonate and brine. After drying (Na₂SO₄), filtration and concentrating *in vacuo*, the crude mixture was purified by chromatography on silica gel using hexane/ethyl acetate (1/1) to give pure 2-(4-chlorophenyl)-1-[4-(methylsulfonyl)phenyl]-4-(trifluoromethyl)-1H-imidazole (4.21 g, 71%) as a white solid: mp (DSC) 183°C. Anal. Calc'd. for C₁₇H₁₂N₂SO₂F₃Cl: C, 50.94, H, 3.02, N, 6.99. Found: C, 50.64, H, 3.03, N, 6.85.

### Example 3

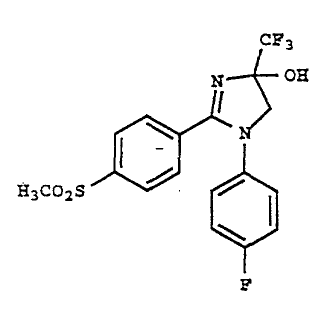

### 1-(4-Fluorophenyl)-4-hydroxy-2-[4-(methylsulfonyl)-phenyl]-4-(trifluoromethyl)-4,5-dihydro-1H-imidazole

### Step 1: Preparation of 4-methylsulfonyl-N-[4-chlorophenyl]benzenecarboximidamide

To a suspension of 4-fluoroaniline (4 mL, 40 mmol) in toluene (120 mL), trimethylaluminum (2M solution in toluene, 21 mL, 42 mmol) was added over 15 minutes. The reaction mixture was warmed to room temperature and stirred for 3 hours. A solution of 4-(methylsulfonyl)benzonitrile (7.65 g, 40 mmol) in methylene chloride (100 mL) was added over 10 minutes and the reaction mixture was heated to 70-75°C. After 48 hours, the reaction mixture was cooled to room temperature and poured over a slurry of silica gel in chloroform. After filtration, the residue was washed with a mixture of methylene chloride/methanol. The combined filtrates were concentrated *in vacuo* and the resulting crude intermediate (7.7 g) was purified by chromatography [silica gel, hexane/ethyl acetate, 25/75] to give 4-methylsulfonyl-N-[4-chlorophenyl]benzenecarboximidamide (4.1 g, 35%) as a white solid: mp (DSC) 182°C. Anal. Calc'd. for C₁₄H₁₃N₂SO₂F: C, 57.52, H, 4.48, N, 9.58, S, 10.97. Found: C, 57.37, H, 4.69, N, 9.21, S, 10.69.

### Step 2: Preparation of 1-(4-fluorophenyl)-4-hydroxy-2-[4-(methylsulfonyl)phenyl]-4-(trifluoromethyl)-4,5-dihydro-1H-imidazole

To a mixture of 4-methylsulfonyl-N-[4-chlorophenyl]benzenecarboximidamide from Step 1 (1 g, 3.42 mmol) and sodium bicarbonate (575 mg, 6.85 mmol) in isopropanol (30 mL), 3-bromo-1,1,1-trifluoroacetone (5.g, 25 mmol) was added. After heating the reaction mixture at 80-90°C for 24 hours, the solvent was removed. The residue was redissolved in methylene chloride and washed with water. The organic fractions were combined, dried over sodium sulfate, filtered and concentrated in vacuo. The crude product (2.34 g) was purified by chromatography [silica gel, hexane/ethyl acetate, 1/1] to give 1-(4-fluorophenyl)-4-hydroxy-2-[4-(methylsulfonyl)phenyl]-4-(trifluoromethyl)-4,5-dihydro-1H-imidazole (650 mg, 47%) as a white solid: mp (DSC) 209°C. Anal. Calc'd. for C₁₇H₁₄N₂SO₃F₄: C, 50.75, H, 3.51, N, 6.96. Found: C, 51.11, H, 3.86, N, 6.57.

### Example 4

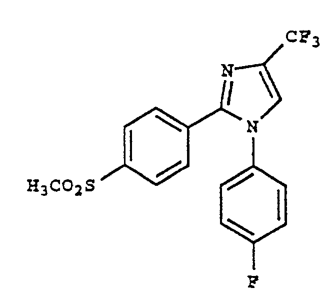

### 1-(4-Fluorophenyl)-2-[4-(methylsulfonyl)phenyl]-4-(trifluoromethyl)-1H-imidazole

A mixture of 1-(4-fluorophenyl)-4-hydroxy-2-[4-(methylsulfonyl)phenyl]-4-(trifluoromethyl)-4,5-dihydro-1H-imidazole (Example 3) (770 mg, 1.9 mmol) and *p*-toluenesulfonic acid monohydrate (88 mg) in toluene (80 mL) was heated to reflux for 20 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration *in vacuo,* the crude mixture (520 mg) was purified by chromatography on silica gel using hexane/ethyl acetate (1/1) to give pure 1-(4-fluorophenyl)-2-[4-(methylsulfonyl)phenyl]-4-(trifluoromethyl)-1H-imidazole (328 mg, 44%) as a white solid: mp (DSC) 183°C. Anal. Calc'd. for C₁₇H₁₂N₂SO₂F₄: C, 53.13, H, 3.15, N, 7.29. Found: C, 53.20, H, 3.22, N, 7.18.

### Example 5

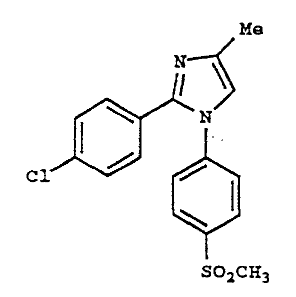

### 2-(4-Chlorophenyl)-1-[4-(methylsulfonyl)phenyl]-4-methyl-1H-imidazole

To a mixture of 4-chloro-N-[4-(methylsulfonyl)phenyl]benzenecarboximidamide (Example 1, Step 1) (240 mg, 0.78 mmol) and sodium bicarbonate (131 mg, 1.56 mmol)-in isopropanol (20 mL), excess chloroacetone (1.5 mL) was added. After heating to reflux, the reaction mixture for 72 hours, the solvent was removed. The residue was redissolved in methylene chloride and washed with aqueous sodium bicarbonate and water. The organic fractions were combined, dried over sodium sulfate, filtered and concentrated *in vacuo*. The crude product (370 mg) was purified by chromatography (silica gel, hexane/ethyl acetate, 25/75) to give pure 2-(4-chlorophenyl)-1-[4-(methylsulfonyl)phenyl]-4-methyl-1H-imidazole (160 mg, 67%): mp (DSC) 166°C. Anal Calc'd. for C₁₇H₁₅N₂SO₂Cl C, 58.87, H, 4.36, N, 8.08 Found: C, 58.78, H, 4.62, N, 7.99.

### Example 6

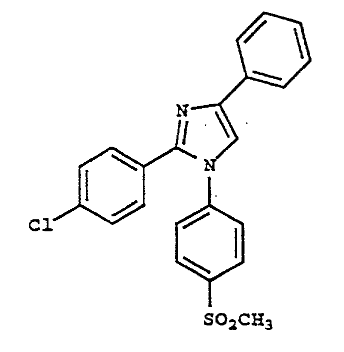

### 2-(4-Chlorophenyl)-1-[4-(methylsulfonyl)phenyl]-4-phenyl-1H-imidazole

To a mixture of 4-chloro-N-[4-(methylsulfonyl)phenyl]benzenecarboximidamide (Example 1, Step 1) (400 mg, 1.29 mmol) and sodium bicarbonate (216 mg, 2.59 mmol) in isopropanol (25 mL), 2-bromoacetophenone (780 mg, 3.87 mmol) was added. After heating the reaction mixture at 55°C for 20 hours, the solvent was removed. The residue was redissolved in methylene chloride and washed with aqueous sodium bicarbonate and water. The organic fractions were combined, dried over sodium sulfate, filtered and concentrated *in vacuo.* The crude product (1.2 g) was purified by chromatography on silica gel with toluene/ethyl acetate (75/25) to give pure 2-(4-chlorophenyl)-1-[4-(methylsulfonyl)phenyl]-4-phenyl-1H-imidazole (300 mg, 57%) as a white solid: mp (DSC) 202°C. Anal. Calc'd. for C₂₂H₁₇N₂SO₂Cl: C, 63.78, H, 4.28, N, 6.76, S, 7.74. Found: C, 63.69, H, 4.11, N, 6.68, S, 7.65.

### Example 7

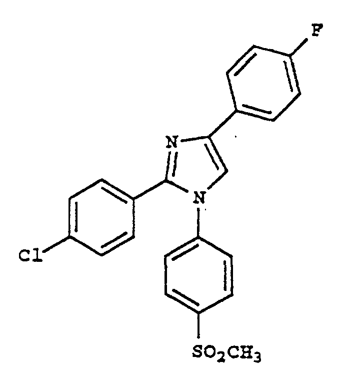

### 2-(4-Chlorophenyl)-4-(4-fluorophenyl)-1-[4-(methylsulfonyl)phenyl]-1H-imidazole

To a mixture of 4-chloro-N-[4-(methylsulfonyl)phenyl]benzenecarboximidamide (Example 1, Step 1) (400 mg, 1.29 mmol) and sodium bicarbonate (216 mg, 2.59 mmol) in isopropanol (25 mL), 2-chloro-4'-fluoroacetophenone (670 mg, 3.87 mmol) was added. After heating the reaction mixture at 80-85°C for 48 hours, the solvent was removed. The residue was redissolved in methylene chloride and washed with aqueous sodium bicarbonate and water. The organic fractions were combined, dried over sodium sulfate, filtered and concentrated *in vacuo*. The crude product (800 mg) was purified by chromatography (silica gel, hexane/ethyl acetate, 1/1) to give 2-(4-chlorophenyl)-4-(4-fluorophenyl)-1-[4-(methylsulfonyl)phenyl]-1H-imidazole (200 mg, 36%) as a pale yellow solid: mp (DSC) 180°C. Anal. Calc'd. for C₂₂H₁₆N₂SO₂FCl: C, 61.90, H, 3.78, N, 6.56, S, 7.51. Found: C, 61.92, H, 3.74, N, 6.43, S, 7.62.

### Example 8

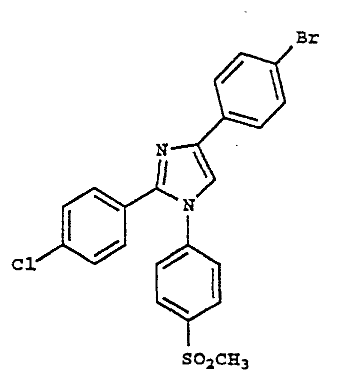

### 4-(4-Bromophenyl)-2-(4-chlorophenyl)-1-[4-(methylsulfonyl)phenyl]-1H-imidazole

To a mixture of 4-chloro-N-[4-(methylsulfonyl)phenyl]benzenecarboximidamide (Example 1, Step 1) (400 mg, 1.29 mmol) and sodium bicarbonate (216 mg, 2.59 mmol) in isopropanol (30 mL), 2,4'-dibromoacetophenone (720 mg, 2.58 mmol) was added. After heating the reaction mixture at 80-85°C for 18 hours, the solvent was removed. The residue was redissolved in methylene chloride and washed with aqueous sodium bicarbonate and water. The organic fractions were combined, dried over sodium sulfate, filtered and concentrated *in vacuo*. The crude product (810 mg) was purified by chromatography (silica gel, hexane/ethyl acetate, 6/4) to give 4-(4-bromophenyl)-2-(4-chlorophenyl)-1-[4-(methylsulfonyl)phenyl]-1H-imidazole (400 mg, 64%) as a pale yellow solid: mp 145-48°C. Anal. Calc'd. for C₂₂H₁₆N₂SO₂BrCl: C, 54.17, H, 3.31, N, 5.74, S, 6.57. Found: C, 54.41, H, 3.33, N, 5.50, S, 6.52.

### Example 9

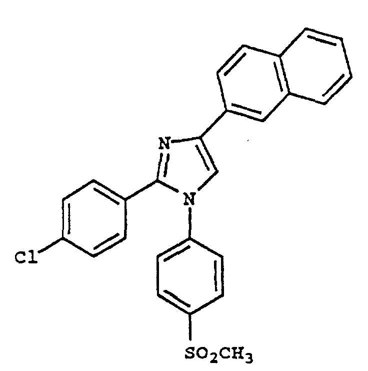

### 2-(4-Chlorophenyl)-1-[4-(methylsulfonyl)phenyl]-4-(2-naphthyl)-1H-imidazole

To a mixture of 4-chloro-N-[4-(methylsulfonyl)phenyl]benzenecarboximidamide (Example 1, Step 1) (400 mg, 1.29 mmol) and sodium bicarbonate (216 mg, 2.59 mmol) in isopropanol (30 mL), 2-bromo-2'-acetonaphthone (970 mg, 3.89 mmol) was-added. After heating the reaction mixture at 80-85°C for 20 hours, the solvent was removed . The residue was redissolved in-methylene chloride and washed with aqueous sodium bicarbonate and water. The organic fractions were combined, dried over sodium sulfate, filtered and concentrated *in vacuo*. The crude product (1.2 g) was purified by chromatography (silica gel, hexane/ethyl acetate, 6/4) to give 2-(4-chlorophenyl)-1-[4-(methylsulfonyl)phenyl]-4-(2-naphthyl)-1H-imidazole (318 mg, 54%) as a pale yellow solid: mp 204-206°C. Anal Calc'd. for C₂₆H₁₉N₂SO₂Cl: C, 68.04, H, 4.17, N, 6.10, S, 6.99. Found: C, 67.65, H, 4.19, N, 5.96, S, 7.10.

### Example 10

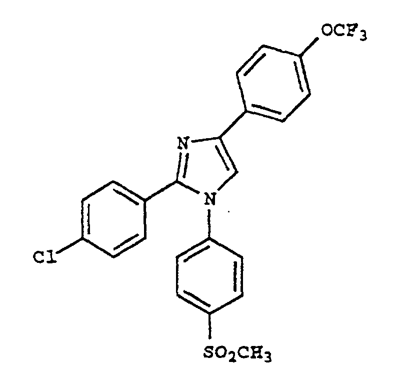

### 2-(4-Chlorophenyl)-1-[4-(methylsulfonyl)phenyl]-4-[4-(trifluoromethoxy)phenyl]-1H-imidazole

To a mixture of 4-chloro-N-[4-(methylsulfonyl)phenyl]benzenecarboximidamide (Example 1, Step 1) (700 mg, 2.24 mmol) and sodium bicarbonate (376 mg, 4.48 mmol) in isopropanol (25 mL), 4-(trifluoromethoxy)phenacyl bromide (950 mg, 3.36 mmol) was added. After heating the reaction mixture at 80-85°C for 22 hours, the solvent was removed. The residue was redissolved in methylene chloride and washed with aqueous sodium bicarbonate and water. The organic fractions were combined, dried over sodium sulfate, filtered and concentrated in vacuo. The crude product was purified by chromatography (silica gel, hexane/ethyl acetate, 6/4) to give 2-(4-chlorophenyl)-1-[4-(methylsulfonyl)phenyl]-4-[4-(trifluoromethoxy)phenyl]-1H-imidazole (467 mg, 42%) as a pale yellow solid: mp 95-97°C. Anal. Calc'd. for C₂₃H₁₆N₂SO₃F₃Cl: C, 56.05, H, 3.27, N, 5.68, S, 6.51. Found: C, 55.90, H, 3.04, N, 5.62, S, 6.74.

### Example 11

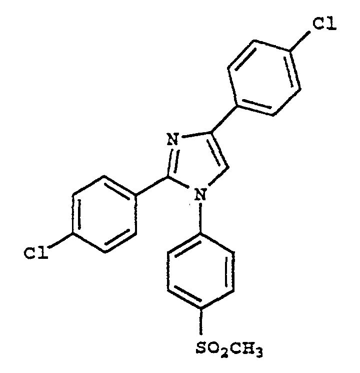

### 2,4-Bis(4-chlorophenyl)-1-[4-(methylsulfonyl)phenyl]-1H-imidazole

To a mixture of 4-chloro-N-[4-(methylsulfonyl)phenyl]benzenecarboximidamide (Example 1, Step 1) (700 mg, 2.24 mmol) and sodium bicarbonate (376 mg, 4.48 mmol) in isopropanol (30 mL), 4-chlorophenacyl bromide (1.05 g, 4.48 mmol) was added. After heating the reaction mixture at 80-85°C for 18 hours, the solvent was removed. The residue was redissolved in methylene chloride and washed with aqueous sodium bicarbonate and water. The organic fractions were combined, dried over sodium sulfate, filtered and concentrated in vacuo. The crude product was purified by chromatography (silica gel, hexane/ethyl acetate, 6/4) to give 2,4-bis-(4-chlorophenyl)-1-[4-(methylsulfonyl)phenyl]-1H-imidazole (545 mg, 55%) as a pale yellow solid: mp 169-171°C. Anal. Calc'd. for C₂₂H₁₆N₂SO₂Cl₂: C, 59.60, H, 3.64, N, 6.32, S, 7.23. Found: C, 59.86, H, 3.80, N, 6.10, S, 7.27.

### Example 12

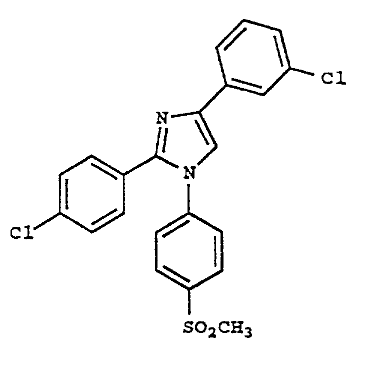

### 2-(4-Chlorophenyl)-4-(3-chlorophenyl)-1-[4-(methylsulfonyl)phenyl]-1H-imidazole

To a mixture of 4-chloro-N-[4-(methylsulfonyl)phenyl]benzenecarboximidamide (Example 1, Step 1) (700 mg, 2.24 mmol) and sodium bicarbonate (376 mg, 4.48 mmol) in isopropanol (35 mL), 3-chlorophenacyl bromide (1.05 g, 4.48 mmol) was added. After-heating the reaction mixture at 80-85°C for 18 hours, the solvent was removed. The residue was redissolved in methylene chloride and washed with aqueous sodium bicarbonate and water. The organic fractions were combined, dried over sodium sulfate, filtered and concentrated *in vacuo.* The crude product was purified by chromatography (silica gel, hexane/ethyl acetate, 6/4) to give 2-(4-chlorophenyl)-4-(3-chlorophenyl)-1-[4-(methylsulfonyl)phenyl]-1H-imidazole (525 mg, 53%) as a pale yellow solid: mp 156-159°C. Anal Calc'd. for C₂₂H₁₆N₂SO₂Cl₂: C, 59.60, H, 3.69, N, 6.32, S, 7.23. Found: C, 59.43, H, 3.59, N, 6.15, S, 7.16.

### Example 13

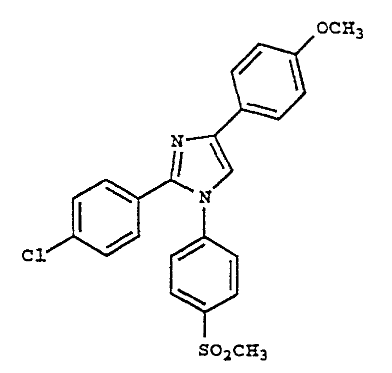

### 2- (4-Chlorophenyl)-4- (4-methoxyphenyl)-1-[4-(methylsulfonyl)phenyl]-1H-imidazole

To a mixture of 4-chloro-N-[4-(methylsulfonyl)phenyl]benzenecarboximidamide (Example 1, Step 1) (700 mg, 2.24 mmol) and sodium bicarbonate (376 mg, 4.48 mmol) in isopropanol (50 mL), 4-methoxyphenacyl bromide (1.03 g, 4.48 mmol) was added.-After heating the reaction mixture at 75-80°C for 20 hours, the solvent was removed. The residue was redissolved in methylene chloride and washed with aqueous sodium bicarbonate and water. The organic fractions were combined, dried over sodium sulfate, filtered and concentrated in *vacuo.* The crude product was purified by chromatography (silica gel, hexane/ethyl acetate, 6/4) to give 2-(4-chlorophenyl)-4-(-4-methoxyphenyl)-1-[4-(methylsulfonyl)phenyl]-4-(4-methoxyphenyl)-1H-imidazole (695 mg, 71%) as a white solid: mp 110-113°C. Anal Calc'd. for C₂₃H₁₉N₂SO₃Cl: C, 62.94, H, 4.36, N, 6.38, S, 7.30. Found: C, 62.54, H, 4.43, N, 6.17, S, 7.15.

### Example 14

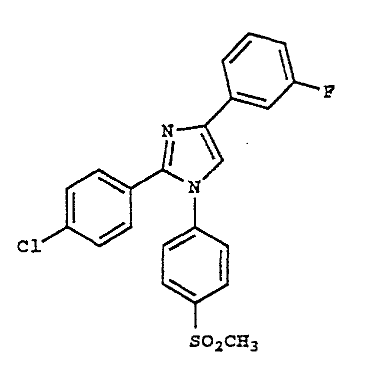

### 2- (4-Chlorophenyl)-4-(3-fluorophenyl)-1-[4-(methylsulfonyl)phenyl-1H-imidazole

To a mixture of 4-chloro-N-[4-(methylsulfonyl) phenyl]benzenecarboximidamide (Example 1, Step 1) (700 mg, 2.24 mmol) and sodium bicarbonate (376 mg, 4.48 mmol) in isopropanol (30 mL), 3-fluorophenacyl bromide (0.97 g, 4.48 mmol) was added. After heating the reaction mixture at 75-80°C for 18 hours, the solvent was removed. The residue was redissolved-in methylene chloride and washed with aqueous sodium bicarbonate and water. The organic fractions were combined, dried over sodium sulfate, filtered and concentrated *in vacuo*. The crude product was purified by chromatography (silica gel, hexane/ethyl acetate, 6/4) to give 2-(4-chlorophenyl)-4- (3-fluorophenyl)-1-[4-(methylsulfonyl)phenyl]-1H-imidazole (481 mg, 50%) as a white solid: mp 194-196°C. Anal. Calc'd. for C₂₂H₁₆N₂SO₂FCl: C, 61.90, H, 3.78, N, 6.56, S, 7.51. Found: C, 61.71, H, 3.59, N, 6.42, S, 7.69.

### Example 15

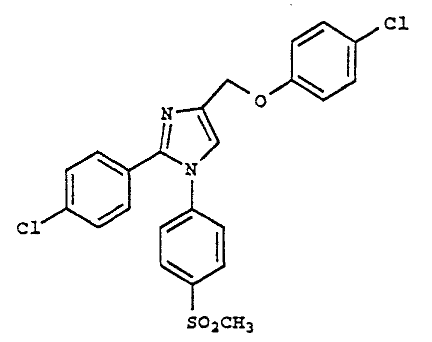

### 2-(4-Chlorophenyl)-4-[(4-chlorophenoxy)methyl]-1-[4-(methylsulfonyl)phenyl]-1H-imidazole

### Step 1: Preparation of 1-(4-chlorophenoxy)-2-chloro-2-propene

To a mixture of 4-chlorophenol (6.1 g, 47.4 mmol) and potassium carbonate (13.1 g, 94.7 mmol) in acetone (200 mL), 2,3-dichloropropene (6.6 mL, 71 mmol) was added. After heating to reflux the reaction mixture for 48 hours, the reaction mixture was cooled and filtered. The residue was washed with more acetone and the combined filtrates were concentrated *in vacuo*. The crude pale brown liquid (11.5 g) was purified by chromatography (silica gel, hexane/ethyl acetate, 85/15) to give 1-(4-chlorophenoxy)-2-chloro-2-propene (8.9 g, 98%) as a white liquid: Anal. Calc'd. for C₉H₈OCl₂: C, 53.23, H, 3.97. Found: C, 53.09, H, 3.95.

### Step 2: Preparation of 1-bromo-3-[(4-chlorophenoxy)phenyl]-2-propanone

To a turbid solution of 1-(4-chlorophenoxy)-2-chloro-2-propene from Step 1 (3 g, 15.7 mmol) in acetonitrile/water (4/1, 100 mL), *N*-bromosuccinimide (4.84 g, 31.4 mmol) was added in one lot. A catalytic amount of 48% HBr (40 µl) was added to the reaction and the yellowish orange mixture was stirred at room temperature. After 24 hours, the reaction mixture was diluted with ether and washed with 5% w/v of sodium thiosulfate. The organic layer was separated and washed with saturated sodium bicarbonate and brine. After drying (MgSO₄), filtration and concentration *in vacuo*, the crude liquid (4.8 g) was purified by chromatography (silica gel, hexane/ethyl acetate, 80/20) to give crude 1-bromo-3-[(4-chlorophenoxy)phenyl]-2-propanone (2.3 g, 54%) which was used in the next step without further purification.

### Step 3 : Preparation of 2-(4-chlorophenyl)-4-hydroxy-1-[4-(methylsulfonyl)phenyl]-4-[(4-chlorophenoxy)methyl]-4,5-dihydro-1H-imidazole

To a mixture of 4-chloro-N-[4-(methylsulfonyl)phenyl]benzenecarboximidamide (Example 1, Step 1) (1 g, 3.24 mmol) and sodium bicarbonate (550 mg, 6.5 mmol) in acetone (100 mL), 1-bromo-3-[(4-chlorophenoxy)phenyl]-2-propanone from Step 2 (1.5 g, 5.8 mmol) was added. After heating to reflux for 24 hours, the reaction mixture was filtered, washed with acetone and concentrated *in vacuo*. The crude mixture (2.5 g) was purified by chromatography (silica gel, toluene/ethyl acetate, 1/1) to give 2-(4-chlorophenyl)-4-hydroxy-1-[4-(methylsulfonyl)phenyl]-4-[(4-chlorophenoxy)methyl]-4,5-dihydro-1H-imidazole (565 mg, 35%) as a white solid.

### Step 4: Preparation of 2-(4-chlorophenyl)-1-[4-(methylsulfonyl)phenyl]-4-[(4-chlorophenoxy)methyl]-1H-imidazole

A mixture of 2-(4-chlorophenyl)-4-hydroxy-1-[4-(methylsulfonyl)phenyl]-4-[(4-chlorophenoxy)methyl]-4,5-dihydro-1H-imidazole from Step 3 (750 mg, 1.5 mmol) and p-toluenesulfonic acid monohydrate (135 mg) in toluene (100 mL) was heated to reflux for 48 hours. The reaction mixture was cooled and the solvent removed under reduced pressure. The crude residue was redissolved in methylene chloride and washed with. water, aqueous sodium bicarbonate and brine. After drying (Na₂SO₄), filtration and concentration *in vacuo*, the crude mixture was purified by chromatography on silica gel using hexane/ethyl acetate (1/1) to give 2-(4-chlorophenyl)-1-[4-(methylsulfonyl)phenyl]-4-[(4-chlorophenoxy)methyl]-1H-imidazole as a white solid: mp (DSC) 173°C. Anal. Calc'd for C₂₃H₁₈N₂Cl₂SO₃·0.25 H₂O: C, 57.81; H, 3.90; N, 5.86; Cl, 14.84. Found: C, 57.67; H, 3.83; N, 5.52; Cl, 15.17.

### Example 16

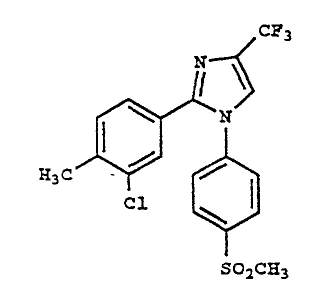

### 2-(3-Chloro-4-methylphenyl)-1-[4-(methylsulfonyl)phenyl]-4-(trifluoromethyl)-1H-imidazole

### Step 1: Preparation of 3-chloro-4-methyl-N-[4-(methylsulfonyl)phenyl]benzenecarboximidamide

To a suspension of 4-(methylsulfonyl)aniline (2.82 g, 16.5 mmol) in toluene (150 mL), trimethylaluminum (2M solution in toluene, 12.5 mL, 24.7 mmol) was added over 15 minutes. The reaction mixture was warmed to room temperature and stirred for 2.5 hours. A solution of 3-chloro-4-methylbenzonitrile (5 g, 33 mmol) in toluene (100 mL) was added over 10 minutes and the reaction mixture was heated to 90-95°C. After 20 hours, the reaction mixture was cooled to room temperature and poured over a slurry of silica gel in chloroform. After filtration, the residue was washed with a mixture of methylene chloride/methanol (2/1). The combined filtrates were concentrated *in vacuo* and the resulting yellowish solid was stirred with a mixture of hexane/ether (2/1, 700 mL). The intermediate was filtered and washed with more of hexane/ether (2/1). The pale yellow solid 3-chloro-4-methyl-N-[4-(methylsulfonyl)phenyl]benzenecarboximidamide (4.7 g, 88%) was used in the next reaction without further purification: mp (DSC) 179°C. Anal. Calc'd. for C₁₅H₁₅N₂SO₂Cl: C, 55.81, H, 4.68, N, 8.68. Found: C, 55.65, H, 4.63, N, 8.59.

### Step 2 : Preparation of 2-(3-chloro-4-methylphenyl)-4-hydroxy-1-[4-(methylsulfonyl)phenyl]-4-(trifluoromethyl)-4,5-dihydro-1H-imidazole

To a mixture of 3-chloro-4-methyl-N-[4-(methylsulfonyl)phenyl]benzenecarboximidamide from Step 1 (2.35 g, 7.3 mmol) and sodium bicarbonate (1.23 g, 14.6 mmol) in isopropanol (100 mL), 3-bromo-1,1,1-trifluoroacetone (5.4 mL, 52 mmol) was added. After heating to reflux the reaction mixture for 24 hours, the solvent was removed. The residue was redissolved in methylene chloride and washed with water. The organic fractions were combined, dried over sodium sulfate, filtered and concentrated in vacuo. The crude mixture (7.3 g) was purified by chromatography (silica gel, toluene/ethyl acetate, 1/1) to give 2-(3-chloro-4-methylphenyl)-4-hydroxy-1-[4-(methylsulfonyl)phenyl]-4-(trifluoromethyl)-4,5-dihydro-1H-imidazole (0.79 g, 25%) as a white solid: mp 201°C. Anal. Calc'd. for C₁₈H₁₆N₂SO₃F₃Cl•0.5 PhCH₃: C, 53.92, H, 4.21, N, 5.81. Found: C, 54.20, H, 4.19, N, 5.67.

### Step 3: Preparation of 2-(3-chloro-4-methylphenyl)-1-[4-(methylsulfonyl)phenyl]-4-(trifluoromethyl)-1H-imidazole

A mixture of 2-(3-chloro-4-methylphenyl)-4-hydroxy-1-[4-(methylsulfonyl)phenyl]-4-(trifluoromethyl)-4,5-dihydro-1H-imidazole from Step 2 (725 mg, 1.7 mmol) and *p*-toluenesulfonic acid monohydrate (150 mg) in toluene (40 mL) was heated to reflux for 48 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration *in vacuo*, the crude mixture (860 mg) was purified by chromatography on silica gel using toluene/ethyl acetate 1/1 to give pure 2-(3-chloro-4-methylphenyl)-1-[4-(methylsulfonyl)phenyl]-4-(trifluoromethyl)-1H-imidazole (660 mg, 95%) as a white solid: mp(DSC) 206°C. Anal. Calc'd. for C₁₈H₁₄N₂SO₂F₃Cl: C, 52.12, H, 3.40, N, 6.75, S, 7.73. Found: C, 52.24, H, 3.45, N, 6.64, S, 7.83.

### Example 17

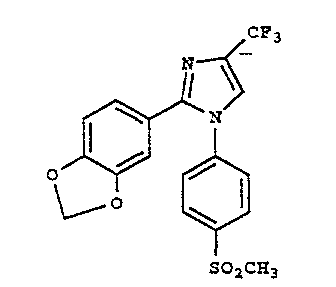

### 5-[1-[4-(Methylsulfonyl)phenyl]-4-(trifluoromethyl)-1H-imidazol-2-yl)-1,3-benzodioxole

### Step 1: Preparation of 3,4-methylenedioxy-N-[4-(methylsulfonyl)phenyl]benzencarboximidamide

To a suspension of (4-methylsulfonyl)aniline (2.82 g, 16.5 mmol) in toluene (150 mL), trimethylaluminum (2M solution in toluene, 12.5 mL, 24.7 mmol) was added over 15 minutes. The reaction mixture was warmed to room temperature and stirred for 2.5 hours. A solution of piperonylonitrile (4.85 g, 33 mmol) in toluene (100 mL) was added over 10 minutes and the reaction mixture was heated to 90-95°C. After 20 hours, the reaction mixture was cooled to room temperature and poured over a slurry of silica gel in chloroform. After filtration, the residue was washed with a mixture of methylene chloride/methanol (2/1). The combined filtrates were concentrated *in vacuo* and the resulting yellowish solid was stirred with a mixture of hexane/ether (2/1, 1000 mL). The product was filtered and washed with more of hexane/ether (2/1). The pale yellow solid 3,4-methylenedioxy-N-[4-(methylsulfonyl)phenyl] benzenecarboximidamide (4.8 g, 91%) was used in the next reaction without further purification: mp (DSC) 214°C. Anal. Calc'd. for C₁₅H₁₄N₂SO₄: C, 56.59, H, 4.43, N, 8.80. Found: C, 56.33, H, 4.28, N, 8.66.

### Step 2: Preparation of 5-[1-[4-(methylsulfonyl)phenyl]-4-hydroxy-4-(trifluoromethyl)-4,5-dihydro-1H-imidazol-2-yl]-1,3-benzodioxole

To a mixture of 3,4-methylenedioxy-N-[4-(methylsulfonyl)phenyl]benzenecarboximidamide from Step 1 (2.32 g, 7.3 mmol) and sodium bicarbonate (1.23 g, 14.6 mmol) in isopropanol (100 mL), 3-bromo-1,1,1-trifluoroacetone (5.4 mL, 52 mmol) was added. After heating the reaction mixture to reflux for 24 hours, the solvent was removed. The residue was redissolved in methylene chloride and washed with water. The organic fractions were combined, dried over sodium sulfate, filtered and concentrated *in vacuo*. The crude mixture (7.1 g) was purified by chromatography (silica gel, toluene/ethyl acetate, 1/1) to give 5-[1-[4-(methylsulfonyl)phenyl]-4-hydroxy-4-(trifluoromethyl)-4,5-dihydro-1H-imidazol-2-yl]-1,3-benzodioxole (1.46 g, 47%) as a white solid: mp 200-202°C. Anal. Calc'd. for C₁₈H₁₅N₂SO₅F₃•0.25 PhCH₃: C, 52.55, H, 3.80, N, 6.21. Found: C, 52.73, H, 3.78, N, 6.01.

### Step 3 : Preparation of 5-[1-[4-(methylsulfonyl)phenyl]-4-(trifluoromethyl)-1H-imidazol-2-yl]-1,3-benzodioxole

A mixture of 5-[1-[4-(methylsulfonyl)phenyl]-4-hydroxy-4-(trifluoromethyl)-4,5-dihydro-1H-imidazol-2-yl]-1,3-benzodioxole from Step 2 (1.26 g, 2.9 mmol) and p-toluenesulfonic acid monohydrate (200 mg) in toluene (50 mL) was heated to reflux for 72 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration *in vacuo*, the crude mixture (1.34 g) was purified by chromatography on silica gel using toluene/ethyl acetate 1/1 to give pure 5- [1-[4-(methylsulfonyl)phenyl]-4-(trifluoromethyl)-1H-imidazol-2-yl]-1,3-benzodioxole (940 mg, 80%) as a white solid: mp (DSC) 165°C. Anal Calc'd. for C₁₈H₁₃N₂SO₄F₃: C, 52.68, H, 3.19, N, 6.83. Found: C, 53.05, H, 3.19, N, 6.65.

### Example 18

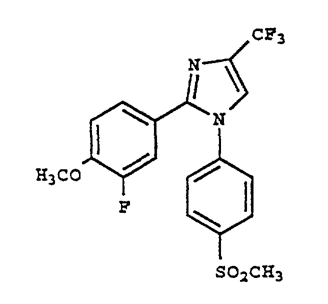

### 2-(3-Fluoro-4-methoxyphenyl)-1-[4-(methylsulfonyl)phenyl]-4-(trifluoromethyl) -1H-imidazole

### Step 1: Preparation of 3-fluoro-4-methoxy-N-[4-(methylsulfonyl)phenyl]benzenecarboximidamide

To a suspension of 4-(methylsulfonyl)aniline (2.82 g, 16.5 mmol) in toluene (150 mL), trimethylaluminum (2M solution in toluene, 12.5 mL, 24.7 mmol) was added over 15 minutes. The reaction mixture was warmed to room temperature and stirred for 2.5 hours. A solution of 3-fluoro-4-methoxybenzonitrile (5 g, 33 mmol) in toluene (100 mL) was added over 10 minutes and the reaction mixture was heated to 80-85°C. After 20 hours, the reaction mixture was cooled to room temperature and poured over a slurry of silica gel in chloroform. After filtration, the residue was washed with a mixture of methylene chloride/methanol (2/1). The combined filtrates were concentrated *in vacuo* and the resulting yellowish solid was stirred with a mixture of hexane/ether (2/1, 1000 mL). The intermediate was filtered and washed with more hexane/ether (2/1). The pale yellow solid 3-fluoro-4-methoxy-N-[4-(methylsulfonyl)phenyl] benzenecarboximidamide (3.95 g, 74%) was used in the next reaction without further purification: mp (DSC) 195°C. Anal. Calc'd. for C₁₅H₁₅N₂SO₃F: C, 55.89, H, 4.69, N, 8.69. Found: C, 55.92, H, 4.74, N, 8.53.

### Step 2: Preparation of 2-(3-fluoro-4-methoxyphenyl)-4-hydroxy-1-[4-(methylsulfonyl)phenyl]-4-(trifluoromethyl)-4,5-dihydro-1H-imidazole

To a mixture of 3-fluoro-4-methoxy-N-[4-(methylsulfonyl)phenyl]benzenecarboximidamide from Step 1 (4.15 g, 12.9 mmol) and sodium bicarbonate (2.16 g, 25.8 mmol) in isopropanol (150 mL), 3-bromo-1,1,1-trifluoroacetone (4.8 mL, 45 mmol) was added. After heating the reaction mixture at 70-75°C for 20 hours, the solvent was removed. The residue was redissolved in methylene chloride and washed with water. The organic fractions were combined, dried over sodium sulfate, filtered and concentrated *in vacuo*. The crude mixture (7.8 g) was purified by chromatography (silica gel, toluene/ethyl acetate, 7/3) to give 2-(3-fluoro-4-methoxyphenyl)-4-hydroxy-1-[4-(methylsulfonyl)phenyl]-4-(trifluoromethyl)-4,5-dihydro-1H-imidazole (3.54 g, 64%) as a white solid: mp (DSC) 210°C. Anal. Calc'd. for C₁₈H₁₆N₂SO₄F₄•0.1 PhCH₃: C, 50.86, H, 3.83, N, 6.34. Found: C, 50.61, H, 3.64, N, 6.16.

### Step 3: Preparation of 2-(3-fluoro-4-methoxyphenyl)-1-[4-(methylsulfonyl)phenyl]-4-(trifluoromethyl)-1H-imidazole

A mixture of 2-(3-fluoro-4-methoxyphenyl)-4-hydroxy-1-[4-(methylsulfonyl)phenyl]-4-(trifluoromethyl)-4,5-dihydro-1H-imidazole from Step 2 (3.4 g, 7.9 mmol) and p-toluenesulfonic acid monohydrate (700 mg) in toluene (200 mL) was heated to reflux for 72 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration in vacuo, the crude mixture (3.6 g) was purified by chromatography on silica gel using toluene/ethyl acetate (8/2) to give pure 2-(3-fluoro-4-methoxyphenyl)-1-[4-(methylsulfonyl)phenyl]-4-(trifluoromethyl)-1H-imidazole (2.12 g, 65%) as a white solid: mp (DSC) 182°C. Anal. Calc'd. for C₁₈H₁₄N₂SO₃F₄: C, 52.17, H, 3.41, N, 6.76, S, 7.74. Found: C, 52.56, H, 3.65, N, 6.53, S, 8.01.

### Example 19

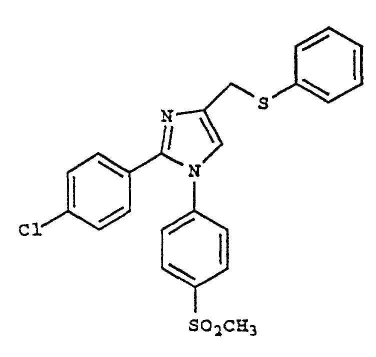

### 2-(4-Chlorophenyl)-4-[(phenylthio)methyl]-1-[4-(methylsulfonyl)phenyl]-1H-imidazole

### Step 1: Preparation of 1-bromo-3-phenylthio-2-propanone

1-Bromo-3-phenylthio-2-propanone is synthesized by reaction of thiophenol with 2,3-dichloropropene followed by treatment of the resulting product with aqueous NBS as described for Example 15.

### Step 2: Preparation of 2-(4-chlorophenyl)-4-hydroxy-1-[4-(methylsulfonyl)phenyl]-4-[(phenylthio)methyl]-4,5-dihydro-1H-imidazole

To a mixture of 4-chloro-N-[4-(methylsulfonyl)phenyl]benzenecarboximidamide (Example 1, Step 1) (1 mmol) and sodium bicarbonate (2 mmol) in acetone (20 mL), 1-bromo-3-phenylthio-2-propanone (1.5 mmol) is added. After heating to reflux for 24 hours, the reaction mixture is filtered, washed with acetone and concentrated *in vacuo.* The crude product is purified by chromatography (silica gel, toluene/ethyl acetate) to give 2-(4-chlorophenyl) -4-hydroxy-1-[4-(methylsulfonyl) phenyl]-4-[(phenylthio)methyl]-4,5-dihydro-1H-imidazole.

### Step 3: Preparation of 2-(4-chlorophenyl)-4-[(phenylthio)methyl]-1-[4-(methylsulfonyl)phenyl]-1H-imidazole

A mixture of 2-(4-chlorophenyl)-4-hydroxy-1-[4-(methylsulfonyl)phenyl]-4-[(phenylthio)methyl]-4,5-dihydro-1H-imidazole (1 mmol) and p-toluenesulfonic acid monohydrate (100 mg) in toluene (70 mL) is heated to reflux for 48 hours. The reaction mixture is cooled and the solvent removed under reduced pressure. The crude residue is redissolved in methylene chloride and washed with water, aqueous sodium bicarbonate and brine. After drying (Na₂SO₄), filtration and concentration *in vacuo*, the crude mixture is purified by chromatography on silica gel using hexane/ethyl acetate to give 2-(4-chlorophenyl)-4-[(phenylthio)methyl]-1-[4-(methylsulfonyl)phenyl]-1H-imidazole.

### Example 20

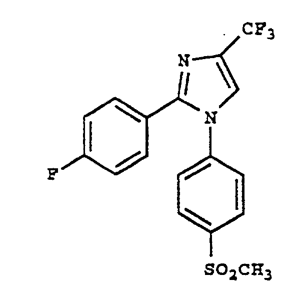

### 2-(4-Fluorophenyl)-1-[4-(methylsulfonyl)phenyl]-4-trifluoromethyl-1H-imidazole

### Step 1: Preparation of 4-fluoro-N-[4-(methylsulfonyl)phenyl]benzenecarboximidamide

To a suspension of 4-(methylsulfonyl)aniline (3.53 g, 20.2 mmol) in toluene (100 mL), trimethylaluminum (15.2 ml, 2M solution in toluene, 30.2 mmol) was added over 15 minutes. The reaction mixture was warmed to room temperature and stirred for 2.5 hours. A solution of 4-fluorobenzonitrile (5 g, 40.3 mmol) in toluene (100 mL) was added over 10 minutes and the reaction mixture heated to 80-85°C. After 20 hours, the reaction mixture was cooled to room temperature and poured over a slurry of silica gel in chloroform. After filtration, the residue was washed with a mixture of methylene chloride/methanol (2/1). The combined filtrates were concentrated *in vacuo* and the resulting yellowish solid stirred with a mixture of hexane/ether (2/1, 1000 mL). The intermediate was filtered and concentrated. The pale yellow solid 4-fluoro-N-[4-(methylsulfonyl)phenyl] benzenecarboximidamide (5.25 g, 87%) was used in the next reaction without further purification: mp (DSC) 206.2°C. Anal. Calc'd for C₁₄H₁₃N₂FSO₃•1.25 H₂O: C, 53.41; H, 4.91; N, 8.90. Found: C, 53.08; H, 4.50; N, 8.61.

### Step 2: Preparation of 2-(4-fluorophenyl)-4-hydroxy-1-[4-(methylsulfonyl)phenyl]-4-trifluoromethyl-4,5-dihvdro-1H-imidazole

To a mixture of 4-fluoro-N-[4-(methylsulfonyl)phenyl]benzenecarboximidamide (Step 1) (4.5 g. 15.4 mmol) and sodium bicarbonate (2.59 g, 30.8 mmol) in isopropanol (200 mL), 3-bromo-1,1,1-trifluoroacetone (3.2 mL) was added. After heating the reaction mixture at 75-80°C for 22 hours, the solvent was removed. The residue was redissolved in methylene chloride and washed with water. The organic fractions were combined, dried over sodium sulfate, filtered and concentrated *in vacuo.* The crude mixture (7.2 g) was purified by chromatography (silica gel, toluene/ethyl acetate, 1/1) to give 2-(4-fluorophenyl)-4-hydroxy-1-[4-(methylsulfonyl)phenyl]-4-trifluoromethyl-4,5-dihydro-1H-imidazole (3.28 g, 53%) as a white solid: mp (DSC) 203°C. Anal. Calc'd for C₁₇H₁₄N₂F₄SO₃ : C, 50.75; H, 3.51; N, 6.96. Found: C, 51.16; H, 3.69; N, 6.54.

### Step 3: Preparation of 2-(4-fluorophenyl)-1-[4-(methylsulfonyl)phenyl]-4-trifluoromethyl-1H-imidazole

A mixture of 2-(4-fluorophenyl)-4-hydroxy-1-[4-(methylsulfonyl)phenyl]-4-trifluoromethyl-4,5-dihydro-1H-imidazole (Step 2) (2.8 g, 7 mmol) and p-toluenesulfonic acid monohydrate (300 mg) in toluene (200 mL) was heated to reflux for 72 hours. The reaction mixture was cooled and the solvent removed under reduced pressure. The crude residue was redissolved in methylene chloride and washed with water, aqueous sodium bicarbonate and brine. After drying (Na₂SO₄), filtration and-concentration *in vacuo,* the crude mixture (3.2 g) was purified by chromatography on silica gel using toluene/ethyl acetate (1/1) to give pure 2-(4-fluorophenyl)-1-[4-(methylsulfonyl)phenyl]-4-trifluoromethyl-1H-imidazole (1.38 g, 52%) as a white solid: mp (DSC) 205.5°C. Anal. Calc'd for C₁₇H₁₂N₂F₄SO₂: C, 53.13; H, 3.15; N, 7.29; S, 8.34. Found: C, 53.18; H, 3.17; N, 7.26; S, 8.57.

### Example 21

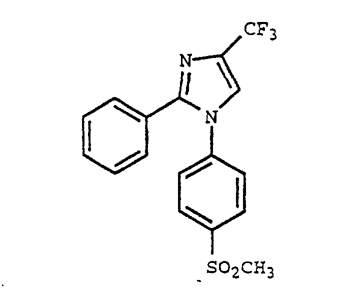

### 1-[4-(Methylsulfonyl)phenyl]-2-phenyl-4-trifluoromethyl-1H-imidazole

### Step 1: Preparation of N-[4-(methylsulfonyl)phenyl]benzenecarboximidamide

To a suspension of 4-(methylsulfonyl)aniline (12 g, 70 mmol) in toluene (400 mL), trimethylaluminum (52.5 ml, 2M solution in toluene, 0.1 mol) was added over 15 minutes. The reaction mixture was warmed to room temperature and stirred for 3.5 hours. A solution of benzonitrile (14.5 g, 0.14 mol) in toluene (300 mL) was added over 10 minutes and the reaction mixture heated to 70-75°C. After 17 hours, the reaction mixture was cooled to room temperature and poured over a slurry of silica gel in chloroform. After filtration, the residue is washed with a mixture of methylene chloride/methanol (2/1). The combined filtrates are concentrated *in vacuo* and the resulting yellowish solid is stirred with a mixture of hexane/ether (2/1, 1000 mL). The intermediate is filtered and washed with more of hexane/ether (2/1). The yellowish solid N-[4-(methylsulfonyl)phenyl]benzenecarboximidamide (16.7 g, 87%) was used in the next reaction without further purification.

### Step 2 : Preparation of 4-hydroxy-1-[4-(methylsulfonyl)phenyl]-2-phenyl-4-trifluoromethyl-4,5-dihydro-1H-imidazole

To a mixture of N-[4-(methylsulfonyl)phenyl] benzenecarboximidamide (Step 1) (16.5 g, 60.1 mmol) and sodium bicarbonate (10.1 g, 0.12 mol) in isopropanol (900 mL), 3-bromo-1,1,1-trifluoroacetone (8.7 ml, 84 mmol) was added. After heating the reaction mixture at 75 -80°C for 20 hours, the solvent was removed. The residue was redissolved in methylene chloride and washed with water. The organic fractions were combined, dried over sodium sulfate, filtered and concentrated *in vacuo.* The crude mixture was purified by chromatography (silica gel, hexane/ethyl acetate, 45/55) to give 4-hydroxy-1-[4-(methylsulfonyl)phenyl]-2-phenyl-4-trifluoromethyl-4,5-dihydro-1H-imidazole (13.6 g, 59%) as a white solid: mp 189 - 190°C. Anal. Calc'd for C₁₇H₁₅N₂F₃SO₃: C, 53.12; H, 3.93; N, 7.29; S, 8.34. Found: C, 53.05; H, 3.90; N, 7.14; S, 8.38.

### Step 3: Preparation of 1-[4-(methylsulfonyl)phenyl]-2-phenyl-4-trifluoromethyl-1H-imidazole

A mixture of 4-hydroxy-1-[4-(methylsulfonyl)phenyl]-2-phenyl-4-trifluoromethyl-4,5-dihydro-1H-imidazole (Step 2) (5.43 g, 14.1 mmol) and *p*-toluenesulfonic acid monohydrate (1.63 g) in toluene (500 mL) was heated to reflux for 96 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration *in vacuo*, the crude mixture was purified by chromatography on silica gel using hexane/ethyl acetate (65/35) to give pure 1-[4-(methylsulfonyl)phenyl]-2-phenyl-4-trifluoromethyl-1H-imidazole (3.12 g, 60%) as a white solid: mp (DSC) 233°C. Anal. Calc'd for C₁₇H₁₃N₂F₃SO₂: C, 55.73; H, 3.58; N, 7.65; S, 8.75. Found: C, 55.49; H, 3.47; N,7.46; S,8.95.

### Example 22

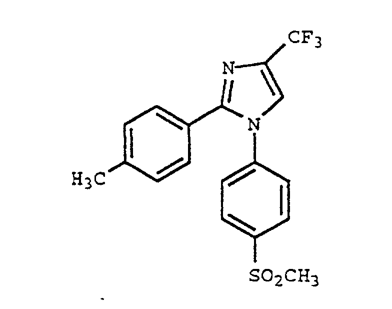

### 2-(4-Methylphenyl)-1-[4-(methylsulfonyl)phenyl]-4-trifluoromethyl-1H-imidazole

### Step 1: Preparation of 4-methyl-N-[4-(methylsulfonyl)phenyl]benzenecarboximidamide

To a suspension of 4-(methylsulfonyl)aniline (3.57 g, 20.9 mmol) in toluene (150 mL), trimethylaluminum (15.6 ml, 2M solution in toluene, 31.4 mmol) was added over 15 minutes. The reaction mixture was warmed to room temperature and stirred for 2.5 hours. A solution of 4-methylbenzonitrile (5 ml, 41.8 mmol) in toluene (100 mL) was added over 10 minutes and the reaction mixture heated to 80-85°C. After 20 hours, the reaction mixture was cooled to room temperature and poured over a slurry of silica gel in chloroform. After filtration, the residue was washed with a mixture of methylene chloride/methanol (2/1). The combined filtrates are concentrated *in vacuo* and the resulting yellowish solid was stirred with a mixture of hexane/ether (2/1, 600 mL). The intermediate was filtered and washed with more of hexane/ether (2/1). The pale yellow solid 4-methyl-N-[4-(methylsulfonyl)phenyl]benzenecarboximidamide (5.3 g, 88%) was used in the next reaction without further purification: mp (DSC) 213°C. Anal. Calc'd for C₁₅H₁₆N₂SO₂: C, 62.48; H, 5.59; N, 9.71. Found: C, 62.00, H, 5.52; N,9.60.

### Step 2 : Preparation of 2-(4-methylphenyl)-4-hydroxy-1-[4-(methylsulfonyl)phenyl]-4-trifluoromethyl-4,5-dihydro-1H-imidazole

To a mixture of 4-methyl-N-[4-(methylsulfonyl)phenyl]benzenecarboximidamide (Step 1) (5 g, 17.4 mmol) and sodium bicarbonate (2.9 g, 34.7 mmol) in isopropanol (200 mL), 3-bromo-1,1,1-trifluoroacetone (3.6 ml, 34.7 mmol) was added. After heating the reaction mixture at 75-80°C for 20 hours, the solvent was removed. The residue was redissolved in methylene chloride and washed with water. The organic fractions were combined, dried over sodium sulfate, filtered and concentrated *in vacuo*. The crude mixture (8.9 g) was purified by chromatography (silica gel, toluene/ethyl acetate 6/4) to give 2-(4-methylphenyl)-4-hydroxy-1-[4-(methylsulfonyl)phenyl]-4-trifluoromethyl-4,5-dihydro-1H-imidazole (3.28 g, 47%) as a white solid: mp 198 - 199°C. Anal. Calc'd for C₁₈H₁₇N₂F₃SO₃ ·0.3 PhMe C, 56.67; H, 4.59; N, 6.58. Found: C, 56.95; H, 4.68; N, 6.13.

### Step 3: Preparation of 2-(4-methylphenyl)-1-[4-(methylsulfonyl)phenyl]-4-trifluoromethyl-1H-imidazole

A mixture of 2-(4-methylphenyl)-4-hydroxy-1-[4-(methylsulfonyl)phenyl]-4-trifluoromethyl-4,5-dihydro-1H-imidazole (Step 2) (0.9 g, 2.3 mmol) and p-toluenesulfonic acid monohydrate (150 mg) in toluene (100 mL) was heated to reflux for 72 hours. The reaction mixture was cooled and the solvent removed under reduced pressure. The crude residue was redissolved in methylene chloride and washed with water, aqueous sodium bicarbonate and brine. After drying (Na₂SO₄), filtration and concentration *in vacuo,* the crude mixture was purified by chromatography on silica gel using toluene/ethyl acetate (1/1) to give pure 2-(4-methylphenyl)-1-[4-(methylsulfonyl)phenyl]-4-trifluoromethyl-1H-imidazole (462 mg, 54%) as a white solid: mp (DSC) 190°C. Anal. Calc'd for C₁₈H₁₅N₂F₃SO₂: C, 56.84; H, 3.97; N, 7.36; S, 8.43. Found: C, 56.66; H, 3.82; N, 7.23; S, 8.45.

### Example 23

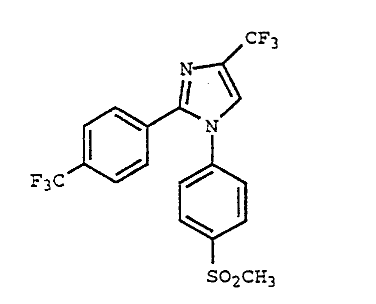

### 1- [4-(Methylsulfonyl)phenyl]-2-(4-trifluoromethylphenyl)-4-trifluoromethyl-1H-imidazole

### Step 1: Preparation of 4-(trifluoromethyl)-N-[4-(methylsulfonyl)phenyl]benzenecarboximidamide

To a suspension of 4-(methylsulfonyl)aniline (10 mmol) in toluene (100 mL), trimethylaluminum (2M solution in toluene, 15 mmol) is added over 15 minutes. The reaction mixture is warmed to room temperature and stirred for 2.5 hours. A solution of 4-trifluoromethylbenzonitrile (20 mmol) in toluene (50 mL) is added over 10 minutes and the reaction mixture is heated to 80-85°C. After 20 hours, the reaction mixture is cooled to room temperature and poured over a slurry of silica gel in chloroform. After filtration, the residue is washed with a mixture of methylene chloride/methanol (2/1). The combined filtrates are concentrated *in vacuo* and the resulting yellowish solid is stirred with a mixture of hexane/ether (2/1, 1000 mL). The intermediate is filtered and washed with more of hexane/ether (2/1). The pale yellow solid 4-(trifluoromethyl)-N-[4-(methylsulfonyl)phenyl]benzenecarboximidamide is used in the next reaction without further purification.

### Step 2: Preparation of 4-hydroxy-1-[4-(methylsulfonyl)phenyl]-2-(4-trifluoromethylphenyl)-4-trifluoromethyl-4,5-dihydro-1H-imidazole

To a mixture of 4-(trifluoromethyl)-N-[4-(methylsulfonyl)phenyl]benzenecarboximidamide (10 mmol) and sodium bicarbonate (20 mmol) in isopropanol (100 mL), 3-bromo-1,1,1-trifluoroacetone (20 mmol) is added. After heating the reaction mixture at 70-75°C for 20 hours, the solvent is removed . The residue is redissolved in methylene chloride and washed with water. The organic fractions are combined, dried over sodium sulfate, filtered and concentrated *in vacuo.* The crude product is purified by chromatography (silica gel, toluene/ethyl acetate, 7/3) to give 4-hydroxy-1-[4-(methylsulfonyl)phenyl]-2-(4-trifluoromethylphenyl)-4-trifluoromethyl-4,5-dihydro-1H-imidazole.

### Step 3: Preparation of 1-[4-(methylsulfonyl)phenyl]-2-(4-trifluoromethylphenyl)-4-trifluoromethyl-1H-imidazole

A mixture of 4-hydroxy-1-[4-(methylsulfonyl)phenyl]-2-(4-trifluoromethylphenyl)-4-trifluoromethyl-4,5-dihydro-1H-imidazole (10 mmol) and p-toluenesulfonic acid monohydrate (1 mmol) in toluene (100 mL) is heated to reflux for 72 hours. The reaction mixture is cooled and the solvent removed under reduced pressure. The crude residue is redissolved in methylene chloride and washed with water, aqueous sodium bicarbonate and brine. After drying (Na₂SO₄), filtration and concentration in vacuo, the crude mixture is purified by chromatography on silica gel using toluene/ethyl acetate to give pure 1-[4-(methylsulfonyl)phenyl]-2-(4-trifluoromethylphenyl)-4-trifluoromethyl-1H-imidazole.

### Example 24

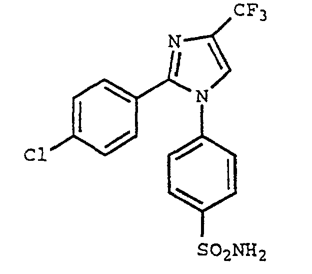

### 4-[2-(4-Chlorophenyl)-4-(trifluoromethyl)-1H-imidazol-1-yl]benzenesulfonamide

To a clear solution of 2-(4-chlorophenyl)-1-[4-(methylsulfonyl)phenyl]-4-(trifluoromethyl)-1H-imidazole from Example 2 (400 mg, 1 mmol) in tetrahydrofuran (THF) (8 mL) at 0°C, *n*-BuMgCl (2M solution in THF, 2 mL, 4 mmol) was added over 10 minutes. After stirring for additional 10 minutes, ice. bath was removed and solution stirred for 1 hour. The reaction mixture was re-cooled to 0°C and triethylborane (1M solution in THF, 5 mL, 5 mmol) was added. After stirring for 2 hours, the reaction was heated to reflux for 48 hours. The reaction mixture was cooled to room temperature and treated with aqueous sodium acetate (1 g in 4 mL water). After stirring for 5 minutes, solid hydroxylamine-O-sulfonic acid (1 g) was added and the mixture stirred for 20 hours. The reaction mixture was diluted with water and extracted with ether (2x250). The ethereal layer was dried over sodium sulfate, filtered and concentrated *in vacuo*. The crude solid (568 mg) was purified by chromatography [silica gel, ethyl acetate/toluene (3/7)] to give 4-[2-(4-chlorophenyl)-4-(trifluoromethyl)-1H-imidazol-l-yl]benzenesulfonamide (260 mg, 65%): mp (DSC) 225°C. Anal. Calc'd. for C₁₆H₁₁N₃SO₂F₃Cl: C, 47.83, H, 2.76 N, 10.46, S, 7.98. Found: C, 48.00, H, 2.83, N, 10.14, S, 7.94.

### Example 25

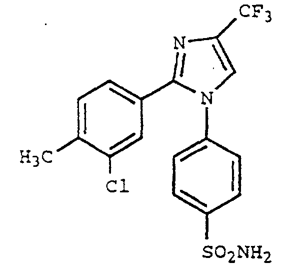

### 4-[2-(3-Chloro-4-methylphenyl)-4-(trifluoromethyl)-1H-imidazol-1-yl]benzenesulfonamide

To a clear solution of 2-(3-chloro-4-methylphenyl)-1-[4-(methylsulfonyl)phenyl]-4-(trifluoromethyl)-1H-imidazole (Example 16) (500 mg, 1.2 mmol) in tetrahydrofuran (10 mL) at 0°C, *n*-BuMgCl (2M solution in THF, 2.4 mL, 4.8 mmol) was added over 10 minutes. After stirring for additional 10 minutes, ice bath was removed and solution stirred for 1 hour. The reaction mixture was re-cooled to 0°C and triethylborane (1M solution in THF, 6 mL, 6 mmol) was added. After stirring for 2 hours, the reaction was heated to reflux for 72 hours. The reaction mixture was cooled to room temperature and treated with aqueous sodium acetate (1 g in 4 mL water). After stirring for 5 minutes, solid hydroxylamine-O-sulfonic acid (1 g) was added and the mixture stirred for 20 hours. The reaction mixture was diluted with water and extracted with ether (2x250). The ethereal layer was dried over sodium sulfate, filtered and concentrated *in vacuo.* The crude product (710 mg) was purified by chromatography (silica gel, ethyl acetate/toluene 3/7) to give pure 4-[2-(3-chloro-4-methylphenyl)-4-(trifluoromethyl)-1H-imidazol-1-yl]benzenesulfonamide (180 mg, 36%) as a white solid: mp(DSC) 222°C. Anal. Calc'd. for C₁₇H₁₃N₃SO₂F₃Cl: C, 49.10, H, 3.15, N, 10.11. Found: C, 49.42, H, 3.19, N, 9.75.

### Example 26

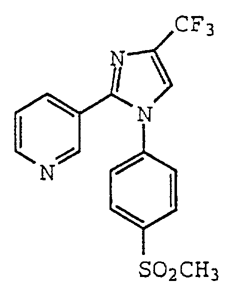

### 3-[1-[4-(Methylsulfonyl)phenyl]-4-trifluoromethyl-1H-imidazol-2-yl]pyridine

### Step 1: Preparation of N-[4-(methylsulfonyl)phenyl]-3-pyridinecarboximidamide

To a suspension of 4-(methylsulfonyl)aniline hydrochloride (6 g, 28.8 mmol) in toluene (150 ml) at 0 °C, trimethylaluminum (2M solution in toluene, 21.6 ml, 43.2 mmol) was added over 15 minutes. The reaction mixture was warmed to room temperature and stirred for 2.5 hours. A solution of 3-cyanopyridine (6 g, 57.6 mmol) in toluene (150 ml) was added over 10 minutes and the reaction mixture was heated to 90-95°C. After 24 hours, the reaction mixture was cooled to room temperature and poured over a slurry of silica gel in chloroform. After filtration, the residue was washed with a mixture of methylene chloride/methanol and later methanol. The combined filtrates were concentrated and the resulting yellowish solid was stirred with ethyl acetate (1000 ml) and filtered. The pale yellow amidine (4.5 g, 34%) was used in the next reaction without further purification: mp (DSC) 265°C. Anal Calc'd. for C₁₃H₁₄N₃SO₂Cl·0.5 H₂O: C, 48.67, H, 4.71, N, 13.10. Found: C, 48.34, H, 4.26, N, 12.77.

### Step 2: Preparation of 3-[4-hydroxy-1-[4-(methylsulfonyl)phenyl]-4-(trifluoromethyl)-4,5-dihydro-1H-imidazol-2-yl]pyridine

To a mixture of the amidine of Step 1 (4.4 g, 16 mmol) and sodium bicarbonate (2.68 g, 32 mmol) in isopropanol (400 ml), 3-bromo-1,1,1-trifluoroacetone (2.5 ml, 24 mmol) was added. After heating at 60-65°C for 36 hours, the reaction mixture was cooled and filtered. The residue was washed with methylene chloride and the combined organic fractions were dried over sodium sulfate, filtered and concentrated. The crude mixture (16.2 g) was purified by chromatography [silica gel, ethyl acetate/acetone (98:2)] to give the compound (3.7 g, 60%) as a white solid. Anal Calc'd. for C₁₆H₁₄N₃SO₃F₃·0.5 H₂O: C, 48.18, H, 3.92, N, 10.53. Found: C, 48.52, H, 3.61, N, 9.79.

### Step 3: Preparation of 3-[1-[4-(methylsulfonyl)phenyl]-4-trifluoromethyl-1H-imidazol-2-yl]pyridine

A mixture of the compound of step 2 (3.6 g, 9.35 mmol) and p-toluenesulfonic acid monohydrate (0.52 g, 2.7 mmol) in toluene (280 ml) was heated to reflux for 24 hours. The reaction mixture was cooled and the solvent removed under reduced pressure. The crude mixture was purified by chromatography on silica gel using ethyl acetate/acetone (98/2) to give pure 3-[1-[4-(methylsulfonyl)phenyl]-4-trifluoromethyl-1H-imidazol-2-yl]pyridine (790 mg, 23%) as a white solid: mp (DSC) 193°C. Anal Calc'd. for C₁₆H₁₂N₃SO₂F₃ : C, 52.30, H, 3.29, N, 11.44, S, 8.73. Found: C, 52.38, H, 3.26, N, 11.30, S, 8.76.

### Example 27

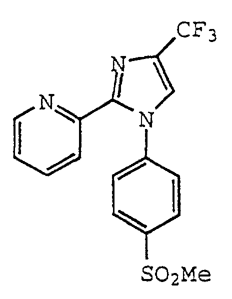

### 2-[1-[4-(Methylsulfonyl)phenyl]-4-trifluoromethyl-1H-imidazol-2-yl]pyridine

### Step 1: Preparation of N-[4-(methylsulfonyl)phenyl]-2-pyridinecarboximidamide

To a suspension of 4-(methylsulfonyl)aniline hydrochloride (6 g, 28.8 mmol) in toluene (150 ml) at 0°C, trimethylaluminum (2M solution in toluene, 21.6 ml, 43.2 mmol) was added over 15 minutes. The reaction mixture was warmed to room temperature and stirred for 2.5 hours. A solution of 2-cyanopyridine (6 g, 57.6 mmol) in toluene (150 ml) was added over 10 minutes and the reaction mixture was heated to 85-90°C. After 24 hours, the reaction mixture was cooled to room temperature and poured over a slurry of silica gel in chloroform. After filtration, the residue was washed with a mixture of methylene chloride/methanol and later methanol. The combined filtrates were concentrated and the resulting yellowish solid was stirred with ethyl acetate (1500 ml) and filtered. The pale yellow solid (5.2 g, 66%) was used in the next reaction without further purification.

### Step 2: Preparation of 2-[4-hydroxy-1-[4-(methylsulfonyl)phenyl]-4-(trifluoromethyl)-4,5-dihydro-1H-imidazol-2-yl]pyridine

To a mixture of the amidine of step 1 (4.4 g, 16 mmol) and sodium bicarbonate (2.7 g, 32 mmol) in isopropanol (400 ml), 3-bromo-1,1,1-trifluoroacetone (2.5 ml, 24 mmol) was added. After heating at 75-80°C for 24 hours, the reaction mixture was cooled and filtered. The residue was washed with methylene chloride and the combined organic fractions were dried over sodium sulfate, filtered and concentrated. The crude product (16.2 g) was purified by chromatography (silica gel, ethyl acetate/toluene 1/1) to give 2-[4-hydroxy-1-[4-(methylsulfonyl)phenyl]-4-(trifluoromethyl)-4,5-dihydro-1H-imidazol-2-yl]pyridine (1.1 g, 18%) as a white solid: mp 195-198°C. Anal. Calc'd. for C₁₆H₁₄N₃SO₃F₃ : C, 49.87, H, 3.66, N, 10.90. Found: C, 50.13 , H, 3.66, N, 10.30.

### Step 3: Preparation of 2-[1-[4-(methylsulfonyl)phenyl]-4-trifluoromethyl-1H-imidazol-2-yl]pyridine

A mixture of 2-[4-hydroxy-1-[4-(methylsulfonyl)phenyl]-4-(trifluoromethyl)-4,5-dihydro-1H-imidazol-2-yl]pyridine from step 2 (1.0 g, 2.6 mmol) and p-toluenesulfonic acid monohydrate (0.2 g, 2.7 mmol) in toluene (100 ml) was heated to reflux for 24 hours. The reaction mixture was cooled and the solvent removed under reduced pressure. The crude mixture (1.2 g) was purified by chromatography on silica gel using ethyl acetate/toluene (1/1) to give pure 2-[1-[4-(methylsulfonyl)phenyl]-4-trifluoromethyl-1H-imidazol-2-yl]pyridine (620 mg, 65%) as a white solid: mp (DSC) 184°C. Anal. Calc'd. for C₁₆H₁₂N₃SO₂F₃ : C, 52.30, H, 3.29, N, 11.44 . Found: C, 52.23, H, 3.23, N, 11.19.

### Example 28

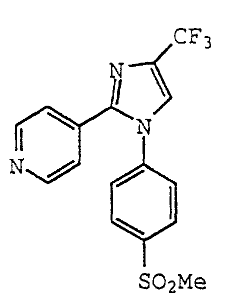

### 4-[1-[4-(Methylsulfonyl)phenyl]-4-trifluoromethyl-1H-imidazol-2-yl]pyridine

### Step 1: Preparation of N-[4-(methvlsulfonyl) phenyl]-4-pyridinecarboximidamide

To a suspension of 4-(methylsulfonyl)aniline hydrochloride (10 g, 48.1 mmol) in toluene (250 ml) at 0°C, trimethylaluminum (2M solution in toluene, 36.1 ml, 72.2 mmol) was added over 10 minutes. The reaction-mixture was warmed to room temperature and stirred for 2.5 hours. A solution of 4-cyanopyridine (10 g, 96.2 mmol) in toluene (250 ml) was added over 10 minutes and the mixture was heated to 70°C. After 24 hours, the reaction mixture was cooled to room temperature and poured over a slurry of silica gel in chloroform. After filtration, the residue was washed with a mixture of methylene chloride/methanol and later with methanol. The combined filtrates were concentrated and the resulting yellowish solid was stirred with ethyl acetate and filtered. The pale yellow solid (4.8 g, 36%) was used in the next reaction without further purification. Anal. Calc'd. for C₁₃H₁₄N₃SClO₂ H₂O: C, 47.34, H, 4.89, N, 12.74, S, 9.72. Found : C, 47.69, H, 4.35, N, 12.77, S, 9.74.

### Step 2: Preparation of 4-[4-hydroxy-1-[4-(methylsulfonyl) phenyl]-4-(trifluoromethyl)-4,5-dihydro-1H-imidazol-2-yl]pyridine

To a mixture of the amidine of step 1 (4.75 g, 16 mmol) and sodium bicarbonate (2.86 g, 34.4 mmol) in isopropanol (400 ml), 3-bromo-1,1,1-trifluoroacetone (2.7 ml, 26 mmol) was added. After heating at 75-80°C for 24 hours, the reaction mixture was cooled and filtered. The residue was washed with methylene chloride and the combined organic fractions were dried over sodium sulfate, filtered and concentrated. The crude product (16.2 g) was purified by chromatography (silica gel, ethyl acetate/isopropanol (95/5)) to give 4-[4-hydroxy-1-[4-(methylsulfonyl)phenyl]-4-(trifluoromethyl)-4,5-dihydro-1H-imidazol-2-yl]pyridine (1.55 g, 23%) as a white solid: mp 219 °C, Anal. Calc'd. for C₁₆H₁₄N₃SO₃F₃ C, 49.87, H, 3.66, N, 10.90, S, 8.32. Found: C, 49.93, H, 3.51, N, 10.79, S, 8.66.

### Step 3: Preparation of 4-[1-[4-(methylsulfonyl)phenyl]-4-trifluoromethyl-1H-imidazol-2-yl]pyridine

A mixture of the 4,5-dihydro-imidazole of step 2 (0.85 g, 2.2 mmol) and *p*-toluenesulfonic acid monohydrate (0.12 g) in toluene (150 ml) was heated to reflux for 24 hours. The reaction mixture was cooled and the solvent removed under reduced pressure. The crude mixture was purified by chromatography on silica gel using ethyl acetate/acetone (96/4) to give pure 4-[1-[4-(methylsulfonyl)phenyl]-4-trifluoromethyl-1H-imidazol-2-yl]pyridine (330 mg, 41%) as a white solid: mp (DSC) 197 °C. Anal. Calc'd. for C₁₆H₁₂N₃SO₂F₃ : C, 52.30, H, 3.29, N, 11.44, S, 8.73. Found: C, 52.19, H, 3.26, N, 11.25, S, 8.99.

### Example 29

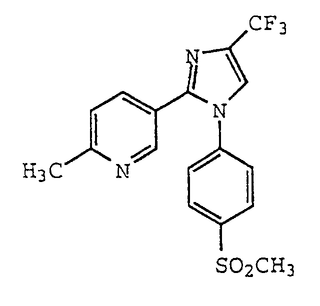

### 2-Methyl-5-[1-[4-(methylsulfonyl)phenyl]-4-trifluoromethyl-1H-imidazol-2-yl]pyridine

### Step 1: Preparation of 2-methyl-N-[4-(methylsultonyl)phenyl]-5-pyridinecarboximidamide

To a suspension of 4-(methylsulfonyl)aniline hydrochloride (8.8 g, 42.3 mmol) in toluene (150 ml) at 0°C, trimethylaluminum (2M solution in toluene, 42.3 ml, 84.6 mmol) was added over 10 minutes. The reaction mixture was warmed to room temperature and stirred for 2.5 hours. A solution of 6-methyl-4-cyanopyridine (10 g, 84.6 mmol) in toluene (150 ml) was added over 10 minutes and the mixture was heated to 80-85°C. After 24 hours, the reaction mixture was cooled to room temperature and poured over a slurry of silica gel in chloroform. After filtration, the residue was washed with a mixture of methylene chloride/methanol and later with methanol. The combined filtrates were concentrated and the resulting yellowish solid was stirred with ethyl acetate and filtered. The pale yellow solid (9.8 g, 80%) was used in the next reaction without further purification. Anal Calc'd. for C₁₄H₁₅N₃SO₂·H₂O: C, 54.71, H, 5.57, N, 13.67. Found: C, 54.62, H, 5.24, N, 13.67.

### Step 2: Preparation of 2-methyl-5-[4-hydroxy-1-[4-(methylsulfonyl)phenyl]-4-(trifluoromethyl)-4,5-dihydro-1H-imidazol-2-yl]pyridine

To a mixture of the amidine of Step 1 (9.8 g, 33.9 mmol) and sodium bicarbonate (5.7 g, 67.8 mmol) in isopropanol (700 ml), 3-bromo-1,1,1-trifluoroacetone (5.3 ml, 50.8 mmol) was added. After heating at 80-85°C for 24 hours, the reaction mixture was cooled and filtered. The residue was washed with methylene chloride and the combined organic fractions were dried over sodium sulfate, filtered and concentrated. The crude material (25.7 g) was purified by chromatography (silica gel, ethyl acetate/acetone, 98/2) to give 2-methyl-5-[4-hydroxy-1-[4-(methylsulfonyl)phenyl]-4-(trifluoromethyl)-4,5-dihydro-1H-imidazol-2-yl]pyridine (6.3 g, 46%) as a white solid:. Anal. Calc'd. for C₁₇H₁₆N₃SO₃F₃ : C, 50.55, H, 4.12, N, 10.40. Found: C, 50.51, H, 3.91, N, 10.25.

### Step 3: Preparation of 2-methyl-5-[1-[4-(methylsulfonyl)phenyl]-4-trifluoromethyl-1H-imidazol-2-yl]pyridine

A mixture of the 4,5-dihydro-imidazole of step 2 (6.2 g, 15.5 mmol) and *p*-toluenesulfonic acid monohydrate (1.6 g, 8.4 mmol) in toluene (550 ml) was heated to reflux for 24 hours. The reaction mixture was cooled and the solvent removed under reduced pressure. The crude mixture (8.2 g) was purified by chromatography on silica gel using ethyl acetate/acetone (98/2) to give pure 2-methyl-5-[1-[4-(methylsulfonyl)phenyl]-4-trifluoromethyl-1H-imidazol-2-yl]pyridine (3.9 g, 66%) as a white solid: mp (DSC) 163°C. Anal Calc'd. for C₁₇H₁₄N₃SO₂F₃ : C, 53.54, H, 3.70, N, 11.02, S, 8.41. Found: C, 53.12, H, 3.56, N, 11.00, S, 8.50.

### Example 30

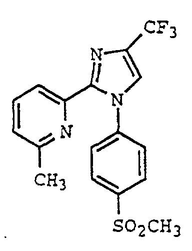

### 2-Methyl-6-[1-[4-(methylsulfonyl)phenyl]-4-trifluromethyl-1H-imidazol-2-yl]pyridine

### Step 1: Preparation of 2-methyl-N-[4-(methylsulfonyl)phenyl]-6-pyridinecarboximidamide

To a suspension of 4-(methylsulfonyl)aniline hydrochloride (4.2 g, 20.3 mmol) in toluene (100 ml) at 0°C, was added trimethylaluminum (2M solution in toluene, 12 ml, 24 mmol) over 10 minutes. The reaction mixture was warmed to room temperature and stirred for 2 hours. A solution of 6-methyl-2-cyanopyridine (3.6 g, 30.5 mmol) in toluene (100 ml) was added over 10 minutes and the mixture was heated to 85-90°C. After 24 hours, the reaction mixture was cooled to room temperature and poured over a slurry of silica gel in chloroform. After filtration, the residue was washed with a mixture of methylene chloride/methanol and later with methanol. The combined filtrates were concentrated and the resulting yellowish solid was stirred with hexane and ethyl acetate (1000 ml) and filtered. The white solid (5.1 g, 87%) was used in the next reaction without further purification. Anal. Calc'd. for C₁₄H₁₆N₃SClO₂ 0.2H₂O: C, 51.05, H, 5.02, N, 12.76. Found: C, 50.97, H, 4.78, N, 12.80.

### Step 2: Preparation of 2-methyl-6-[4-hydroxy-1-[4-(methylsulfonyl)phenyl]-4-(trifluoromethyl)-4,5-dihydro-1H-imidazol-2-yl]pyridine

To a mixture of the amidine of Step 1 (4.9 g, 16.95 mmol) and sodium bicarbonate (2.85 g, 33.9 mmol) in isopropanol (300 ml), 3-bromo-1,1,1-trifluoroacetone (2.65 ml, 25.4 mmol) was added. After heating at 80-85°C for 24 hours, the reaction mixture was cooled and filtered. The residue was washed with methylene chloride and the combined organic fractions were dried over sodium sulfate, filtered and concentrated. The crude mixture (9 g) was purified by chromatography (silica gel, ethyl acetate/isopropanol/ammonium hydroxide 95/5/0.5) to give 2-methyl-6-[4-hydroxy-1-[4-(methylsulfonyl)phenyl]-4-(trifluoromethyl)-4,5-dihydro-1H-imidazol-2-yl]pyridine (1.4 g, 21%) as a white solid: Anal. Calc'd. for C₁₇H₁₆N₃SO₃F₃: C, 51.12, H, 4.02, N, 10.52. Found: C, 51.43, H, 3.96, N, 10.06.

### Step 3: Preparation of 2-methyl-6-[1-[4-(methylsulfonyl)phenyl]-4-trifluoromethyl-1H-imidazol-2-yl]pridine

A mixture of the 4,5-dihydro-imidazole of step 2 (1.3 g, 3.26 mmol) and *p*-toluenesulfonic acid monohydrate (0.26 g, 1.36 mmol) in toluene (200 ml) was heated to reflux for 24 hours. The reaction mixture was cooled and the solvent removed under reduced pressure. The crude mixture (1.56 g) was purified by chromatography on silica gel using ethyl acetate/acetone (98/2) to give pure 2-methyl-6-[1-[4-(methylsulfonyl)phenyl]-4-trifluoromethyl-1H-imidazol-2-yl]pyridine (0.48 g, 38%) as a white solid: mp (DSC) 205°C. Anal. Calc'd. for C₁₇H₁₄N₃SO₂F₃ 0.25H₂O: C, 52.91, H, 3.79, N, 10.89, S, 8.31. Found: C, 52.67, H, 3.55, N, 10.64, S, 8.68.

### Example 31

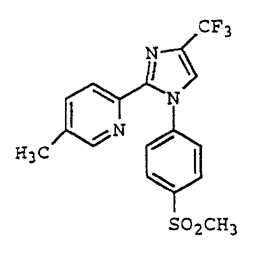

### 5-Methyl-2-[1-(4-(methylsulfonyl)phenyl]-4-trifluoromethyl-1H-imidazol-2-yl]pyridine

### Step 1: Preparation of 5-methyl-2-cyanopyridine

To a solution of 2-fluoro-5-methylpyridine (39 g; 351.5 mmol) in 141 ml of dimethylsulfoxide was added 17.23 g of sodium cyanide ( 351.5 mmol). After stirring for 3 days at 150°C, an additional 3 g of sodium cyanide was added and heating was continued for 5 hours. The reaction mixture was cooled to 25°C then poured into 525 ml of ice water. The solution was filtered through a coarse fritted funnel and a dark brown solid was collected. The solid was air dried to give 17 g of the desired cyanopyridine: Anal Calc'd. for C₇H₆N₂: C, 71.17; H, 5.12; N, 23.71. Found: C, 69.91; H, 5.24; N, 23.26.

### Step 2: Preparation of 5-methyl-N-[4-(methylthio)phenyl]-2-pyridinecarboximidamide

To a solution of 4-thiomethylaniline (8.25 g; 59 mmol) in 1,2-dichloroethane (164 ml) at 0°C, triethylaluminum (1.9M solution in toluene, 31.2 ml, 59 mmol) was added over 10 minutes. The reaction mixture was warmed to room temperature and stirred for 1.5 hours. A solution of 5-methyl-2-cyanopyridine (Step 1) (59 mmol) in 1,2-dichloroethane (62 ml) was added over 10 minutes and the mixture was heated to reflux. After 12 hours, the reaction mixture was cooled to room temperature and 50 g of silica gel were added. The suspension was stirred for 1-2 hours at 25°C and 12 ml of methanol was added and stirred at 25°C. After filtration through Celite®, the residue was washed with a mixture of methylene chloride/methanol and later with methanol. The combined filtrates were concentrated and the resulting solid stirred with hexane/ethyl acetate (1000 ml) and filtered. The solid obtained (7g) was used in the next reaction without further purification: Anal Calc'd. for C₁₄H₁₅N₃•0.3 H₂O: C, 63.99; H, 5.98; N, 15.99. Found: C, 64.05; H, 6.06; N, 16.11.

### Step 3: Preparation of 5-methyl-2-[4-hydroxy-1-[4-(methylthio)phenyl]-4-(trifluoromethyl)-4,5-dihydro-1H-imidazol-2-yl]pyridine

To a mixture of the amidine of step 2 (10 g, 52.41 mmol) and sodium bicarbonate (8.6 g, 103 mmol) in isopropanol (1200 ml), 3-bromo-1,1,1-trifluoroacetone (10 g, 52 mmol) was added. After heating at reflux for 22 hours, the reaction mixture was cooled and filtered. The residue was dissolved in methylene chloride and washed water than brine. The organic extracts were dried (MgSO₄), filtered and concentrated. The crude mixture was purified by chromatography (silica gel, 100% ethyl acetate) to give the desired dihydro-imidazole (5.1 g): Anal Calc'd. for C₁₇H₁₆N₃SOF₃: C, 55.58; H, 4.39; N, 11.44. Found: C, 55.54; H, 4.35; N, 11.20.

### Step 4: Preparation of 5-methyl-2-[1-[4-(methylthio)phenyl]-4-trifluoromethyl-1H-imidazol-2-yl]pyridine

A mixture of the dihydro-imidazole of step 3 (3. 95 g, 13.9 mmol) and p-toluenesulfonic acid monohydrate (785 mg, 4 mmol) in toluene (500 ml) was heated to reflux for 4-5 hours. The reaction mixture was cooled and 10 ml of triethylamine was added and the solvent removed under reduced pressure. The crude mixture was purified by chromatography on silica gel using ethyl acetate to give the desired product which was used in the next reaction without further purification.

### Step 5: Preparation of 5-methyl-2-[1-[4-(methylsulfonyl)phenyl]-4-trifluoromethyl-1H-imidazol-2-yl]pyridine

To solution of the methylthio compound from step 4 (3.95 g, 11.5 mmol) in 45 ml of methanol was added an aqueous solution of Oxone® (6.94 g dissolved in 28 ml of water). After stirring at 25°C for 4-5 hours, the solvent was removed under reduced pressure, redissolved in 50 ml of methylene chloride and extracted with 50 ml of an aqueous solution of sodium bicarbonate. The organic extracts were dried (MgSO₄), filtered and concentrated. The crude material was purified by chromatography (silica gel; 50% ethyl acetate/ toluene) to provide 1.6 g of the desired product: mp 196°C. Anal Calc'd. for C₁₇H₁₄N₃SO₂F₃: C, 53.54; H, 3.70; N, 11.02; S, 8.41. Found: C, 53.09; H, 3.43; N, 10.75; S, 8.69.

### Example 32

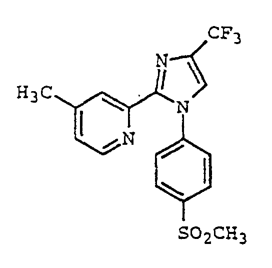

### 4-Methyl-2-[1-[4-(methylsulfonyl)phenyl]-4-trifluoromethyl-1H-imidazol-2-yl]pyridine

### Step 1: Preparation of 2-cvano-4-methylpyridine

To a suspension of 4-picoline *N*-oxide (13.64 g, 0.124 mole) in 82 ml of THF, under an inert atmosphere, was added trimethylsilyl cyanide (20.1 ml, 0.15 mole) followed by 1,8-diazabicyclo[5.4.0]undec-7-ene (DBU) (4.4 ml, 0.028 mole). After stirring at 25 °C for 12 hours, the reaction mixture was heated to reflux. After 4.5 hours, the solvent was removed under reduced pressure and the crude sample was eluted with methylene chloride through a pad of Florisil®. The solvent was removed under reduced pressure to provide (8.7 g, 60%) of 2-cyano-4-methyl pyridine, a white crystalline solid: mp 88-89 °C Anal. Calc'd. for C₇H₆N₂: C, 71.17; H, 5.12; N, 23.71. Found: C, 70.17; H, 5.12; N, 23.44.

### Step 2: Preparation of 4-methyl-N-[4-(methylsulfonyl)phenyl]-2-pyridinecarboximidamide

To a solution of 4-methylsulfonyl aniline (7.62 g, 44.5 mmol) in 40 ml of 1,2-dichloroethane, was added 23.4 ml of a 1.9 M solution of triethylaluminum in toluene. After stirring for 1.5 hours at 0 °C, 2-cyano-4-methyl-pyridine from step 1 (5.26 g, 44.5 mmol) was added. The reaction mixture was heated to reflux for 20 hours and poured onto a pad of silica gel, in a fritted filter funnel, pre-wetted and washed with 50% methanol/methylene chloride. The filtrates were evaporated under reduced pressure to provide 11.05 g (85%) of the desired amidine as a light brown solid: mp 180-184°C Anal. Calc'd.. for C₁₄H₁₅N₃O₂S: C, 58.11; H, 5.23; N, 14.52. Found: C, 57.56; H, 5.15; N, 14.35.

### Step 3: Preparation of 4-methyl-2-[4-hydroxy-1-[4-(methylsulfonyl)phenyl]-4-(trifluoromethyl)-4,5-dihydro-1H-imidazol-2-yl]pyridine

To the amidine of step 2 (12.9 g, 44.67 mmol) and sodium bicarbonate (7.15 g, 85.1 mmol) in 1L of isopropanol, 3-bromo-1,1,1-trifluoro-acetone (12.3 g, 64.4 mmol) was added. The mixture was heated to reflux. After 24 hours, the solvent was removed under reduced pressure and the resulting residue was partitioned between methylene chloride and brine. The organic extracts were dried (MgSO₄), filtered and the solvent was removed under reduced pressure to provide a dark brown oil which was purified by flash chromatography (SiO₂, 5% isopropanol/methylene chloride) to provide 3.81 g (24%) of the 4,5-dihydro-imidazole as a brown solid.

### Step 4: Preparation of 4-methyl-2-[1-[4-(methylsulfonyl)phenyl]-4-trifluoromethyl-1H-imidazol-2-yl]pyridine

To a suspension of the 4,5-dihydro-imidazole of step 3 (3.82 g, 10.78 mmol) in 700 ml of toluene was added 0.62 g of p-toluenesulfonic acid. After heating at reflux for 12 hours, an additional 0.3 g of *p*-toluenesulfonic acid was added. After 12 hours, 2.7 ml of triethylamine was added and the solvent was removed under reduced pressure to provide 5.17 g of crude compound. Crude compound was purified by chromatography twice (SiO₂; 30% heptane/ethyl acetate) by HPLC to provide 263 mg of the targeted compound. Impure fractions containing the desired product were recombined and repurified by chromatography using HPLC (SiO₂; 50% ethyl acetate/toluene) to provide an additional 639.5 mg of the desired compound: mp (DSC) 195 °C. Anal. Calc'd. for C₁₇H₁₄F₃N₃O₂S: C, 53.54; H, 3.70; N, 11.02; S, 8.41. Found: C, 53.21; H, 3.71; N, 10.77; S, 8.63.

### Example 33

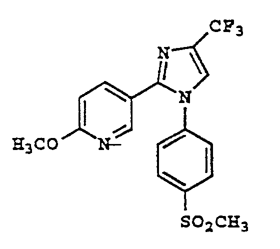

### 2-Methoxy-5-[1-[4-(methylsulfonyl)phenyl]-4-trifluoromethyl-1H-imidazol-2-yl]pyridine

### Step 1: Preparation of 2-methoxy-N-[4-(methylsulfonyl)phenyl]-5-pyridinecarboximidamide

To a suspension of 4-(methylsulfonyl)aniline hydrochloride (1.8 g, 8.7 mmol) in toluene (50 ml) at 0°C, trimethylaluminum (2M solution in toluene, 5.2 ml, 10.4 mmol) was added over 10 minutes. The reaction mixture was warmed to room temperature and stirred for 2 hours. A solution of 6-methoxy-3-cyanopyridine (1.75 g, 13 mmol) in toluene (100 ml) was added over 10 minutes and the mixture was heated to 85-90°C. After 24 hours, the reaction mixture was cooled to room temperature and poured over a slurry of silica gel in chloroform. After filtration, the residue was washed with a mixture of methylene chloride and methanol and later with methanol. The combined filtrates were concentrated and the resulting yellowish solid was stirred with ethyl acetate (1000 ml) and filtered. The white solid (2 g, 75%) was used in the next reaction without further purification. Anal. Calc'd. for C₁₄H₁₆N₃SClO₃.0.5 H₂O: C, 47.93, H, 4.88, N, 11.98. Found: C, 48.01, H, 4.82, N, 11.32.

### Step 2: Preparation of 2-methoxy-5-[4-hydroxy-1-[4-(methylsulfonyl)phenyl]-4-(trifluoromethyl)-4,5-dihydro-1H-imidazol-2-yl]pyridine

To a mixture of the amidine of step 1 (1.9 g, 6.23 mmol) and sodium bicarbonate (1.05 g, 12.46 mmol) in isopropanol (150 ml), 3-bromo-1,1,1-trifluoroacetone (0.97 ml, 9.34 mmol) was added. After heating at 85-90°C for 48 hours, the reaction mixture was cooled and filtered. The residue was washed with methylene chloride and the combined organic fractions were dried over sodium sulfate, filtered and concentrated. The crude mixture (4.25 g) was purified by chromatography (silica gel, methylene chloride/methanol/ammonium hydroxide, 95/5/0.5) to give the 4,5-dihydro-imidazole (1.1 g, 42%) as a white solid: Anal. Calc'd. for C₁₇H₁₆N₃SO₄F₃·0.5 EtOAc: C, 49.67, H, 4.39, N, 9.15. Found: C, 49.80, H, 4.06, N, 9.33.

### Step 3: Preparation of 2-methoxy-5-[1-[4-(methylsulfonyl)phenyl]-4-trifluoromethyl-1H-imidazol-2-yl]pyridine

A mixture of the 4,5-dihydro-imidazole of step 2 (0.8 g, 1.93 mmol) and p-toluenesulfonic acid monohydrate (0.2 g, 1.04 mmol) in toluene (150 ml) was heated to reflux for 24 hours. The reaction mixture was cooled and the solvent removed under reduced pressure. The crude mixture (1.1 g) was purified by chromatography on silica gel using ethyl acetate/toluene (1/1) to give pure 2-methoxy-5-[1-[4-(methylsulfonyl)phenyl]-4-trifluoromethyl-lH-imidazol-2-yl]pyridine (0.38 g, 49%) as a white solid: mp (DSC) 166°C. Anal. Calc'd. for C₁₇H₁₄N₃SO₃F₃ : C, 51.38, H, 3.55, N, 10.57. Found: C, 51.38, H, 3.25, N, 10.41.

### Example 34

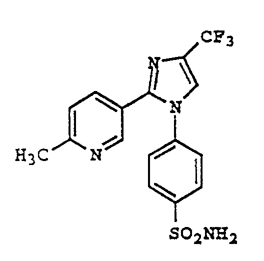

### 4-[2-(6-Methylpyridin-3-yl)-4-(trifluoromethyl)-1H-imidazol-1-yl]benzenesulfonamide

To a clear solution of Example 31 (2.4 g, 6.3 mmol) in tetrahydrofuran (60 ml) at 0°C, *n*-BuMgCl (2M solution in THF, 15.7 ml, 31.5 mmol) was added over 10 minutes. After stirring for additional 20 minutes, the ice bath was removed and the solution was stirred for 2 hours. The reaction mixture was recooled to 0°C and triethylborane (1M solution in THF, 38 ml, 38 mmol) was added. After stirring for 1 hour, the reaction was heated to reflux for 72 hours. The reaction mixture was cooled to room temperature and treated with aqueous sodium acetate (5.5 g in 22 ml water). After stirring for 5 minutes, solid hydroxylamine-O-sulfonic acid (5.5 g) was added and the mixture stirred for 24 hours. The reaction mixture was diluted with water and extracted with ether. The ethereal layer was dried over sodium sulfate, filtered and concentrated. The crude solid (13.3 g) was purified by chromatography (silica gel, hexane/isopropanol, 7/3) to give 4-[2-(6-methylpyridin-3-yl)-4-(trifluoromethyl)-1H-imidazol-1-yl]benzenesulfonamide (298 mg, 12%): mp (DSC) 203°C. Anal. Calc'd. for C₁₆H₁₃N₄SO₂F₃·0.25 H₂O: C, 49.68, H, 3.52 N, 14.48, S, 8.29. Found: C, 49.88, H, 3.39, N, 13.94, S, 8.47.

### Example 35

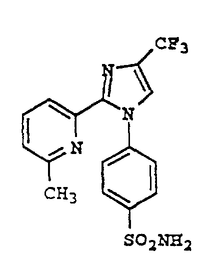

### 4-(2-(6-Methylpyridin-2-yl)-4-(trifluoromethyl)-1H-imidazol-1-yl]benzenesulfonamide

To a clear solution of Example 32 (10 mmol) in tetrahydrofuran (60 ml) at 0°C, *n*-BuMgcl (2M solution in THF, 25 ml, 50 mmol) is added over 10 minutes. After stirring for an additional 20 minutes, the ice bath is removed and the solution is stirred for 2 hours. The reaction mixture is recooled to 0°C and triethylborane (1M solution in THF, 60 ml, 60 mmol) is added. After stirring for 1 hour, the reaction is heated to reflux for 72 hours. The reaction mixture is cooled to room temperature and treated with aqueous sodium acetate (5.5 g in 22 ml water). After stirring for 5 minutes, solid hydroxylamine-O-sulfonic acid (5.5 g) is added and the mixture stirred for 24 hours. The reaction mixture is diluted with water and extracted with ether. The ethereal layer is dried over sodium sulfate, filtered and concentrated. The crude solid is purified by chromatography on silica gel using mixtures of hexane and isopropanol to give the desired product.

### Example 36

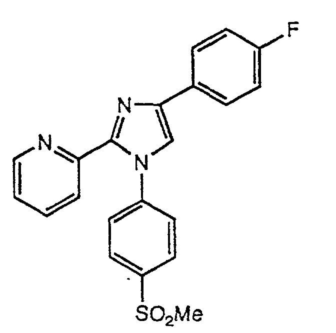

### 2-[4-(4-Fluorophenyl)-1-[4-(methylsulfonyl)phenyl]-1H-imidazol-2 yl]pyridine

### Step 1: Preparation of N-[4-(methylsulfonyl)phenyl]-2-pyridinecarboximidamide

To a suspension of 5.00 g (24.0 mmol) of 4-methylsulfonylaniline hydrochloride in 150 ml of toluene stirring in an ice bath under nitrogen, was added dropwise 18.0 ml (containing 36.0 mmol) of a 2M solution of trimethylaluminum in toluene. After stirring for 30 minutes, a solution of 3.75 g (36.0 mmol) of 2-cyanopyridine in 20 ml of toluene. The resulting solution was stirred overnight at room temperature, and then at 85° for four hours. After cooling, the toluene was decanted and evaporated. The residue was taken up in 150 ml of methylene chloride and added back to the reaction flask. Methanol (150 ml) was cautiously added, and the mixture was filtered through a bed of silica gel using 50-50 methanol/methylene chloride as eluent. Evaporation of the solvent gave the amidine (6.85 g) as a yellow solid, which was used in the next reaction without further purification.

### Step 2: Preparation of 2-[4-(4-fluorophenyl)-1-[4-(methylsulfonyl)phenyl]-1H-imidazol-2-yl]pyridine

A mixture of the amidine of Step 1 (2.00 g, 7.27 mmol), 2-bromo-4'-fluoroacetophenone (3.16 g, 14.5 mmol) and sodium bicarbonate (1.22 g, 14.5 mmol) in isopropanol (70 ml) was stirred at reflux for two days. After cooling, the solvent was evaporated. The residue was partitioned between methylene chloride and aqueous sodium chloride, and the aqueous layer further extracted with methylene chloride. The combined organic extracts were dried over sodium sulfate, filtered, and evaporated. Chromatography of the residue over silica gel using 40% ethyl acetate/toluene followed by a second chromatography over silica gel using 40% ethyl acetate/methylene chloride as eluant gave 2-[4-(4-fluorophenyl)-1-[4-(methylsulfonyl)phenyl]-1H-imidazol-2-yl]pyridine (190 mg) as a light tan solid: m.p. 88-91°C. Anal. Calc'd for C₂₁H₁₆FN₃O₂S (M.W. 393.44): C, 64.11;, H, 4.10, N, 10.68. Found: C, 63.80; H, 4.16, N, 10.23.

### Example 37

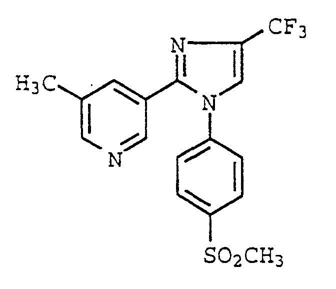

### 3-Methyl-5-[1-[4-(methylsulfonyl)phenyl]-4-(trifluoromethyl)-1H-imidazol-2-yl]pyridine

### Step 1: Preparation of 5-methylnicotinic acid:

The 5-methylnicotinic acid was prepared by the method of E. P. Kyba et al., *J. Org. Chem.*, **53**, 3513-3521 (1988)]. To a solution of KMnO₄ in water (1.1 L) was added lutidine (25.0 g, 0.233 mol) and the mixture was stirred mechanically at 45°C overnight. The reaction mixture was cooled and filtered through Celite® to remove MnO₂. The filtrate was concentrated to about 150 mL and acidified with a 2N HCl solution. White solid precipitated and was removed by filtration and washed with water (2 x 50 mL). The filtrate and washings were evaporated to dryness. The residue was boiled with ethanol (200 mL) and filtered repeatedly. The combined filtrate was concentrated to give of 5-methylnicotinic acid as a white solid (14.8 g, 46%): mp 213-215°C.

### Step 2: Preparation of 5-methylpyridinylcarboxamide

A solution of 5-methylnicotinic acid from step 1 (14.5 g, 0.106 mol) in 125 mL of thionyl chloride was heated to reflux for 5 hours. Excess thionyl chloride was removed by distillation and the residue was suspended in 75 mL of dichloroethane. Ammonia was bubbled into the mixture at -30°C for half hour and the mixture was stirred at room temperature overnight. Solvent was evaporated and the residue was treated with methanol and filtered. The filtrate was concentrated and the residue was extracted with boiling hot ethyl acetate (3 x 150 mL) to separate product from ammonium chloride. The extracts were filtered and concentrated to afford 10.6 g of 5-methylpyridinylcarboxamide as a brown solid (73%) : mp 160-163°C.

### Step 3: Preparation of 3-cyano-5-methylpyridine

To a suspension of 5-methylpyridinylcarboxamide from step 2 (10.5 g, 0.077 mol) in triethylamine (23.3 g, 0.23 mol) and 400 mL of methylene chloride was added trifluoroacetic anhydride (21.0-g, 0.100 mol) rapidly at 0°C. The reaction was completed after a few minutes. Water was added and the aqueous layer was extracted with methylene chloride. The combined organic layers were washed with water, brine and dried over magnesium sulfate. After filtration, the filtrate was concentrated to give 9.18 g of 3-cyano-5-methylpyridine crude, which was used in the next step without purification.

### Step 4: Preparation of 3-methyl-5-[4-hydroxy-1-[4-(methylsulfonyl)phenyl]-4-(trifluoromethyl)-4,5-dihydro-1H-imidazol-2-yl]pyridine

To a suspension of 4-(methylsulfonyl)aniline hydrochloride (10.5 g, 0.051 mol) in toluene (500 mL) was added trimethylaluminum (2M solution in toluene, 75.0 mL, 0.150 mol) over 15 minutes at 0°C. The reaction mixture was warmed up to room temperature and stirred for 2 hours. A solution of 3-cyano-5-methylpyridine from step 3 in 90 mL of toluene was added over 10 minutes and the mixture was stirred at 85-90°C for 16 hours. The reaction mixture was cooled to room temperature and filtered through a slurry of silica gel. After filtration, the residue was washed with methanol (800 mL). The combined filtrate was concentrated under reduced pressure and the residue was treated with a mixture of ether and hexane (2/1, 1000 mL). The brownish solid was filtered and washed with more ether and hexane to give 11.8 g of N-[4-(methylsulfonyl)phenyl]-5-methylnicotinamidine (80%). To a mixture of the above crude amidine (11.3 g, 0.039 mol) and sodium bicarbonate (9.83 g, 0.12 mol) in isopropanol (400 mL) was added 3-bromo-1,1,1-trifluoroacetone (11.2 g, 0.059 mol) quickly at room temperature. After heating the reaction mixture at 75-80°C for 16 hours, the solvent was removed and the residue was partitioned between water and methylene chloride. The organic layers were washed with brine, dried over anhydrous magnesium sulfate, filtered and concentrated under reduced pressure. The crude was purified by chromatography on silica gel (ethyl acetate/acetone, 98:2) to give pure 3-methyl-5-[4-hydroxy-1-[4-(methylsulfonyl)phenyl]-4-(trifluoromethyl)-4,5-dihydro-1H-imidazol-2-yl]pyridine as a yellow solid (3.85 g, 25%): mp (DSC) 237-239°C; Anal. Calc'd. for C₁₇H₁₆F₃N₃O₃S: C, 51.12, H, 4.04, N, 10.52, S, 8.03. Found: C, 51.02, H, 3.94, N, 10.19, S, 8.11.

### Step 5: Preparation of 3-methyl-5-[1-[4-(methylsulfonyl)phenyl]-4-(trifluoromethyl)-1H-imidazol-2-yl]pyridine

A mixture of 3-methyl-5-[4-hydroxy-1-[4-(methylsulfonyl)phenyl]-4-(trifluoromethyl)-4,5-dihydro-1H-imidazol-2-yl]pyridine from step 4 (3.8 g, 9.5 mmol) and p-toluenesulfonic acid monohydrate (0.91 g, 4.8 mmol) in 150 mL of toluene was heated to reflux for 24 hours. The reaction mixture was cooled and the solvent was removed under reduced pressure. The residue was partitioned between water and methylene chloride. The organic layer was washed with water, saturated sodium bicarbonate solution and brine, dried over magnesium sulfate and filtered. The filtrate was evaporated *in vacuo* and the crude product was purified by flash chromatography on silica gel (ethyl acetate/acetone, 98:2) to give 3-methyl-5-[1-[4-(methylsulfonyl)phenyl]-4-(trifluoromethyl)-1H-imidazol-2-yl]pyridine as a yellow solid (1.7 g, 47%): mp (DSC) 196-198°C; Anal. Calc'd. for C₁₇H₁₄F₃N₃O₂S: C, 53.54, H, 3.70, N, 11.02, S, 8.41. Found: C, 53.50, H, 3.65, N, 10.82, S, 8.55.

### Example 38

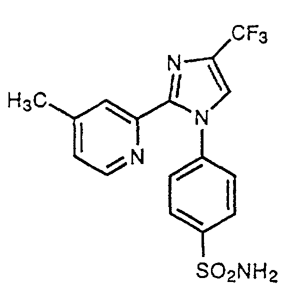

### 4-[2-(4-Methylpyridin-2-yl)-4-(trifluoromethyl)-1H-imidazol-1- yl]benzenesulfonamide

To a stirred solution of the product of Example 34 (294.5 mg, 0.77 mmol) in 11 ml of freshly distilled THF at 0 °C was added 1.54 ml of butyl magnesium chloride (2.0 M solution in THF) over a period of 6 minutes. After stirring at 25 °C for 2.5 hours, the reaction was cooled to 0 °C and 3.85 ml of triethylborane (1.0 M solution in THF) was added over 30 minutes. The reaction mixture was stirred at 25°C for 1.5 hours and heated to reflux. After 72 hours, the reaction mixture was diluted with 50 ml of ethyl acetate and washed with aqueous sodium bicarbonate (2 x 50 ml). The organic extracts were dried (MgSO₄), filtered and the solvent removed under reduced pressure to provide 359 of an orange solid, which was purified by chromatography (SiO₂; 40% toluene/ethyl acetate) to provide 68.1 mg of a light yellow solid. Preparative thin layer chromatography (SiO₂; 50% ethyl acetate/toluene) of 22 mg of this material yielded 14 mg of 4-[2-(4-methylpyridin-2-yl)-4-(trifluoromethyl)-1H-imidazol-1-yl]benzenesulfonamide: mp (DSC) 283 °C. Anal. Calc'd. for C₁₆H₁₃F₃N₄O₂S: C, 50.26; H, 3.43; N, 14.65; S, 8.50. Found: C, 50.41; H, 3.37; N, 14.18; S, 8.51.

### Example 39

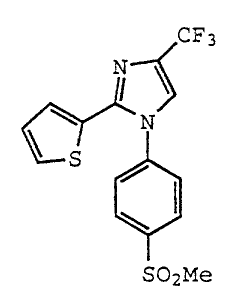

### 2-[1-[4-(Methylsulfonyl)phenyl]-4-(trifluoromethyl)-1H-imidazol-2-yl]thiophene

### Step 1: Preparation of N-[4-(methylsulfonyl) phenyl]-2-thiophenecarboximidamide

To a suspension of 4-(methylsulfonyl)aniline (10.4 g, 61.1 mmol) in toluene (400 ml) at 0°C, trimethylaluminum (2M solution in toluene, 46.8 ml, 91.6 mmol) was added over 15 minutes. The reaction mixture was warmed to room temperature and stirred for 2 hours. A solution of 2-thiophenecarbonitrile (10.0 g, 91.6 mmol) in toluene (200 ml) was added over 10 minutes and the mixture was heated to 80-85°C. After 16 hours, the reaction mixture was cooled to room temperature and poured over a slurry of silica gel in chloroform. After filtration, the residue was washed with a mixture of methylene chloride/methanol and later with methanol. The combined filtrates were concentrated and the resulting yellow solid was stirred with ethyl acetate and filtered. The pale yellow solid (9.8 g, 57%) was used in the next reaction without further purification: m.p. (DSC) 182°C. Anal. Calc'd. for C₁₂H₁₂N₂S₂O₂: C, 51.41, H, 4.31, N, 9.99, S, 22.87. Found: C, 51.02, H, 4.37, N, 9.80, S, 22.93.

### Step 2: Preparation of 2-[4-hydroxy-1-[4-(methylsulfonyl)phenyl]-4-(trifluoromethyl)-4,5-dihydro-1H-imidazol-2-yl]thiophene

To a mixture of the-amidine of step 1 (2.0 g, 7.1 mmol) and sodium bicarbonate (1.2 g, 14.3 mmol) in isopropanol (200 ml), 3-bromo-1,1,1-trifluoroacetone (1.1 ml, 10.7 mmol) was added. After heating at 80-85°C for 16 hours, the reaction mixture was cooled and filtered. The residue was washed with methylene chloride and the combined organic fractions were dried over sodium sulfate, filtered and concentrated. The crude mixture (25.7 g) was purified by chromatography (silica gel, ethyl acetate/hexane 55/45) to give the 4,5-dihydro-imidazole (1.1 g, 38%) as a white solid: mp (DSC) 214°C. Anal. Calc'd. for C₁₅H₁₃N₂S₂O₃F_{3:} C, 46.15, H, 3.36, N, 7.18, S, 16.43. Found: C, 46.09, H, 3.26, N, 7.07, S, 16.71.

### Step 3: Preparation of 2-[1-[4-(methylsulfonyl) phenyl]-4-(trifluoromethyl)-1H-imidazol-2-yl]thiophene

A mixture of the 4,5-dihydro-imidazole of step 2 (0.60 g, 1.54 mmol) and *p*-toluenesulfonic acid monohydrate (0.12 g, 0.63 mmol) in toluene (100 ml) was heated to reflux for 4.5 hours. The reaction mixture was cooled and the solvent removed under reduced pressure. The crude mixture (1.2 g) was purified by chromatography on silica gel using ethyl acetate/hexane 50/50 to give pure 2-[1-[4-(methylsulfonyl)phenyl]-4-(trifluoromethyl)-1H-imidazol-2-yl]thiophene (0.47 g, 82%) as a white solid: mp (DSC) 182°C. Anal. Calc'd. for C₁₅H₁₁N₂S₂O₂F₃ C, 48.38, H, 2.98, N, 7.52, S, 17.22. Found: C, 48.36, H, 3.02, N, 7.42, S, 17.47.

### Example 40

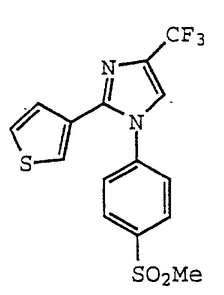

### 3-[1-[4-(Methylsulfonyl)phenyl]-4-(trifluoromethyl)-1H-imidazol-2-yl]thiophene

### Step 1: Preparation of N-[4-(methylsulfonyl)phenyl]-2-thiophenecarboximidamide

To a suspension of 4-(methylsulfonyl)'aniline (3.3 g, 19.5 mmol) in toluene (200 ml) at 0°C, trimethylaluminum (2M solution in toluene, 14.7 ml, 29.3 mmol) was added over 15 minutes. The reaction mixture was warmed to room temperature and stirred for 2 hours. A solution of 3-thiophenecarbonitrile (3.2 g, 29.3 mmol) in toluene (50 ml) was added over 10 minutes and the mixture was heated to 80-85°C. After 16 hours, the reaction mixture was cooled to room temperature and poured over a slurry of silica gel in chloroform. After filtration, the residue was washed with a mixture of methylene chloride/methanol and later with methanol. The combined filtrates were concentrated and the resulting yellow solid was stirred with ethyl acetate and filtered. The pale yellow solid (2.7 g, 49%) was used in the next reaction without further purification: mp (DSC) 213 °C, Anal. Calc'd. for C₁₂H₁₂N₂S₂O₂: C, 51.41, H, 4.31, N, 9.99, S, 22.87. Found: C, 51.28, H, 4.06, N, 9.86, S, 23.14.

### Step 2: Preparation of 3-[4-hydroxy-1-[4-(methylsulfonyl)phenyl]-4-(trifluoromethyl)-4,5-dihydro-1H-imidazol-2-yl]thiophene

To a mixture of the amidine of step 1 (3.5 g, 12.5 mmol) and sodium bicarbonate (2.1 g, 25.0 mmol) in isopropanol (200 ml), 3-bromo-1,1,1-trifluoroacetone 1.96 ml, 18.7 mmol) was added. After heating at 80-85°C for 16 hours, the reaction mixture was cooled and filtered. The residue was washed with methylene - chloride and the combined organic fractions were dried over sodium sulfate, filtered and concentrated. The crude material was purified by chromatography (silica gel, ethyl acetate/toluene (6/4)) to give 3-[4-hydroxy-1-[4-(methylsulfonyl)phenyl]-4-(trifluoromethyl) -4,5-dihydro-1H-imidazol-2-yl]thiophene (1.7 g, 35%) as a white solid: mp (DSC) 226°C. Anal. Calc'd. for C₁₅H₁₃N₂S₂O₃F₃ : C, 46.15, H, 3.36, N, 7.18, S, 16.43. Found: C, 46.56, H, 3.39, N, 7.01, S, 16.88.

### Step 3: Preparation of 3-[1-[4-(methylsulfonyl)phenyl]-4-(trifluoromethyl)-1H-imidazol-2-yl]thiophene

A mixture of the 4,5-dihydro-imidazole of step 2 (1.5 g, 3.8 mmol) and *p*-toluenesulfonic acid monohydrate (0.30 g, 1.5 mmol) in toluene (250 ml) was heated to reflux for 40 hours. An additional *p*-toluenesulfonic acid monohydrate (0.15 g, 0.78 mmol) was added. The reaction mixture was heated to reflux for 18 hours. The reaction mixture was cooled and the solvent removed under reduced pressure. The crude mixture (3.5 g) was purified by chromatography on silica gel using ethyl acetate/toluene (55/45) to give pure 3-[1-[4-(methylsulfonyl)phenyl]-4-(trifluoromethyl)-1H-imidazol-2-yl]thiophene (0.90 g, 64%) as a white solid: mp 194-197°C. Anal. Calc'd. for C₁₅H₁₁N₂S₂O₂F₃: C, 48.38, H, 2.98, N, 7.52, S, 17.22. Found: C, 48.74, H, 2.98, N, 7.56, S, 17.45 .

### Example 41

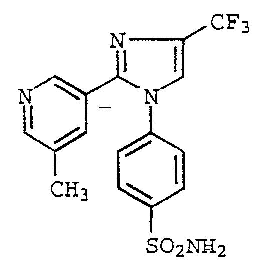

### 4-[2-(5-Methylpyridin-3-yl)-4-(trifluoromethyl)-1H-imidazol-1-yl]benzenesulfonamide

To a solution of 3-methyl-5-[1-[4-(methylsulfonyl)phenyl]-4-(trifluoromethyl)-1H-imidazol-2-yl]pyridine (Example 39) (1.9 mmol) in 25 mL of dry THF was added n-BuMgCl (3.8 mL of 2.0 M THF solution, 7.5 mmol) slowly at 0 °C. After stirring for additional 15 minutes, the solution was stirred at room temperature for 2 hours. The reaction mixture was re-cooled to 0°C and triethylborane (9.5 mL of 1.0 M THF solution, 9.5 mmol) was added. After stirring at for 2 hours, the mixture was heated to reflux for 72 hours. The reaction mixture was cooled to room temperature and treated with a solution of sodium acetate (2.3 g) in 10 mL of water. After stirring for 5 minutes, hydroxylamine-*O*-sulfonic acid (2.3 g) was added and the mixture was stirred for 20 hours. The reaction mixture was extracted with ether (2 x 100 mL). The ethereal layer was dried over MgSO₄, filtered and concentrated *in vacuo.* The crude product was purified by chromatography on silica gel (isopropanol/toluene, 5:95) to give 0.07 g of 4-[2-(5-methylpyridin-3-yl)-4-(trifluoromethyl)-1H-imidazol-1-yl]benzenesulfonamide as a colorless solid (8%): mp 242-243°C. Anal. Calc'd. For C₁₆H₁₃F₃N₄O₂S: C, 50.26, H, 3.43, N, 14.65, S, 8.39. Found: C, 50.02, H, 3.63, N, 14.26, S, 8.41.

### Example 42

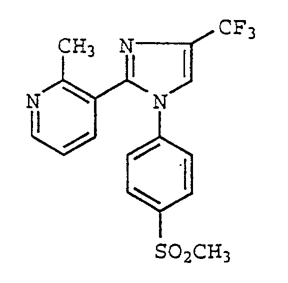

### 2-Methyl-3-[1-[4-(methylsulfonyl)phenyl]-4-(trifluoromethyl)-1H-imidazol-2-yl]pyridine

### Step 1: Preparation of 2-methylnicotinamide:

To a stirred mixture of 2-methylnicotinic acid (15.0 g, 0.111 mol) and 1,1'-carbonyldiimidazole (36.0 g, 0.222 mol) was added 300 mL of methylene chloride dropwise. The reaction mixture was stirred at room temperature overnight. Ammonia gas was distilled into the reaction mixture for 30 minutes using a dry ice condenser and the mixture was stirred at room temperature for an additional hour. Solvent was removed under vacuum and the residue was dissolved with 500 mL of acetonitrile. The solution was concentrated to half volume at low temperature and the product precipitated out as white solid. The crude mixture was recrystallized from ethanol/ether to give 11.5 g of 2-methylnicotinamide as a colorless crystal (76%): mp 160-163°C. Anal. Calc'd. For C₇H₈N₂O: C, 61.75, H, 5.92, N, 20.57. Found: C, 61.44, H, 6.14, N, 20.66.

### Step 2: Preparation of 3-cyano-2-methylpyridine:

To a suspension of 2-methylnicotinamide from step 1 (11.1 g, 0.081 mol) in triethylamine (24.8 g, 0.243 mol) and 400 mL of methylene chloride was added trifluoroacetic anhydride (21.0 g, 0.100 mol) rapidly at 0°C. The reaction was complete after a few minutes at this temperature. Water was added and the aqueous layer was extracted with methylene chloride. The combined organic layers were washed with water, brine and dried over magnesium sulfate. After filtration, the filtrate was concentrated and the residue was purified by chromatography on silica gel (ethyl acetate/hexane, 1:1) to give 7.2 g of 3-cyano-2-methylpyridine as a pale yellow solid (75%): mp(DSC) 56-58°C.

### Step 3: Preparation of 2-methyl-3-[4-hydroxy-1-[4-(methylsulfonyl)phenyl]-4-(trifluoromethyl)-4,5-dihydro-1H-imidazol-2-yl]pyridine:

To a suspension of 4-(methylsulfonyl)aniline hydrochloride (6.85 g, 0.040 mol) in dichloroethane (400 mL) was added triethylaluminum (1.9M solution in toluene, 32.0 mL, 60 mmol) over 15 minutes at 0°C. The reaction mixture was warmed to room temperature and stirred for 2 hours. A solution of 3-cyano-2-methylpyridine, from step 2, in 70 mL of dichloroethane was added over 10 minutes and the mixture was stirred at 75°C for 16 hours. The reaction mixture was cooled to room temperature and treated with 50 g of silica gel. The mixture was stirred for 30 minutes and filtered. The filtrate and washings were concentrated under reduced pressure and the residue was washed with ether to give 7.3 g of crude 2-methyl-N-[4-(methylsulfonyl)phenyl]-3-pyridinecarboximidamide (60%). To a mixture of the above crude amidine (7.0 g, 0.024 mol) and sodium bicarbonate (4.0 g, 0.048 mol) in isopropanol (350 mL) was added 3-bromo-1,1,1-trifluoroacetone (6.9 g, 0.036 mol) rapidly at room temperature. After heating the reaction mixture at 75-80°C for 16 hours, the solvent was removed and the residue was partitioned between water and methylene chloride. The organic layers were washed with brine, dried over anhydrous magnesium sulfate, filtered and concentrated under reduced pressure. The crude was purified by chromatography on silica gel (ethyl acetate/acetone, 98:2) to give 4.02 g of pure 2-methyl-3-[4-hydroxy-1-[4-(methylsulfonyl)phenyl]-4-(trifluoromethyl)-4,5-dihydro-1H-imidazol-2-yl]pyridine as a yellow solid (25%): mp (DSC) 237-239°C. Anal. Calc'd. for C₁₇H₁₆F₃N₃O₃S: C, 51.12, H, 4.04, N, 10.52, S, 8.03. Found: C, 50.92, H, 4.12, N, 10.04, S, 7.83.

### Step 4: Preparation of 2-methyl-3-[1-[4-(methylsulfonyl)phenyl]-4-(trifluoromethyl)-1H-imidazol-2-yl]pyridine:

A mixture of 2-methyl-3-[4-hydroxy-1-[4-(methylsulfonyl)phenyl]-4-(trifluoromethyl)-4,5-dihydro-1H-imidazol-2-yl]pyridine from step 3 (3.97 g, 0.01 mol) and *p*-toluenesulfonic acid monohydrate (0.60 g, 0.0032 mol) in 250 mL of toluene was heated to reflux for 24 hours. The reaction mixture was cooled and the solvent was removed under reduced pressure. The residue was partitioned between water and methylene chloride. The organic layer was washed with water, saturated sodium bicarbonate solution and brine, dried over magnesium sulfate and filtered. The filtrate was evaporated in vacuo and the crude product was purified by recrystallization from ethyl-acetate/hexane to give 2.8 g of 2-methyl-3-[1-[4-(methylsulfonyl)phenyl]-4-(trifluoromethyl)-1H-imidazol-2-yl]pyridine (73%): mp 160-161°C. Anal. Calc'd. for C₁₇H₁₄F₃N₃O₂S: C, 53.54, H, 3.70, N, 11.02, S, 8.41. Found: C, 53.58, H, 3.88, N, 11.02, S, 8.51.

### Example 43

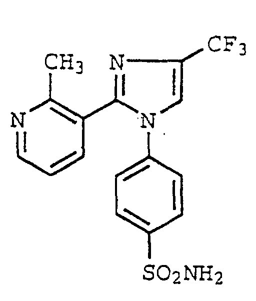

### 4-[2-(2-Methylpyridin-3-yl)-4-(trifluoromethyl)-1H-imidazol-1-yl]benzenesulfonamide

### Step 1: Preparation of 2-methyl-3-[1-[4-[[2-(trimethylsilyl)ethyl]sulfonyl]phenyl]-4-(trifluoromethyl)-1H-imidazol-2-yl]pyridine

To a solution of diisopropylamine (0.7 mL, 0.005 mol) in 9 mL of dry THF was added butyllithium (BuLi) (2.83 mL of 1.62M solution in hexane, 4.6 mmol) at 0°C. The solution was stirred at this temperature for 5 minutes and cooled to -78°C with a dry ice/isopropanol bath. A solution of 2-methyl-3-[1-(4-(methylsulfonyl)phenyl]-4-(trifluoromethyl)-1H-imidazol-2-yl]pyridine (Example 44) (1.46 g, 3.8 mmol) in 12 mL of dry THF was added over 10 minutes and the reaction mixture was stirred at -78°C for 1 hour. (Iodomethyl)trimethylsilane (1.23 g, 57 mmol) was added dropwise and the reaction mixture was warmed to room temperature and was stirred overnight. The reaction was quenched with 50 mL of 1 N HCl and the aqueous phase was extracted with ethyl acetate (3 x 60 mL). The combined organic layers were washed with brine, dried over MgSO₄, filtered and concentrated. The crude mixture was purified by chromatography on silica gel (ethyl acetate/hexane, 65:35) to give 1.30 g of 2-methyl-3-[1-[4-[[2-(trimethylsilyl)ethyl]sulfonyl]phenyl]-4-(trifluoromethyl)-1H-imidazol-2-yl]pyridine as a white solid (74%): mp(DSC) 155-157°C. Anal. Calc'd. for C₂₁H₂₄F₃N₃O₂SSi: C, 53.94; H, 5.17; N, 8.99; S, 6.86. Found: C, 53.77; H, 4.94; N, 8.75; S, 6.98.

### Step 2: Preparation of 4-[2-(2-methylpyridin-3-yl)-4-(trifluoromethyl)-1H-imidazol-1-yl]benzenesulfonamide:

To a solution of 2-methyl-3-[1-[4-[[2-(trimethylsilyl)ethyl]sulfonyl]phenyl]-4-(trifluoromethyl)-1H-imidazol-2-yl]pyridine from Step 1 (0.234 g, 0.5 mmol) in 1.5 mL of dry THF was added n-Bu₄NF (1.5 mL of 1.0M THF solution, 1.5 mmol). The mixture was heated to reflux for 1 hour and cooled to room temperature. A solution of sodium acetate (0.19 g, 2.3 mmol) in 3 mL of water and hydroxylamine-*O*-sulfonic acid (0.28 g, 2.5 mmol) were added sequentially and the mixture was stirred for 1 hour. Water (7 mL) and ethyl acetate (7 mL) were added. The organic phase was separated and washed with sat. NaHCO₃ solution, water, and brine, dried over MgSO₄ and filtered. The filtrate was concentrated and the residue was purified by chromatography on silica gel (ethyl acetate/acetone, 95:5) to give 0.16 g of 4-[2-(2-methylpyridin-3-yl)-4-(trifluoromethyl)-1H-imidazol-1-yl]benzenesulfonamide as a colorless solid (84%): mp 235-237°C. Anal. Calc'd. for C₁₆H₁₃F₃N₄O₂S: C, 50.26; H, 3.43; N, 14.65; S, 8.39. Found: C, 50.06; H, 3.29; N, 14.44; S, 8.52.

### Example 44

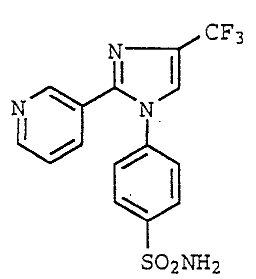

### 4-[2-(Pyridin-3-yl)-4-(trifluoromethyl)-1H-imidazol-1-yl]benzenesulfonamide

3-[1-[4-[[2-(Trimethylsilyl)ethyl]sulfonyl]phenyl]-4-(trifluoromethyl)-1H-imidazol-2-yl]pyridine was prepared with the product of Example 28 with a method similar to that described in Example 45, Step 1. To a solution of 3-[1-[4-[[2-(trimethylsilyl)ethyl]sulfonyl]phenyl]-4-(trifluoromethyl)-1H-imidazol-2-yl]pyridine (0.200 g, 0.46 mmol) in 1.0 mL of dry THF was added n-Bu₄NF (1.38 mL of 1.0 M THF solution, 1.38 mmol). The mixture was heated to reflux for 1 hour and cooled to room temperature. A solution of sodium acetate (0.17 g, 2.1 mmol) in 3 mL of water and hydroxylamine-*O*-sulfonic acid (0.26 g, 2.3 mmol) were added sequentially and the mixture was stirred for 1 hour. Water (7 mL) and ethyl acetate (7 mL) were added. The organic phase was separated and washed with saturated NaHCO₃ solution, water, brine, dried over MgSO₄ and filtered. The filtrate was concentrated and the residue was purified by chromatography on silica gel (ethyl acetate/acetone, 95:5) to give 0.147 g of 4-[2-(pyridin-3-yl)-4-(trifluoromethyl)-1H-imidazol-1-yl]benzenesulfonamide as a colorless solid (87%): mp(DSC) 213-215 °C. Anal. Calc'd. for C₁₅H₁₁F₃N₄O₂S: C, 48.91; H, 3.01; N, 15.21; S, 8.71. Found: C, 48.58; H, 2.99; N, 14.87; S, 8.85.

The following imidazole derivatives could be prepared by the procedure described in Example 26 or 45:
Example 45: 4-[2-(4-chlorophenyl) -4-methyl-1H-imidazol-1-yl]benzenesulfonamide;
Example 46: 4-[2-(4-chlorophenyl)-4-phenyl-1H-imidazol-1-yl]benzenesulfonamide;
Example 47: 4-[2-(4-chlorophenyl)-4-(4-fluorophenyl)-1H-imidazol-1-yl]benzenesulfonamide;
Example 48: 4-[2-(4-chlorophenyl)-4-(4-bromophenyl)-1H-imidazol-1-yl]benzenesulfonamide;
Example 49: 4-[2-(4-chlorophenyl) -4-(2-naphthyl)-1H-imidazol-1-yl]benzenesulfonamide;
Example 50: 4-[2-(4-chlorophenyl)-4-[4-(trifluoromethoxy)phenyl]-1H-imidazol-1-yl]benzenesulfonamide;
Example 51: 4-[2,4-bis(4-chlorophenyl)-1H-imidazol-1-yl]benzenesulfonamide;
Example 52: 4-[2-(4-chlorophenyl)-4-(3-chlorophenyl)-1H-imidazol-1-yl]benzenesulfonamide;
Example 53: 4-[2-(4-chlorophenyl)-4-[4-(methoxy)phenyl]-1H-imidazol-1-yl]benzenesulfonamide;
Example 54: 4-[2-(4-chlorophenyl)-4-(3-fluorophenyl)-1H-imidazol-1-yl]benzenesulfonamide;
Example 55: 4-[2-(4-chlorophenyl)-4-[(4-chlorophenoxy)methyl]-1H-imidazol-1-yl]benzenesulfonamide;
Example 56: 4-[2-(3,4-methylenedioxyphenyl)-4-(trifluoromethyl)-1H-imidazol-l-yl]benzenesulfonamide;
Example 57: 4-[2-(3-fluoro-4-methoxyphenyl)-4-(trifluoromethyl)-1H-imidazol-1-yl]benzenesulfonamide;
Example 58: 4-[2-(4-chlorophenyl)-4-[ (phenylthio)methyl]-1H-imidazol-1-yl]benzenesulfonamide;
Example 59: 4-[2-(4-chlorophenyl)-4-[(N-methyl-N-phenylamine)methyl]-1H-imidazol-1-yl]benzenesulfonamide;
Example 60: 4-[2-(4-chlorophenyl)-4-[(2-quinolylmethoxy)methyl]-1H-imidazol-1-yl]benzenesulfonamide;
Example 61: 4-[2-(4-chlorophenyl)-4-methoxymethyl-1H-imidazol-1-yl]benzenesulfonamide;
Example 62: 4-[2-(4-fluorophenyl)-4-(trifluoromethyl)-1H-imidazol-1-yl]benzenesulfonamide;
Example 63: 4-[2-phenyl-4-(trifluoromethyl)-1H-imidazol-1-yl]benzenesulfonamide; and
Example 64: 4-[2-(4-trifluoromethylphenyl)-4-(trifluoromethyl)-1H-imidazol-1-yl]benzenesulfonamide.

The following imidazole derivative was prepared by the procedure described in Example 26:
Example 65: 1-methyl-3-[1-[4-(methylsulfonyl)phenyl]-4-trifluoromethyl-1H-imidazol-2-yl]-1H-indole.

The following imidazole derivatives in Tables I-IV were obtained according to procedures of Schemes I-XIV. Many of these were synthesized by using the experimental conditions given in Examples 1-5. The sulfonamide derivatives were synthesized from the corresponding sulfones using experimental procedure given for Examples 24-24 and 43.

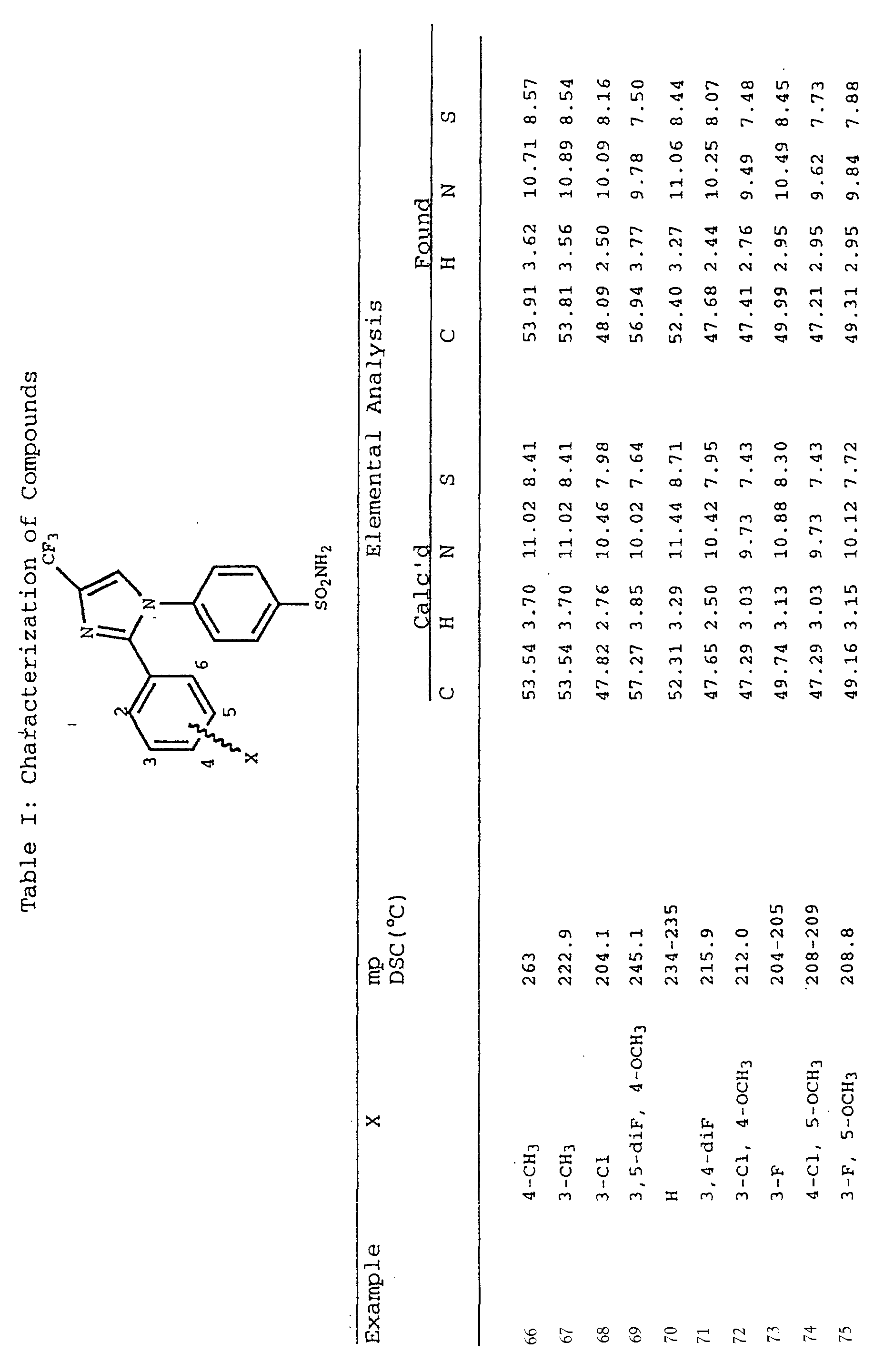

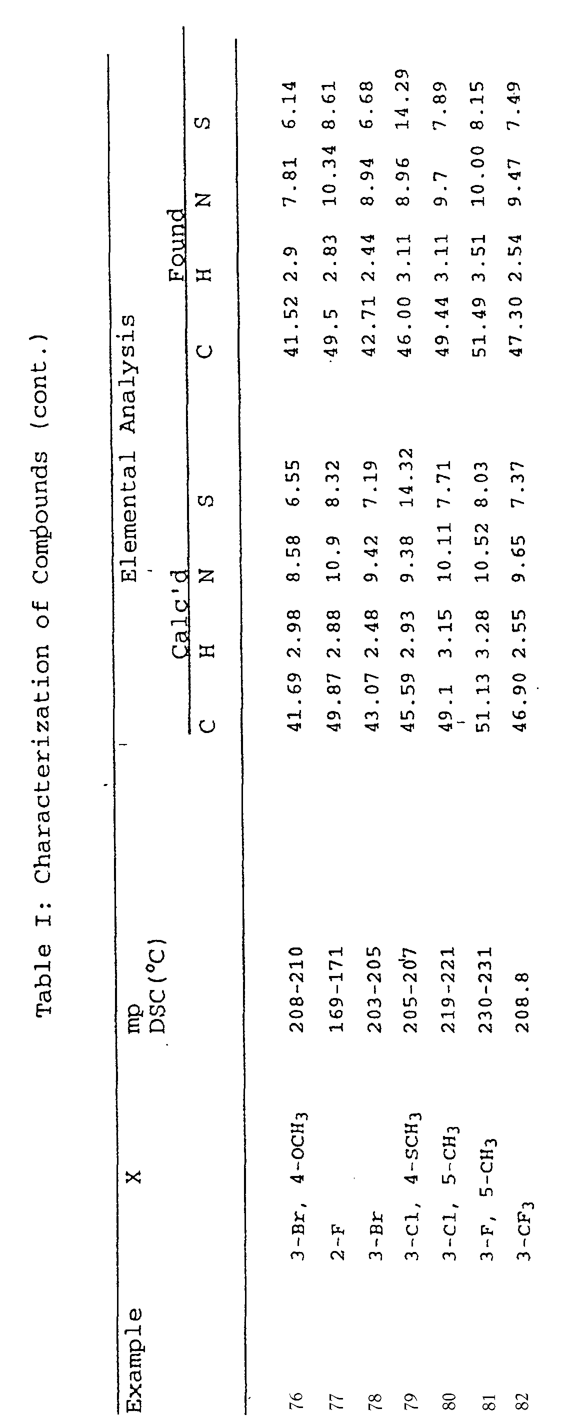

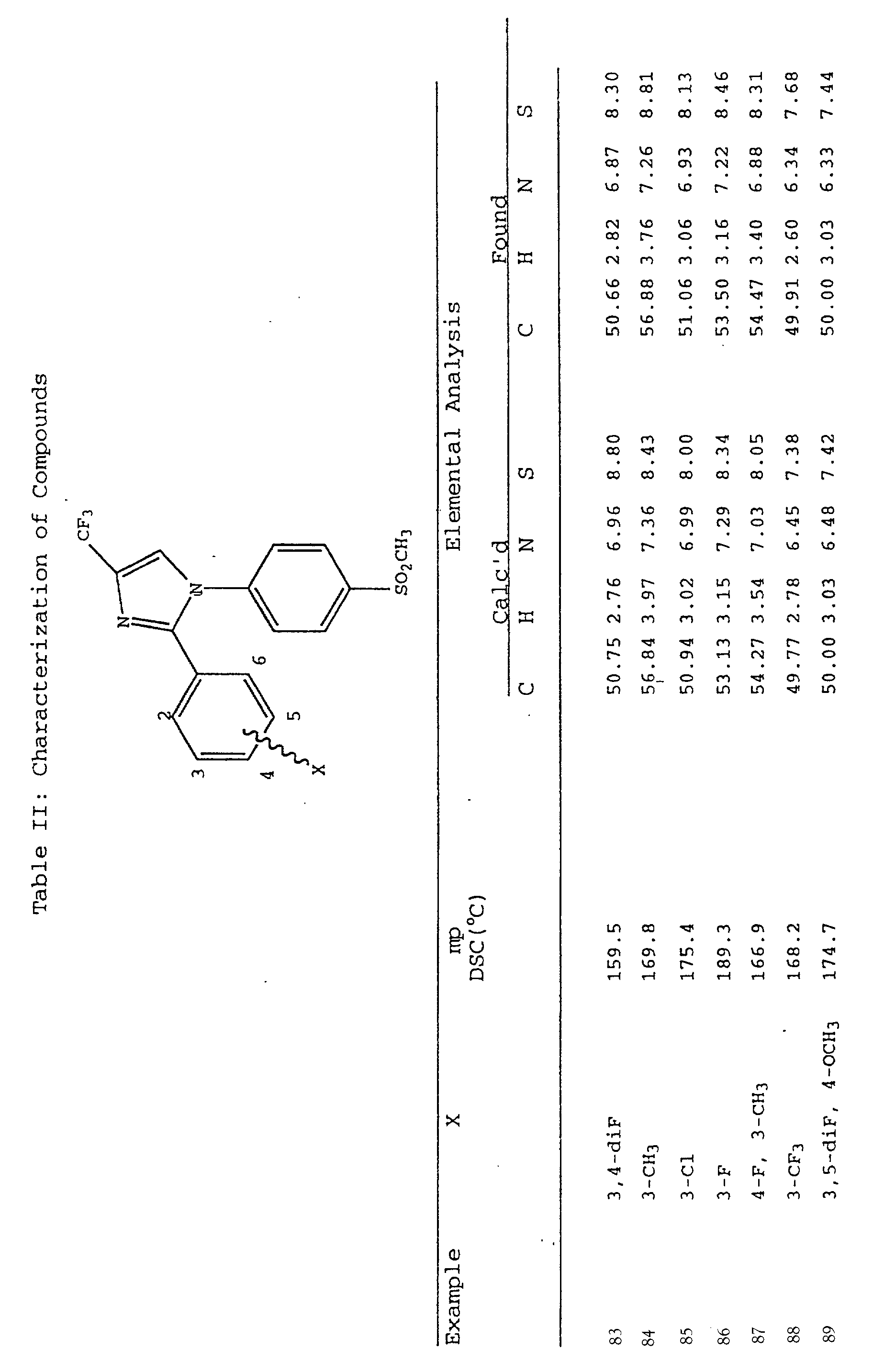

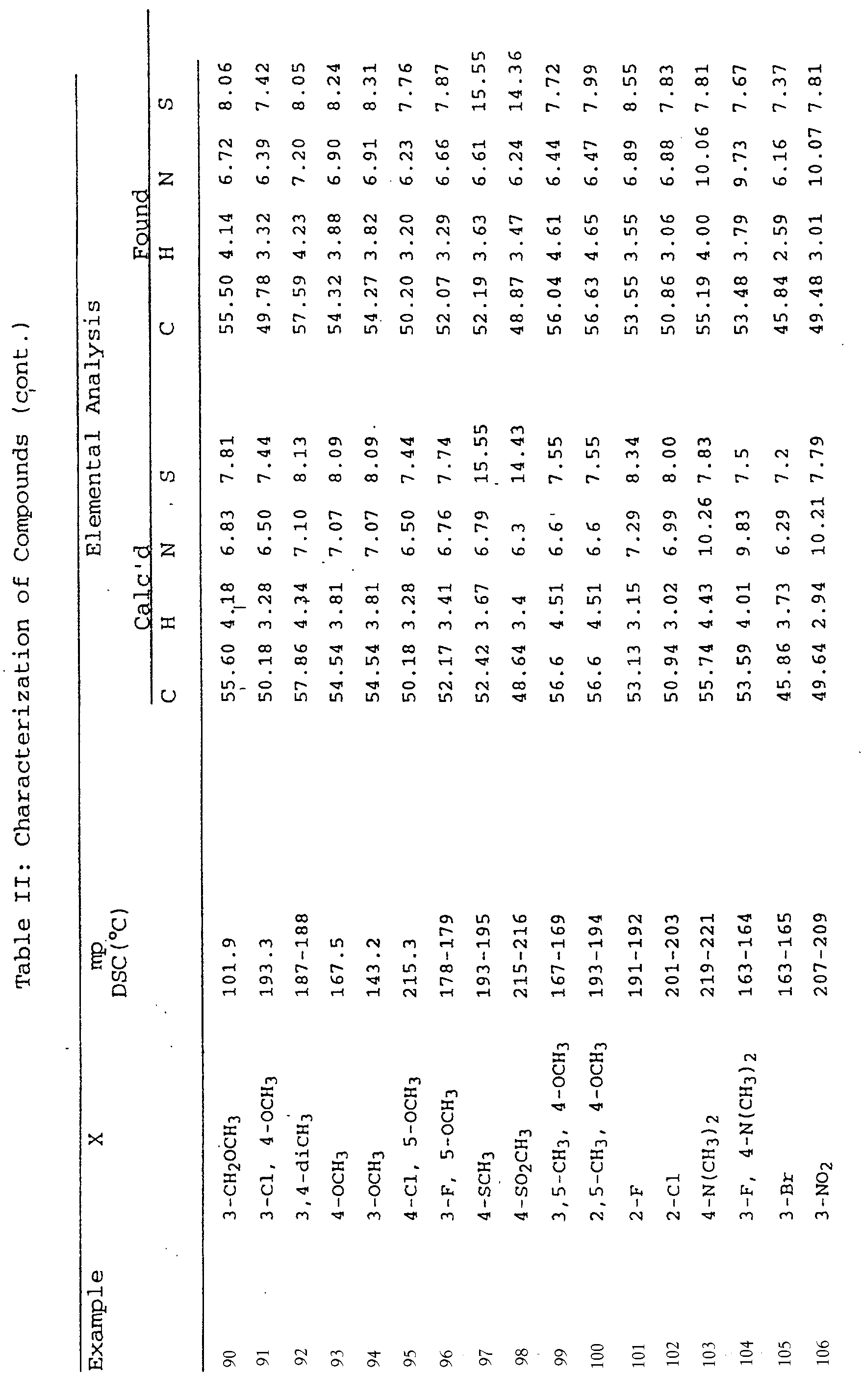

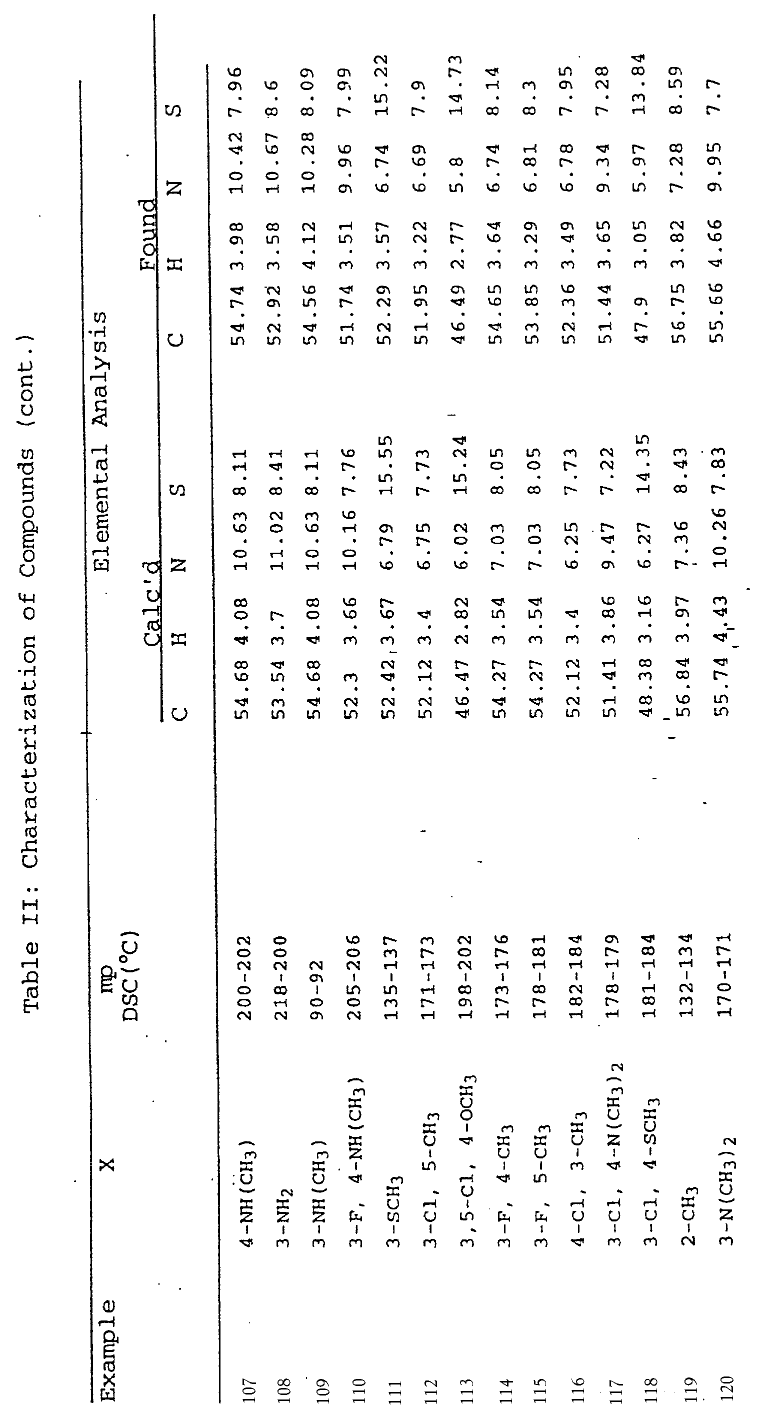

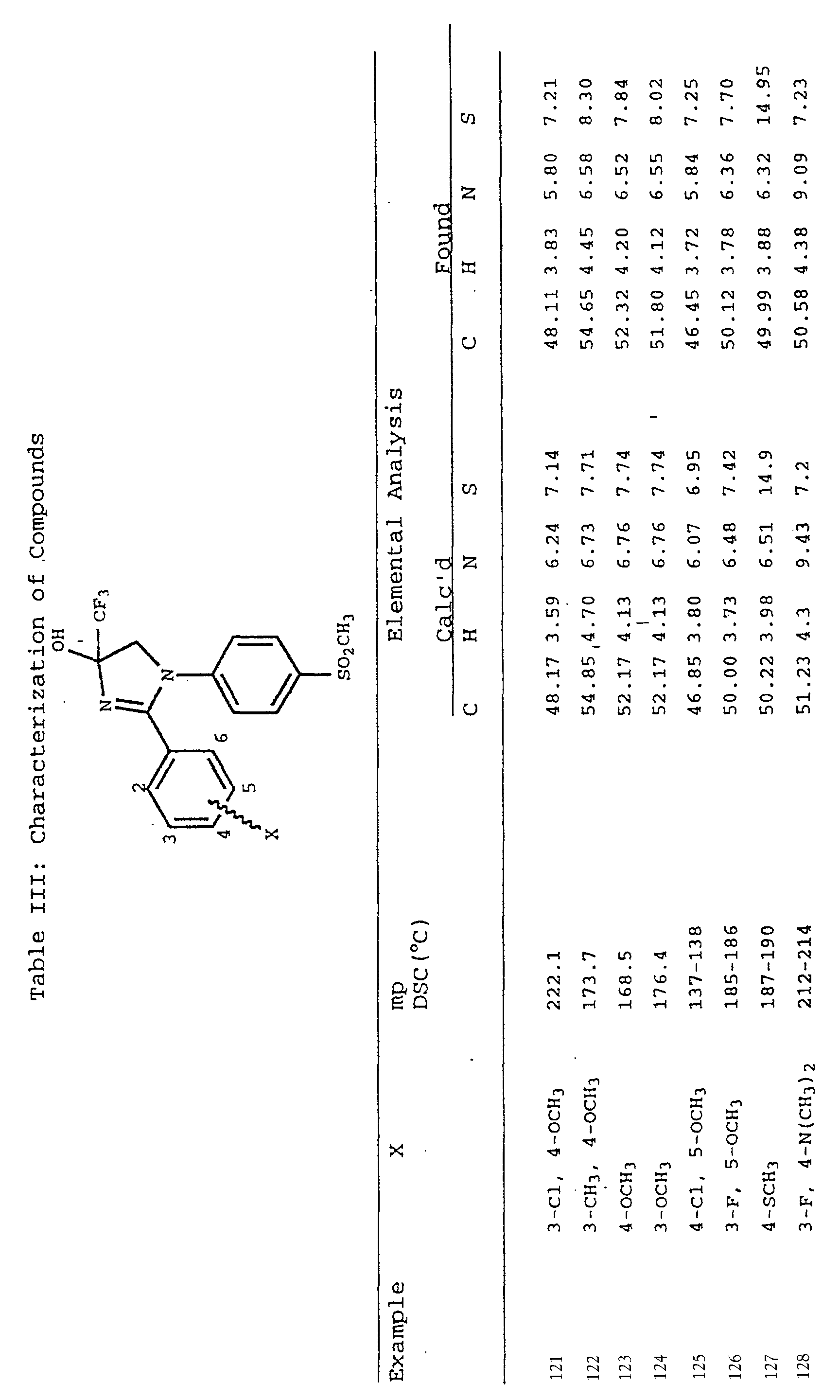

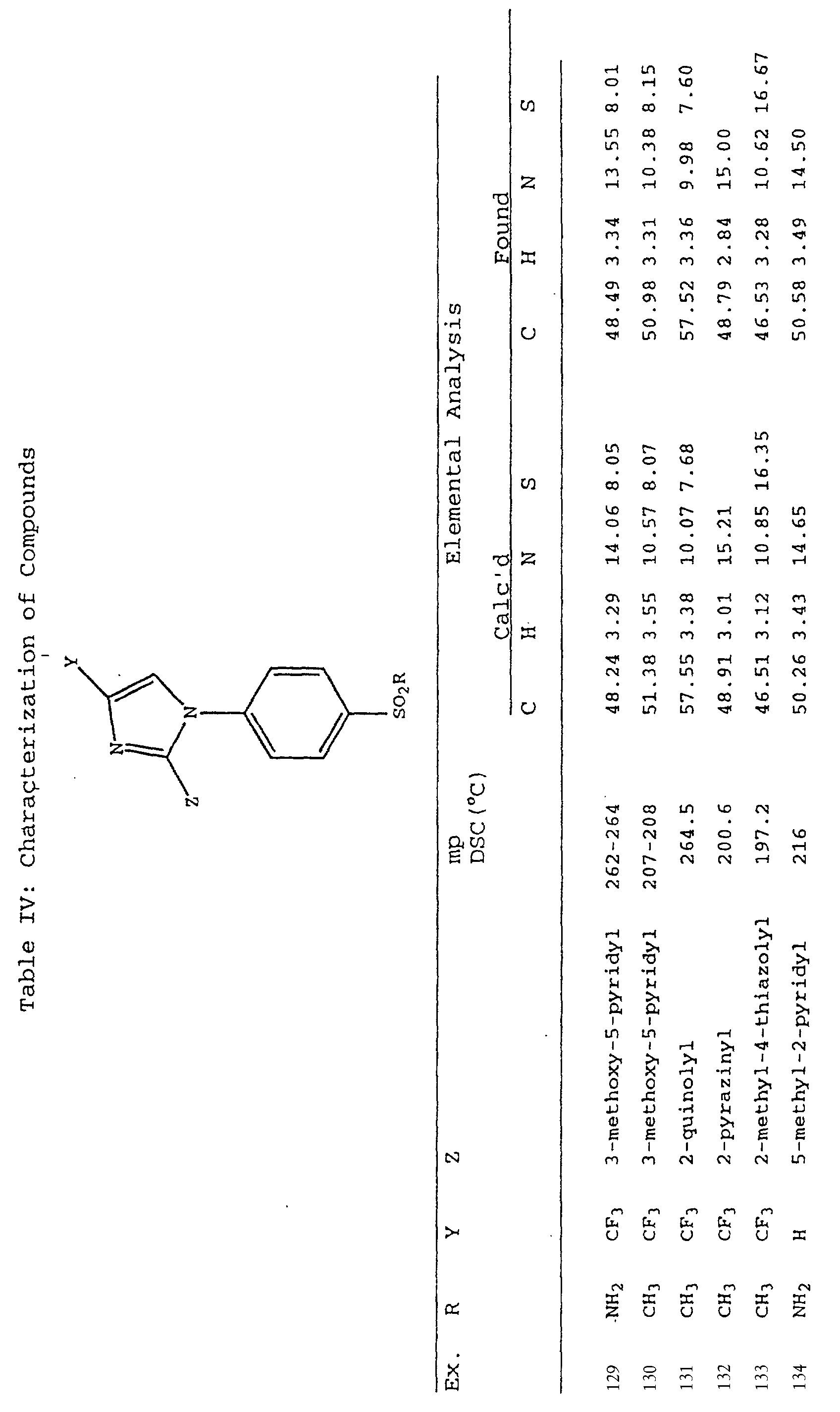

### Example 135

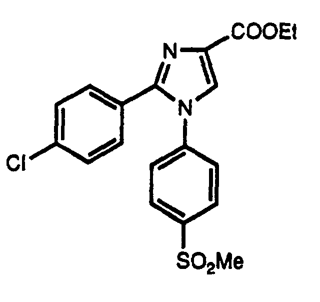

### Ethyl [2-(4-chlorophenyl)-1-[4-(methylsulfonyl)phenyl]-1H-imidazol-4-yl] carboxylate

A mixture of 4-chloro-N-[4-(methylsulfonyl)phenyl] benzenecarboximidamide (Example 1, step 1) (12.1 g, 39.2 mmol), sodium bicarbonate (6.58 g, 78.3 mmol), and 90% ethyl bromopyruvate (16.9 g) in 480 ml of 2-propanol was stirred at reflux overnight. After cooling, the mixture was concentrated. The residue was partitioned between dichloromethane and water and the aqueous layer further extracted with dichloromethane. The combined organic extracts were dried over sodium sulfate, filtered, and evaporated. Trituration of the residue with ethyl acetate gave the title compound as a pale beige, crystalline solid (6.61 g): mp (DSC) 218°C. Anal. Calc'd. for C₁₉H₁₇ClN₂O₄S (MW 404.87): C, 56.37; H, 4.23; N, 6.92. Found: C, 56.28; H, 4.13; N, 6.80.

### Example 136

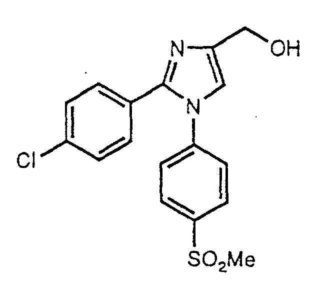

### 2- (4-Chlorophenyl)-1-[4-(methylsulfonyl)phenyl]-1H-imidazole-4-methanol

To a solution of 4.00 g (9.88 mmol) of Example 138 in 125 ml of dichloromethane stirring in a dry ice/isopropanol bath was added 24.7 ml of 1M diisobutyl aluminum hydride in toluene (containing 24.7 mmol). The mixture was warmed to room temperature overnight. Excess reagent was quenched with methanol, and the resulting mixture was washed with 15% aqueous acetic acid. The aqueous layer was further extracted with dichloromethane, and the combined organic extracts were dried over sodium sulfate. Alter filtration and evaporation, the residue was triturated with 50% ethyl acetate/hexane, and the alcohol was obtained as a white solid: m.p. 205-208°C. Anal. Calc'd. for C₁₇H₁₅ClN₂O₃S•1/2 H₂O (MW 371.84): C, 54.91; H, 4.07; N, 7.53. Found: C, 54.75; H, 3.96; N, 7.17.

### Example 137

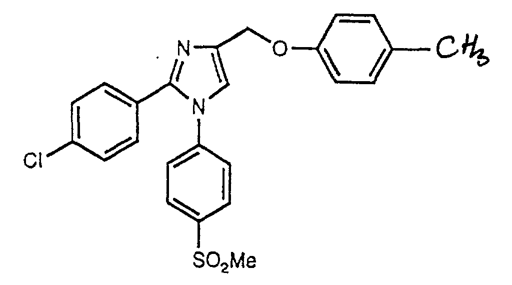

### 2- (4-Chlorophenyl)-4- [(4-methylphenoxy)methyl]-1-[4-(methylsulfonyl)phenyl]-1H-imidazole

### Step 1: Preparation of 4-chloromethyl-2-(4-chlorophenyl)-1-(4-(methylsulfonyl)phenyl]-1H-imidazole

A suspension of the title product of Example 139 (1.82 g, 4.96 mmol) in 10 ml of chloroform was treated with thionyl chloride (1.18 g, 9.92 mmol), and the resulting mixture was stirred at reflux for 1 hour. Another 1.18 g of thionyl chloride was added, and reflux continued for 1 hour. After cooling, the mixture was evaporated and the residue was chromatographed over silica gel using 50% ethyl acetate/hexane as eluent to give the chloromethyl compound as a very pale yellow crystalline solid (1.26 g): m.p. 166-169°C.

### Step 2: Preparation of 2-(4-Chlorophenyl)-4-[(4-methylphenoxy)methyl]-1-[4-(methylsulfonyl)phenyl]-1H-imidazole

A mixture of 122 mg (0.32 mmole) of 4-chloromethyl-2- (4-chlorophenyl)-1-[4-(methylsulfonyl)phenyl]-1H-imidazole (Step 1), p-cresol (69 mg, 0.64 mmole), and potassium carbonate (110 mg, 0.8 mmole) in 5 ml of dimethylformamide was stirred at 85-90 °C for 6 hours. After cooling, the mixture was partitioned between ethyl acetate and aqueous sodium chloride, and the aqueous layer was extracted with ethyl acetate. The combined organic extracts were washed with brine, dried over sodium sulfate, filtered, and evaporated. Chromatography of the residue over silica gel using mixtures of ethyl acetate and hexane gave 2-(4-chlorophenyl)-4-[(4-methylphenoxy)methyl]-1-[4-(methylsulfonyl)phenyl]-1H-imidazole as a pure white solid (118 mg): m.p. (DSC) 193 °C. Anal. Calc'd. for C₂₄H₂₁ClN₂O₃S (MW 452.96): C, 63.64; H, 4.67; N, 6.18. Found: C, 63.42; H, 4.64; N, 5.79.

### Example 138

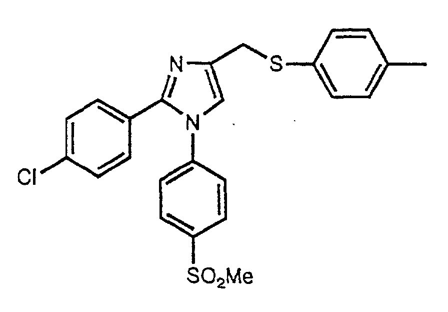

### 2-(4-Chlorophenyl)-4-[[(4-methylphenoxy)thio]methyl]-1-[4-(methylsulfonyl)phenyl]-1H-imidazole

To a solution of 4-chloromethyl-2-(4-chlorophenyl)-1-[4-(methylsulfonyl)phenyl]-1H-imidazole (Example 140, Step 1) in 5 ml of dimethylformamide was added p-thiocresol (98 mg, 0.79 mmole) and anhydrous potassium carbonate (136 mg, 0.985 mmole), and the mixture was stirred rapidly overnight. The mixture was partitioned between ethyl acetate and water, and the aqueous layer was extracted with ethyl acetate The combined organic extracts were dried over sodium sulfate, filtered and evaporated. Chromatography of the residue over silica gel using mixtures of ethyl acetate and hexane as eluent gave the title compound as a glassy solid: m.p. (DSC) 51 °C. Anal. Calc'd. for C₂₄H₂₁ClN₂O₂S₂ (MW 469.03): C, 61.46; H, 4.51; N, 5.97. Found: C, 61.38; H, 4.68; N, 5.81.

### Example 139

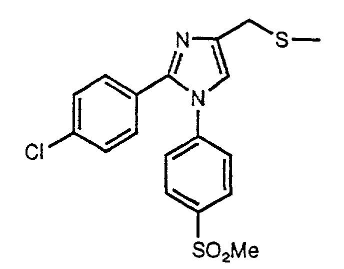

### 2-(4-Chlorophenyl)-1-[4-(methylsulfonyl)phenyl]-4-[(4-methylthio)methyl]-1H-imidazole

To a solution of of 4-chloromethyl-2-(4-chlorophenyl)-1-[4-(methylsulfonyl)phenyl]-1H-imidazole (Example 140, Step-1) (150 mg, 0.394 mmole) in 5 ml of dimethylformamide was added sodium thiomethoxide (55 mg, 0.79 mmole) and the mixture was stirred for three days. The mixture was partitioned between ethyl acetate and water, and the aqueous layer was extracted with ethyl acetate. The combined organic extracts were washed with brine, dried over sodium sulfate, filtered, and evaporated. Chromatography of the residue over silica gel using mixtures of ethyl acetate and hexane as eluents gave the title compound as a pale yellow oil (64 mg) : Anal. Calc'd. for C₁₈H₁₇ClN₂O₂S₂•1/2 H₂O (MW 401.93): C, 53.79; ⁻H, 4.26; N, 6.97. Found: C, 53.97; H, 4.43; N, 6.84.

### Example 140

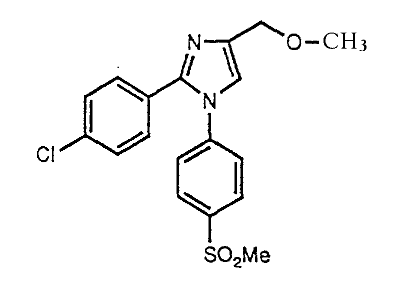

### 2-(4-Chlorophenyl)-4-(4-methoxymethyl)-1-[4-(methylsulfonyl)phenyl]-1H-imidazole

To a solution of 46 mg (2.0 mmol) of sodium metal in 2 ml of methanol was added a solution of 4-chloromethyl-2- (4-chlorophenyl)-1-[4-(methylsulfonyl)phenyl]-1H-imidazole (Example 140, Step 1) (167 mg, 0.438 mmole) and the mixture was stirred overnight. The mixture was partitioned between ethyl acetate and water, and the aqueous layer was extracted with ethyl acetate. The combined organic extracts were washed with brine, dried over sodium sulfate, filtered, and evaporated. Chromatography of the residue over silica gel using ethyl acetate as the eluent gave the title compound as a white crystalline solid (72%): mp 171-172 °C. Anal. Calc'd. for C₁₈H₁₇ClN₂O₃S (MW 376.86): C, 57.37; H, 4.55; N, 7.43. Found: C, 57.29; H, 4.42; N, 7.33.

### Example 141

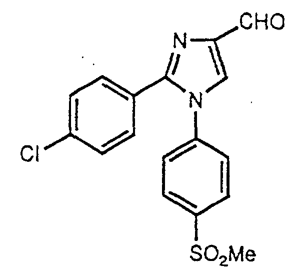

### 2-(4-chlorophenyl)-1-[4-(methylsulfonyl)phenyl]-1H-imidazole-4-carboxaldehyde

To 8 ml of a 1:1 mixture of dimethyl sulfoxide and dichloromethane stirring in a dry ice/isopropanol bath under nitrogen was added dropwise oxalyl chloride (321 µl, 3.69 mmol). After stirring for 10 minutes, a solution of 2-(4-chlorophenyl)-1-[4-(methylsulfonyl]phenyl]-1H-imidazole-4-methanol (Example 139) (670 mg, 1.85 mmol) in 25 ml of a 1:1 mixture of dimethyl sulfoxide and dichloromethane. Stirring was continued while warming to 0 °C, where it was maintained for 15 minutes. Triethylamine (1.87 g, 18.5 mmol) was added, and the mixture was stirred overnight while warming to room temperature. The mixture was partitioned between dichloromethane and water, the organic layer was washed with water and then brine, dried over sodium sulfate, filtered, and evaporated. Chromatography of the residue over silica gel using mixtures of ethyl acetate and hexane gave the title compound as an off-white solid (330 mg): mp (DSC) 203 °C. Anal. Calc'd. for C₁₇H₁₃ClN₂O₃S (MW 360.82): C, 56.59; H, 3.63; N, 7.76. Found: C, 56.24; H, 3.62; N, 7.50.

### Example 142

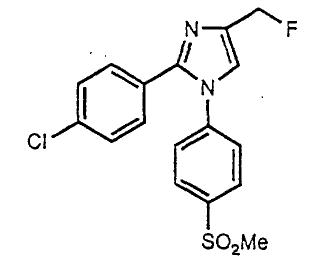

### 2-(4-Chlorophenyl)-4-fluoromethyl-1-[4-(methylsulfonyl)phenyl]-1H-imidazole

To a suspension of 2-(4-chlorophenyl)-1-[4-(methylsulfonyl)phenyl]-1H-imidazole-4-methanol (Example 139) (250 mg, 0.689 mmole) in 5 ml of dichloromethane was added dropwise a solution of diethylamino sulfur trifluoride (DAST) (166 mg, 1.03 mmole) in 1 ml of dichloromethane. As the addition proceeded, the mixture became homogeneous. After stirring for two hours, water was added, the layers separated and the aqueous layer was extracted with dichloromethane. The combined organic extracts were dried over sodium sulfate, filtered and evaporated. Chromatography of the residue over silica gel using 60% ethyl acetate in hexane gave the title compound as a very slightly yellow solid (106 mg): mp (DSC) 165 °C. Anal. Calc'd. for C₁₇H₁₄ClFN₂O₂S•1/4 H₂O (MW 369.33): C, 55.29; H, 3.82; N, 7.59. Found: C, 55.15; H, 3.82; N, 7.42.

### Example 143

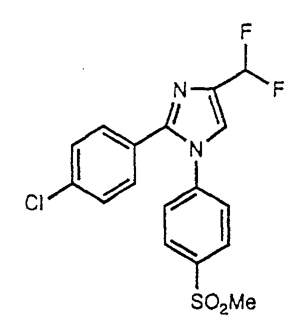

### 2-(4-Chlorophenyl)-4-difluoromethyl-1-[4-(methylsulfonyl)phenyl]-1H-imidazole

To a suspension of 2-(4-chlorophenyl)-1-[4-(methylsulfonyl)phenyl]-1H-imidazole-4-carboxaldehyde (Example 144) (150 mg, 0.416 mmole) in 5 ml of dichloromethane was added dropwise a solution of DAST (201 mg, 1.25 mmol) of in 1 ml of dichloromethane, producing a homogeneous solution. After stirring at room temperature for 30 minutes, the mixture was partitioned between dichloromethane and water, and the aqueous layer was extracted with dichloromethane. The combined organic extracts were dried over sodium sulfate, filtered, and evaporated. Chromatography of the residue over silica gel using 50% ethyl acetate in hexane as the eluent, followed by crystallization from ethyl acetate and hexane gave the title compound as very small pale beige plates (21 mg): m.p. 179-180 °C. Anal. Calc'd. for C₁₇H₁₃ClF₂N₂O₂S (MW 382.82): C, 53.34; H, 3.42; N, 7.32. Found: C, 53.42; H, 3.26; N, 7.08.

### Example 144

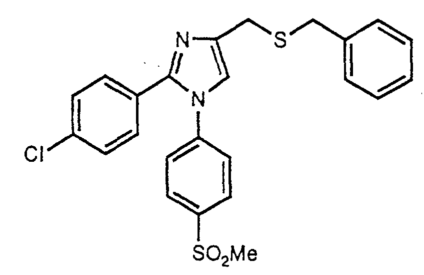

### 2-(4-Chlorophenyl)-1-[4-(methylsulfonyl)phenyl]-4-[[(4-phenylmethyl)thio]methyl]-1H-imidazole

To a solution of benzyl mercaptan (195 mg, 1.6 mmol) in 5 ml of dimethylformamide was added 63 mg of a 60% dispersion of sodium hydride in mineral oil. After gas evolution ceased, 4-chloromethyl-2-(4-chlorophenyl)-1-[4-(methylsulfonyl)phenyl]-1H-imidazole (Example 140, Step 1) (300 mg, 0.787 mmole) was added as a solid and stirring continued overnight. The mixture was partitioned between ethyl acetate and water, and the aqueous layer was extracted with ethyl acetate. The combined organic extracts were washed with brine, dried over sodium sulfate, filtered, and evaporated. Radial chromatography of the residue over a 2 mm layer of silica gel using 50% ethyl acetate in hexane as the eluent gave the title compound as a pale yellow solid (343 mg): mp(DSC) 41°C. Anal. Calc'd. for C₂₄H₂₁ClN₂O₂S₂ (MW 469.03): C, 61.46; H, 4.51; N, 5.97. Found: C, 61.06; H, 4.34; N, 5.80.

### Example 145

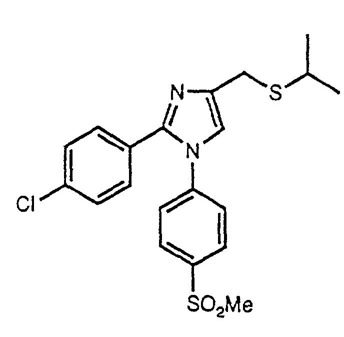

### 2-(4-Chlorophenyl)-4-[[(1-methylethyl)thio]methyl]-1-[4-(methylsulfonyl)phenyl]-1H-imidazole

The title compound was prepared as a white solid by the method of Example 148 except that 2-mercaptopropane was used in place of benzyl mercaptan: mp (DSC) 118 °C. Anal. Calc'd. for C₂₀H₂₁ClN₂O₂S₂ (MW 420.98): C, 57.06; H, 5.03; N, 6.65. Found: C, 56.72; H, 4.89; N, 6.42.

### Example 146

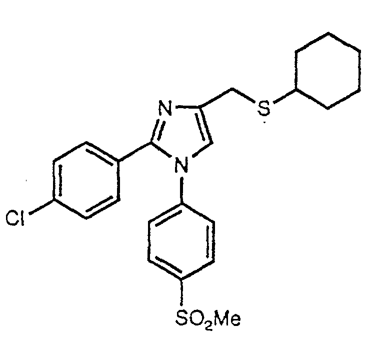

### 2-(4-Chlorophenyl)-4-[[(cyclohexyl)thio]methyl]-1-[4-(methylsulfonyl)phenyl]-1H-imidazole

The title compound was prepared as a white solid by the method of Example 148 except that cyclohexyl mercaptan was used in place of benzyl mercaptan, and that 40% ethyl acetate in hexane was used as the chromatography eluent: mp (DSC) 48 °C. Anal. Calc'd. for C₂₃H₂₅ClN₂O₂S₂ (MW 461.05): C, 59.92; H, 5.47; N, 6.08. Found: C, 59.63; H, 5.52; N, 5.96.

### Example 147

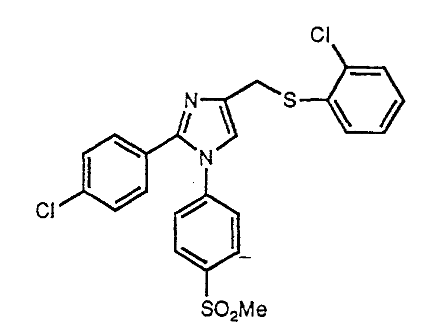

### 2-(4-Chlorophenyl)-4-[[(2-chlorophenyl)thio]methyl]-1-[4-(methylsulfonyl)phenyl]-1H-imidazole

To a solution of 4-chloromethyl-2-(4-chlorophenyl)-1-[4-(methylsulfonyl)phenyl]-1H-imidazole (Example 140, Step 1) (250 mg, 0.656 mmol) and 2-chlorothiophenol (190 mg, 1.31 mmol) in 5 ml of dimethylformamide was added potassium carbonate (226 mg, 1.64 mmol). The mixture was stirred rapidly overnight at room temperature and partitioned between ethyl acetate and water. The aqueous layer was extracted with ethyl acetate, the combined organic extracts were washed with brine, and dried over sodium sulfate. The solution was filtered and then concentrated. Chromatography of the residue over silica gel using 50% ethyl acetate in hexane as the eluent followed by crystallization gave the title compound as a pure white crystalline solid (147 mg): mp (DSC) 153 °C. Anal. Calc'd. for C₂₃H₁₈Cl₂N₂O₂S₂ (MW 489.44): C, 56.44; H, 3.71; N, 5.72. Found: C, 56.51; H, 3.54; N, 5.57.

### Example 148

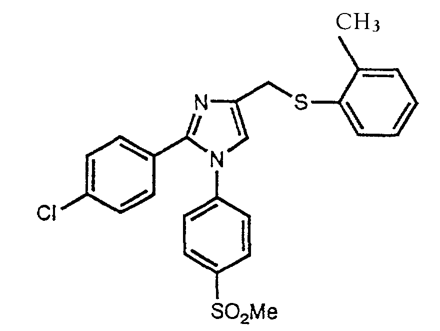

### 2- (4-Chlorophenyl) -4-[[(2-methylphenyl)thio]methyl]-1-[4-(methylsulfonyl)phenyl]-1H-imidazole

To a solution of 4-chloromethyl-2-(4-chlorophenyl)-1-[4-(methylsulfonyl)phenyl]-1H-imidazole (Example 140, Step 1) (250 mg, 0.656 mmol) and o-thiocresol (163 mg, 1.31 mmol) in 5 ml of dry dimethylformamide was added anhydrous potassium carbonate (226 mg, 1.64 mmol). The mixture was stirred rapidly overnight at room temperature. The mixture was then partitioned between ethyl acetate and water. The aqueous layer was further extracted with ethyl acetate, the combined organic extracts washed with brine, and dried over sodium sulfate. The solution was filtered and concentrated. Chromatography of the residue over silica gel using 40% ethyl acetate in hexane gave the title compound as a very pale yellow solid (210 mg): mp (DSC) 51 °C. Anal. Calc'd. for C₂₄H₂₁ClN₂O₂S₂ (MW 469.03): C, 61.46; H, 4.51; N, 5.97. Found: C, 61.16; H, 4.50; N, 5.86.

### Example 149

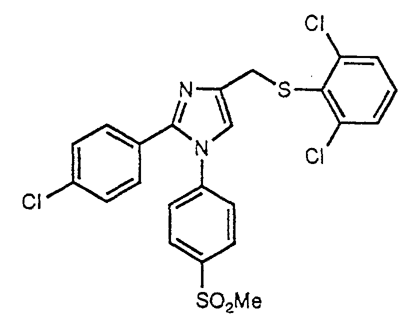

### 2-(4-Chlorophenyl)-4-[[(2,6-dichlorophenyl)thio]methyl]-1-[4-(methylsulfonyl)phenyl]-1H-imidazole

To a solution of 4-chloromethyl-2-(4-chlorophenyl)-1-[4-(methylsulfonyl)phenyl]-1H-imidazole (Example 140, Step 1) (250 mg, -0.656 mmole) and 2,6-dichlorothiophenol in 5 ml of dimethylformamide (235 mg, 1.31 mmol) was added 226 mg (1.64 mmol) of potassium carbonate. The resulting mixture was stirred rapidly at room temperature for two days. The mixture was partitioned between ethyl acetate and water and the aqueous layer was further extracted with ethyl acetate. The combined organic extracts were washed with brine, dried over sodium sulfate, filtered, and evaporated. Chromatography of the residue over silica gel using 50% ethyl acetate in hexane gave the title compound, 282 mg, as a pure white solid: mp (DSC) 202 °C. Anal. Calc'd. for C₂₃H₁₇Cl₃N₂O₂S₂ (MW 523.89): C, 52.73; H, 3.27; N, 5.35. Found: C, 52.55; H, 2.98; N, 5.19.

### Example 150

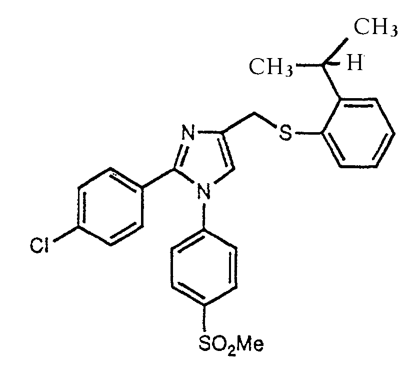

### 2-(4-Chlorophenyl)-4-[[[2-(1-methylethyl)phenyl]thio]methyl]-1-[4-(methylsulfonyl)phenyl]-1H-imidazole

The title compound was prepared as a white solid by the method of Example 153 except that 2-isopropylthiophenol was used in place of 2,6-dichlorothiophenol and that 40% ethyl acetate in hexane was used as chromatography eluent: m.p. 68-70 °C. Anal. Calc'd. for C₂₆H₂₅ClN₂O₂S₂·1/4H₂O (MW 501.58): C, 62.26; H, 5.02; N, 5.59. Found: C, 62.36; H, 5.11; N, 5.45.

### Example 151

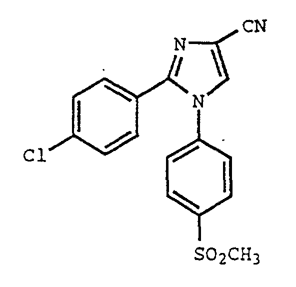

### 2-(4-Chlorophenyl)-1-[4-(methylsulfonyl)phenyl]-1H-imidazole-4-carbonitrile

A solution of 82 mg (0.23 mmole) of 2-(4-chlorophenyl)-1-[4-(methylsulfonyl)phenyl]-1H-imidazole-4-carboxaldehyde (Example 144) and 51 mg (0.45 mmole) of hydroxylamine O-sulfonic acid in 10 ml of absolute ethanol and 1 ml of pyridine was stirred at reflux overnight. After cooling, the mixture was evaporated, and the residue was taken up in dichloromethane. The solution was washed with aqueous sodium bicarbonate, dried over sodium sulfate, filtered, and evaporated. Chromatography of the residue over silica gel using 50% ethyl acetate in hexane as the eluent gave the title compound, 71 mg, as a pure white crystalline solid: mp (DSC) 205 °C. Anal. Calc'd. for C₁₇H₁₂ClN₃O₂S•1/4 H₂O (MW 362.32): C, 56.36; H, 3.34; N, 11.60. Found: C, 56.49; H, 3.27; N, 11.45.

### Example 152

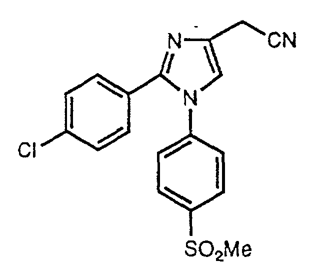

### 2-(4-Chlorophenyl)-1-[4-(methylsulfonyl)phenyl]-1H-imidazole-4-acetonitrile

A mixture of 250 mg (0.656 mmole) of 4-chloromethyl-2-(4-chlorophenyl)-1-[4-(methylsulfonyl)phenyl]-1H-imidazole (Example 140, Step 1) and 86 mg (1.3 mmol) of potassium cyanide in 4 ml of dimethylformamide was stirred at 85 °C for 24 hours. An additional 86 mg of potassium cyanide was added, and stirring continued for 8 hours. After cooling, the mixture was partitioned between dichloromethane and water and the aqueous layer further extracted with dichloromethane. The combined organic extracts were washed with brine, dried over sodium sulfate, filtered, and evaporated. Chromatography of the residue over silica gel using 60% ethyl acetate in toluene, followed by trituration with ethyl acetate gave the title compound, 59 mg, as a very pale yellow crystalline solid: mp (DSC) 197 °C. Anal. Calc'd. for C₁₈H₁₄ClN₃O₂S (MW 371.85): C, 58.14; H, 3.80; N, 11.30. Found: C, 57.92; H, 3.57; N, 11.01.

### Example 153

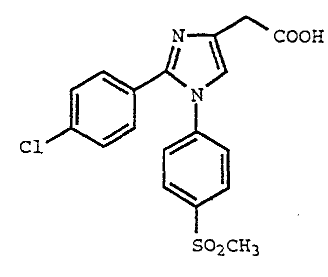

### 2-(4-Chlorophenyl)-1-[4-(methylsulfonyl)phenyl]-1H-imidazole-4-acetic acid

A mixture of 50 mg (0.13 mmole) of the title product of Example 156 and 5 ml of concentrated hydrochloric acid was stirred at reflux for one hour. After cooling, the mixture was evaporated and the residue taken up in water. The mixture was basified with aqueous sodium bicarbonate solution, and the pH then adjusted to 4 with acetic acid. The mixture was extracted with dichloromethane and the combined organic extracts dried over sodium sulfate. After filtration, the solution was evaporated and the residue azeotropically distilled with toluene. Trituration of the residue with ethyl acetate gave the title compound, 31 mg, as a white solid: m.p. 263-264 °C. Anal. Calc'd. for C₁₈H₁₅ClN₂O₄S•1/4H₂O (MW 395.35): C, 54.69; H, 3.82; N, 7.09. Found: C, 54.39; H, 3.88; N, 6.72.

### Example 154

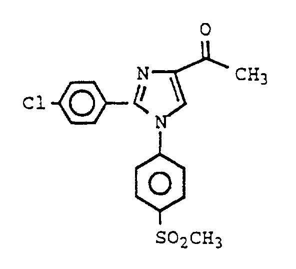

### 1-[2-(4-Chlorophenyl)-1-[4-(methylsulfonyl)phenyl]-1H-imidazol-4-yl]-1-ethanone

### Step 1- Preparation of 2-(4-chlorophenyl)-1-[4-(methylsulfonyl)phenyl]-1H-imidazole-4-carboxylic acid

A suspension of ethyl [2-(4-chlorophenyl)-1-[4-(methylsulfonyl)phenyl]-1H-imidazol-4-yl] carboxylate (Example 138) (929 mg, 2.29 mmol) in 16 ml of methanol and 16 ml of 1N aqueous sodium hydroxide was stirred at reflux for one hour. After cooling, the mixture was concentrated, water was added, and the resulting mixture was acidified with acetic acid. The mixture was extracted with dichloromethane, and the combined organic extracts were dried over sodium sulfate, filtered, and evaporated. Acetic acid was removed by azeotropic distillation with toluene to give the title compound, 520 mg, as a white crystalline solid: mp (DSC) 121 °C. Anal. Calc'd. for C₁₇H₁₃ClN₂O₄S•H₂O (MW 394.83): C, 51.71; H, 3.32; N, 7.10. Found: C, 51.89; H, 3.29; N, 6.97.

### Step 2 - Preparation of 2-(4-chlorophenyl)-N-methoxy-N-methyl-1-[4-(methylsulfonyl)phenyl]-1H-imidazole-4-carboxamide

Oxalyl chloride (0.34 g, 2.65 mmole) in 5 ml acetonitrile was added to 16 ml acetonitrile containing dimethylformamide (0.25 g, 3.46 mmole) cooled to 0°C. After 15 minutes, 2-(4-chlorophenyl)-1-[4-(methylsulfonyl)phenyl]-1H-imidazole-4-carboxylic acid from step 1 (1.0 g, 2.65 mmole) was added with 20 ml acetonitrile. After warming to room temperature N,0-dimethylhydroxylamine HCl (0.28 g, 2.92 mmole) and pyridine (0.42 g, 5.31 mmole) were added and the mixture was stirred at room temperature for three days. The reaction mixture was concentrated to give an oily solid. The amide was purified by silica gel chromatography: Anal. Calc'd. C₁₉H₁₈N₃O₄SCl (419.89); C, 54.35; H⁻, 4.32; N, 10.01. Found: C, 53.96; H, 4.30; N, 9.68.

### Step 3 - Preparation of 1-[2-(4-chlorophenyl)-1-[4-(methylsulfonyl)phenyl]-1H-imidazol-4-yl]-1-ethanone

Methyl lithium·LiBr complex (1.5 M in ethyl ether) (0.47 ml, 0.7 mmol) was added by syringe to a cold (-70°C) solution of 2-(4-chlorophenyl)-N-methoxy-N-methyl-1-[4-(methylsulfonyl)phenyl]-1H-imidazole-4-carboxamide from step 2 (250 mg, 0.6 mmol) in 50 ml tetrahydrofuran. The reaction was warmed to 0°C and re-cooled to -60°C before additional, methyl lithium (0.47 ml) was added. The reaction was warmed to room temperature. After stirring for two days, 50 ml of 10% acetic acid was added and the mixture was concentrated to a gum. The gum was dissolved in 50 ml ethyl acetate, washed with water (2 x 50 ml), dried over Na₂SO₄, filtered and concentrated. The product was purfied by silica gel chromatography: Anal. Calc'd. C₁₈H₁₅N₂O₃SCl·1/4 H₂O: C, 56.99; H, 4.12; N, 7.38. Found: C, 56.88; H, 4.05; N, 7.6838

### Example 155

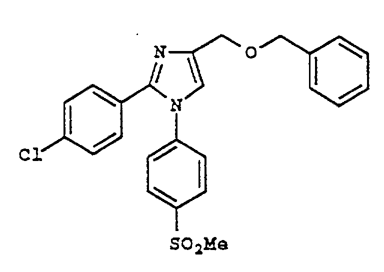

### 2-(4-Chlorophenyl)-1-[4-(methylsulfonyl)phenyl]-4-(phenylmethoxymethyl)-1H-imidazole

To a suspension of 58 mg of 60% sodium hydride in mineral oil (containing 35 mg, 1.4 mmol) in 2 ml of dimethylformamide was added a solution of 142 mg (1.31 mmol) of benzyl alcohol in 0.5 ml of dimethyl formamide. The mixture was stirred while heating to 40°C. After 15 minutes, 250 mg (0.656 mmole) of the title product of Example 139 was added as a solid and stirred while heating to 85°C. The temperature was maintained for 6 hours and then the mixture was cooled. The mixture was partitioned between ethyl acetate and water and the aqueous layer further extracted with ethyl acetate. The combined organic extracts were washed with brine, dried over sodium sulfate, filtered, and evaporated. Chromatography of the residue over silica gel using 50% ethyl acetate in hexane as the eluent gave the title compound, 60 mg, as pure white crystalline solid, m.p. 64-65°C. Anal. Calc'd. for C₂₄H₂₁ClN₂O₃S•1/4H₂O (MW 457.56): C, 63.01; H, 4.63; N, 6.12. Found: C, 62.76; H, 4.43; N, 6.20.

### BIOLOGICAL EVALUATION

### Rat Carrageenan Foot Pad Edema Test

The carrageenan foot edema test was performed with materials, reagents and procedures essentially as described by Winter, et al., (*Proc. Soc. Exp. Biol. Med.,* **111**, 544 (1962)). Male Sprague-Dawley rats were selected in each group so that the average body weight was as close as possible. Rats were fasted with free access to water for over sixteen hours prior to the test. The rats were dosed orally (1 ml) with compounds suspended in vehicle containing 0.5% methylcellulose and 0.025% surfactant, or with vehicle alone. One hour later a subplantar injection of 0.1 ml of 1% solution of carrageenan/sterile 0.9% saline was administered and the volume of the injected foot was measured with a displacement plethysmometer connected to a pressure transducer with a digital indicator. Three hours after the injection of the carrageenan, the volume of the foot was again measured. The average foot swelling in a group of drug-treated animals was compared with that of a group of placebo-treated animals and the percentage inhibition of edema was determined (Otterness and Bliven, *Laboratory Models for Testing NSAIDs,* in *Non-steroidal Anti-Inflammatory Drugs,* (J. Lombardino, ed. 1985)). The % inhibition shows the % decrease from control paw volume determined in this procedure and the data for selected compounds in this invention are summarized in Table V.

### Rat Carrageenan-induced Analgesia Test

The rat carrageenan analgesia test was performed with materials, reagents and procedures essentially as described by Hargreaves, et al., (*Pain,* **32**, 77 (1988)). Male Sprague-Dawley rats were treated as previously described for the Carrageenan Foot Pad Edema test.

Three hours after the injection of the carrageenan, the rats were placed in a special plexiglass container with a transparent floor having a high intensity lamp as a radiant heat source, positionable under the floor. After an initial twenty minute period, thermal stimulation was begun on either the injected foot or on the contralateral uninjected foot. A photoelectric cell turned off the lamp and timer when light was interrupted by paw withdrawal. The time until the rat withdraws its foot was then measured. The withdrawal latency in seconds was determined for the control and drug-treated groups, and percent inhibition of the hyperalgesic foot withdrawal determined. Results are shown in Table V.

**TABLE V.**

| | **RAT PAW EDEMA** | **ANALGESIA** |
|---|---|---|
| | % Inhibition @ 30mg/kg body weight | % Inhibition @ 10mg/kg body weight |
| Example | | |
| 2 | 9 | |
| 5 | 21 | |
| 6 | 23.5 | |
| 7 | 27 | |
| 18 | 36 | 13 |
| 21 | 38 | 25 |
| 22 | 24 | 19 |
| 24 | 51 | 47 |
| 25 | 40 | 21 |
| 26 | 57 | 51 |
| 27 | 37 | |
| 29 | 28 | 36 |
| 30 | 30 | |
| 34 | 68 | |
| 38 | 42 | |
| 41 | 45* | 18 |
| 43 | 49 | 47 |
| 57 | 34 | 27 |
| 67 | 43 | 32 |
| 68 | 34* | 35 |
| 70 | 55 | 28 |
| 72 | 48 | |
| 85 | 25 | |
| 84 | 36 | 7 |
| 85 | 28* | 5 |
| 89 | 16 | |
| 91 | 16 | 4 |
| 115 | 51 | |

| | | |
|---|---|---|
| * 10 mg/kg | | |

### Evaluation of COX-1 and COX-2 activity in vitro

The compounds of this invention exhibited inhibition *in vitro* of COX-2. The COX-2 inhibition activity of the compounds of this invention illustrated in the Examples was determined by the following methods.

### a. Preparation of recombinant COX baculoviruses

Recombinant COX-1 and COX-2 were prepared as described by Gierse et al, [J. *Biochem.,* **305**, 479-84 (1995)]. A 2.0 kb fragment containing the coding region of either human or murine COX-1 or human or murine COX-2 was cloned into a BamHl site of the baculovirus transfer vector pVL1393 (Invitrogen) to generate the baculovirus transfer vectors for COX-1 and COX-2 in a manner similar to the method of D.R. O'Reilly et al (*Baculovirus Expression Vectors: A Laboratory Manual* (1992)). Recombinant baculoviruses were isolated by transfecting 4 µg of baculovirus transfer vector DNA into SF9 insect cells (2x10⁸) along with 200 ng of linearized baculovirus plasmid DNA by the calcium phosphate method. See M.D. Summers and G.E. Smith, *A Manual of Methods for Baculovirus Vectors and Insect Cell Culture Procedures,* Texas Agric. Exp. Station Bull. 1555 (1987). Recombinant viruses were purified by three rounds of plaque purification and high titer (10⁷ - 10⁸ pfu/ml) stocks of virus were prepared. For large scale production, SF9 insect cells were infected in 10 liter fermentors (0.5 x 10⁶/ml) with the recombinant baculovirus stock such that the multiplicity of infection was 0.1. After 72 hours the cells were centrifuged and the cell pellet homogenized in-Tris/Sucrose (50 mM: 25%, pH 8.0) containing 1% 3-[(3-cholamidopropyl)dimethylammonio] -1-propanesulfonate (CHAPS). The homogenate was centrifuged at 10,000xG for 30 minutes, and the resultant supernatant was stored at -80°C before being assayed for COX activity.

### b. Assay for COX-1 and COX-2 activity

COX activity was assayed as PGE₂ formed/µg protein/time using an ELISA to detect the prostaglandin released. CHAPS-solubilized insect cell membranes containing the appropriate COX enzyme were incubated in a potassium phosphate buffer (50 mM, pH 8.0) containing epinephrine, phenol, and heme with the addition of arachidonic acid (10 µM). Compounds were pre-incubated with the enzyme for 10-20 minutes prior to the addition of arachidonic acid. Any reaction between the arachidonic acid and the enzyme was stopped after ten minutes at 37°C/room temperature by transferring 40 µl of reaction mix into 160 µl ELISA buffer and 25 µM indomethacin. The PGE2 formed was measured by standard ELISA technology (Cayman Chemical). Results are shown in Table VI.

**TABLE VI.**

| **Example** | **Human COX-2** **ID**_{**50**} **µM** | **Human COX-1** **ID**_{**50**} **µM** |
|---|---|---|
| 1 | 4 | >100 |
| 2 | 0.1 | 23 |
| 3 | 40 | >100 |
| 4 | 4.7 | >100 |
| 5 | 0.2 | >100 |
| 6 | 0.3 | >100 |
| 7 | 0.1 | >100 |
| 9 | 0.3 | >100 |
| 10 | 0.5 | >100 |
| 12 | 0.2 | >100 |
| 13 | 1.6 | >100 |
| 14 | 0.2 | >100 |
| 16 | <0.1 | >100 |
| 17 | 0.2 | 1.0 |
| 18 | 0.2 | 49 |
| 21 | 0.1 | >100 |
| 22 | 0.2 | 26 |
| 24 | <0.1 | 1.6 |
| 25 | <0.1 | 0.6 |
| 26 | 1.8 | >100 |
| 27 | 1.5 | >100 |
| 28 | >100 | >100 |
| 29 | 1.8 | >100 |
| 30 | 2.9 | >100 |
| 32 | 0.5 | >100 |
| 33 | 1.2 | 49 |
| 34 | 0.3 | 88.5 |
| 38 | 0.4 | >100 |
| 39 | 0.5 | >100 |
| 41 | 0.5 | >100 |
| 43 | 9.6 | >100 |
| 54 | 0.1 | 3.6 |
| 55 | <0.1 | 0.9 |
| 57 | <0.1 | 3.6 |
| 65 | 1.1 | >100 |
| 66 | 0.2 | 4.6 |
| 67 | <0.1 | 2.8 |
| 68 | <0.1 | 6.2 |
| 70 | <0.1 | 19.3 |
| 71 | <0.1 | 29.8 |
| 72 | <0.1 | 5.8 |
| 73 | <0.1 | 67.7 |
| 74 | <0.1 | 8.6 |
| 76 | <0.1 | 2.7 |
| 77 | 0.1 | 31.2 |
| 78 | <0.1 | 7.0 |
| 79 | <0.1 | 3.6 |
| 80 | <0.1 | >100 |
| 8 | <0.1 | 82.0 |
| 83 | 0.1 | >100 |
| 84 | <0.1 | 78.1 |
| 85 | <0.1 | - >100 |
| 86 | <0.1 | >100 |
| 87 | 0.2 | 24.1 |
| 88 | 0.2 | >100 |
| 89 | 0.2 | >100 |
| 91 | 0.1 | >100 |
| 92 | 0.2 | 29.9 |
| 93 | 0.6 | 3.0 |
| 94 | 0.4 | >100 |
| 95 | 0.3 | >100 |
| 96 | 1.0 | >100 |
| 97 | 0.2 | 2.1 |
| 99 | 0.7 | >100 |
| 101 | 0.5 | >100 |
| 102 | 0.9 | >100 |
| 103 | 0.8 | 4.5 |
| 104 | 0.3 | 17.1 |
| 105 | <0.1 | >100 |
| 106 | 0.6 | >100 |
| 107 | 1.48 | 53.5 |
| 110 | 0.7 | >100 |
| 111 | 0.3 | >100 |
| 112 | <0.1 | >100 |
| 113 | 0.1 | >100 |
| 114 | 0.1 | >100 |
| 115 | 0.1 - | >100 |
| 116 | <0.1 | 7.9 |
| 117 | 0.3 | 1.6 |
| 118 | <0.1 | >100 |
| 137 | <0.1 | >100 |
| 138 | <0.1 | >100 |
| 139 | 0.6 | >100 |
| 141 | 1.6 | >100 |
| 142 | 0.4 | >100 |
| 143 | 0.6 | >100 |
| 144 | 0.1 | >100 |
| 145 | 0.6 | >100 |
| 146 | 0.1 | >100 |
| 147 | <0.1 | >100 |
| 148 | <0.1 | >100 |
| 149 | <0.1 | 13.7 |
| 150 | <0.1 | >100 |
| 151 | 0.3 | >100 |
| 152 | 1.5 | >100 |
| 155 | 0.5 | 100 |

Biological paradigms for testing the cytokine-inhibiting activity of these compounds are found in WO-A-9513067, published 18 May 1995.

Also embraced within this invention is a class of pharmaceutical compositions comprising the active compounds of this combination therapy in association with one or more non-toxic, pharmaceutically-acceptable carriers and/or diluents and/or adjuvants (collectively referred to herein as "carrier" materials) and, if desired, other active ingredients. The active compounds of the present invention may be administered by any suitable route, preferably in the form of a pharmaceutical composition adapted to such a route, and in a dose effective for the treatment intended. The active compounds and composition may, for example, be administered orally, intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The active ingredient may also be administered by injection as a composition wherein, for example, saline, dextrose or water may be used as a suitable carrier.

The amount of therapeutically active compounds that are administered and the dosage regimen for treating a disease condition with the compounds and/or compositions of this invention depends on a variety of factors, including the age, weight, sex and medical condition of the subject, the severity of the disease, the route and frequency of administration, and the particular compound employed, and thus may vary widely. The pharmaceutical compositions may contain active ingredients in the range of 0.1 to 2000 mg, preferably in the range of 0.5 to 500 mg and most preferably between 1 and 100 mg. A daily dose of 0.01 to 100 mg/kg body weight, preferably between 0.5 and 20 mg/kg body weight and most preferably between 0.1 to 10 mg/kg body weight, may be appropriate. The daily dose can be administered in one to four doses per day.

In the case of psoriasis and other skin conditions, it may be preferable to apply a topical preparation of compounds of this invention to the affected area two to four times a day.

For inflammations of the eye or other external tissues, e.g., mouth and skin, the formulations are preferably applied as a topical ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base. Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The antiinflammatory active ingredients are preferably present in such formulations in a concentration of 0.5 to 20%, advantageously 0.5 to 10% and particularly about 1.5% w/w.

For therapeutic purposes, the active compounds of this combination invention are ordinarily combined with one or more adjuvants appropriate to the indicated route of administration. If administered *per* os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

## Claims

1. A compound of Formula (I) 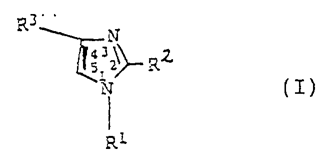 wherein R³ is a radical selected from C₁-C₆-alkyl, C₁-C₆-haloalkyl, aryl-C₁-C₆-alkyl, formyl, C₂-C₆-alkanoyl, cyano, C₁-C₆-alkylthio-C₁-C₆-alkyl, C₃-C₁₀-cycloalkylthio-C₁-C₆-alkyl, C₁-C₆-hydroxyalkyl, C₁-C₆-alkoxy-C₁-C₆-alkyl, arylcarbonyl, aryl-C₁-C₆-alkylcarbonyl, C₁-C₆-cyanoalkyl, C₁-C₆-carboxyalkyl, C₁-C₆-alkoxycarbonyl-C₁-C₆-alkyl, C₁-C₆-alkoxycarbonyl, C₁-C₆-haloalkylcarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxy-C₁-C₆-alkyl and aryl;
wherein R¹ and R² are independently selected from aryl and heteroaryl, wherein R¹ and R² are optionally substituted at a substitutable position with one or more radicals independently selected from C₁-C₆-alkylsulfonyl, aminosulfonyl, halo, C₁-C₆-alkylthio, C₁-C₆-alkyl, cyano, carboxyl, C₁-C₆-alkoxycarbonyl, C₁-C₆-haloalkyl, hydroxyl, C₁-C₆-alkoxy, C₁-C₆-hydroxyalkyl, C₁-C₆-alkoxy-C₁-C₆-alkyl, C₁-C₆-haloalkoxy, amino, mono or di C₁-C₆-alkylamino, arylamino and nitro; provided at least one of R¹ and R² is 4-methylsulfonylphenyl or 4-aminosulfonylphenyl; wherein aryl whenever occurring means a phenyl or naphthyl ring; wherein heteroaryl whenever occuring means an unsaturated heterocyclic radical selected from pyrrolyl, pyrrolinyl, imidazolyl, pyrazolyl, 2-pyridyl, 3-pyridyl, 4-pyridyl, pyrimidyl, pyrazinyl, pyridazinyl, triazolyl tetrazolyl, indolyl, isoindolyl, indolizinyl, benzimidazolyl, quinolyl, isoquinolyl, indazolyl, benzotriazolyl, tetrazolopyridazinyl, pyranyl, 2-furyl, 3-furyl, 2-thienyl, 3-thienyl, oxazolyl, isoxazolyl, oxadiazolyl, benzoxazolyl, benzoxadiazolyl, thiazolyl, thiadiazolyl, benzothiazolyl, benzothiadiazolyl, benzofuran, benzothiophene, wherein said heterocyclic radical may be substituted at a substitutable position with one or more substituents selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and halo-C₁-C₆-alkoxy;
or a pharmaceutically-acceptable salt thereof.

2. Compounds of Formula (I) 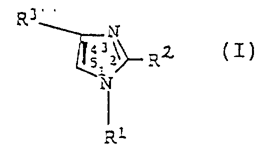 wherein R³ is a radical selected from methyl, ethyl, isopropyl, cert-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, benzyl, phenylethyl, phenylpropyl, formyl, cyano, hydroxymethyl, hydroxyethyl, methoxymethyl, ethoxymethyl, methylthiomethyl, isopropylthiomethyl, cyclohexylthiomethyl, methylcarbonyl, ethylcarbonyl, phenylcarbonyl, trifluoromethylcarbonyl, difluoromethylcarbonyl, fluoromethylcarbonyl, benzylcarbonyl, cyanomethyl, cyanobutyl, methoxycarbonylmethyl, ethoxycarbonylethyl, carboxymethyl, carboxypropyl, phenylthiomethyl, 2-chlorophenylthiomethyl, 2,6-dichlorophenylthiomethyl, 4-methylphenylthiomethyl, 2-isopropylphenylthiomethyl, 2-methylphenylthiomethyl, phenyloxymethyl, 4-chlorophenyloxymethyl, 4-methylphenyloxymethyl, benzyloxymethyl, 4-methoxybenzyloxymethyl, naphthyl and phenyl,
wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trifluoromethoxy;
wherein R¹ and R² is selected from phenyl, imidazolyl, thienyl, thiazolyl, pyrrolyl, oxazolyl, isoxazolyl, triazolyl, pyrazinyl, pyrimidinyl, quiolinyl, indolyL, benzimidazolyl, pyrazolyl and pyridyl, wherein R¹ and R² are optionally substituted at a substitutable position with one or more radicals independently selected from methylsulfonyl, aminosulfonyl, fluoromethylsulfonyl, difluoromethylsulfonyl, fluoro, chloro, bromo, methylthio, methylsulfinyl, methyl, ethyl, isopropyl, *tert*-butyl, isobutyl, pentyl, hexyl, cyano, carboxyl, methoxycarbonyl, ethoxycarbonyl, isopropoxycarbonyl, *tert*-butoxycarbonyl, propoxycarbonyl, butoxycarbonyl, isobutoxycarbonyl, pentoxycarbonyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl, methoxymethyl, ethoxymethyl, trifluoromethoxy, amino, methylamino, N,N-diethylamino, phenylamino and nitro; provided that at least one of R¹ or R² is 4-methyl-sulfonylphenyl or 4-amino sulfonylphenyl or a pharmaceutically-acceptable salt thereof.

3. Compounds of Claim 2 wherein R³ is a radical selected from methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl;
wherein R¹ is phenyl substituted with 4-methylsulfonyl or 4-aminosulfonyl; and wherein R² is selected from imidazolyl, thienyl, thiazolyl, pyrrolyl, oxazolyl, isoxazolyl, triazolyl, pyrimidinyl, quiolinyl, indolyl, benzimidazolyl, pyrazolyl and pyridyl, wherein R² is optionally substituted at a substitutable position with one or more radicals independently selected from methylthio, methyl, ethyl, isopropyl, *tert*-butyl, isobutyl, pentyl, hexyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, methylenedioxy, ethoxy, propoxy, n-butoxy, hydroxymethyl, hydroxyethyl, methoxymethyl, ethoxymethyl, and trifluoromethoxy; or a pharmaceutically-acceptable salt thereof.

4. A compound of Claim 1 selected from compounds, and their pharmaceutically acceptable salts, of the group consisting of
1-methyl-3-[1-[4-(methylsulfonyl)phenyl]-4-trifluoromethyl-1H-imidazol-2-yl]-1H-indole;
2-[1-[4-(methylsulfonyl)phenyl]-4-(trifluoromethyl)-1H-imidazol-2-yl]thiophene;
3-[1-[4-(methylsulfonyl)phenyl]-4-(trifluoromethyl)-1H-imidazol-2-yl]thiophene;
2-methyl-3-[1-[4-(methylsulfonyl)phenyl]-4-(trifluoromethyl)-1H-imidazol-2-yl]pyridine;
3-fluoro-5- [1-[4-(methylsulfonyl)phenyl]-4-(trifluoromethyl)-1H-imidazol-2-yl]pyridine;
3-chloro-5-[1-[4-(methylsulfonyl)phenyl]-4-(trifluoromethyl)-1H-imidazol-2-yl]pyridine;
5-methyl-2-[1-[4-(methylsulfonyl)phenyl]-4-(trifluoromethyl)-1H-imidazol-2-yl]pyridine;
4-methyl-2-[1-[4-(methylsulfonyl)phenyl]-4-(trifluoromethyl)-1H-imidazol-2-yl]pyridine;
2-methoxy-6-[1-[4-(methylsulfonyl)phenyl]-4-(trifluoromethyl)-1H-imidazol-2-yl]pyridine;
5-methoxy-2-[1-[4-(methylsulfonyl)phenyl]-4-(trifluoromethyl)-1H-imidazol-2-yl]pyridine;
4-methoxy-2-[1-[4-(methylsulfonyl)phenyl]-4-(trifluoromethyl)-1H-imidazol-2-yl]pyridine;
2-chloro-6-[1-[4-(methylsulfonyl)phenyl]-4-(trifluoromethyl)-1H-imidazol-2-yl]pyridine;
5-chloro-2-[1-[4-(methylsulfonyl)phenyl]-4-(trifluoromethyl)-1H-imidazol-2-yl]pyridine;
4-chloro-2-[1-[4-(methylsulfonyl)phenyl]-4-(trifluoromethyl)-1H-imidazol-2-yl]pyridine;
2-fluoro-6-[1-[4-(methylsulfonyl)phenyl]-4-(trifluoromethyl)-1H-imidazol-2-yl]pyridine;
4-fluoro-2-[1-[4-(methylsulfonyl)phenyl]-4-(trifluoromethyl)-1H-imidazol-2-yl]pyridine;
4-fluoro-2-[1-[4-(methylsulfonyl)phenyl]-4-(trifluoromethyl)-1H-imidazol-2-yl]pyridine;
3-methoxy-5-[1-[4-(methylsulfonyl)phenyl]-4-(trifluoromethyl)-1H-imidazol-2-yl]pyridine;
3-[4-methyl-1-[4-(methylsulfonyl)phenyl]-4-(trifluoromethyl)-1H-imidazol-2-yl]pyridine;
3-[4-[4-(fluorophenyl)-1-[4-(methylsulfonyl)phenyl]-1H-imidazol-2-yl]pyridine;
5-[1-[4-(methylsulfonyl)phenyl]-4-(trifluoromethyl)-1H-imidazol-2-yl]-2-(methylthio)pyridine;
3-[4-(difluoromethyl)-1-[4-(methylsulfonyl)phenyl]-1H-imidazol-2-yl]pyridine;
3-[1-[4-(methylsulfonyl)phenyl]-4-(trifluoromethyl)-1H-imidazol-2-yl]pyridine;
2-[1-[4-(methylsulfonyl)phenyl-4-(trifluoromethyl)-1H-imidazol-2-yl]pyridine;
2-methyl-4-[1-[4-(methylsulfonyl)phenyl-4-(trifluoromethyl)-1H-imidazol-2-yl]pyridine;
4-[1-[4-(methylsulfonyl)phenyl-4-(trifluoromethyl)-1H-imidazol-2-yl]pyridine;
2-methyl-6-[1-[4-(methylsulfonyl)phenyl-4-(trifluoromethyl)-1H-imidazol-2-yl]pyridine;
3-methyl-5-[1-[4-(methylsulfonyl)phenyl]-4-(trifluoromethyl)-1H-imidazol-2-yl]pyridine;
2-methoxy-5-[1-[4-(methylsulfonyl)phenyl]-4-(trifluoromethyl)-1H-imidazol-2-yl]pyridine; and
2-[4-(4-fluorophenyl-1-[4-(methylsulfonyl)phenyl]-4-(trifluoromethyl)-1H-imidazol-2-yl]pyridine.

5. A Compound of Claim 1 selected from compounds, and their pharmaceutically acceptable salts, of the group consisting of
4-[2-(4-methylpyridin-2-yl]-4-(trifluoromethyl)-1H-imidazol-1-yl]benzenesulfonamide;
4-[2-(5-methylpyridin-3-yl)-4-(trifluoromethyl)-1H-imidazol-1-yl]benzenesulfonamide;
4-[2-(2-methylpyridin-3-yl)-4-(trifluoromethyl)-1H-imidazol-1-yl]benzenesulfonamide;
4-[2-(5-fluoropyridin-3-yl)-4-(trifluoromethyl)-1H-imidazol-1-yl]benzenesulfonamide;
4-[2-(5-chloropyridin-3-yl)-4-(trifluoromethyl)-1H-imidazol-1-yl]benzenesulfonamide;
4-[2-(5-methylpyridin-2-yl)-4-(trifluoromethyl)-1H-imidazol-1-yl]benzenesulfonamide;
4-[2-(4-methylpyridin-2-yl)-4-(trifluoromethyl)-1H-imidazol-1-yl]benzenesulfonamide;
4-[2-(6-methoxypyridin-2-yl)-4-(trifluoromethyl)-1H-imidazol-1-yl]benzenesulfonamide;
4-[2-(5-methoxypyridin-2-yl)-4-(trifluoromethyl)-1H-imidazol-1-yl]benzenesulfonamide;
4-[2-(4-methoxypyridin-2-yl)-4-(trifluoromethyl)-1H-imidazol-1-yl]benzenesulfonamide;
4-[2-(6-chloropyridin-2-yl)-4-(trifluoromethyl)-1H-imidazol-1-yl]benzenesulfonamide;
4-[2-(5-chloropyridin-2-yl)-4-(trifluoromethyl)-1H-imidazol-1-yl]benzenesulfonamide;
4-[2-(4-chloropyridin-2-yl)-4-(trifluoromethyl)-1H-imidazol-1-yl]benzenesulfonamide;
4-[2-(6-fluoropyridin-2-yl)-4-(trifluoromethyl)-1H-imidazol-1-yl]benzenesulfonamide;
4-[2-(5-fluoropyridin-2-yl)-4-(trifluoromethyl)-1H-imidazol-1-yl]benzenesulfonamide;
4-[2-(4-fluoropyridin-2-yl)-4-(trifluoromethyl)-1H-imidazol-1-yl]benzenesulfonamide;
4-[2-(5-methoxypyridin-3-yl)-4-(trifluoromethyl)-1H-imidazol-1-yl]benzenesulfonamide;
4-[4-methyl-2-(3-pyridinyl)-1H-imidazol-1-yl]benzenesulfonamide;
4-[2-(pyridin-3-yl)-4-(trifluoromethyl)-1H-imidazol-1-yl]benzenesulfonamide;
4-[4-(4-fluorophenyl)-2-(3-pyridinyl)-1H-imidazol-1-yl]benzenesulfonamide;
4-[2-[6-(methylthio)pyridin-3-yl]-4-trifluoromethyl)-1H-imidazol-1-yl]benzenesulfonamide;
4-[4-(difluoromethyl)-2-(3-pyridinyl)-1H-imidazol-1-yl]benzenesulfonamide; and
4-[2-(6-methylpyridin-3-yl)-4-(trifluoromethyl)-1H-imidazol-1-yl]benzenesulfonamide.

6. Compounds of Claim 1 having Formula II 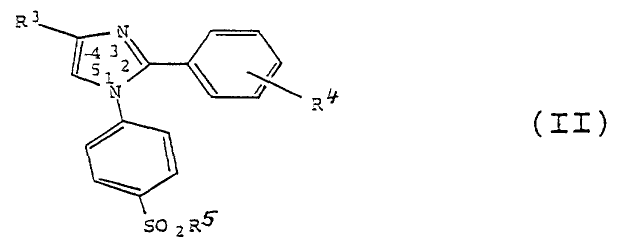 wherein R³ is a radical selected from C₁-C₆-alkyl, C₁-C₆-haloalkyl, formyl, C₂-C₆-alkanoyl, arylcarbonyl, cyano, C₁-C₆-alkylthio-C₁-C₆-alkyl, C₃-C₁₀-cycloalkylthio-C₁-C₆-alkyl, C₁-C₆-hydroxyalkyl, C₁-C₆-alkoxy-C₁-C₆-alkyl, C₁-C₆-cyanoalkyl, C₁-C₆-carboxyalkyl, C₁-C₆-alkoxycarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxy-C₁-C₆-alkyl and aryl; wherein R⁵ is a radical selected from methyl and amino; and wherein R⁴ is one or more radicals selected from hydrido, halo, C₁-C₆-alkyl, C₁-C₆-haloalkyl, C₁-C₆-alkoxy, amino, C₁-C₆-haloalkoxy, cyano, carboxyl, hydroxyl, C₁-C₆-hydroxyalkyl, C₁-C₆-alkoxy-C₁-C₆-alkyl, C₁-C₆-alkylamino, nitro and C₁-C₆-alkylthio; or a pharmaceutically-acceptable salt thereof.

7. Compounds of Formula II 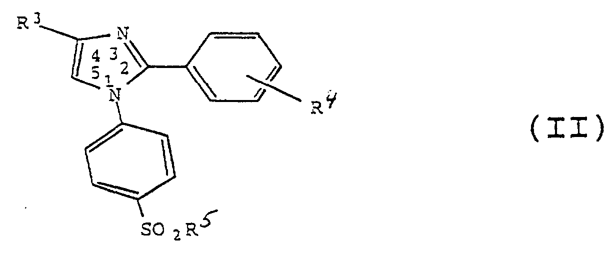 wherein R³ is selected from methyl, ethyl, isopropyl, *tert*-butyl, isobutyl, pentyl, hexyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, formyl, cyano, hydroxymethyl, hydroxyethyl, methoxymethyl, ethoxymethyl, methylthiomethyl, isopropylthiomethyl, cyclohexylthiomethyl, methylcarbonyl, cyanomethyl, cyanobutyl, methoxycarbonyl, ethoxycarbonyl, isopropoxycarbonyl *tert*-butoxycarbonyl, propoxycarbonyl, carboxymethyl, carboxypropyl, phenylthiomethyl, 2-chlorophenylthiomethyl, 2,6-dichlorophenylthiomethyl, 4-methylphenylthiomethyl, 2-isopropylphenylthiomethyl, 2-methylphenylthiomethyl, phenyloxymethyl, 4-chlorophenyloxymethyl, 4-methylphenyloxymethyl, benzyloxymethyl, 4-methoxybenzyloxymethyl, naphthyl and phenyl, wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trifluoromethoxy; wherein R⁵ is methyl or amino; and wherein R⁴ is a radical selected from hydrido, methylsulfonyl, fluoro, chloro, bromo, methylthio, methyl, ethyl, isopropyl, *tert*-butyl, isobutyl, pentyl, hexyl, cyano, carboxyl, methoxycarbonyl, ethoxycarbonyl, isopropoxycarbonyl, *tert*-butoxycarbonyl, propoxycarbonyl, butoxycarbonyl, isobutoxycarbonyl, pentoxycarbonyl, fluoromethyl, dirluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl, methoxymethyl, ethoxymethyl, trifluoromethoxy, amino, methylamino, N,N-dimechylamino, phenylamino and nitro; or a pharmaceutically-acceptablesalt thereof.

8. A compound of Claim 7 selected from compounds, and their pharmaceutically acceptable salts, of the group consisting of
2-(4-fluorophenyl)-1-[4-(methylsulfonyl)phenyl]-4-(trifluoromethyl)-1H-imidazole;
2-(3,4-difluorophenyl)-1-[4-(methylsulfonyl)phenyl]-4-(trifluoromethyl)-1H-imidazole;
2-(2-fluorophenyl)-1-[4-(methylsulfonyl)phenyl]-4-(trifluoromethyl)-1H-imidazole;
2-(3-fluorophenyl)-1-[4-(methylsulfonyl)phenyl]-4-(trifluoromethyl)-1H-imidazole;
2-(4-fluoro-3-methylphenyl)-1-[4-(methylsulfonyl)phenyl]-4-(trifluoromethyl)-1H-imidazole;
2-(3-chloro-4-(N,N-dimethylamino)phenyl)-1-[4-(methylsulfonyl)phenyl]-4-(trifluoromethyl)-1H-imidazole;
2-(3-fluoro-4-(N, N-dimethylamino)phenyl)-1-[4-(methylsulfonyl)phenyl]-4-(trifluoromethyl)-1H-imidazole;
2-(3-bromophenyl)-1-[4-(methylsulfonyl)phenyl]-4-(trifluoromethyl)-1H-imidazole;
2-(3-nitrophenyl)-1- [4-(methylsulfonyl)phenyl]-4-(trifluoromethyl)-1H-imidazole;
2-(4-chlorophenyl)-1-[4-(methylsulfonyl)phenyl]-4-(trifluoromethyl)-1H-imidazole;
2-(4-chlorophenyl)-1-[4-(methylsulfonyl)phenyl]-4-methyl-1H-imidazole;
2-(4-chlorophenyl)-1-[4-(methylsulfonyl)phenyl]-4-phenyl-1H-imidazole;
2-(4-chlorophenyl)-4-(4-fluorophenyl)-1-[4-(methylsulfonyl)(phenyl]-1H-imidazole;
2-(3-fluoro-4-methoxyphenyl)-1-[4-(methylsulfonyl)phenyl-4-(trifluoromethyl)-1H-imidazole;
1-[4-(methylsulfonyl)phenyl]-2-phenyl-4-trifluoromethyl-1H-imidazole;
2-(4-methylphenyl)-1-[4-(methylsulfonyl)phenyl]-4-trifluoromethyl-1H-imidazole;
2-(3-fluoro-5-methylphanyl)-1-[4-(methylsulfonyl)phenyl]-4-(trifluoromethyl)-1H-imidazole;
2-(3-methylphenyl)-1-[4-(methylsulfonyl)phenyl]-4-trifluoromethyl-1H-imidazole;
2-(3,5-difluoro-4-methoxyphenyl)-1-[4-(methylsulfonyl)phenyl]-4-trifluoromethyl-1H-imidazole;
1-[4-(methylsulfonyl)phenyl]-2-(3-chlorophenyl)-4-trifluoromethyl-1H-imidazole;
1-[4-(methylsulfonyl)phenyl]-2-(4-methoxy-3-chlorophenyl)-4-trifluoromethyl-1H-imidazole; and
2-(2-methylphenyl)-1-[4-(methylsulfonyl)phenyl]-4-trifluoromethyl-1H-imidazole.

9. A compound of Claim 7 selected from compounds, and their pharmaceutically acceptable salts, of the group consisting of
4-[2-(4-fluorophenyl)-4-(trifluoromethyl)-1H-imidazol-1-yl]benzenesulfonamide;
4-[2-(4-methylphenyl)-4-(trifluoromethyl)-1H-imidazol-1-yl]benzenesulfonamide;
4-[2-(3,4-difluorophenyl)-4-(trifluoromethyl)-1H-imidazol-1-yl]benzenesulfonamide;
4-[2-(2-fluorophenyl)-4-(trifluoromethyl)-1H-imidazol-1-yl]benzenesulfonamide;
4-[2-(3-fluorophenyl)-4-(trifluoromethyl)-1H-imidazol-1-yl]benzenesulfonamide;
4-[2-(4-chloro-3-methoxyphenyl)-4-(trifluoromethyl)-1H-imidazol-1-yl]benzenesulfonamide;
4-[2-(3,5-difluoro-4-methoxyphenyl)-4-(trifluoromethyl)-1H-imidazol-1-yl]benzenesulfonamide;
4-[2-(4-chlorophenyl)-4-(trifluoromethyl)-1H-imidazol-1-yl]benzenesulfonamide;
4-[2-(3-chloro-4-methylphenyl)-4-(trifluoromethyl)-1H-imidazol-1-yl]benzenesulfonamide;
4-[2-(3-fluoro-4-methoxyphenyl)-4-(trifluoromethyl)-1H-imidazol-1-yl]benzenesulfonamide;
4-[2-(3-fluoro-5-methylphenyl)-4-(trifluoromethyl)-1H-imidazol-1-yl]benzenesulfonamide;
4- [2-(3-methylphenyl)-4-trifluoromethyl-1H-imidazol-1-yl]benzenesulfonamide;
4-[2-(3-chlorophenyl)-4-trifluoromethyl-1H-imidazol-1-yl]benzenesulfonamide;
4-[2-phenyl-4-trifluoromethyl-1H-imidazol-1-yl]benzenesulfonamide; and
4- [2-(4-methoxy-3-chlorophenyl)-4-trifluoromethyl-1H-imidazol-1-yl]benzenesulfonamide.

10. Compounds of Claim 1 having Formula III 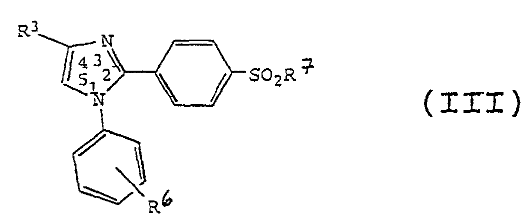 wherein R³ is a radical selected from C₁-C₆-alkyl, C₁-C₆-haloalkyl, formyl, C₂-C₆-alkanoyl, arylcarbonyl, cyano, C₁-C₆-alkylthio-C₁-C₆-alkyl, C₃-C₁₀-cycloalkylthio-C₁-C₆-alkyl, C₁-C₆-hydroxyalkyl, C₁-C₆-alkoxyalkyl, C₁-C₆-alkylcarbonyl, C₁-C₆-cyanoalkyl, C₁-C₆-carboxyalkyl, C₁-C₆-alkoxycarbonyl, arylthio-C₁-C₆-alkyl, aryloxy-C₁-C₆-alkyl, aryl-C₁-C₆-alkylthio-C₁-C₆-alkyl, aryl-C₁-C₆-alkoxyalkyl and aryl; wherein the aryl radicals are optionally substituted at a substitutable position with one or more radicals selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and C₁-C₆-haloalkoxy; wherein R⁶ is one or more radicals selected from hydrido, halo, C₁-C₆-alkyl, C₁-C₆-haloalkyl, C₁-C₆-alkoxy, amino, C₁-C₆-haloalkoxy, cyano, carboxyl, hydroxyl, C₁-C₆-hydroxyalkyl, C₁-C₆-alkoxy-C₁-C₆-alkyl,C₁-C₆-alkylamino, nitro and C₁-C₆-alkylthio; and wherein R⁷ is a radical selected from methyl and amino; or a pharmaceutically-acceptable salt thereof.

11. Compounds of Claim 10 wherein R³ is selected from methyl, ethyl, isopropyl, *tert*-butyl, isobutyl, pentyl, hexyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, formyl, cyano, hydroxymethyl, hydroxyethyl, methoxymethyl, ethoxymethyl, methylthiomethyl, methylcarbonyl, cyanomethyl, cyanobutyl, azidomethyl, methoxycarbonyl, ethoxycarbonyl, isopropoxycarbonyl, *tert*-butoxycarbonyl, propoxycarbonyl, phenylthiomethyl, phenyloxymethyl, 4-chlorophenyloxymethyl, 4-methylphenyloxymethyl, benzyloxymethyl, 4-methoxybenzyloxymethyl, naphthyl and phenyl, wherein the phenyl radicals are optionally substituted at a substitutable position with one or more radicals selected from fluoro, chloro, bromo, methylthio, methylsulfinyl, methyl, ethyl, isopropyl, *tert*-butyl, isobutyl, pentyl, hexyl, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxyl, methoxy, methylenedioxy, ethoxy, propoxy, n-butoxy, hydroxymethyl, hydroxyethyl and trifluoromethoxy;
wherein R⁶ is a radical selected from hydrido, fluoro, chloro, bromo, iodo, methyl, ethyl, isopropyl, tert-butyl, isobutyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, ethoxy, isopropoxy, *tert*-butoxy, propoxy, butoxy, isobutoxy, pentoxy, methylenedioxy, amino, trifluoromethoxy, cyano, carboxyl, hydroxyl, nitro, hydroxymethyl, methoxymethyl, ethoxymethyl, methylamino, methylthio, ethylthio, propylthio and butylthio; and wherein R⁷ is methyl, fluoromethyl or amino, or a pharmaceutically-acceptable salt thereof.

12. Compounds of Formula IV 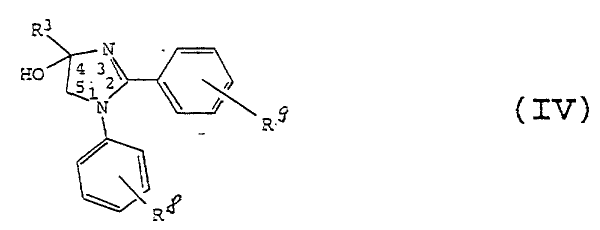 wherein R³ is selected from C₁-C₆-haloalkyl; and wherein R⁸ and R⁹ are independently selected from hydrido, halo, C₁-C₆-alkyl, C₁-C₆-haloalkyl, C₁-C₆-alkoxy, C₁-C₆-alkylsulfonyl, C₁-C₆-haloalkylsulfonyl and sulfamyl;
or a pharmaceutically-acceptable salt thereof.

13. Compounds of Claim 12 wherein R³ is selected from fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl; and wherein R⁸ and R⁹ are independently selected from hydrido, fluoro, chloro, bromo, iodo, methyl, ethyl, isopropyl, *tert*-butyl, isobutyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methoxy, ethoxy, isopropoxy, *tert*-butoxy, propoxy, butoxy, isobutoxy, pentoxy, methylenedioxy, methylsulfonyl, fluoromethylsulfonyl and sulfamyl; or a pharmaceutically-acceptable salt thereof.

14. A pharmaceutical composition comprising a therapeutically-effective amount of a compound, said compound selected from a family of compounds according to any of Claims 1-13; or a pharmaceutically-acceptable salt thereof.

15. Use of a compound according to any of Claims 1-13 or a pharmaceutically-acceptable salt thereof for peparing a medicament for treating inflammation or an inflammation-associated disorder in a subject.

16. Use according to Claim 15 wherein the inflammation-associated disorder is arthritis.

17. Use according to Claim 15 wherein the inflammation-associated disorder is pain.

18. Use according to Claim 15 wherein the inflammation-associated disorder is fever.

19. A process of making a sulphonamide of Formula I according to claim 1 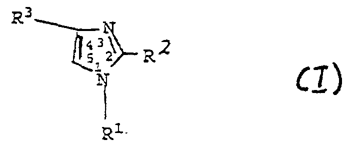 or a pharmaceutically, acceptable salt thereof,
wherein R¹, R² and R³ are defined as in claim 1
said method comprising the steps of
treating a compound of Formula I wherein one of R¹ and R² is 4-methylsulfonylphenyl with a base and an substituted trialkylsilane in an appropriate solvent to form a silylalkylsulfone,
treating said silylalkylsulfone with an alkylammonium halide to form a sulfinic acid salt, and
forming said sulfonamide by treating the sulfinic acid salt with an aminating agent.

## Patentansprüche

1. Eine Verbindung der Formel (I) 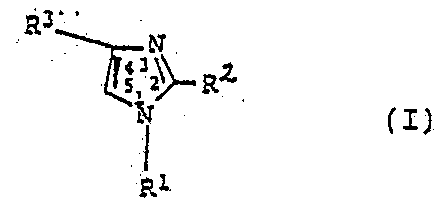 worin R³ ein Rest ist ausgewählt aus C₁-C₆-Alkyl, C₁-C₆-Haloalkyl, Aryl-C₁-C₆-alkyl, Formyl, C₂-C₆-Alkanoyl, Cyano, C₁-C₆-Alkylthio-C₁-C₆-alkyl, C₁-C₁₀-Cycloalkylthio-C₁-C₆-alkyl, C₁-C₆-Hydroxyalkyl, C₁-C₆-Alkoxy-C₁-C₆-alkyl, Arylcarbonyl, Aryl-C₁-C₆-alkylcarbonyl, C₁-C₆-Cyanoalkyl, C₁-C₆-Carboxyalkyl, C₁-C₆-Alkoxycarbonyl-C₁-C₆-alkyl, C₁-C₆-Alkoxycarbonyl, C₁-C₆-Haloalkylcarbonyl, Arylthio-C₁-C₆-alkyl, Aryloxy-C₁-C₆-alkyl, Aryl-C₁-C₆-alkylthio-C₁-C₆-alkyl, Aryl-C₁-C₆alkoxy-C₁-C₆-alkyl und Aryl;
worin R¹ und R² unabhängig voneinander ausgewählt sind aus Aryl und Heteroaryl, worin R¹ und R² gegebenenfalls substituiert sind an einer substituierbaren Position mit einem oder mehreren Resten unabhängig ausgewählt aus C₁-C₆-Alkylsulfonyl, Aminosulfonyl, Halo, C₁-C₆-Alkylthio, C₁-C₆-Alkyl, Cyano, Carboxyl, C₁-C₆-Alkoxycarbonyl, C₁-C₆-Haloalkyl, Hydroxyl, C₁-C₆-Alkoxy, C₁-C₆-Hydroxyalkyl, C₁-C₆-Alkoxy-C₁-C₆-alkyl, C₁-C₆-Haloalkoxy, Amino, mono- oder di-C₁-C₆-Alkylamino, Arylamino und Nitro;
mit der Maßgabe, dass mindestens eines von R¹ und R² 4-Methylsulfonylphenyl oder 4-Aminosulfonylphenyl ist; worin Aryl wann immer es erscheint einen Phenyl- oder Naphthylring bedeutet; worin Heteroaryl, wann immer es erscheint einen ungesättigten heterocyclischen Rest bedeutet ausgwählt aus Pyrrolyl, Pyrrolinyl, Imidazolyl, Pyrazolyl, 2-Pyridyl, 3-Pyridyl, 4-Pyridyl, Pyrimidyl, Pyrazinyl, Pyridazinyl, Triazolyl, Tetrazolyl, Indolyl, Isoindolyl, Indolizinyl, Benzimidazolyl, Chinolyl, Isochinolyl, Indazolyl, Benzotriazolyl, Tetrazolopyridazinyl, Pyranyl, 2-Furyl, 3-Furyl, 2-Thienyl, 3-Thienyl, Oxazolyl, Isoxazolyl, Oxadiazolyl, Benzoxazolyl, Benzoxadiazolyl, Thiazolyl, Thiadiazolyl, Benzothiazolyl, Benzothiadiazolyl, Benzofuran, Benzothiophen, worin dieser heterocyclische Rest substituert sein kann an einer substituierbaren Position mit einem oder mehreren Substituenten ausgewählt aus Halo, C₁-C₆-Alkylthio, C₁-C₆-Alkylsulfinyl, C₁-C₆-Alkyl, Cyano, C₁-C₆-Haloalkyl, Hydroxyl, C₁-C₆-Alkoxy, C₁-C₆-Hydroxyalkyl und Halo-C₁-C₆-alkoxy oder ein pharmazeutisch verträgliches Salz davon.

2. Verbindungen der Formel (I) 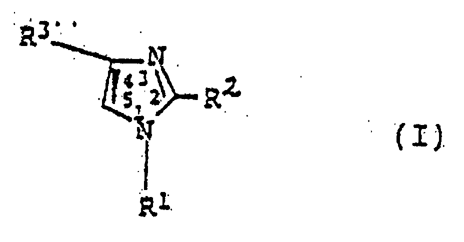 worin R³ ein Rest ist ausgewählt aus Methyl, Ethyl, Isopropyl, *tert*-Butyl, Isobutyl, Pentyl, Hexyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Benzyl, Phenylethyl, Phenylpropyl, Formyl, Cyano, Hydroxymethyl, Hydroxyethyl, Methoxymethyl, Ethoxymethyl, Methylthiomethyl, Isopropylthiomethyl, Cyclohexylthiomethyl, Methylcarbonyl, Ethylcarbonyl, Phenylcarbonyl, Trifluormethylcarbonyl, Difluormethylcarbonyl, Fluormethylcarbonyl, Benzylcarbonyl, Cyanomethyl, Cyanobutyl, Methoxycarbonylmethyl, Ethoxycarbonylethyl, Carboxymethyl, Carboxypropyl, Phenylthiomethyl, 2-Chlorphenylthiomethyl, 2,6-Dichlorphenylthiomethyl, 4-Methylphenylthiomethyl, 2-Isopropylphenylthiomethyl, 2-Methylphenylthiomethyl, Phenyloxymethyl, 4-Chlorphenyloxymethyl, 4-Methylphenyloxymethyl, Benzyloxymethyl, 4-Methoxybenzyloxymethyl, Naphthyl und Phenyl,
worin die Phenylreste gegebenenfalls substituiert sind an einer substituierbaren Position mit einem oder mehreren Resten ausgewählt aus Fluor, Chlor, Brom, Methylthio, Methylsulfinyl, Methyl, Ethyl, Isopropyl, *tert*-Butyl, Isobutyl, Pentyl, Hexyl, Cyano, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Hydroxyl, Methoxy, Methylendioxy, Ethoxy, Propoxy, n-Butoxy, Hydroxymethyl, Hydroxyethyl und Trifluormethoxy;
worin R¹ und R² ausgewählt sind aus Phenyl, Imidazolyl, Thienyl, Thiazolyl, Pyrrolyl, Oxazolyl, Isoxazolyl, Triazolyl, Pyrazinyl, Pyrimidinyl, Chinolinyl, Indolyl, Benzimidazolyl, Pyrazolyl und Pyridyl,
worin R¹ und R² gegebenenfalls substituiert sind an einer substituierbaren Position mit einem oder mehreren Resten unabhängig voneinander ausgewählt aus Methylsulfonyl, Aminosulfonyl, Fluormethylsulfonyl, Difluormethylsulfonyl, Fluor, Chlor, Brom, Methylthio, Methylsulfinyl, Methyl, Ethyl, Isopropyl, *tert*-Butyl, Isobutyl, Pentyl, Hexyl, Cyano, Carboxyl, Methoxycarbonyl, Ethoxycarbonyl, Isopropoxycarbonyl, *tert*-Butoxycarbonyl, Propoxycarbonyl, Butoxycarbonyl, Isobutoxycarbonyl, Pentoxycarbonyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Hydroxyl, Methoxy, Methylendioxy, Ethoxy, Propoxy, n-Butoxy, Hydroxymethyl, Hydroxyethyl, Methoxymethyl, Ethoxymethyl, Trifluormethoxy, Amino, Methylamino, N,N-Diethylamino, Phenylamino und Nitro,
mit der Maßgabe, dass mindestens eines von R¹ oder R² 4-Methylsulfonylphenyl oder 4-Aminosulfonylphenyl ist oder ein pharmazeutisch verträgliches Salz davon.

3. Verbindungen des Anspruchs 2, worin R³ ein Rest ist ausgewählt aus Methyl, Ethyl, Isopropyl, *tert*-Butyl, Isobutyl, Pentyl, Hexyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl-, Difluorpropyl, Dichlorethyl und Dichlorpropyl;
worin R¹ Phenyl ist substituiert mit 4-Methylsulfonyl oder 4-Aminosulfonyl und
worin R² ausgewählt ist aus Imidazolyl, Thienyl, Thiazolyl, Pyrrolyl, Oxazolyl, Isoxazolyl, Triazolyl, Pyrimidinyl, Chinolinyl, Indolyl, Benzimidazolyl, Pyrazolyl und Pyridyl,
worin R² gegebenenfalls substituiert ist an einer substituierbaren Position mit einem oder mehreren Resten unabhängig voneinander ausgewählt aus Methylthio, Methyl, Ethyl, Isopropyl, *tert*-Butyl, Isobutyl, Pentyl, Hexyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Methoxy, Methylendioxy, Ethoxy, Propoxy, n-Butoxy, Hydroxymethyl, Hydroxyethyl, Methoxymethyl, Ethoxymethyl und Trifluormethoxy oder ein pharmazeutisch verträgliches Salz davon.

4. Eine Verbindung des Anspruchs 1 ausgewählt aus Verbindungen und deren pharmazeutisch verträglichen Salzen der Gruppe bestehend aus
1-Methyl-3-[1-[4-(methylsulfonyl)phenyl]-4-trifluormethyl-1Himidazol-2-yl]-1H-indol;
2-[1-[4-(Methylsulfonyl)phenyl]-4-(trifluormethyl)-1Himidazol-2-yl]thiophen;
3-[1-[4-(Methylsulfonyl)phenyl]-4-(trifluormethyl)-1 H-imidazol-2-yl]thiophen;
2-Methyl-3-[1-[4-(methylsulfonyl)phenyl]-4-(trifluormethyl)-1H-imidazol-2-yl]pyridin;
3-Fluor-5-[1-[4-(methylsulfonyl)phenyl]-4-(trifluormethyl)-1H-imidazol-2-yl]pyridin;
3-Chlor-5-[1-[4-(methylsulfonyl)phenyl]-4-(trifluormethyl)-1H-imidazol-2-yl]pyridin;
5-Methyl-2-[1-[4-(methylsulfonyl)phenyl]-4-(trifluormethyl)-1H-imidazol-2-yl]pyridin;
4-Methyl-2-[1-[4-(methylsulfonyl)phenyl]-4-(trifluormethyl)-1H-imidazol-2-yl]pyridin;
2-Methoxy-6-[1-[4-(methylsulfonyl)phenyl]-4-(trifluormethyl)-1H-imidazol-2-yl]pyridin;
5-Methoxy-2-[1-[4-(methylsulfonyl)phenyl]-4-(trifluormethyl)-1H-imidazol-2-yl]pyridin;
4-Methoxy-2-[1-[4-(methylsulfonyl)phenyl]-4-(trifluormethyl)-1H-imidazol-2-yl]pyridin;
2-Chlor-6-[1-[4-(methylsulfonyl)phenyl]-4-(trifluormethyl)-1H-imidazol-2-yl]pyridin;
5-Chlor-2-[1-[4-(methylsulfonyl)phenyl]-4-(trifluormethyl)-1H-imidazol-2-yl]pyridin;
4-Chlor-2-[1-[4-(methylsulfonyl)phenyl]-4-(trifluormethyl)-1H-imidazol-2-yl]pyridin;
2-Fluor-6-[1-[4-(methylsulfonyl)phenyl]-4-(trifluormethyl)-1H-imidazol-2-yl]pyridin;
4-Fluor-2-[1-[4-(methylsulfonyl)phenyl]-4-(trifluormethyl)-1H-imidazol-2-yl]pyridin;
4-Fluor-2-[1-(4-(methylsulfonyl)phenyl]-4-(trifluormethyl)-1H-imidazol-2-yl]pyridin;
3-Methoxy-5-[1-[4-(methylsulfonyl)phenyl]-4-(trifluormethyl)-1H-imidazol-2-yl]pyridin;
3-[4-Methyl-1-[4-(methylsulfonyl)phenyl]-4-(trifluormethyl)-1H-imidazol-2-yl]pyridin;
3-[4-[4-(Fluorphenyl)-1-[4-(methylsulfonyl)phenyl]-1Himidazol-2-yl]pyridin;
5-[1-[4-(Methylsulfonyl)phenyl]-4-(trifluormethyl)-1Himidazol-2-yl]-2-(methylthio)pyridin;
3-[4-(Difluormethyl)-1-[4-(methylsulfonyl)phenyt]-1 H-imidazol-2-yl]pyridin;
3-[1 -[4-(Methylsulfonyl)phenyl]-4-(trifluormethyl)-1Himidazol-2-yl]pyridin;
2-[1-[4-(Methylsulfonyl)phenyl-4-(trifluormethyl)-1H-imidazol-2-yl]pyridin;
2-Methyl-4-[1-[4-(methylsulfonyl)phenyl]-4-(trifluormethyl)-1Himidazol-2-yl]pyridin;
4-[1-[4-(Methylsulfonyl)phenyl]-4-(trifluormethyl)-1H-imidazol-2-yl]pyridin;
2-Methyl-6-[1-[4-(methylsulfonyl)phenyl]-4-(trifluormethyl)-1Himidazol-2-yl]pyridin;
3-Methyl-5-[1-[4-(methylsulfonyl)phenyl]-4-(trifluormethyl)-1H-imidazol-2-yl]pyridin;
2-Methoxy-5-[1-[4-(methylsulfonyl)phenyl]-4-(trifluormethyl)-1H-imidazol-2-yl]pyridin;
2-[4-(4-Fluorphenyl-1-[4-(methylsulfonyl)phenyl]-4-(trifluormethyl)-1H-imldazol-2-yl]pyridin.

5. Eine Verbindung des Anspruchs 1 ausgewählt aus Verbindungen und deren pharmazeutisch verträglichen Salzen der Gruppe bestehend aus
4-[2-(4-Methylpyridin-2-yl)-4-(trifluormethyl)-1H-imidazol-1-yl]benzolsulfonamid;
4-[2-(5-Methylpyridin-3-yl)-4-(trifluormethyl)-1H-imidazol-1-yl]benzolsulfonamid;
4-[2-(2-Methylpyridin-3-yl)-4-(trifluormethyl)-1H-imidazol-1-yl]benzolsulfonamid;
4-[2-(5-Fluorpyridin-3-yl)-4-(trifluormethyl)-1H-imidazol-1-yl]benzolsulfonamid;
4-[2-(5-Chlorpyridin-3-yl)-4-(trifluormethyl)-1H-imidazol-1-yl]benzolsulfonamid;
4-[2-(5-Methylpyridin-2-yl)-4-(trifluormethyl)-1H-imidazol-1-yl]benzolsulfonamid;
4-[2-(4-Methylpyridin-2-yl)-4-(trifluormethyl)-1H-imidazol-1-yl]benzolsulfonamid;
4-[2-(6-Methoxypyridin-2-yl)-4-(trifluormethyl)-1H-imidazol-1-yl]benzolsulfonamid;
4-[2-(5-Methoxypyridin-2-yl)-4-(trifluormethyl)-1H-imidazol-1-yl]benzolsulfonamid;
4-[2-(4-Methoxypyridin-2-yl)-4-(trifluormethyl)-1H-imidazol-1-yl]benzolsulfonamid;
4-[2-(6-Chlorpyridin-2-yl)-4-(trifluormethyl)-1H-imidazol-1-yl]benzolsulfonamid;
4-[2-(5-Chlorpyridin-2-yl)-4-(trifluormethyl)-1H-imidazol-1-yl]benzolsulfonamid;
4-[2-(4-Chlorpyridin-2-yl)-4-(trifluormethyl)-1H-imidazol-1-yl]benzolsulfonamid;
4-[2-(6-Fluorpyridin-2-yl)-4-(trifluormethyl)-1 H-imidazol-1-yl]benzolsulfonamid;
4-[2-(5-Fluorpyridin-2-yl)-4-(trifluormethyl)-1H-imidazol-1-yl]benzolsulfonamid;
4-[2-(4-Fluorpyridin-2-yl)-4-(trifluormethyl)-1H-imidazol-1-yl]benzolsulfonamid;
4-[2-(5-Methoxypyridin-3-yl)-4-(trifluormethyl)-1 H-imidazol-1-yl]benzolsulfonamid;
4-[4-Methyl-2-(3-pyridinyl)-1H-imidazol-1-yl]benzolsulfonamid;
4-[2-(Pyridin-3-yl)-4-(trifluormethyl)-1H-imidazol-1-yl]benzolsulfonamid;
4-[4-(4-Fluorphenyl)-2-(3-pyridinyl)-1H-imidazol-1-yl]benzolsulfonamid;
4-[2-[6-(Methylthio)pyridin-3-yl]-4-(trifluormethyl)-1Himidazol-1yl]benzolsulfonamid;
4-[4-(Difluormethyl)-2-(3-pyridinyl)-1 H-imidazol-1-yl]benzolsulfonamid und
4-[2-(6-Methylpyridin-3-yl)-4-(trifluormethyl)-1H-imidazol-1-yl]benzolsulfonamid.

6. Verbindungen des Anspruchs 1 der Formel II 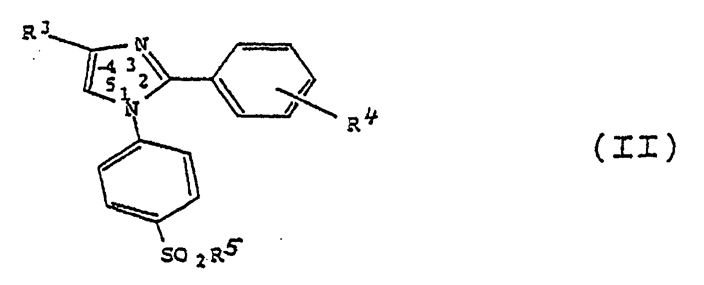 worin R³ ein Rest ist ausgewählt aus C₁-C₆-Alkyl, C₁-C₆-Haloalkyl, Formyl, C₂-C₆-Alkanoyl, Arylcarbonyl, Cyano, C₁-C₆-Alkylthio-C₁-C₆-alkyl, C₃-C₁₀-Cycloalkylthio-C₁-C₆-alkyl, C₁-C₆-Hydroxyalkyl, C₁-C₆-Alkoxy-C₁-C₆-alkyl, C₁-C₆-Cyanoalkyl, C₁-C₆-Carboxyalkyl, C₁-C₆-Alkoxycarbonyl, Arylthlo-C₁-C₆-alkyl, Aryloxy-C₁-C₆-alkyl, Aryl-C₁-C₆-alkylthio-C₁-C₆-alkyl, Aryl-C₁-C₆-alkoxy-C₁-C₆alkyl und Aryl;
worin R⁵ ein Rest ist ausgewählt aus Methyl und Amino und
worin R⁴ ein oder mehrere Reste ist ausgewählt aus Hydrido, Halo, C₁-C₆-Alkyl, C₁-C₆-Haloalkyl, C₁-C₆-Alkoxy, Amino, C₁-C₆-Haloalkoxy, Cyano, Carboxyl, Hydroxyl, C₁-C₆-Hydroxyalkyl, C₁-C₆-Alkoxy-C₁-C₆-alkyl, C₁-C₆-Alkylamino, Nitro und C₁-C₆-Alkylthio oder ein pharmazeutisch verträgliches Salz davon.

7. Verbindungen der Formel II 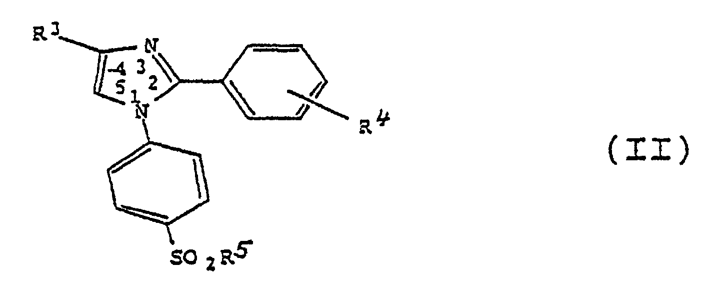 worin R³ ausgewählt ist aus Methyl, Ethyl, Isopropyl, *tert*-Butyl, isobutyl, Pentyl, Hexyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Formyl, Cyano, Hydroxymethyl, Hydroxyethyl, Methoxymethyl, Ethoxymethyl, Methylthiomethyl, Isopropylthiomethyl, Cyclohexylthiomethyl, Methylcarbonyl, Cyanomethyl, Cyanobutyl, Methoxycarbonyl, Ethoxycarbonyl, Isopropoxycarbonyl, *tert*-Butoxycarbonyl, Propoxycarbonyl, Carboxymethyl, Carboxypropyl, Phenylthiomethyl, 2-Chlorphenylthiomethyl, 2,6-Dichlorphenylthiomethyl, 4-Methylphenylthiomethyl, 2-Isopropylphenylthiomethyl, 2-Methylphenylthiomethyl, Phenyloxymethyl, 4-Chlorphenyloxymethyl, 4-Methylphenyloxymethyl, Benzyloxymethyl, 4-Methoxybenzyloxymethyl, Naphthyl und Phenyl,
worin die Phenylreste gegebenenfalls substituiert sind an einer substituierbaren Position mit einem oder mehreren Resten ausgewählt aus Fluor, Chlor, Brom, Methylthio, Methylsulfinyl, Methyl, Ethyl, Isopropyl, *tert*-Butyl, Isobutyl, Pentyl, Hexyl, Cyano, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Hydroxyl, Methoxy, Methylendioxy, Ethoxy, Propoxy, n-Butoxy, Hydroxymethyl, Hydroxyethyl und Trifluormethoxy;
worin R⁵ Methyl oder Amino ist und worin R⁴ ein Rest ist ausgewählt aus Hydrido, Methylsulfonyl, Fluor, Chlor, Brom, Methylthio, Methyl, Ethyl, Isopropyl, *tert*-Butyl, Isobutyl, Pentyl, Hexyl, Cyano, Carboxyl, Methoxycarbonyl, Ethoxycarbonyl, Isopropoxycarbonyl, *tert*-Butoxycarbonyl, Propoxycarbonyl, Butoxycarbonyl, Isobutoxycarbonyl, Pentoxycarbonyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Hydroxyl, Methoxy, Methylendioxy, Ethoxy, Propoxy, n-Butoxy, Hydroxymethyl, Hydroxyethyl, Methoxymethyl, Ethoxymethyl, Trifluormethoxy, Amino, Methylamino, N,N-Dimethylamino, Phenylamino und Nitro oder ein pharmazeutisch verträgliches Salz davon.

8. Eine Verbindung des Anspruchs 7 ausgewählt aus Verbindungen und deren pharmazeutisch verträglichen Salzen der Gruppe bestehend aus
2-(4-Fluorphenyl)-1-[4-(methylsulfonyl)phenyl]-4-(trifluormethyl)-1H-imidazol;
2-(3,4-Difluorphenyl)-1-[4-(methylsulfonyl)phenyl]-4-(trifluormethyl)-1H-imidazol;
2-(2-Fluorphenyl)-1-[4-(methylsulfonyl)phenyl]-4-(trifluormethyl)-1H-imidazol;
2-(3-Fluorphenyl)-1-[4-(methylsulfonyl)phenyl]-4-(trifluormethyl)-1H-imidazol;
2-(4-Fluor-3-methylphenyl)-1-[4-(methylsulfonyl)phenyl]-4-(trifluormethyl)-1H-imidazol;
2-(3-Chlor-4-(N,N-dimethylamino)phenyl)-1-[4-(methylsulfonyl)phenyl]-4-(trifluormethyl)-1H-imidazol;
2-(3-Fluor-4-(N,N-dimethylamino)phenyl)-1-[4-(methylsulfonyl)phenyl]-4-(trifluormethyl)-1 H-imidazol;
2-(3-Bromphenyl)-1-[4-(methylsulfonyl)phenyl]-4-(trifluormethyl)-1H-imidazol;
2-(3-Nitrophenyl)-1-[4-(methylsulfonyl)phenyl]-4-(trifluormethyl)-1H-imidazol;
2-(4-Chlorphenyl)-1-[4-(methylsulfonyl)phenyl]-4-(trifluormethyl)-1H-imidazol;
2-(4-Chlorphenyl)-1-[4-(methylsulfonyl)phenyl]-4-methyl-1Himidazol;
2-(4-Chlorphenyl)-1-[4-(methylsulfonyl)phenyl]-4-phenyl-1Himidazol;
2-(4-Chlorphenyl)-4-(4-fluorphenyl)-1-[4-(methylsulfonyl)phenyl]-1H-imidazol;
2-(3-Fluor-4-methoxyphenyl)-1-[4-(methylsulfonyl)phenyl-4-(trifluormethyl)-1H-imidazol;
1-[4-(Methylsulfonyl)phenyl]-2-phenyl-4-trifluormethyl-1Himidazol;
2-(4-Methylphenyl)-1-[4-(methylsulfonyl)phenyl]-4-trifluormethyl-1H-imidazol;
2-(3-Fluor-5-methylphenyl)-1-[4-(methylsulfonyl)phenyl]-4-(trifluormethyl)-1H-imidazol;
2-(3-Methylphenyl)-1-[4-(methylsulfonyl)phenyl]-4-trifluormethyl-1H-imidazol;
2-(3,5-Difluor-4-methoxyphenyl)-1-[4-(methylsulfonyl)phenyl]-4-trifluormethyl-1H-imidazol;
1-[4-(Methylsulfonyl)phenyl]-2-(3-chlorphenyl)-4-trifluormethyl-1H-imidazol;
1-[4-(Methylsulfonyl)phenyl]-2-(4-methoxy-3-chlorphenyl)-4-trifluormethyl-1H-imidazol und
2-(2-Methylphenyl)-1-[4-(methylsulfonyl)phenyl]-4-trifluormethyl-1H-imidazol.

9. Eine Verbindung des Anspruchs 7 ausgewählt aus Verbindungen und deren pharmazeutisch verträglichen Salzen der Gruppe bestehend aus
4-[2-(4-Fluorphenyl)-4-(trifluormethyl)-1H-imidazol-1-yl]benzolsulfonamid;
4-[2-(4-Methylphenyl)-4-(trifluormethyl)-1H-imidazol-1-yl]benzolsulfonamid;
4-[2-(3,4-Difluorphenyl)-4-(trifluormethyl)-1H-imidazol-1-yl]benzolsulfonamid;
4-[2-(2-Fluorphenyl)-4-(trifluormethyl)-1H-imidazol-1-yl]benzolsulfonamid;
4-[2-(3-Fluorphenyl)-4-(trifluormethyl)-1H-imidazol-1-yl]benzolsulfonamid;
4-[2-(4-Chlor-3-methoxyphenyl)-4-(trifluormethyl)-1Himidazol-1-yl]benzolsulfonamid;
4-[2-(3,5-Difluor-4-methoxyphenyl)-4-(trifluormethyl)-1Himidazol-1-yl]benzolsulfonamid;
4-[2-(4-Chlorphenyl)-4-(trifluormethyl)-1H-imidazol-1-yl]benzolsulfonamid;
4-[2-(3-Chlor-4-methylphenyl)-4-(trifluormethyl)-1H-imidazol-1-yl]benzolsulfonamid;
4-[2-(3-Fluor-4-methoxyphenyl)-4-(trifluormethyl)-1Himidazol-1-yl]benzolsulfonamid;
4-[2-(3-Fluor-5-methylphenyl)-4-(trifluormethyl)-1Himidazol-1-yl]benzolsulfonamid;
4-[2-(3-Methylphenyl)-4-trifluormethyl-1H-imidazol-1-yl]benzolsulfonamid;
4-[2-(3-Chlorphenyl)-4-trifluormethyl-1H-imidazol-1-yl]benzolsulfonamid;
4-[2-Phenyl-4-trifluormethyl-1H-imidazol-1-yl]benzolsulfonamid und
4-[2-(4-Methoxy-3-chlorphenyl)-4-trifluormethyl-1H-imidazol-1-yl]benzolsulfonamid.

10. Verbindungen des Anspruchs 1 der Formel III 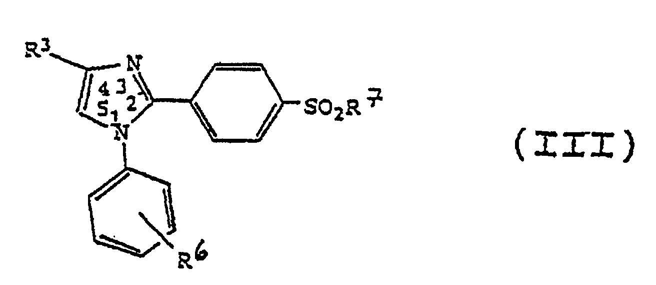 worin R³ ein Rest ist ausgewählt aus C₁-C₆-Alkyl, C₁-C₆-Haloalkyl, Formyl, C₂-C₆-Alkanoyl, Arylcarbonyl, Cyano, C₁-C₆-Alkylthio-C₁-C₆-alkyl, C₃-C₁₀-Cycloalkylthio-C₁-C₈-alkyl, C₁-C₆-Hydroxyalkyl, C₁-C₆-Alkoxyalkyl, C₁-C₆-Alkylcarbonyl, C₁-C₆-Cyanoalkyl, C₁-C₆-Carboxyalkyl, C₁-C₆-Alkoxycarbonyl, Arylthio-C₁C₆-alkyl, Aryloxy-C₁-C₆-alkyl, Aryl-C₁-C₆-alkylthio-C₁-C₆-alkyl, Aryl-C₁-C₆alkoxyalkyl und Aryl;
worin die Arylreste gegebenenfalls substituiert sind an einer substituierbaren Position mit einem oder mehreren Resten ausgewählt aus Halo, C₁-C₆-Alkylthio, C₁-C₆-Alkylsulfinyl, C₁-C₆-Alkyl, Cyano, C₁-C₆-Haloalkyl, Hydroxyl, C₁-C₆-Alkoxy, C₁-C₆-Hydroxyalkyl und C₁-C₆-Haloalkoxy; worin R⁶ ein oder mehrere Reste ist ausgewählt aus Hydrido, Halo, C₁-C₆-Alkyl, C₁-C₆-Haloalkyl, C₁-C₆-Alkoxy, Amino, C₁-C₆-Haloalkoxy, Cyano, Carboxyl, Hydroxyl, C₁-C₆-Hydroxyalkyl, C₁-C₆-Alkoxy-C₁-C₆-alkyl, C₁-C₆-Alkylamino, Nitro und C₁-C₆-Alkylthio; und
worin R⁷ ein Rest ist ausgewählt aus Methyl und Amino oder ein pharmazeutisch verträgliches Salz davon.

11. Verbindungen des Anspruchs 10 worin R³ ausgewählt ist aus Methyl, Ethyl, Isopropyl, *tert*-Butyl, Isobutyl, Pentyl, Hexyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Formyl, Cyano, Hydroxymethyl, Hydroxyethyl, Methoxymethyl, Ethoxymethyl, Methylthiomethyl, Methylcarbonyl, Cyanomethyl, Cyanobutyl, Azidomethyl, Methoxycarbonyl, Ethoxycarbonyl, Isopropoxycarbonyl, *tert*-Butoxycarbonyl, Propoxycarbonyl, Phenylthiomethyl, Phenyloxymethyl, 4-Chlorphenyloxymethyl, 4-Methylphenyloxymethyl, Benzyloxymethyl, 4-Methoxybenzyloxymethyl, Naphthyl und Phenyl
worin die Phenylreste gegebenenfalls substituiert sind an einer substituierbaren Position mit einem oder mehreren Resten ausgewählt aus Fluor, Chlor, Brom, Methylthio, Methylsulfinyl, Methyl, Ethyl, Isopropyl, *tert*-Butyl, Isobutyl, Pentyl, Hexyl, Cyano, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Hydroxyl, Methoxy, Methylendioxy, Ethoxy, Propoxy, n-Butoxy, Hydroxymethyl, Hydroxylethyl und Trifluormethoxy;
worin R⁶ ein Rest ist ausgewählt aus Hydrido, Fluor, Chlor, Brom, lod, Methyl, Ethyl, Isopropyl, *tert*-Butyl, Isobutyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Methoxy, Ethoxy, Isopropoxy, *tert*-Butoxy, Propoxy, Butoxy, Isobutoxy, Pentoxy, Methylendioxy, Amino, Trifluormethoxy, Cyano, Carboxyl, Hydroxyl, Nitro, Hydroxymethyl, Methoxymethyl, Ethoxymethyl, Methylamino, Methylthio, Ethylthio, Propylthio und Butylthio; und
worin R⁷ Methyl, Fluormethyl oder Amino ist oder ein pharmazeutisch verträgliches Salz davon.

12. Verbindungen der Formel IV 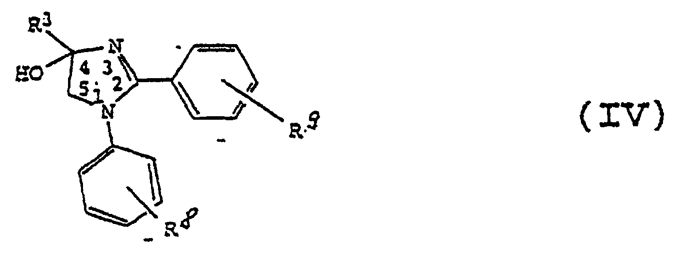 worin R³ ausgewählt ist aus C₁-C₆-Haloalkyl und
worin R⁸ und R⁹ unabhängig voneinander ausgewählt sind aus Hydrido, Halo, C₁-C₆-Alkyl, C₁-C₆-Haloalkyl, C₁-C₆-Alkoxy, C₁-C₆-Alkylsulfonyl, C₁-C₆-Haloalkylsulfonyl und Sulfamyl oder ein pharmazeutisch verträgliches Salz davon.

13. Verbindungen des Anspruchs 12, worin R³ ausgewählt ist aus Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl; und
worin R⁸ und R⁹ unabhängig voneinander ausgewählt sind aus Hydrido, Fluor, Chlor, Brom, Iod, Methyl, Ethyl, Isopropyl, *tert*-Butyl, Isobutyl, Fluormethyl, Difluormethyl, Trifluormethyl, Chlormethyl, Dichlormethyl, Trichlormethyl, Pentafluorethyl, Heptafluorpropyl, Difluorchlormethyl, Dichlorfluormethyl, Difluorethyl, Difluorpropyl, Dichlorethyl, Dichlorpropyl, Methoxy, Ethoxy, Isopropoxy, *tert*-Butoxy, Propoxy, Butoxy, Isobutoxy, Pentoxy, Methylendioxy, Methylsulfonyl, Fluormethylsulfonyl und Sulfamyl oder ein pharmazeutisch verträgliches Salz davon.

14. Eine pharmazeutische Zusammensetzung, enthaltend eine therapeutisch wirksame Menge einer Verbindung, wobei diese Verbindung ausgewählt ist aus einer Familie von Verbindungen gemäß einer der Ansprüche 1-13 oder ein pharmazeutisch verträgliches Salz davon.

15. Verwendung einer Verbindung gemäß einem der Ansprüche 1-13 oder ein pharmazeutisch verträgliches Salz davon zur Herstellung eines Medikamentes zur Behandlung von Entzündung oder einer entzündungsbedingten Störung in einem Subjekt.

16. Verwendung gemäß Anspruch 15, worin die entzündungsbedingte Störung Arthritis ist.

17. Verwendung gemäß Anspruch 15, worin die entzündungsbedingte Störung Schmerz ist.

18. Verwendung gemäß Anspruch 15, worin die entzündungsbedingte Störung Fieber ist.

19. Ein Verfahren zur Herstellung eines Sulfonamids der Formel I gemäß Anspruch I 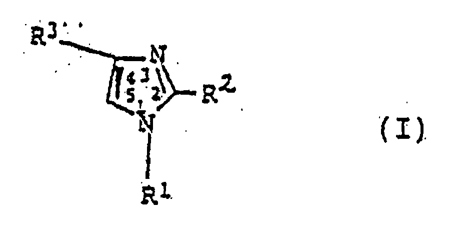 oder ein pharmazeutisch verträgliches Salz davon, worin R¹, R² und R³ wie in Anspruch 1 definiert sind,
wobei diese Methode die Stufen umfaßt:
Behandlung einer Verbindung der Formel I, worin eines von R¹ und R² 4-Methylsulfonylphenyl ist mit einer Base und einem substituierten Trialkylsilan in einem geeigneten Lösungsmittel unter Bildung eines Silylalkylsulfons,
Behandlung dieses Silylalkylsulfons mit einem Alkylammoniumhalogenid unter Bildung eines Sulfinsäuresalzes und
Bildung dieses Sulfonamids durch Behandlung des Sulfinsäuresalzes mit einem Aminierungsmittel.

## Revendications

1. Composé de la formule (I) 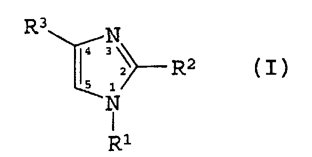 dans laquelle R³ est un radical choisi parmi alkyle en C₁ à C₆, haloalkyle en C₁ à C₆, arylalkyle en C₁ à C₆, formyle, alcanoyle en C₂ à C₆, cyano, (C₁-C₆-alkyl)thioalkyle en C₁ à C₆, (C₁-C₁₀-cycloalkyl)thioalkyle en C₁ à C₆, hydroxyalkyle en C₁ à C₆, (C₁-C₆-alcoxy)alkyle en C₁ à C₆, arylcarbonyle, aryl(C₁-C₆-alkyl)carbonyle, cyanoalkyle en C₁ à C₆, carboxyalkyle en C₁ à C₆, (C₁-C₆-alcoxy)-carbonylalkyle en C₁ à C₆, alcoxycarbonyle en C₁ à C₆, halo(C₁-C₆-alkyl)carbonyle, arylthioalkyle en C₁ à C₆, aryloxyalkyle en C₁ à C₆, aryl(C₁-C₆-alkyl)thioalkyle en C₁ à C₆, aryl (C₁-C₆-alcoxy)alkyle en C₁ à C₆ et aryle ;
dans laquelle R¹ et R² sont choisis indépendamment parmi aryle et hétéroaryle, R¹ et R² étant le cas échéant substitués dans une position substituable par un ou plusieurs radicaux choisis indépendamment parmi alkylsulfonyle en C₁ à C₆, aminosulfonyle, halo, alkylthio en C₁ à C₆, alkyle en C₁ à C₆, cyano, carboxyle, alcoxycarbonyle en C₁ à C₆, haloalkyle en C₁ à C₆, hydroxyle, alcoxy en C₁ à C₆, hydroxyalkyle en C₁ à C₆, (C₁-C₆-alcoxy)alkyle en C₁ à C₆, haloalcoxy en C₁ à C₆, amino, mono- ou dialkylamino en C₁ à C₆, arylamino et nitro ; à la condition que l'un au moins de R¹ et R² soit 4-méthylsulfonylphényle ou 4-aminosulfonylphényle ; où l'aryle chaque fois qu'il est présent désigne un noyau phényle ou naphtyle ; où l'hétéroaryle chaque fois qu'il est présent désigne un radical hétérocyclique insaturé choisi parmi pyrrolyle, pyrrolinyle, imidazolyle, pyrazolyle, 2-pyridyle, 3-pyridyle, 4-pyridyle, pyrimidyle, pyrazinyle, pyridazinyle, triazolyle, tétrazolyle, indolyle, isoindolyle, indolizinyle, benzimidazolyle, quinoléyle, isoquinoléyle, indazolyle, benzotriazolyle, tétrazolopyridazinyle, 2-furyle, 3-furyle, 3-furyle, 2-thiényle, 3-thiényle, oxazolyle, isoxazolyle, oxadiazolyle, benzoxazolyle, benzoxadiazolyle, thiazolyle, thiadiazolyle, benzothiazolyle, benzothiadiazolyle, benzofurane, benzothiophène, ledit radical hétérocyclique pouvant être substitué dans une position substituable par un ou plusieurs substituants choisis parmi halo, alkylthio en C₁ à C₆, alkylsulfinyle en C₁ à C₆, alkyle en C₁ à C₆, cyano, haloalkyle en C₁ à C₆, hydroxyle, alcoxy en C₁ à C₆, hydroxyalkyle en C₁ à C₆ et haloalcoxy en C₁ à C₆ ;
ou un sel pharmaceutiquement acceptable de celui-ci.

2. Composés de la formule (I) 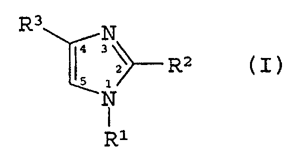 dans laquelle R³ est un radical choisi parmi méthyle, éthyle, isopropyle, tert-butyle, isobutyle, pentyle, hexyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, benzyle, phényléthyle, phénylpropyle, formyle, cyano, hydroxyméthyle, hydroxyéthyle, méthoxyméthyle, éthoxyméthyle, méthylthiométhyle, isopropylthiométhyle, cyclohexylthiométhyle, méthylcarbonyle, éthylcarbonyle, phénylcarbonyle, trifluorométhylcarbonyle, difluorométhylcarbonyle, fluorométhylcarbonyle, benzylcarbonyle, cyanométhyle, cyanobutyle, méthoxycarbonylméthyle, éthoxycarbonyléthyle, carboxyméthyle, carboxypropyle, phénylthiométhyle, 2-chlorophénylthiométhyle, 2,6-dichlorophénylthiométhyle, 4-méthylphénylthiométhyle, 2-isopropylphénylthiométhyle, 2-méthylphénylthiométhyle, phényloxyméthyle, 4-chlorophényloxyméthyle, méthylphényloxyméthyle, benzyloxyméthyle, 4-méthoxybenzyloxyméthyle, naphtyle et phényle, les radicaux phényle étant le cas échéant substitués dans une position substituable par un ou plusieurs radicaux choisis parmi fluoro, chloro, bromo, méthylthio, méthylsulfinyle, méthyle, éthyle, isopropyle, tert-butyle, isobutyle, pentyle, hexyle, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyle, méthoxy, méthylènedioxy, éthoxy, propoxy, n-butoxy, hydroxyméthyle, hydroxyéthyle et trifluorométhoxy ; dans laquelle R¹ et R² sont choisis parmi phényle, imidazolyle, thiényle, thiazolyle, pyrrolyle, oxazolyle, isoxazalyle, triazolyle, pyrazinyle, pyrimidinyle, quinolinyle, indolyle, benzimidazolyle, pyrazolyle et pyridyle, R¹ et R² étant le cas échéant substitués dans une position substituable par un ou plusieurs radicaux choisis indépendamment parmi méthylsulfonyle, aminosulfonyle, fluorométhylsulfonyle, difluorométhylsulfonyle, fluoro, chloro, bromo, méthylthio, méthylsulfinyle, méthyle, éthyle, isopropyle, tert-butyle, isobutyle, pentyle, hexyle, cyano, carbonyle, méthoxycarbonyle, éthoxycarbonyle, isopropoxycarbonyle, tert-butoxycarbonyle, propoxycarbonyle, butoxycarbonyle, isobutoxycarbonyle, pentoxycarbonyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyle, méthoxy, méthylènedioxy, éthoxy, propoxy, n-butoxy, hydroxyméthyle, hydroxyéthyle, méthoxyméthyle, éthoxyméthyle, trifluorométhoxy, amino, méthylamino, N,N-diéthylamino, phénylamino et nitro ; à la condition que l'un au moins de R¹ et R² soit 4-méthylsulfonylphényle ou 4-aminosulphonylphényle ;
ou un sel pharmaceutiquement acceptable de celui-ci.

3. Composés selon la revendication 2, dans lesquels R³ est un radical choisi parmi méthyle, éthyle, isopropyle, tert-butyle, isobutyle, pentyle, hexyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle et dichloropropyle ;
dans lesquels R¹ est phényle substitué par un 4-méthylsulfonyle ou 4-aminosulfonyle ; et dans lesquels R² est choisi parmi imidazolyle, thiényle, thiazolyle, pyrrolyle, oxazolyle, isoxazolyle, triazolyle, pyrimidinyle, quinolinyle, indolyle, benzimidazolyle, pyrazolyle et pyridyle, R² étant le cas échéant substitué dans une position substituable par un ou plusieurs radicaux choisis indépendamment parmi méthylthio, méthyle, éthyle, isopropyle, tert-butyle, isobutyle, pentyle, hexyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, méthoxy, méthylènedioxy, éthoxy, propoxy, n-butoxy, hydroxyméthyle, hydroxyéthyle, méthoxyméthyle, éthoxyméthyle et trifluorométhoxy ;
ou un sel pharmaceutiquement acceptable de celui-ci.

4. Composé selon la revendication 1, choisi parmi les composés, ainsi que leurs sels pharmaceutiquement acceptables, du groupe comprenant les
1-méthyl-3-[1-[4-(méthylsulfonyl)phényl]-4-trifluorométhyl-1H-imidazol-2-yl]-1H-indole ;
2-[1-[4-(méthylsulfonyl)phényl]-4-(trifluorométhyl)-1H-imidazol-2-yl]thiophène ;
3-[1-[4-(méthylsulfonyl)phényl]-4-(trifluorométhyl)-1H-imidazol-2-yl]thiophène ;
2-méthyl-3-[1-[4-(méthylsulfonyl)phényl]-4-(trifluorométhyl)-1H-imidazol-2-yl]pyridine ;
3-fluoro-5-[1-[4-(méthylsulfonyl)phényl]-4-(trifluorométhyl)-1H-imidazol-2-yl]pyridine ;
3-chloro-5-[1-[4-(méthylsulfonyl)phényl]-4-(trifluorométhyl)-1H-imidazol-2-yl]pyridine:
5-méthyl-2-[1- [4- (méthylsulfonyl)phényl]-4-(trifluorométhyl)-1H-imidazol-2-yl]pyridine ;
4-méthyl-2-[1- [4- (méthylsulfonyl)phényl)-4-(trifluorométhyl)-1H-imidazol-2-yl]pyridine ;
2-méthoxy-6- [1-[4-(méthylsulfonyl)phényl] -4-(trifluorométhyl)-1H-imidazol-2-yl]pyridine ;
5-méthoxy-2-[1-[4-(méthylsulfonyl]phényl]-4-(trifluorométhyl)-1H-imidazol-2-yl]pyridine ;
4-méthoxy-2-[1-[4-(méthylsulfonyl)phényl]-4-(trifluorométhyl)-1H-imidazol-2-yl]pyridine ;
2-chloro-6-[1-[4-(méthylsulfonyl)phényl]-4-(trifluorométhyl)-1H-imidazol-2-yl]pyridine ;
5-chloro-2-[1-[4-(méthylsulfonyl)phényl]-4-(trifluorométhyl)-1H-imidazol-2-yl]pyridine ;
4-chloro-2-[1-[4-(méthylsulfonyl)phényl]-4-(trifluorométhyl)-1H-imidazol-2-yl]pyridine ;
2-fluoro-6-[1-[4-(méthylsulfonyl]phényl]-4-(trifluorométhyl)-1H-imidazol-2-yl]pyridine ;
4-fluoro-2-[1-[4-(méthylsulfonyl]phényl]-4-(trifluorométhyl)-1H-imidazol-2-yl]pyridine ;
4-fluoro-2-[1-[4-(méthylsulfonyl)phényl]-4-(trifluorométhyl)-1H-imidazol-2-yl]pyridine ;
3-méthoxy-5-[1-[4-(méthylsulfonyl)phényl]-4-(trifluorométhyl)-1H-imidazol-2-yl]pyridine ;
3-[4-méthyl-1-[4-(méthylsulfonyl)phényl]-4-(trifluorométhyl)-1H-imidazol-2-yl]pyridine ;
3-[4-[4-(fluorophényl)-1-[4-(méthylsulfonyl)phényl]-1H-imidazol-2-yl]pyridine ;
5-[1-[4-(méthylsulfonyl)phényl]-4-(trifluorométhyl)-1H-imidazol-2-yl]-2-(méthylthio)pyridine ;
3-[4-(difluorométhyl)-1-[4-(méthylsulfonyl)phényl]-1H-imidazol-2-yl]pyridine ;
3-[1-[4-(méthylsulfonyl)phényl]-4-(trifluorométhyl)-1H-imidazol-2-yl]pyridine ;
2-[1-[4-(méthylsulfonyl]phényl]-4-(trifluorométhyl)-1H-imidazol-2-yl]pyridine ;
2-méthyl-4-[1-[4-(méthylsulfonyl)phényl]-4-(trifluorométhyl]-1H-imidazol-2-yl]pyridine ;
4-[1-[4-(méthylsulfonyl)phényl-4-(trifluorométhyl)-1H-imidazol-2-yl]pyridine ;
2-méthyl-6-[1-[4-(méthylsulfonyl)phényl-4-(trifluorométhyl)-1H-imidazol-2-yl]pyridine ;
3-méthyl-5-[1-[4-(méthylaulfonyl)phényl]-4-(trifluorométhyl)-1H-imidazol-2-yl]pyridine ;
2-méthoxy-5-[1-[4-(méthylsulfonyl)phényl]-4-(trifluorométhyl]-1H-imidazol-2-yl]pyridine ;
2-[4-(4-fluorophényl)-1-[4-(méthylsulfonyl)phényl]-4-(trifluorométhyl]-1H-imidazol-2-yl]pyridine.

5. Composé selon la revendication 1, choisi parmi les composés, ainsi que leurs sels pharmaceutiquement acceptables, du groupe comprenant les
4-[2-(4-méthylpyridin-2-yl)-4-(trifluorométhyl)-1H-imidazol-1-yl]benzènesulfonamide ;
4- [2-(5-méthylpyridin-3-yl)-4-(trifluorométhyl)-1H-imidazol-1-yl]benzènesulfonamide ;
4-[2-(2-méthylpyridin-3-yl)-4-(trifluorométhyl)-1H-imidazol-1-yl]benzènesulfonamide ;
4-[2-(5-fluoropyridin-3-yl)-4-(trifluorométhyl)-1H-imidazol-1-yl]benzènesulfonamide ;
4-[2-(5-chloropyridin-3-yl)-4-(trifluorométhyl)-1H-imidazol-1-yl]benzènesulfonamide ;
4-[2-(5-méthylpyridin-2-yl)-4-(trifluorométhyl)-1H-imidazol-1-yl]benzènesulfonamide ;
4-[2-(4-méthylpyridin-2-yl)-4-(trifluorométhyl)-1H-imidazol-1-yl]benzènesulfonamide ;
4-[2-(6-méthoxypyridin-2-yl)-4-(trifluorométhyl)-1H-imidazol-1-yl]benzènesulfonamide ;
4-[2-(5-méthoxypyridin-2-yl)-4-(trifluorométhyl)-1H-imidazol-1-yl]benzènesulfonamide ;
4-[2-(4-méthoxypyridin-2-yl)-4-(trifluorométhyl)-1H-imidazol-1-yl]benzènesulfonamide ;
4- [2-(6-chloropyridin-2-yl)-4-(trifluorométhyl)-1H-imidazol-1-yl]benzènesulfonamide ;
4-[2-(5-chloropyridin-2-yl)-4-(trifluorométhyl)-1H-imidazol-1-yl]benzènesulfonamide ;
4- [2- (4-chloropyridin-2-yl) -4-(trifluorométhyl)-1H-imidazol-1-yl]benzènesulfonamide ;
4-[2-(6-fluoropyridin-2-yl)-4-(trifluorométhyl)-1H-imidazol-1-yl]benzènesulfonamide ;
4-[2-(5-fluoropyridin-2-yl)-4-(trifluorométhyl)-1H-imidazol-1-yl]benzènesulfonamide ;
4[2-(4-fluoropyridin-2-yl)-4-(trifluorométhyl)-1H-imidazol-1-yl]benzènesulfonamide ;
4- [2-(5-méthoxypyridin-3-yl)-4-(trifluorométhyl)-1H-imidazol-1-yl]benzènesulfonamide ;
4-[4-méthyl-2-(3-pyridinyl)-1H-imidazol-1-yl]benzènesulfonamide ;
4-[2-(pyridin-3-yl)-4-(trifluorométhyl)-1H-imidazol-1-yl]benzènesulfonamide ;
4-[4-(4-fluorophényl)-2-(3-pyridinyl)-1H-imidazol-1-yl]benzènesulfonamide ;
4-[2-[6-(méthylthio)pyridin-3-yl]-4-(trifluorométhyl)-1H-imidazol-1-yl]benzènesulfonamide ;
4-[4-(difluorométhyl)-2-(3-pyridinyl)-1H-imidazol-1-yl]benzènesulfonamide ; et
4-[2-(6-méthylpyridin-3-yl)-4-(trifluorométhyl)-1H-imidazol-1-yl]benzènesulfonamide ;

6. Composés selon la revendication 1, ayant la formule II 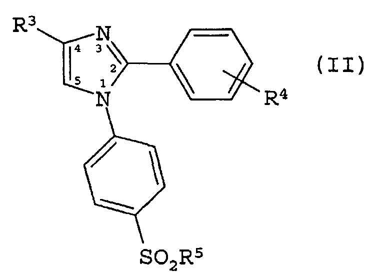 dans laquelle R³ est un radical choisi parmi alkyle en C₁ à C₆, haloalkyle en C₁ à C₆, formyle, alcanoyle en C₂ à C₆, arylcarbonyle, cyano, (C₁-C₆-alkyl)thioalkyle en C₁ à C₆, (C₃-C₁₀-cycloalkyl)thioalkyle en C₁ à C₆, hydroxyalkyle en C₁ à C₆, (C₁-C₆-alcoxy)alkyle en C₁ à C₆, cyanoalkyle en C₁ à C₆, carboxyalkyle en C₁ à C₆, alcoxycarbonyle en C₁ à C₆, arylthioalkyle en C₁ à C₆, aryloxyalkyle en C₁ à C₆, aryl(C₁-C₆-alkyl)thioalkyle en C₁ à C₆, aryl(C₁-C₆-alcoxy)-alkyle en C₁ à C₆ et aryle ; dans laquelle R⁵ est un radical choisi parmi méthyle et amino ; et dans laquelle R⁴ représente un ou plusieurs radicaux choisis parmi hydrido, halo, alkyle en C₁ à C₆, haloalkyle en C₁ à C₆, alcoxy en C₁ à C₆, amino, haloalcoxy en C₁ à C₆, cyano, carboxyle, hydroxyle, hydroxyalkyle en C₁ à C₆, (C₁-C₆-alcoxy)alkyle en C₁ à C₆, alkylamino en C₁ à C₆, nitro et alkylthio en C₁ à C₆ ; ou un sel pharmaceutiquement acceptable de celui-ci.

7. Composé de la formule II 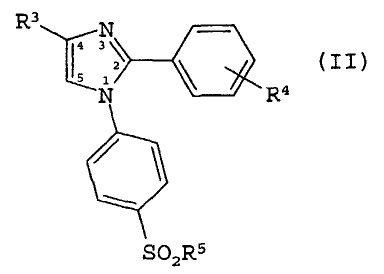 dans laquelle R³ est choisi parmi méthyle, éthyle, isopropyle, tert-butyle, isobutyle, pentyle, hexyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, formyle, cyano, hydroxyméthyle, hydroxyéthyle, méthoxyméthyle, éthoxyméthyle, méthylthiométhyle, isopropylthiométhyle, cyclohexylthiométhyle, méthylcarbonyle, cyanométhyle, cyanobutyle, méthoxycarbonyle, éthoxycarbonyle, isopropoxycarbonyle, tert-butoxycarbonyle, propoxycarbonyle, carboxyméthyle, carboxypropyle, phénylthiométhyle, 2-chlorophénylthiométhyle, 2,6-dichlorophénylthiométhyle, 1-méthylphénylthiométhyle, 2-isopropylphénylthiométhyle, 2-méthylphénylthiométhyle, phényloxyméthyle, 4-chlorophényloxyméthyle, 4-méthylphényloxyméthyle, benzyloxyméthyle, 4-méthoxybenzyloxyméthyle, naphtyle et phényle, les radicaux phényle étant le cas échéant substitués dans une position substituable par un ou plusieurs radicaux choisis parmi fluoro, chloro, bromo, méthylthio, méthylsulfinyle, méthyle, éthyle, isopropyle, tert-butyle, isobutyle, pentyle, hexyle, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyle, méthoxy, méthylènedioxy, éthoxy, propoxy, n-butoxy, hydroxyméthyle, hydroxyéthyle et trifluorométhoxy ; dans laquelle R⁵ est méthyle ou amino ; et dans laquelle R⁴ est un radical choisi parmi hydrido, méthylsulfonyle, fluoro, chloro, bromo, méthylthio, méthyle, éthyle, isopropyle, tert-butyle, isobutyle, pentyle, hexyle, cyano, carboxyle, méthoxycarbonyle, éthoxycarbonyle, isopropoxycarbonyle, tert-butoxycarbonyle, propoxycarbonyle, butoxycarbonyle, isobutoxycarbonyle, pentoxycarbonyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyle, méthoxy, méthylènedioxy, éthoxy, propoxy, n-butoxy, hydroxyméthyle, hydroxyéthyle, méthoxyméthyle, éthoxyméthyle, trifluorométhoxy, amino, méthylamino, N,N-diméthylamino, phénylamino et nitro ; ou un sel pharmaceutiquement acceptable de celui-ci.

8. Composé selon la revendication 7, choisi parmi les composés, ainsi que leurs sels pharmaceutiquement acceptables, du groupe comprenant les
2-(4-fluorophényl)-1-[4-(méthylsulfonyl)phényl]-4-(trifluorométhyl)-1H-imidazole ;
2-(3,4-difluorophényl)-1-[4-(méthylsulfonyl)phényl]-4-(trifluorométhyl)-1H-imidazole ;
2-(2-fluorophényl)-1-[4-(méthylsulfonyl)phényl]-4-(trifluorométhyl)-1H-imidazole ;
2-(3-fluorophényl)-1-[4-(méthylsulfonyl)phényl]-4-(trifluorométhyl)-1H-imidazole ;
2-(4-fluoro-3-méthylphényl)-1-[4-(méthylsulfonyl)-phényl]-4-(trifluorométhyl)-1H-imidazole ;
2-(3-chloro-4-(N,N-diméthylamino)phényl)-1-[4-(méthylsulfonyl)phényl]-4-(trifluorométhyl)-1H-imidazole ;
2-(3-fluoro-4-(N,N-diméthylamino)phényl)-1-[4-(méthylsulfonyl)phényl]-4-(trifluorométhyl)-1H-imidazole ;
2-(3-bromophényl)-1-[4-(méthylsulfonyl)phényl]-4-(méthylsulfonyl)phényl]-4-(trifluorométhyl)-1H-imidazole ;
2-(3-nitrophényl)-1-[4- (méthylsulfonyl)phényl] -4-(trifluorométhyl)-1H-imidazole ;
2-(4-chlorophényl)-1-[4-(méthylsulfonyl)phényl]-4-(trifluorométhyl)-1H-imidazole ;
2-(4-chlorophényl)-1-[4-(méthylsulfonyl)phényl]-4-méthyl-1H-imidazole ;
2-(4-chlorophényl)-1-[4-(méthylsulfonyl)phényl]-4-phényl-1H-imidazole ;
2-(4-chlorophényl)-4-(4-fluorophényl)-1-[4-(méthylsulfonyl)phényl]-1H-imidazole ;
2-(3-fluoro-4-méthoxyphényl)-1-[4-(méthylsulfonyl)phényl)-4-(trifluorométhyl)-1H-imidazole ;
1-[4-(méthylsulfonyl)phényl]-2-phényl-4-trifluorométhyl-1H-imidazole ;
2-(4-méthylphényl)-1-[4-(méthylsulfonyl)phényl]-trifluorométhyl-1H-imidazole ;
2-(3-fluoro-5-méthylphényl)-1-[4-(méthylsulfonyl)phényl]-4-trifluorométhyl-1H-imidazole ;
2-(3-méthylphényl)-1-[4-(méthylsulfonyl)phényl]-4-trifluorométhyl-1H-imidazole ;
2-(3,5-difluoro-4-méthoxyphényl)-1-[4-(méthylsulfonyl)phényl]-4-trifluorométhyl-1H-imidazole ;
1-[4-(méthylsulfonyl)phényl]-2-(3-chlorophényl)-4-trifluorométhyl-1H-imidazole ;
1-[4-(méthylsulfonyl)phényl]-2-(4-méthoxy-3-chlorophényl)-4-trifluorométhyl-1H-imidazole ; et
2-(2-méthylphényl)-1-[4-(méthylsulfonyl]phényl] -4-trifluorométhyl-1H-imidazole.

9. Composé selon la revendication 7, choisi parmi les composés, ainsi que leurs sels pharmaceutiquement acceptables, du groupe comprenant les
4-[2-(4-fluorophényl)-4-(trifluorométhyl)-1H-imidazol-1-yl]benzènesulfonamide ;
4-[2-(4-méthylphényl)-4-(trifluorométhyl)-1H-imidazol-1-yl]benzènesulfonamide ;
4-[2-(3,4-difluorophényl)-4-(trifluorométhyl)-1H-imidazol-1-yl]benzènesulfonamide ;
4-[2-2-fluorophényl)-4-(trifluorométhyl)-1H-imidazol-1-yl]benzènesulfonamide ;
4-[2-(3-fluorophényl)-4-(trifluorométhyl)-1H-imidazol-1-yl]benzènesulfonamide ;
4-[2-(4-chloro-3-méthoxyphényl-4-(trifluorométhyl)-1H-imidazol-1-yl]benzènesulfonamide ;
4-[2-(3,5-difluoro-4-méthoxyphényl)-4-(trifluorométhyl)-1H-imidazol-1-yl]benzènesulfonamide ;
4-[2-(4-chlorophényl)-4-(trifluorométhyl)-1H-imidazol-1-yl]benzènesulfonamide ;
4-[2-(3-chloro-4-méthylphényl]-4-(trifluorométhyl)-1H-imidazol-1-yl]benzènesulfonamide ;
4-[2-(3-fluoro-4-méthoxyphényl)-4-(trifluorométhyl)-1H-imidazol-1-yl]benzènesulfonamide ;
4-[2-(3-fluoro-5-méthylphényl)-4-(trifluorométhyl)-1H-imidazol-1-yl]benzènesulfonamide ;
4-[2-(3-méthylphényl)-4-(trifluorométhyl)-1H-imidazol-1-yl]benzènesulfonamide ;
4- [2-(3-chlorophényl)-4- (trifluorométhyl)-1H-imidazol-1-yl]benzènesulfonamide ;
4-[2-phényl-4-trifluorométhyl-1H-imidazol-1-yl]benzènesulfonamide ; et
4-[2-(4-méthoxy-3-chlorophényl]-4-(trifluorométhyl)-1H-imidazol-1-yl]benzènesulfonamide.

10. Composés selon la revendication 1, ayant la formule (III) 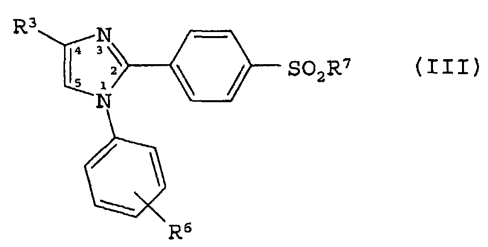 dans laquelle R³ est un radical choisi parmi alkyle en C₁ à C₆, haloalkyle en C₁ à C₆, formyle, alcanoyle en C₂ à C₆, arylcarbonyle, cyano, (C₁-C₆-alkyl)thioalkyle en C₁ à C₆, (C₃-C₁₀-cycloalkyl)thioalkyle en C₁ à C₆, hydroxyalkyle en C₁ à C₆, alcoxyalkyle en C₁ à C₆, alkylcarbonyle en C₁ à C₆, cyanoalkyle en C₁ à C₆, carboxyalkyle en C₁ à C₆, alcoxycarbonyle en C₁ à C₆, arylthioalkyle en C₁ à C₆, aryloxyalkyle en C₁ à C₆, aryl(C₁-C₆-alkyl)thioalkyle en C₁ à C₆, aryl(C₁-C₆-alcoxy)alkyle et aryle ; les radicaux aryle étant le cas échéant substitués dans une position substituable par un ou plusieurs radicaux choisis parmi halo, alkylthio en C₁ à C₆, alkylsulfinyle, alkyle en C₁ à C₆, cyano, haloalkyle en C₁ à C₆, hydroxyle, alcoxy en C₁ à C₆, hydroxyalkyle en C₁ à C₆ et haloalcoxy en C₁ à C₆ ; dans laquelle R⁶ représente un ou plusieurs radicaux choisis parmi hydrido, halo, alkyle en C₁ à C₆, haloalkyle en C₁ à C₆, alcoxy en C₁ à C₆, amino, haloalcoxy en C₁ à C₆, carboxyle, hydroxyle, hydroxyalkyle en C₁ à C₆, (C₁-C₆-alcoxy)alkyle en C₁ à C₆, alkylamino en C₁ à C₆, nitro et alkylthio en C₁ à C₆ ; et dans laquelle R⁷ est un radical choisi parmi méthyle et amino ; ou un sel pharmaceutiquement acceptable de celui-ci.

11. Composés selon la revendication 10, dans lesquels R³ est choisi parmi méthyle, éthyle, isopropyle, tert-butyle, isobutyle, hexyle, trifluorométhyle, chlorométhyle, dichlorométhyle, trichlorométhyle, pentafluoroéthyle, heptafluoropropyle, difluorochlorométhyle, dichlorofluorométhyle, difluoropropyle, dichloroéthyle, dichloropropyle, formyle, cyano, hydroxyméthyle, hydroxyéthyle, méthoxyméthyle, méthylthiométhyle, méthylcarbonyle, cyanométhyle, cyanobutyle, azidométhyle, méthoxycarbonyle, éthoxycarbonyle, isopropoxycarbonyle, butoxycarbonyle, propoxycarbonyle, phénylthiométhyle, phényloxyméthyle, 4-chlorophényloxyméthyle, 4-méthylphényloxyméthyle, benzyloxyméthyle, 4-méthoxybenzyloxyméthyle, naphtyle et phényle, les radicaux phényle étant le cas échéant substitués dans une position substituable par un ou plusieurs radicaux choisis parmi fluoro, chloro, bromo, méthylthio, méthylsulfinyle, méthyle, éthyle, isopropyle, tert-butyle, isobutyle, pentyle, hexyle, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyle, méthoxy, méthylènedioxy, éthoxy, propoxy, n-butoxy, hydroxyméthyle, hydroxyéthyle et trifluorométhoxy ;
dans laquelle R⁶ est un radical choisi parmi hydrido, fluoro, chloro, bromo, iodo, méthyle, éthyle, isopropyle, tert-butyle, isobutyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, méthoxy, éthoxy, isopropoxy, tert-butoxy, propoxy, butoxy, isobutoxy, pentoxy, méthylènedioxy, amino, trifluorométhoxy, cyano, carboxyle, hydroxyle, nitro, hydroxyméthyle, méthoxyméthyle, éthoxyméthyle, méthylamino, méthylthio, éthylthio, propylthio et butylthio ; et dans laquelle R⁷ est un méthyle, fluorométhyle ou amino, ou un sel pharmaceutiquement acceptable de celui-ci.

12. Composés de la formule IV 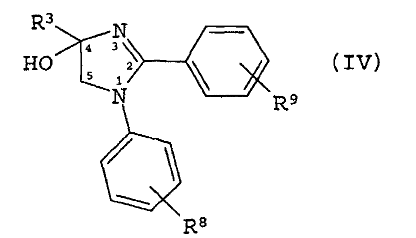 dans laquelle R³ est choisi parmi haloalkyle en C₁ à C₆ ; et dans laquelle R⁸ et R⁹ sont choisis indépendamment parmi hydrido, halo, alkyle en C₁ à C₆, haloalkyle en C₁ à C₆, alcoxy en C₁ à C₆, alkylsulfonyle en C₁ à C₆, haloalkylsulfonyle en C₁ à C₆ et sulfamyle ; ou un sel pharmaceutiquement acceptable de celui-ci.

13. Composés selon la revendication 12, dans lesquels R³ est choisi parmi fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle ; et dans laquelle R⁸ et R⁹ sont choisis indépendamment parmi hydrido, fluoro, chloro, bromo, iodo, méthyle, éthyle, isopropyle, tert-butyle, isobutyle, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, méthoxy, éthoxy, isopropoxy, tert-butoxy, propoxy, butoxy, isobutoxy, pentoxy, méthylènedioxy, méthylsulfonyle, fluorométhylsulfonyle et sulfamyle ; ou un sel pharmaceutiquement acceptable de celui-ci.

14. Composition pharmaceutique comprenant une quantité thérapeutiquement efficace d'un composé, ledit composé étant choisi dans une famille de composés selon l'une quelconque des revendications 1 à 13, ou d'un sel pharmaceutiquement acceptable de celui-ci.

15. Utilisation d'un composé selon l'une quelconque des revendications 1 à 13 ou d'un sel pharmaceutiquement acceptable de celui-ci pour préparer un médicament pour traiter une inflammation ou un trouble associé à une inflammation chez un sujet.

16. Utilisation selon la revendication 15, dans laquelle le trouble associé à une inflammation est l'arthrite.

17. Utilisation selon la revendication 15, dans laquelle le trouble associé à une inflammation est la douleur.

18. Utilisation selon la revendication 15, dans laquelle le trouble associé à une inflammation est la fièvre.

19. Procédé pour préparer un sulfonamide de la formule I selon la revendication 1 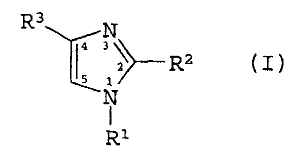 ou un sel pharmaceutiquement acceptable de celui-ci,
dans laquelle R¹, R² et R³ sont tels que définis dans la revendication 1,
ledit procédé comprenant les étapes consistant à
traiter le composé de la formule I, dans laquelle l'un de R¹ et R² est 4-méthylsulfonylphényle, avec une base et un trialkylsilane substitué dans un solvant approprié afin de former une silylalkylsulfone,
traiter ladite silylalkylsulfone avec un halogénure d'alkylammonium afin de former un sel d'acide sulfinique et
former ledit sulfonamide en traitant le sel d'acide sulfinique avec un agent aminant.
